(19) Europäisches Patentamt
European Patent Office
Office européen des brevets

(11) EP 1 568 691 A1

(12) EUROPEAN PATENT APPLICATION
published in accordance with Art. 158(3) EPC

(43) Date of publication:
**31.08.2005 Bulletin 2005/35**

(21) Application number: **03777283.7**

(22) Date of filing: **05.12.2003**

(51) Int Cl.[7]: **C07D 211/58**, C07D 401/06, C07D 401/14, C07D 405/14, A61K 31/4468, A61K 31/4545, A61K 31/444, A61P 7/00, A61P 7/06, A61P 43/00

(86) International application number:
**PCT/JP2003/015589**

(87) International publication number:
**WO 2004/052859 (24.06.2004 Gazette 2004/26)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **06.12.2002 US 431234 P**

(71) Applicant: **Kowa Co., Ltd.**
**Aichi 460-8625 (JP)**

(72) Inventors:
- **IMAGAWA, Shigehiko**
**TSUKUBA-SHI, Ibaraki 305-0821 (JP)**
- **DOI, Takeshi**
**HIGASHIMURAYAMA-SHI, Tokyo 189-0022 (JP)**
- **TAMURA, Masahiro**
**MACHIDA-SHI, Tokyo 194-0003 (JP)**
- **OHKUCHI, Masao**
**TOKOROZAWA-SHI, Saitama 359-0041 (JP)**

(74) Representative: **Hartz, Nikolai**
**Wächtershäuser & Hartz**
**Patentanwälte**
**Weinstrasse 8**
**80333 München (DE)**

# (54) ERYTHROPOIETIN PRODUCTION ACCELERATOR

(57) The present invention relates to a preventive or therapeutic agent for pathological conditions caused by reduced production of erythropoietin, or for anemia, or for chronic anemia, renal anemia, aplastic anemia, or pure red cell aplasia, the agent comprising, as an active ingredient, a cyclic amine compound represented by the following formula (1):

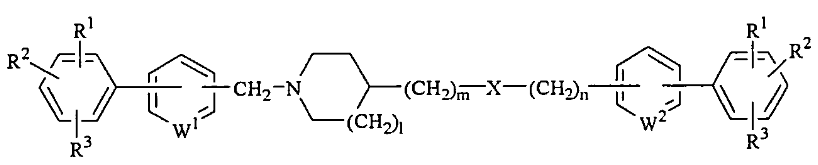

(1)

wherein, $R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;

$W^1$ and $W^2$ each independently represent N or CH;

X represents O, $NR^4$, $CONR^4$ or $NR^4CO$;

$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and

l, m and n each represents a number of 0 or 1, or a salt thereof or a solvate thereof.

EP 1 568 691 A1

Fig. 1

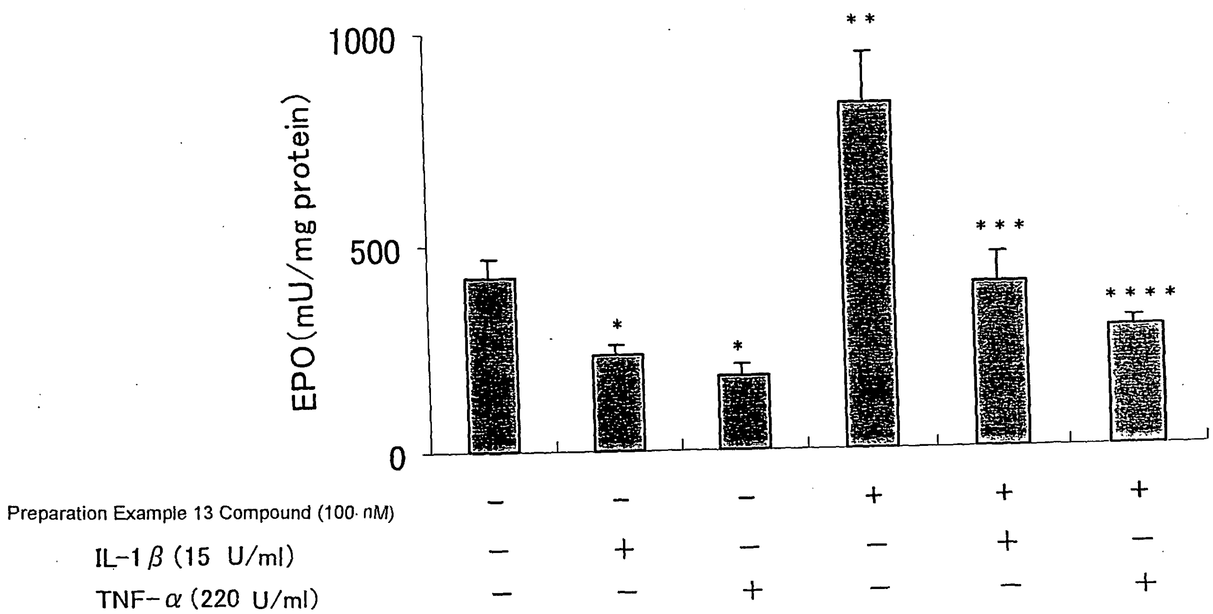


* ; p<0.005、Against control group * * * ; p<0.025、Against IL-1β (15 U/ml) group
* * ; p<0.01、Against control group * * * * ; p<0.01、Against TNF-α (220 U/ml) group

## Description

Technical Field

The present invention relates to an erythropoietin production potentiator, and more particularly to a preventive or therapeutic agent for pathological conditions caused by reduced production of erythropoietin, such as anemia.

Background Art

Erythropoietin (EPO) is a glycoprotein hormone, which participates in maturation and differentiation of an erythroid progenitor cell to a matured red blood cell. EPO is a 165-amino-acid polypeptide, which is found in nature in the form of a monomer [Lin, F-K. et al., Proc. Natl. Acad. Sci. USA 82:7580-7584 (1985)].

Human erythropoietin plays a key role in proliferation and differentiation of red blood cells. Therefore, the hormone is useful for treatment of blood diseases primarily involving reduced production of red blood cells. Clinically, EPO is used in treatment of anemia associated with chronic renal failure (CRF), autologous blood storage, or anemia of prematurity (Eschbach JW, Egrie JC, Downing MR, et al., NEJM, 316:73-78 (1987); Eschbach JW, Abdulhadi MH, Browne JK et al., Ann. Intern. Med., 111:992 (1989); Egrie JC, Eschbach JW, McGuire T, Adamson JW, Kidney Intl., 33:262 (1988); and Lim VS, Degowin RL, Zavala D, et al., Ann. Intern. Med., 110:108-114 (1989)). EPO is also used in treatment of patients suffering AIDS or patients having cancer and receiving chemotherapy (Danna RP, Rudnick SA, Abels RI, Garnick MB, Erythropoietin in Clinical Applications - An International Perspective, New York: Marcel Dekker; 1990, pp. 301-324). EPO has been found to be effective in treatment of chronic anemia.

Some protein used for therapy such as EPO has a short plasma half-life and is susceptible to degradation in the presence of protease [Spivak JL and Hogans BB, Blood, 73:90 (1989); McMahon FG et al., Blood, 76:1718 (1990)]; i.e., EPO exhibits poor bioavailability. Accordingly, in a protein therapy employing EPO, intravenous injection must be performed frequently in order to maintain the effective therapeutic blood level of the protein in circulation. Subcutaneous injection may be an alternative administration route. However, when administered subcutaneously, the agent is absorbed slowly from the administration site. Thus, plasma level of the protein is significantly low as compared with the case of intravenous administration, although effect of sustained release may be appreciable. Therefore, in order to obtain a therapeutic effect of similar level, subcutaneous injection must be performed frequently as in the case of intravenous administration.

Accordingly, demand exists for a method for potentiating erythropoietin production through administration of a compound other than erythropoietin, which exhibits poor bioavailability.

Disclosure of the Invention

In view of the foregoing, the present inventors have performed studies on effects of various compounds on erythropoietin production, and, quite unexpectedly, have found that compounds represented by the general formula (1) below exhibits erythropoietin production potentiating effect, and thus is useful as a preventive or therapeutic drug for anemia, thereby leading to completion of the present invention.

The present invention provides a preventive or therapeutic agent for pathological conditions caused by reduced production of erythropoietin containing as an active ingredient, a cyclic amine compound represented by the following general formula (1):

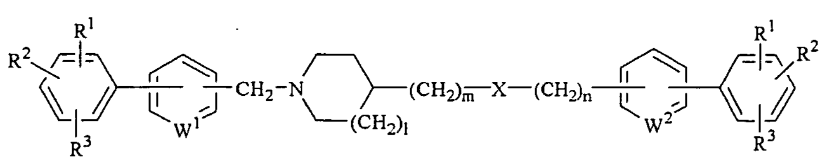

(1)

wherein,

$R^1$,$R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;

$W^1$ and $W^2$ each independently represent N or CH ;

X represents O, $NR^4$, $CONR^4$ or $NR^4CO$ ;

$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and

l, m and n each represents a number of 0 or 1, or a salt thereof or a solvate thereof

The present invention also provides an erythropoietin production potentiator containing, as an active ingredient, the cyclic amino compound of formula (1), a salt thereof or solvates thereof.

The present invention further provides use of the cyclic amino compound of formula (1), a salt thereof or solvates thereof for the preparation of an erythropoietin production potentiator.

The present invention further provides use of the cyclic amino compound of formula (1), a salt thereof or solvates thereof for the preparation of a preventive or therapeutic agent for diseases caused by the lowering of an erythropoietin production.

The present invention further provides a method of potentiating the erythropoietin production in a patient in need thereof, including administering an effective amount of the cyclic amino compound of formula (1), a salt thereof or solvates thereof.

The present invention further provides a method of treating the diseases caused by the lowering of erythropoietin production, including administering to a patient in need thereof, an effective amount of the cyclic amino compound of formula (1), a salt thereof or solvates thereof.

In brief, the present invention provides a novel erythropoietin production potentiator, and preventive or therapeutic agent for the diseases, such as anemia, caused by the lowering of erythropoietin production.

Brief Description of the Drawings

Fig. 1 is a view showing the effect of addition of medicaments on the amount of a EPO protein produced by Hep3B cells under 1%$O_2$.

Fig. 2 is a view showing the effect of addition of medicaments on the activity of EPO promotor.

Fig. 3 is a view showing the effect of addition of medicaments on Ht value in anemia model mice.

Fig. 4 is a view showing the effect of addition of medicaments on Hb amount in anemia model mice.

Fig. 5 is a view showing the effect of addition of medicaments on the number of reticulocytes in anemia model mice.

Fig. 6 is a view showing the effect of addition of medicaments on the EPO amount in blood serum.

Best Mode for Carrying out the Invention

In the general formula (1), the halogen atoms for $R^1$,$R^2$ and $R^3$ include fluorine, chlorine, bromine and iodine atoms.

The alkyl group for $R^1$, $R^2$, $R^3$ and $R^4$ typically includes straight, branched or cyclic $C_1$-$C_8$ alkyl groups, such as straight or branched $C_1$-$C_8$ alkyl groups, for example, methyl, ethyl, propyl, butyl, pentyl, hexyl, heptyl and octyl groups, and $C_3$-$C_8$ cycloalkyl groups, for example, cyclopropyl, cyclobutyl, cyclopentyl, cyclohexyl, cyclohexylmethyl and cyclohexylethyl groups. Among them, particularly preferred are $C_1$-$C_6$ alkyl groups such as methyl, ethyl, n-propyl, isopropyl, n-butyl and the like.

The halogen-substituted alkyl group for $R^1$, $R^2$ and $R^3$ typically includes $C_1$-$C_8$ alkyl groups substituted with 1 to 3 halogen atoms. Among them, particularly preferred are $C_1$-$C_6$ alkyl groups substituted with 1 to 3 halogen atoms, such as trifluoromethyl, 2,2,2-trifluoroethyl, etc.

The alkoxy group typically includes straight, branched or cyclic $C_1$-$C_8$ alkoxy groups, such as straight or branched $C_1$-$C_8$ alkoxy groups, for example, methoxy, ethyoxy, n-propoxy, isopropoxy, n-butoxy, isobutoxy, sec-butoxy, tert-butoxy, pentyloxy and hexyloxy groups; and $C_3$-$C_8$ cycloalkyloxy groups, for example, cyclopropyloxy, cyclobutyloxy, cyclopentyloxy, cyclohexyloxyl, cyclohexylmethyloxy and cyclohexylethyloxy groups. Among them, particularly preferred are $C_1$-$C_6$ alkoxy groups such as methoxy, ethoxy, n-propoxy, isopropoxy and n-butyloxy groups.

The alkylthio group typically includes $C_1$-$C_8$ alkylthio groups, and is preferably a $C_1$-$C_6$ alkylthio group such as, for example, methylthio, ethylthio, n-propylthio, isopropylthio or the like.

The alkoxycarbonyl group typically includes $C_1$-$C_6$ alkoxycarbonyl groups, and is preferably a $C_1$-$C_4$ alkoxycarbonyl group such as methoxycarbonyl, ethoxycarbonyl, tert-butoxycarbonyl or the like.

The alkanoyl group typically includes $C_1$-$C_6$ alkanoyl groups and is preferably a $C_1$-$C_4$ alkanoyl group such as acetyl, propionyl, butyryl, isobutyryl or the like.

The alkenyl group for $R^4$ typically includes $C_3$-$C_8$ alkenyl groups and is preferably a $C_3$-$C_6$ alkenyl group such as 2-propenyl, 3-butenyl or the like. The alkynyl group typically includes $C_3$-$C_8$ alkynyl groups and is preferably a $C_3$-$C_6$ alkynyl group such as 2-propynyl, 3-butynyl or the like.

The aryl group for $R^4$ typically includes $C_6$-$C_{14}$ aryl groups and is preferably phenyl, naphthyl, anthryl, indenyl, indanyl, 5,6,7,8-tetrahydronaphthyl or the like.

The heteroaryl group for $R^4$ typically includes heteroaryl groups of 5- or 6-membered ring containing 1 to 4

nitrogen atoms in the ring, and is preferably imidazolyl, pyridyl, pyrimidinyl or the like. The aralkyl group typically includes $C_6$-$C_{14}$ aryl-$C_1$-$C_6$ alkyl groups such as phenyl $C_1$-$C_6$ alkyl groups and naphthyl $C_1$-$C_6$ alkyl groups, for example, benzyl, naphthylmethyl, phenylethyl, phenylpropyl, etc. The heteroaralkyl group for $R^4$ typically includes 5- or 6-membered ring heteroaryl containing 1 to 4 nitrogen atoms-$C_1$-$C_6$ alkyl groups, such as imidazolyl-$C_1$-$C_6$ alkyl, pyridyl-$C_1$-$C_6$ alkyl, pyrimidinyl -$C_1$-$C_6$ alkyl, etc.

The groups which can substitute the above-mentioned aryl, heteroaryl, aralkyl or heteroaralkyl include 1 to 3 groups or atoms selected from alkyl, alkoxy, halogen-substituted alkoxy, alkylthio, alkylsulfinyl, alkylsulfonyl, halogen, nitro, amino, acetylamino, trifluoromethyl and alkylenedioxy, wherein said alkyl, alkoxy and alkylthio include those illustrated for $R^1$~$R^3$. The alkyl group contained in the alkylsulfinyl and alkylsulfonyl groups include $C_1$-$C_3$ alkyl groups such as methyl, ethyl, n-propyl and isopropyl groups. The halogen-substituted alkoxy includes $C_1$-$C_8$ alkoxy groups substituted by 1 to 3 halogen atoms, and is preferably a $C_1$-$C_4$ alkoxy group substituted by 1 to 3 halogen atoms such as trifluoromethoxy or 2,2,2-trifluoroethoxy. The alkylenedioxy group typically includes $C_1$-$C_3$ alkylenedioxy groups such as methylenedioxy, ethylenedioxy and propylenedioxy groups.

Preferably, X represents $NR^4$. More preferably, $R^4$ represents a $C_1$-$C_8$ alkyl group or a substituted or unsubstituted $C_6$-$C_{14}$ aryl group or a substituted or unsubstituted 5- or 6-membered ring heteroaryl group containing 1 to 4 nitrogen atoms in the ring or a substituted or unsubstituted $C_6$-$C_{14}$ aryl -$C_1$-$C_6$ alkyl group or a substituted or unsubstituted 5- or 6-membered ring heteroaryl -$C_1$-$C_6$ alkyl group containing 1 to 4 nitrogen atoms in the ring.

Preferably, $R^1$, $R^2$ and $R^3$ are attached to the phenyl group at the 3, 4 and 5-positions thereof. In this case, it is particularly preferable that $R^1$ and $R^3$ (at the 3- and 5- positions of the phenyl ring) are an alkoxy group or a halogen. It is also preferable that $R^2$ (at the 4- position of the phenyl ring) is a hydrogen atom, a halogen atom, or a hydroxy, alkyl, halogen-substituted alkyl, alkoxy, alkylthio, carboxy, alkoxycarbonyl or alkanoyl group.

I denotes 0 or 1, and is preferably 1.

Preferably, $W^1$ represents N. Preferably, $W^2$ represents N.

Preferable compounds include the compounds of the formula (1), wherein X represents $NR^4$, and $R^4$ represents a $C_1$-$C_8$ alkyl group or a substituted or unsubstituted $C_6$-$C_{14}$ aryl group or a substituted or unsubstituted 5- or 6-membered ring heteroaryl group containing 1 to 4 nitrogen atoms in the ring ring or a substituted or unsubstituted $C_6$-$C_{14}$ aryl -$C_1$-$C_6$ alkyl group or a substituted or unsubstituted 5- or 6-membered ring heteroaryl -$C_1$-$C_6$ alkyl group containing 1 to 4 nitrogen atoms in the ring. Particularly preferably, $R^4$ represents a phenyl or pyridyl group which may be substituted with one or two groups or atoms selected from halogen, alkyl, alkoxy, alkylthio, trifluoromethyl and alkylenedioxy.

Among the compound (1), preferred are 4-[N-(4-methoxyphenyl)-N-[5-(3, 4, 5-trimethoxyphenyl)pyridin-3-yl]methyl]amino]-1-[[2-(3, 4, 5-trimethoxyphenyl)pyridin-4-yl]methyl]piperidine, 4-[N-(3, 5-dimethoxyphenyl)-N-[[2-(3, 4, 5-trimethoxyphenyl)pyridin-4-yl]methyl]amino]-1-[[2-(3, 4, 5-trimethoxyphenyl)pyridin-4-yl]methyl]piperidine, 4-[N-(3, 4-methylenedioxyphenyl)-N-[[2-(3, 4, 5-trimethoxyphenyl)pyridin-4-yl]methyl]amino]-1-[[2-(3, 4, 5-trimethoxyphenyl) pyridin-4-yl]methyl]piperidine, 4-[N-methyl-N-[[2-(3, 4, 5-trimethoxyphenyl)pyridin-4-yl]methyl]amino]-1-[[2-(3, 4, 5-trimethoxyphenyl)pyridin-4-yl]methyl]piperidine, 4-[N-(4-methylthio)phenyl]-N-[[5-(3, 4, 5-trimethoxyphenyl)pyridin-3-yl] methyl]amino]-1-[[2-(3, 4, 5-trimethoxyphenyl)pyridin-4-yl]methyl]piperidine, or a salt thereof.

No particular limitation is imposed on the acid addition salts of the compounds (1) according to the invention as long as they are pharmaceutically acceptable salts. Examples include the acid-addition salts of mineral acids, such as hydrochlorides, hydrobromides, hydroiodides, sulfates and phosphates; and acid-addition salts of organic acids, such as benzoates, methanesulfonates, ethanesulfonates, benzenesulfonates, p-toluenesulfonates, oxalates, maleates, fumarates, tartrates, citrates and acetates.

The compounds of formula (1) may be present in the form of solvates typified by hydrates, and the solvates are embraced in the present invention.

The compounds of formula (1) can be prepared in accordance with the following processes A-L:

Process A: Preparation of the compound of the formula (1) wherein I = 1, m =0, n=1 and X=$CONR^4$

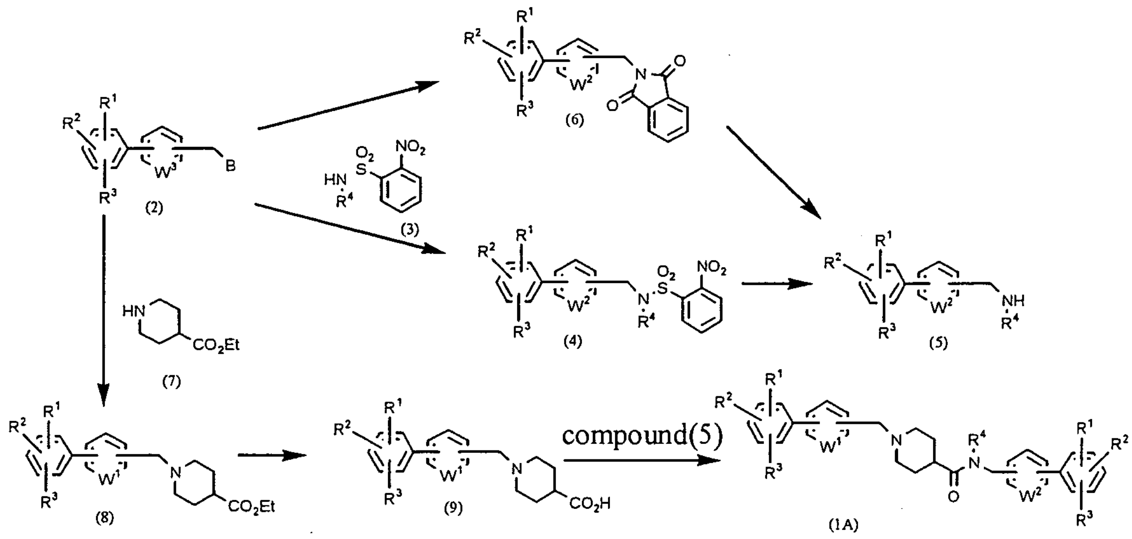


wherein, $W^1$, $W^2$ , $R^1$, $R^2$, $R^3$ and $R^4$ are as defined above, $W^3$ has the same meaning as $W^1$ or $W^2$, and B denotes a leaving group such as a halogen atom, or methanesulfonyloxy or p-toluenesulfonyloxy group.

Compound (2) and an N-(2-nitro)benzenesulfonylamine derivative (3) are reacted to give compound (4). The resulting compound (4) is treated with thiophenol in the presence of a base such as potassium carbonate to eliminate the 2-nitrobenzenesulfonyl group, thereby giving amine compound (5). Alternatively, when $R^4$ is H, it is possible to react compound (2) with potassium phthalimide and then treat the resulting phthalimide derivative (6) with hydrazine to give the corresponding amine compound (5).

On the other hand, compound (2) is reacted with ethyl isonipecotate (7) in a solvent such as acetonitrile, N, N-dimethylformamide (DMF), dimethyl sulfoxide (DMSO), tetrahydrofuran (THF), dioxane, toluene, benzene, etc. in the presence of a base such as potassium carbonate or the like at a temperature between 0°C and a reflux temperature for several hours to several days, preferably at room temperature overnight, to give compound (8). The compound (8) is subjected to a usual alkaline hydrolysis to give the corresponding carboxylic acid compound (9).

The carboxylic acid compound (9) is reacted with the amine compound (5) using a dehydration condensing agent such as 1-(3-dimethylaminopropyl)-3-ethylcarbodiimide hydrochloride (water-soluble carbodiimide), 2-(1H-benzotriazol -1-yl)-1,1,3,3-tetramethyluronium hexafluorophosphate (HBTU) or the like in a solvent such as chloroform, dichloroethane, THF, dioxane, acetonitrile, etc. at a temperature between 0°C and a reflux temperature for several hours to several days, preferably at room temperature for 12 hours, to give an end product (1A).

Process B: Preparation of the compound of the formula (1) wherein l = 1, m =0, n=1 and X=O

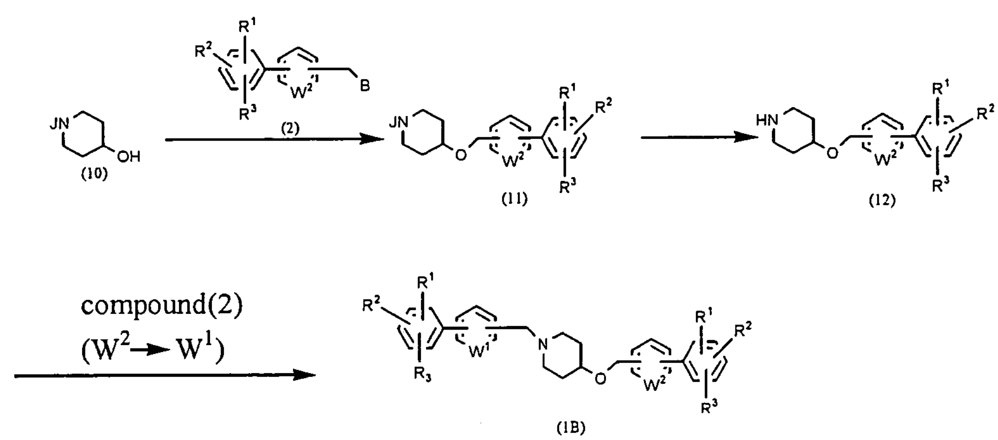


wherein, B, $W^1$, $W^2$ , $R^1$, $R^2$ and $R^3$ are as defined above, and J denotes a protecting group such as benzyloxycarbonyl, tert-butoxycarbonyl, acetyl, benzoyl or benzyl group.

Incidentally, in the reaction schemes shown above and below, the expression "($W^2 \rightarrow W^1$)" means that $W^2$ in the formula representing compound (2) is changed to $W^1$. The similarly applies to the reaction schemes shown below.

4-Hydroxypiperidine compound (10) with a protected amino group is reacted with compound (2) in the presence of sodium hydride and potassium iodide in a solvent such as DMF, DMSO, etc. at a temperature between 0°C and a reflux temperature for several hours to several days, preferably at room temperature for 2 days, to give compound (11). The protecting group in the compound (11) is removed in a known manner. The resulting compound (12) is reacted with compound (2) in the presence of a base such as potassium carbonate in a solvent such as acetonitrile, DMF, DMSO, THF, dioxane, etc. at a temperature between 0°C and a reflux temperature for several hours to several days, preferably at room temperature for 4 hours, to give an end product (1B).

Process C: Preparation of the compound of the formula (1) wherein l = 1, m =0, n=0, X=$NR^4CO$ and $R^4$=H or Me

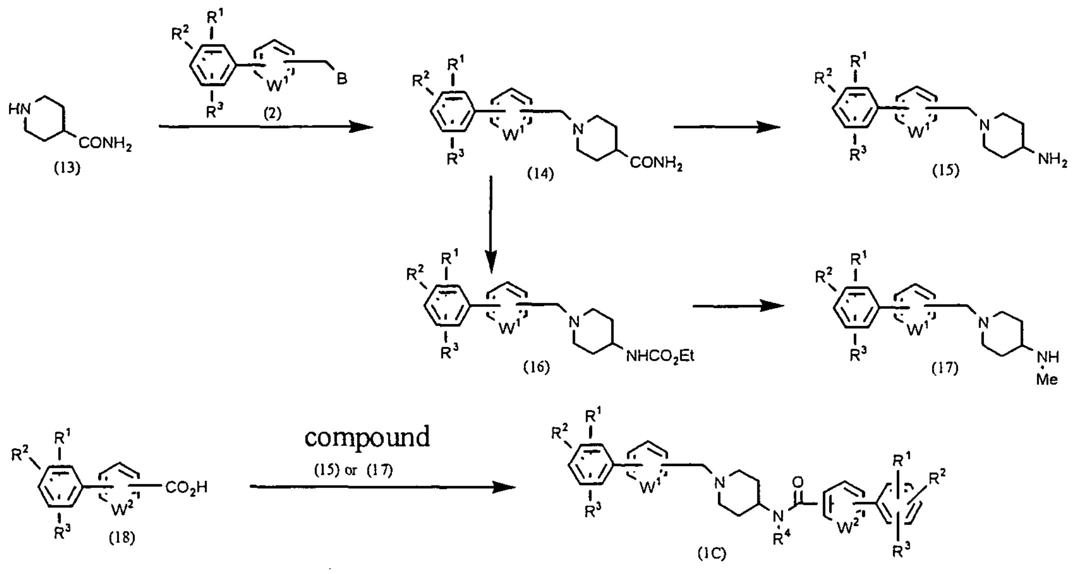

(13) (2) (14) (15) (16) (17) (18) (1C)

wherein, B, $W^1$, $W^2$, $R^1$, $R^2$ and $R^3$ are as defined above, and $R^4$ denotes a hydrogen atom or methyl group.

Isonipecotamide (13) is reacted with compound (2) in the presence of a base such as potassium carbonate, sodium carbonate or the like in a solvent such as acetonitrile, DMF, DMSO, THF, dioxane, etc. at a temperature between 0°C and a reflux temperature for several hours to several days, preferably at room temperature for 4 hours, to give compound (14). The compound (14) is subjected to Hofmann rearrangement reaction to give amine compound (15).

On the other hand, by subjecting the compound (14) to Hofmann rearrangement reaction in ethanol, carbamate compound (16) is obtained. Then, by subjecting the compound (16) to a reduction reaction using lithium aluminum hydride, methylamine compound (17) is obtained.

By reacting carboxylic acid compound (18) with the amine compound (15) or methylamine compound (17) similarly to the condensation reaction in Process A, an end compound (1C) is obtained.

Process D: Preparation of the compound of the formula (1) wherein l = 1, m =0, n=1 and X=$NR^4$

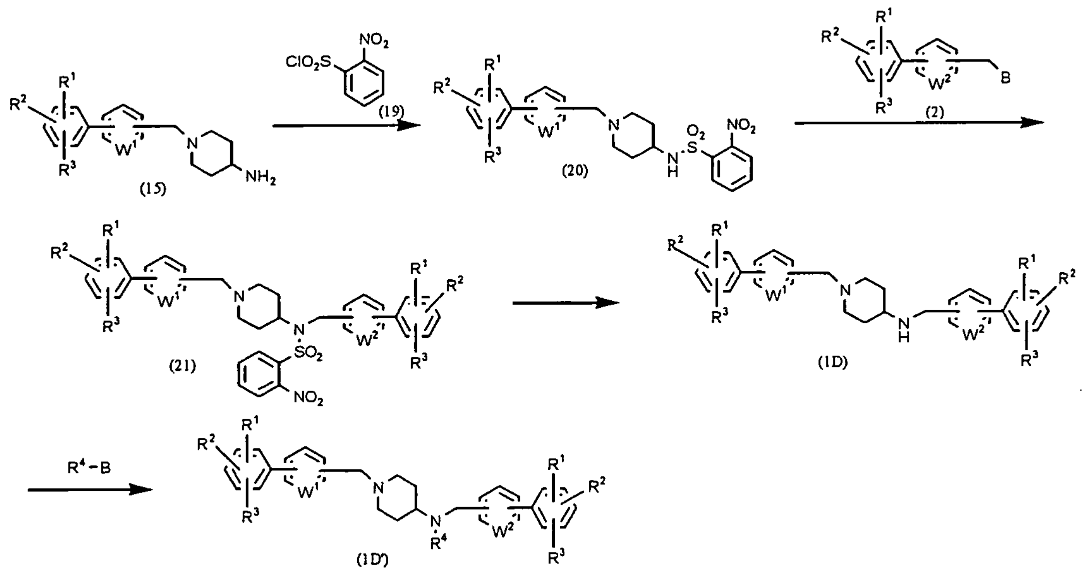


(15) (20) (21) (1D) (1D')

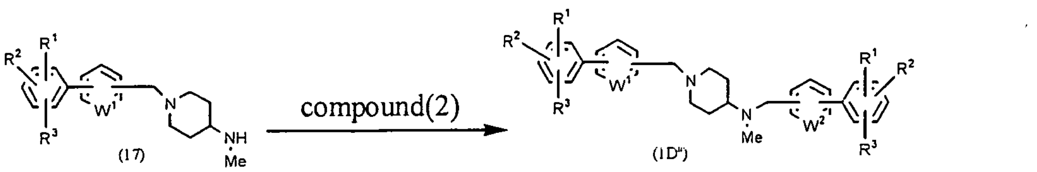


(17) (1D")

wherein, B, $W^1$, $W^2$, $R^1$, $R^2$ and $R^3$ are as defined above, and $R^4$ denotes an alkyl, alkenyl, alkynyl, aralkyl or heteroaralkyl group.

The amine compound (15) mentioned in the above is reacted with 2-nitrobenzenesulfonyl chloride (19) according to a known manner to give compound (20). The compound (20) is reacted with compound (2) in the presence of a base such as potassium carbonate in a solvent such as acetonitrile, DMF, DMSO, THF, dioxane or the like at a temperature between 0°C and a reflux temperature for several hours to several days, preferably at room temperature for 4 hours, to give compound (21). The benzenesulfonyl group of the compound (21) is removed similarly to the procedure for the compound (4) in Process A to give an end compound (1D) ($R^4$=H). The compound (1D) is reacted with $R^4$-B in the presence of a base such as sodium carbonate, sodium bicarbonate, potassium carbonate, cesium carbonate or the like in a solvent such as acetonitrile, THF, dioxane, chloroform, dichloromethane, DMF, DMSO or the like at a temperature between 0°C and a reflux temperature for several hours to several days, preferably at 80°C for 12 hours, to give compound (1D').

On the other hand, the methylamine compound (17) is reacted compound (2) in the presence of a base such as potassium carbonate in a solvent such as acetonitrile, DMF, DMSO, THF, dioxane or the like at a temperature between 0°C and a reflux temperature for several hours to several days, preferably at room temperature for 4 hours, to give an end compound (1D") ($R^4$=Me).

Process E: Preparation of the compound of the formula (1) wherein l = 1, m =0 or 1, n=1 and X=$NR^4$,

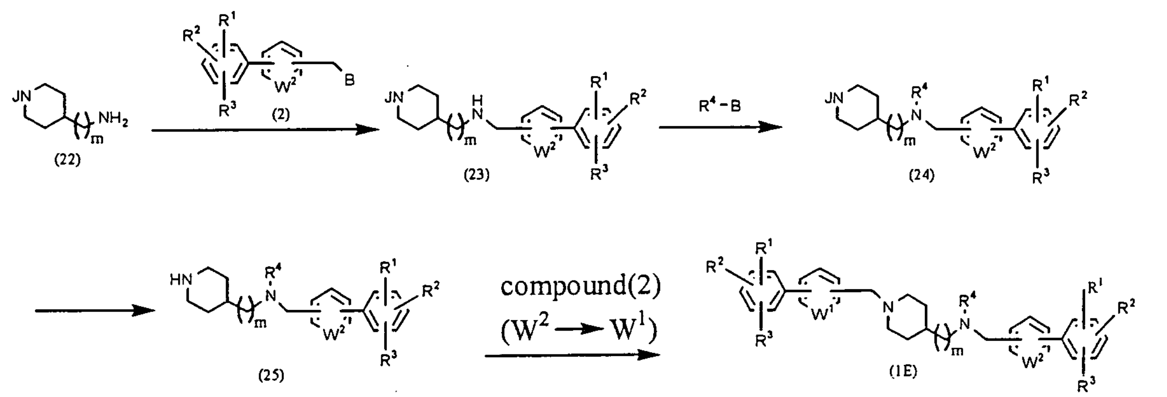


wherein, B, J, $W^1$, $W^2$ , $R^1$, $R^2$ and $R^3$ are as defined above, and $R^4$ denotes an alkyl, alkenyl, alkynyl, aralkyl or heteroaralkyl group.

Aminopiperidine derivative (22) in which the amino group on the ring is protected is reacted with compound (2) in the presence of a base such as potassium carbonate in a solvent such as acetonitrile, DMF, DMSO, THF, dioxane or the like at a temperature between 0°C and a reflux temperature for several hours to several days, preferably at room temperature for 4 hours, to give compound (23). The compound (23) is reacted with $R^4$-B in the presence of a base such as sodium carbonate, sodium bicarbonate, potassium carbonate, cesium carbonate or the like in a solvent such as acetonitrile, THF, dioxane, chloroform, dichloroethane, DMF, DMSO or the like at a temperature between 0°C and a reflux temperature for several hours to several days, preferably at 80°C for 12 hours, to give compound (24). After removal of the protecting group in the compound (24), the resulting compound is reacted with compound (2) in the presence of a base such as potassium carbonate in a solvent such as acetonitrile, DMF, DMSO, THF, dioxane or the like at a temperature between 0°C and a reflux temperature for several hours to several days, preferably at room temperature for 4 hours, to give compound (1E).

Process F: Preparation of the compound of the formula (1) wherein l = 1, m =0, n=1 and X=$NR^4$,

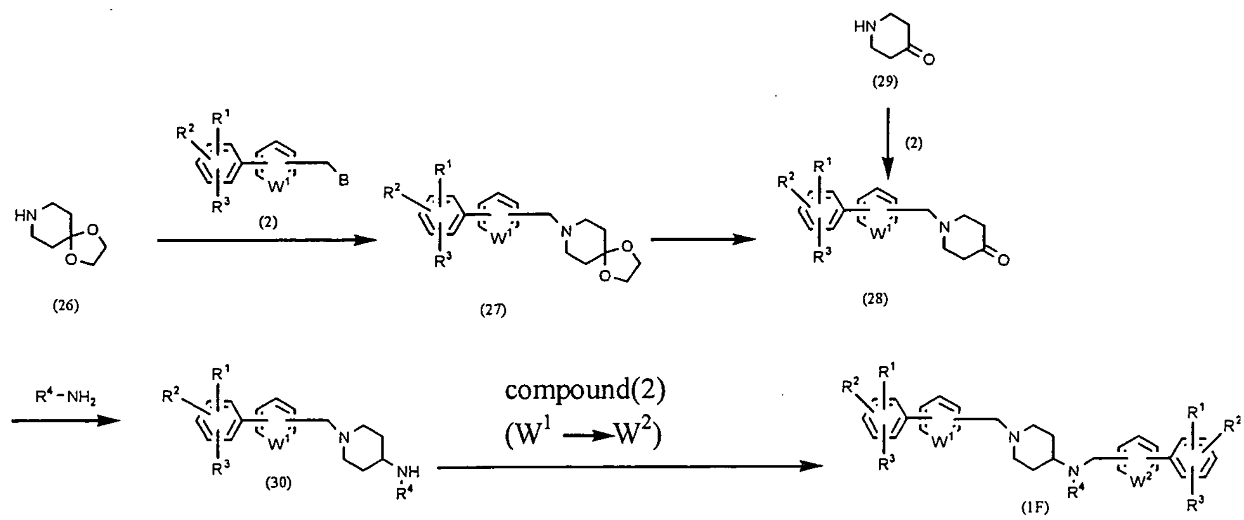


wherein, B, $W^1$, $W^2$ , $R^1$, $R^2$ and $R^3$ are as defined above, and $R^4$ denotes an alkyl, alkenyl, alkynyl, aralkyl, heteroaralkyl, aryl or heteroaryl group.

4-Piperidone ethylene ketal (26) is reacted with compound (2) in the presence of a base such as potassium carbonate in a solvent such as acetonitrile, DMF, DMSO, THF, dioxane, etc. at a temperature between 0°C and a reflux temperature for several hours to several days, preferably at room temperature for 4 hours, to give compound (27), which in turn is deketalized by using an acid to give ketone compound (28).

On the other hand, 4-piperidone (29) is reacted compound (2) in the presence of a base such as potassium carbonate in a solvent such as acetonitrile, DMF, DMSO, THF, dioxane or the like at a temperature between 0°C and a reflux temperature for several hours to several days, preferably at room temperature for 4 hours, to give

compound (28). Using the compound (28), amine compound (30) can be prepared according to either of the following two synthesis processes:

Synthesis process 1: The compound (28) is reacted with an amine compound of the formula: $R^4$-$NH_2$ in the presence of molecular sieves in toluene or benzene at a temperature between 0°C and a reflux temperature for several hours to several days, preferably at reflux temperature for 12 hours, followed by reaction with a reducing agent such as sodium borohydride or sodium cyanoborohydride in a solvent such as methanol, ethanol, propanol, isopropanol etc. at a temperature between 0°C and a reflux temperature for several minutes to several days, preferably at room temperature for 1 hour, to give the amine compound (30).

Synthesis process 2: The compound (28) is reacted with an amine compound of the formula: $R^4$-$NH_2$ in the presence of a reducing agent such as sodium triacetoxy boron hydride in a solvent such as dichloromethane, 1,2-dichloroethane, methanol, ethanol, etc. at a temperature between 0°C and a reflux temperature for several minutes to several days, preferably at room temperature for 4 hours, to give the amine compound (30).

The resulting compound (30) is reacted with compound (2) in a solvent such as acetonitrile, DMF, DMSO, THF, dioxane, etc. at a temperature between 0°C and a reflux temperature for several hours to several days, preferably at room temperature for 4 hours, to give an end product (IF).

Process G: Preparation of the compound of the formula (1) wherein l = 1, m =0, n=1 and X=$NR^4$

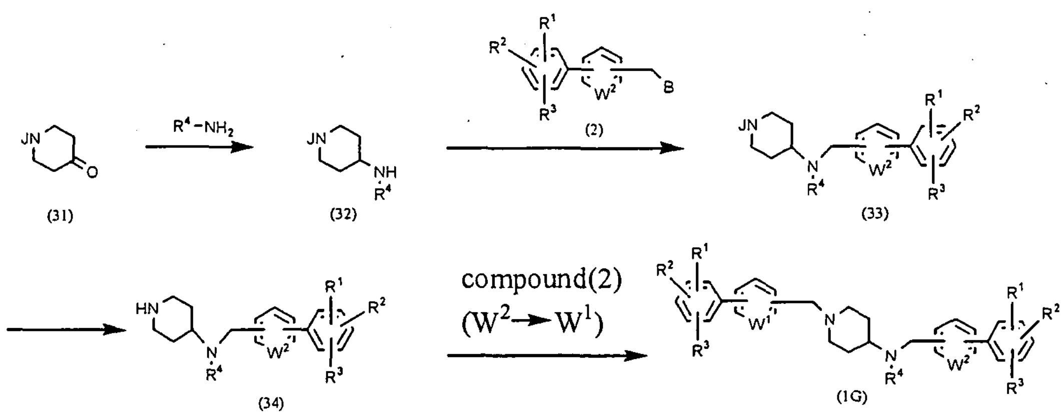


(31) (32) (2) (33) (34) (1G)

wherein, B, J, $W^1$, $W^2$ , $R^1$, $R^2$ and $R^3$ are as defined above, and $R^4$ denotes an alkyl, alkenyl, alkynyl, aralkyl, heteroaralkyl, aryl or heteroaryl group.

4-Piperidone derivative (31) in which the amino group is protected is reacted with an amine compound $R^4$-$NH_2$ similarly to the procedure for preparation of compound (30) in Process F to give compound (32). The compound (32) is reacted with compound (2) in the presence of a base such as potassium carbonate in a solvent such as acetonitrile, DMF, DMSO, THF, dioxane, etc. at a temperature between 0°C and a reflux temperature for several hours to several days, preferably at room temperature for 4 hours, to give compound (33). After removal of the protecting group from the compound (33), the resulting compound (34) is reacted with compound (2) in the presence of a base such as potassium carbonate in a solvent such as acetonitrile, DMF, DMSO, THF, dioxane, etc. at a temperature between 0°C and a reflux temperature for several hours to several days, preferably at room temperature for 4 hours, to give an end product (1G).

Process H: Preparation of the compound of the formula (1) wherein l = 0, m =0, n=1 and X=NH

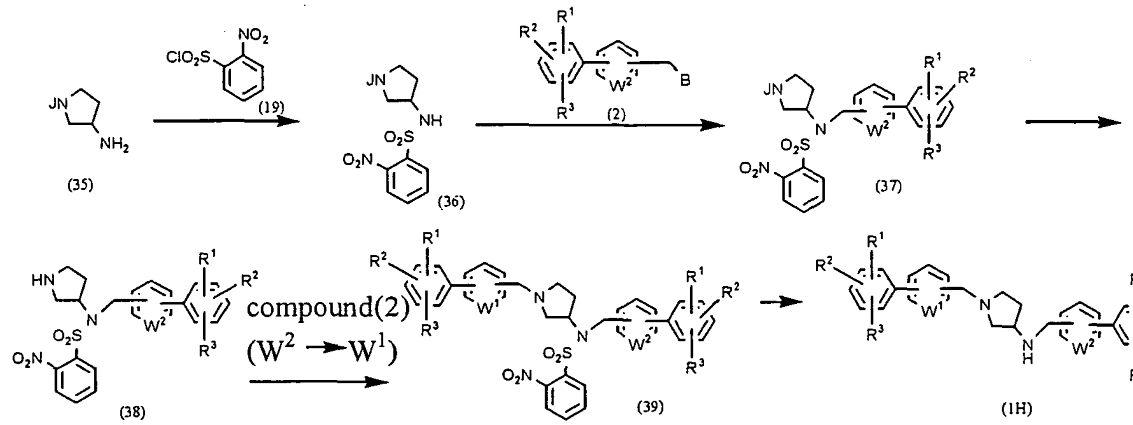


wherein, B, J, $W^1$, $W^2$ , $R^1$, $R^2$ and $R^3$ are as defined above.

3-Aminopyrrolidine derivative (35) with a protected amino group on the ring is reacted with 2-nitrobenzenesulfonyl chloride (19) under usual conditions to give a benzenesulfonyl derivative (36). The derivative (36) is reacted with compound (2) in the presence of a base such as potassium carbonate in a solvent such as acetonitrile, DMF, DMSO, THF, dioxane, etc. at a temperature between 0°C and a reflux temperature for several hours to several days, preferably at room temperature for 4 hours, to give compound (37). The protecting group of the amino group is removed from the compound (37) to give compound (38), which in turn is reacted with compound (2) in the presence of a base such as potassium carbonate in a solvent such as acetonitrile, , DMF, DMSO, THF, dioxane, etc. at a temperature between 0°C and a reflux temperature for several hours to several days, preferably at room temperature for 4 hours, to give compound (39). By subjecting the compound (39) to a reaction similar to that in the preparation of compound (5) in Process A, an end product (1H) is obtained.

Process I: Preparation of the compound of the formula (1) wherein l = 0, m =0, n=1 and X=$NR^4$

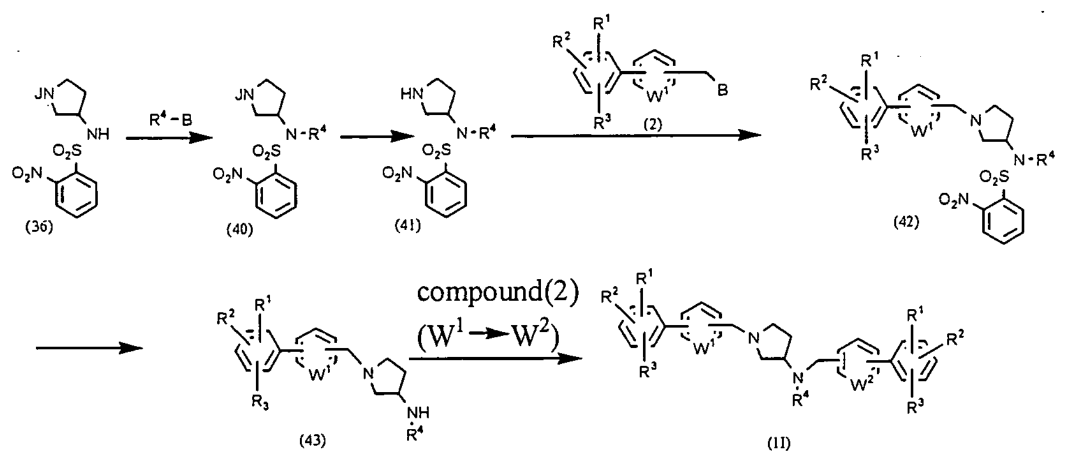


wherein, B, J, $W^1$, $W^2$ , $R^1$, $R^2$ and $R^3$ are as defined above, and $R^4$ denotes an alkyl, alkenyl, alkynyl or aralkyl group.

Compound (36) is reacted with $R^4$-B in the presence of a base such as sodium carbonate, potassium carbonate, etc. in a solvent such as acetonitrile, THF, dioxane, chloroform, dichloroethane, DMF, DMSO, etc. at a temperature between 0°C and a reflux temperature for several hours to several days, preferably at 80°C for 12 hours, to give compound (40). The amino-protecting group is removed from the compound (40), and the resulting compound (41) is reacted with compound (2) in the presence of a base such as potassium carbonate in a solvent such as acetonitrile, DMF, DMSO, THF, dioxane, etc. at a temperature between 0°C and a reflux temperature for several hours to several days, preferably at room temperature for 4 hours, to give compound (42). By subjecting the compound (42) to a reaction similar to that in the preparation of compound (5) in Process A, compound (43) is obtained. The compound (43) is reacted with compound (2) in the presence of a base such as potassium carbonate in a solvent such as acetonitrile, DMF, DMSO, THF, dioxane, etc. at a temperature between 0°C and a reflux temperature for several hours to several days, preferably at room temperature for 4 hours, to give an end product (1I).

Process J: Preparation of the compound of the formula (1) wherein $R^2$=OH

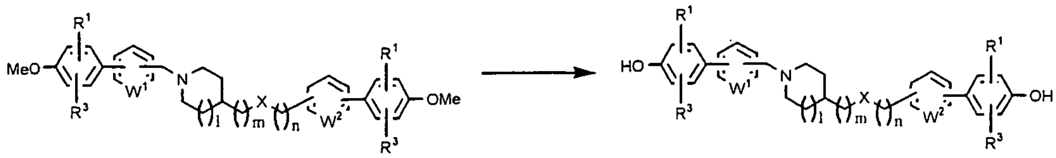

(1J) (1J')

wherein, X, $W^1$, $W^2$ , $R^1$, $R^3$, 1, m and n have the same meanings as defined above.

By reacting methoxy compound (1J) with iodotrimethylsilane in a solvent such as toluene, benzene, chloroform, dichloromethane, etc. at a temperature between -25°C and a reflux temperature for several minutes to several days, preferably at 0°C for 2 hours, there can be obtained an end product (1J').

Process K: Preparation of the compound of the formula (1) wherein l = 1, m = 0, n = 0 and X = $NR^4CO$

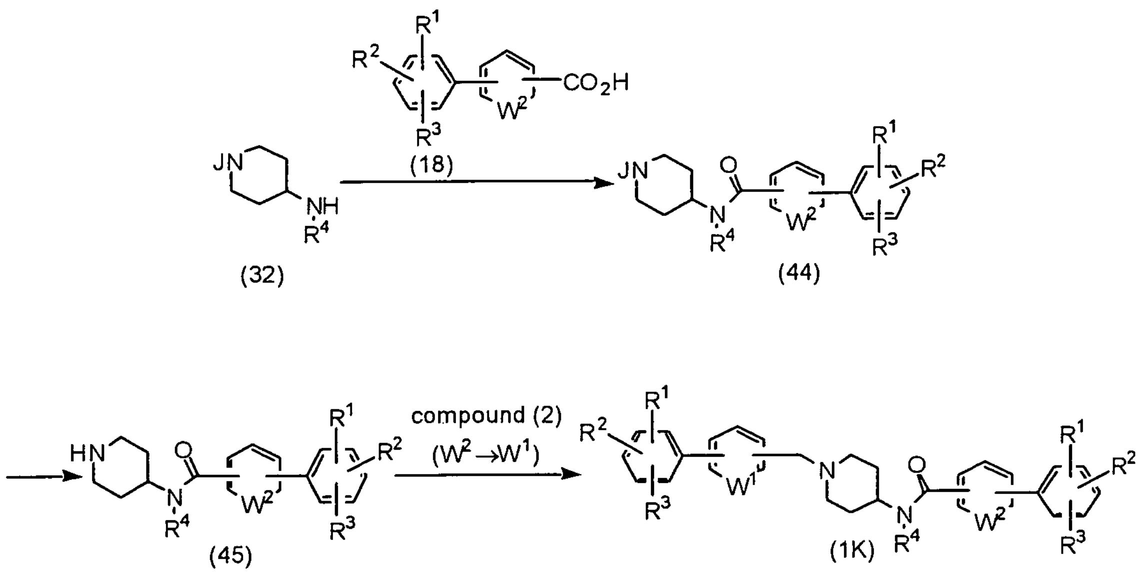


(18) (32) (44) (45) (1K)

wherein, B, J, $W^1$, $W^2$, $R^1$, $R^2$ and $R^3$ are as defined above, and $R^4$denotes an alkyl, alkenyl, alkynyl, aralkyl, heteroaralkyl, aryl or heteroaryl group.

Compound (32), which is described in the Process G, is reacted with compound (18) in the similar procedure as described in the preparation of compound (1A) in Process A to give compound (44). After removal of the protecting group from the compound (44), the resulting compound (45) is reacted with compound (2) in the presence of a base such as potassium carbonate in a solvent such as acetonitrile, DMF, DMSO, THF, dioxane, etc. at a temperature between 0°C and a reflux temperature for several hours to several days, preferably at room temperature for 4 hours, to give an end product (1K).

Process L: Oreparation of the compound of the formula (1) wherein l=1, m=0, n=1 and X=alkylsulfonylphenyl amino group

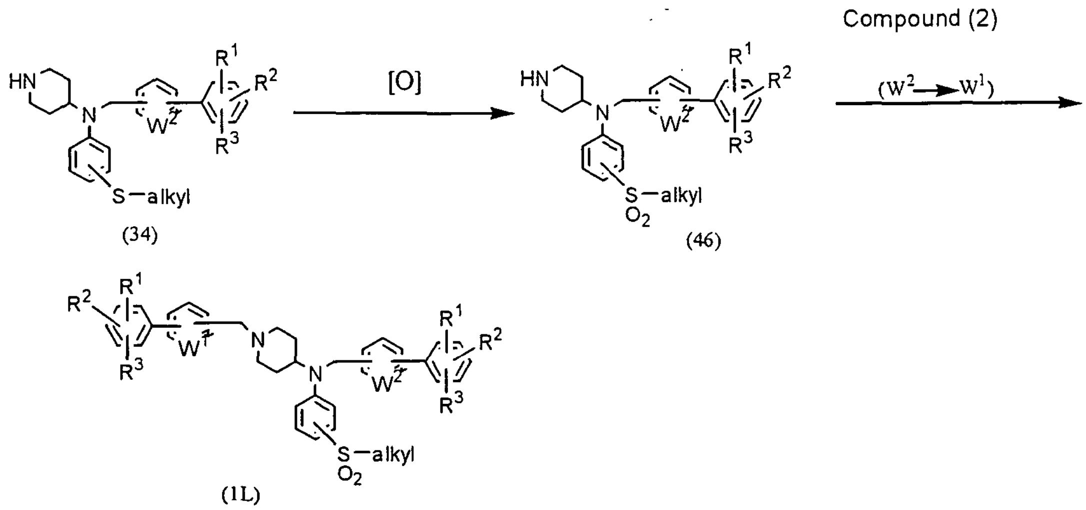


(34) (46) (1L)

wherein, B, $W^1$, $W^2$, $R^1$, $R^2$ and $R^3$ are as defined above.

The compound (34) which is synthesized according to process G and in which X is alkylthiophenylamino group is reacted with an oxidizing agent such as 3-chloropherbenzoic acid, peracetic acid or hydrogen peroxice according to a known manner to give an alkylsulfoxide derivative (46). The compound (46) is reacted with compound (2) in the presence of a base such as potassium carbonate in a solvent such as acetonitrile, DMF, DMSO, THF or dioxane at a temperature between 0°C and a reflux temperature for several hours to several days, preferably at 70°C overnight, to give an end product (1L).

The compounds (1) according to the present invention are obtained by any of the above-described processes and may further be purified by using an ordinary purification means such as recrystallization or column chromatography as needed. As needed, the compounds may also be converted into the desired salts or solvates in a method known *per se* in the art.

When the compounds (1) have an asymmetric carbon atom, the present invention includes any configurational isomers.

The compounds (1) according to the present invention have an action for potentiating the production of erythropoietin strongly, and are useful as preventive or therapeutic agents for treating pathological conditions caused by reduced production of erythropoietin in mammalian including human, such as anemia, especially chronic anemia, renal anemia, aplastic anemia, or pure red cell aplasia.

The medicine according to the present invention comprises a compound (1), a salt thereof, or a solvate thereof as an active ingredient. The form of administration may be suitably selected as necessary for the therapeutic application intended without any particular limitation, including oral preparations, injections, suppositories, ointments, inhalants, eye drops, nose drops and plasters. A composition suitable for use in these administration forms can be prepared by blending a pharmaceutically acceptable carrier in accordance with the conventional preparation method publicly known by those skilled in the art.

When an oral solid preparation is formulated, an excipient, and optionally, a binder, disintegrator, lubricant, colorant, a taste corrigent, a smell corrigent and the like are added to compound (1) and the resulting composition can be formulated into tablets, coated tablets, granules, powders, capsules, etc. in accordance with methods known in the art. As such additives described above, any additives may be used which are generally used in the pharmaceutical field. Examples include excipients such as lactose, sucrose, sodium chloride, glucose, starch, calcium carbonate, kaolin, microcrystalline cellulose and silicic acid; binders such as water, ethanol, propanol, simple syrup, glucose solution, starch solution, gelatin solution, carboxymethyl cellulose, hydroxypropyl cellulose, hydroxypropyl starch, methyl cellulose, ethyl cellulose, shellac, calcium phosphate and polyvinyl pyrrolidone; disintegrators such as dry starch, sodium alginate, agar powder, sodium hydrogencarbonate, calcium carbonate, sodium lauryl sulfate, monoglyceryl stearate and lactose; lubricants such as purified talc, stearic acid salts, borax and polyethylene glycol; and taste corrigents such as sucrose, orange peel, citric acid and tartaric acid.

When an oral liquid preparation is formulated, a taste corrigent, buffer, stabilizer, smell corrigent and/or the like are added to compound (1) and the resulting composition can be formulated into internal liquid preparations, syrup preparations, elixirs, etc. in accordance with methods known in the art. In this case, the taste corrigent may be used those as described above. As the buffer, sodium citrate may be mentioned. As examples of the stabilizer, tragacanth, gum arabic and gelatin may be mentioned.

When an injection is formulated, a pH adjustor, buffer, stabilizer, isotonicity agent, local anesthetic and the like may be added to compound (1) according to the present invention, and the resultant composition can be formulated into subcutaneous, intramuscular and intravenous injections in accordance with methods known in the art. Examples of the pH adjustor and buffer in this case include sodium citrate, sodium acetate and sodium phosphate. Examples of the stabilizer include sodium pyrosulfite, EDTA, thioglycolic acid and thiolactic acid. Examples of the local anesthetic include procaine hydrochloride and lidocaine hydrochloride. Examples of the isotonicity agent include sodium chloride and glucose.

When a suppository is formulated, a carrier for a preparation known in the art, for example, polyethylene glycol, lanoline, cacao butter, fatty acid triglyceride or the like, and optionally, a surfactant such as Tween (registered trade mark) and the like are added to the compound (1), and the resultant composition can be formulated into suppositories in accordance with methods known in the art.

When an ointment is formulated, a base material, stabilizer, wetting agent, preservative and the like, which are generally used, are blended with compound (1) as needed, and the resulting blend is mixed and formulated into ointments in accordance with a known method. Examples of the base material include liquid paraffin, white vaseline, bleached beeswax, octyldodecyl alcohol and paraffin. Examples of the preservative include methyl p-hydroxybenzoate, ethyl p-hydroxybenzoate and propyl p-hydroxybenzoate.

Besides the above preparations, inhalants, eye drops and nose drops may also be formulated in accordance with known methods.

The dose of the medicine according to the present invention varies according to the age, sex, weight and condition of the patient to be treated, the administration method, the number of times of administration, and the like. It is however preferred that the medicine is generally orally or parenterally administered at once or in several portions in a dose of 0.01 to 1,000 mg per day, preferably, 0.1 to 100 mg per day in terms of compound (1), for an adult.

Examples

The present invention will hereinafter be described in more detail by Examples. However, the present invention is not limited to these examples.

Referential Example 1

Synthesis of ethyl 2-(3,4,5-trimethoxyphenyl)isonicotinate:

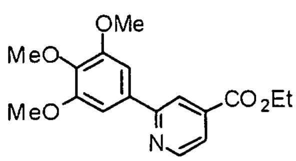

3,4,5-Trimethoxyphenylboronic acid (20.10 g) and ethyl 2-chloroisonicotinate (18.56g) were suspended in a mixted solvent of toluene (200 mL) and THF(100mL), and to the suspension 2 M sodium carbonate (200 mL) and tetrakis(triphenyl phosphine) palladium(0) (5.78 g) were added. The mixture was stirred at 90°C overnight under an argon atmosphere. Ethyl acetate was added to the reaction mixture for extraction, and the organic layer was washed with brine, dried over anhydrous magnesium sulfate and concentrated under reduced pressure. The residue was purified by column chromatography on silica gel using hexane-ethyl acetate (5:1) to give the title compound.
Yield: 27.99 g (88%).
$^1$H-NMR (400 MHz, $CDCl_3$) δ: 1.45 (t, 3H, J=7.0 Hz), 3.92 (s, 3H), 3.99 (s, 6H), 4.46 (q, 2H, J=7.0 Hz), 7.30 (s, 2H), 7.76 (dd, 1H, J=5.1 Hz, 1.6 Hz), 8.24 (dd, 1H, J=1.6 Hz, 0.8 Hz), 8.81 (dd, 1H, J=5.1 Hz, 0.8 Hz).

Referential Example 2

Synthesis of 4-hydroxymethyl-2-(3,4,5-trimethoxyphenyl)pyridine:

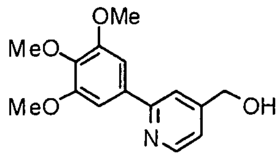

Ethyl 2-(3,4,5-trimethoxyphenyl)isonicotinate (24.57 g) was dissolved in dry THF (200 mL), and to the solution lithium aluminum hydride (2.94 g) was added at 0°C under an argon atmosphere. The mixture was stirred at 0°C for 1 hour as it is. A small amount of water and then sodium sulfate were added to the reaction mixture, and the resulting insoluble matters were filtered off through celite. The filtrate was concentrated under reduced pressure and the reultant crude crystals were recrystalized from ethyl acetate-hexane to give the title compound.
Yield: 17.53 g (82%).
$^1$H-NMR (400 MHz, $CDCl_3$) δ: 3.90 (s, 3H), 3.95 (s, 6H), 4.79 (s, 2H), 7.19 (d, 1H, J=5.1 Hz), 7.21 (s, 2H), 7.66 (s, 1H), 8.60 (d, 1H, J=5.1 Hz).

Referential Example 3

Synthesis of 4-chloromethyl-2-(3,4,5-trimethoxyphenyl)pyridine:

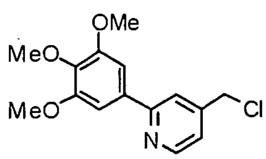

4-Hydroxymethyl-2-(3,4,5-trimethoxyphenyl)pyridine(19.18g) was dissolved in chloroform (100 mL), and to the solution thinly chloride (10.2 mL) was added at 0°C. After 30 minutes, the mixture was warmed to room temperature and stirred for 4 hours. The reaction mixture was washed with aqaueous saturated sodium hydrogendcarbonate and saturated brine, dried over anhydrous sodium sulfate and concentrated under reduced pressure. The crystalline residue was then recrystallized from ethyl acetate-hexane to give the title compound as pale yellow crystalline powder.
Yield: 18.24 g (89%).
$^1$H-NMR (400 MHz, $CDCl_3$) δ: 3.91 (s, 3H), 3.97 (s, 6H), 4.61 (s, 2H), 7.24 (s, 2H), 7.26 (d, 1H, J=5.1 Hz), 7.68 (s, 1H), 8.67 (d, 1H, J=5.1 Hz).

Referential Example 4

Synthesis of N-[[2-(3,4,5-trimethoxyphenyl)pyridin-4-yl]methyl]phthalimide:

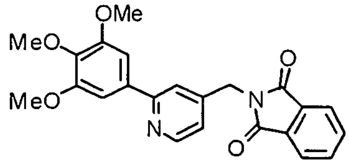

To a solution of 4-chioromethyl-2-(3,4,5-trimethoxyphenyl)pyridine (881 mg) in chloroform (10 mL) was added

potassium phthalimide (556 mg). The mixture was stirred at room temperature overnight and water was added. After separating the organic layer, the aqueous layer was extracted with chloroform. Organic layers were combined, dried over anhydrous magnesium sulfate and concentrated under reduced pressure to give the title compound as white powder.
Yield: 1.16 g (96%).

Referential Example 5

Synthesis of 4-aminomethyl-2-(3,4,5-trimethoxyphenyl)pyridine:

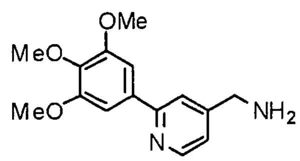

To a suspension of N-[[2-(3,4,5-trimethoxyphenyl)pyridin-4-yl]methyl] phthalimide (1.16 g) in ethanol (30 mL) was added hydrazine monohydrate (1 mL).
The mixture was refluxed for 3 hours. After cooling, insoluble matters were filtered off. The filtrate was concentrated under reduced pressure and the residue was dissolved in chloroform. The solution was washed with saturated aqueous sodium hydrogen carbonate and brine, dried over anhydrous magnesium sulfate and concentrated under reduced pressure to give the title compound as pale yellow oil.
Yield: 418 mg (53%).

Referential Example 6

Synthesis of ethyl 1-[[2-(3,4,5-trimethoxyphenyl)pyridin-4-yl]methyl]piperidine-4-carboxylate:

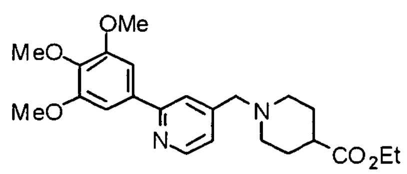

Ethyl piperidine-4-carboxylate (514 mg), 4-chloromethyl-2-(3,4,5-trimethoxyphenyl)pyridine (969 mg) and potassium carbonate (452 mg) were suspended in acetonitrile (20 mL). The suspension was stirred at room temperature for 4 hours. The reaction mixture was concentrated under reduced pressure and then chloroform and water were added thereto to separate an organic layer. An aqueous layer was extracted with chloroform. Organic layers were combined, and dried over anhydrous magnesium sulfate. After concentration under reduced pressure, the residue was purified by column chromatography on silica gel eluting with hexane-ethyl acetate (1:1) and then chloroform-methanol (40:1) to give the title compound as white prisms .
Yield: 1.20 g (88%).
$^1$H-NMR (400 MHz, $CDCl_3$) δ: 1.25 (t, 3H, J=7.0 Hz), 1.72-1.93 (m, 4H), 2.10 (t, 2H, J=9.8 Hz), 2.27-2.35 (m, 1H), 2.86 (d, 2H, J=11.3 Hz), 3.55 (s, 2H), 3.91 (s, 3H), 3.98 (s, 6H), 4.14 (q, 2H, J=7.0 Hz), 7.21 (d, 1H, J=4.9 Hz), 7.24 (s, 2H), 7.63 (s, 1H), 8.59 (d, 1H, J=5.1 Hz).

Referential Example 7

Synthesis of 1-[[2-(3,4,5-trimethoxyphenyl)pyridin-4-yl]methyl]piperidine-4-carboxylic acid:

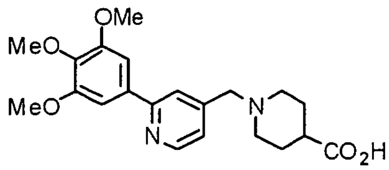

To a solution of ethyl 1-[[2-(3,4,5-trimethoxyphenyl)pyridin-4-yl] piperidine-4-carboxylate (760 mg) in ethanol (10 mL) was added 1 M sodium hydroxide (10 mL). The mixture was stirred at room temperature for 4 hours and ethanol was distilled away under reduced pressure. To the residue was added water (20 mL) and 5% aqueous potassium hydrogen sulfate was gradually added until pH of the solution became 7. Precipitated crystals were collected by filtration and the product was used for the next steps without further purification.
Yield: 779 mg (theoretical amount).

Preparation Example 1

Synthesis of 1-[[2-(3,4,5-trimethoxyphenyl)pyridin-4-yl]methyl]-4-[[2-(3,4,5-trimethoxyphenyl)pyridin-4-yl] methylaminocarbonyl]piperidine maleate:

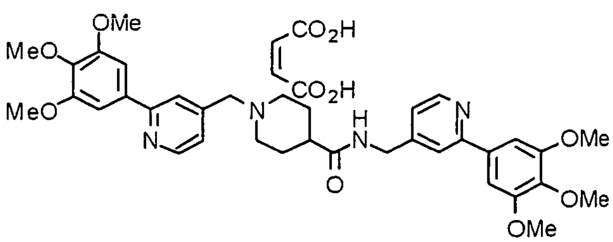

To a solution of 1-[[2-(3,4,5-trimethoxyphenyl)pyridin-4-yl]methyl] piperidine-4-caroxylic acid (97 mg) and 4-aminomethyl-2-(3,4,5-trimethoxyphenyl) pyridine (68 mg) in acetonitrile (5 mL) was added 2-(1H-benzotriazol-1-yl)-1,1,3,3-tetramethyluroniumhexafluorophosphate (95 mg). The mixture was stirred at room temperature for 12 hours and concentrated under reduced pressure. The residue was dissolved in chloroform, washed with saturated aqueous sodium hydrogen carbonate and saturated brine, dried over anhydrous magnesium sulfate and concentrated under reduced pressure. The resulting residue was purified by column chromatography on silica gel eluting with chloroform-methanol (40:1~20:1). The title compound was obtained as a free base. The free base was then converted to a maleate adding maleic acid.
Yield: 93 mg (49%).
$^1$H-NMR (400 MHz, measured as a maleate, DMSO-$d_6$) δ: 1.87-2.01 (m, 4H), 2.48-2.56 (m, 1H), 2.78-2.86 (m, 2H), 3.26-3.31 (m, 2H), 3.78 (s, 3H), 3.79 (s, 3H), 3.87 (s, 6H), 3.90 (s, 6H), 4.15 (s, 2H), 4.39 (d, 2H, J=5.9 Hz), 6.16 (s, 2H), 7.16 (d, 1H, J=5.9 Hz), 7.35 (s, 2H), 7.39 (d, 1H, J=5.9 Hz), 7.39 (s, 2H), 7.73 (s, 1H), 7.95 (s, 1H), 8.15 (d, 1H, J=5.9 Hz), 8.54 (d, 1H, J=4.9 Hz), 8.68 (d, 1H, J=4.9 Hz).

Referential Example 8

Synthesis of 1-(benzyloxycarbonyl)-4-[[2-(3,4,5-trimethoxyphenyl)pyridin-4-yl] methyloxy]piperidine:

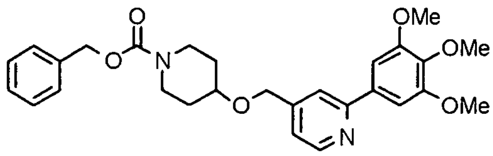

To a solution of 1-(benzyloxycarbonyl)-4-hydroxypiperidine (1.0 g) in DMF (20 mL) was added sodium hydride (55% dispersion in mineral oil, 222 mg). The mixture was stirred at room temperature for 1 hour and then, 4-chlolromethyl-2-(3,4,5-trimethoxyphenyl)pyridine (1.37 g) and potassium iodide (755 mg) were added. The mixture was stirred at 70°C overnight, added with water and extracted with chloroform. The organic layer was washed with saturated brine, dried over anhydrous sodium sulfate and concentrated under reduced pressure. The residue was purified by column chromatography on silica gel using chloroform-methanol (99:1) as an eluent to give the title compound.
Yield: 213 mg (10%).
$^1$H NMR (400MHz, $CDCl_3$) δ: 1.63 (br, 2H), 1.89 (br, 2H), 3.20-3.35 (m, 2H), 3.57-3.68 (m, 1H), 3.84-3.92 (m, 5H), 3.94 (s, 6H), 4.62 (s, 2H), 5.11 (s, 2H), 7.21-7.35 (m, 8H), 7.61 (s, 1H), 8.61 (d, 1H, J=5.0Hz).

Referential Example 9

Synthesis of 4-[[2-(3,4,5-trimethoxyphenyl)pyridin-4-yl]methyloxy]piperidine:

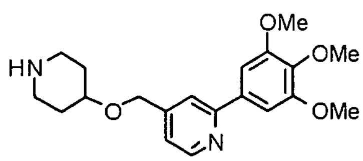

To a solution of 1-(benzyloxycarbonyl)-4-[[2-(3,4,5-trimethoxyphenyl) pyridin-4-yl]methyloxy]piperidine (213 mg) in methanol (10 mL) was added 40% aqueous potassium hydroxide (10 mL). The mixture was stirred at 100°C for 3 hours. After concentrating under reduced pressure, water was added to the residue and the residue was extracted with chloroform. The organic layer was washed with saturated brine, dried over anhydrous sodium sulfate and concentrated under reduced pressure. The residue was purified by column chromatography on silica gel eluting with chloroform-ammonia saturated methanol (20:1) to give the title compound.
Yield: 93 mg (60%).
$^1$H NMR (400MHz, $CDCl_3$) δ: 1.55-1.68 (m, 2H), 2.01 (br, 2H), 2.67-2.72 (m, 2H), 3.13-3.18 (m, 2H), 3.50-3.60 (m, 1H), 3.91 (s, 3H), 3.97 (s, 6H), 4.64 (s, 2H), 7.22 (d, 1H, J=4.3 Hz), 7.24 (s, 2H), 7.64 (s, 1H), 8.63 (d, 1H, J=5.1 Hz).

Preparation Example 2

Synthesis of 1-[2-[(3,4,5-trimethoxyphenyl)pyridin-4-yl]methyl]-4-[[2-(3,4,5-Trimethoxyphenyl)pyridin-4-yl]methyloxy] piperidine trihydrochloride:

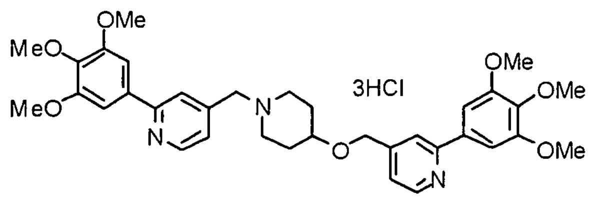

4-[[2-(3,4,5-trimethoxyphenyl)pyridin-4-yl]methyloxy]piperidine (70 mg), 4-chloromethyl-2-(3,4,5-trimethoxyphenyl)pyridine (22 mg), potassium carbonate (56 mg) and potassium iodide (40 mg) were suspended in acetonitrile (5 mL). The mixture was stirred at room temperature for 5 hr and concentrated under reduced pressure. Chloroform and water were added to the residue and the organic layer was separated. Aqueous layer was then extracted with chloroform and the organic layers were combined, dried over anhydrous magnesium sulfate and concentrated under reduced pressure. The residue was purified by column chromatography on silica gel using chloroform-methanol (40:1) as an eluent. The resulting free base was converted to a trihydrochloride according to a conventional method.
Yield: 42 mg (39%).
$^1$H NMR (400MHz, measured as a free base, $CDCl_3$) δ: 1.53-2.42 (m, 6H), 2.80 (br, 2H), 3.57 (br, 3H), 3.88 (s, 6H), 3.94 (s, 6H), 3.95 (s, 6H), 4.60 (s, 2H), 7.18-7.24(m, 6H), 7.61 (s, 2H), 8.58-8.61 (m, 2H).

Referential Example 10

Synthesis of (3S)-1-(*tert*-butoxycarbonyl)-3-[(2-nitrobenzene)sulfonylamino] pyrrolidine:

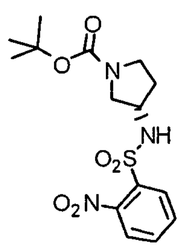

To an ice-cooled solution of(3S)-3-amino-1-(*tert*-butoxycarbonyl) pyrrolidine (404 mg) and triethylamine (220 mg) in THF (5 mL) was added 2-nitrobenzenesulfonyl chloride (481 mg). The mixture was stirred at room temperature for 30 minutes and concentration under reduced pressure. Ethyl acetate was added to the residue. The solution was washed with water and saturated brine, dried over anhydrous sodium sulfate and concentrated under reduced pressure. The residue was purified by column chromatography on silica gel using chroloform-methanol (20:1) as an eluent to give the title compound as pale yellow amorphous substance.
Yield: 597 mg (74%).
$^1$H-NMR (400 MHz, $CDCl_3$) δ: 1.44 (s, 9H), 1.80-2.12 (m, 2H), 3.14-3.44 (m, 4H), 4.02 (br, 1H), 5.48 (d, 1H, J=7.2 Hz), 7.77 (t, 2H, J=4.4 Hz), 7.87-7.90 (m, 1H), 8.17-8.19 (m, 1H).

Referential Example 11

Synthesis of (3S)-1-(*tert*-butoxycarbonyl)-3-[N-methyl-N-(2-nitrobenzenesulfonyl)amino]pyrrolidine :

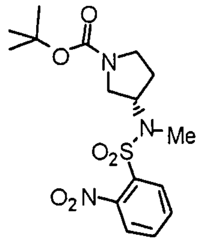

To a suspension of (3S)-1-(*tert*-butoxycarbonyl)-3-[(2-nitrobenzene sulfonyl)amino]pyrrolidine (371 mg) and potassium carbonate (138 mg) in acetonitrile (10 mL) was added methyl iodide (141 mg). The mixture was stirred at 60°C for 2 hours and concentrated under reduced pressure. Ethyl acetate was added to the mixture. The solution was washed with saturated aqueous sodium hydrogen carbonate and saturated brine, dried over anhydrous sodium sulfate and cencentrated under reduced pressure. The residue was applied to column chromatography on silica gel using hexane-ethyl acetate (2:1) as an eluent for purification to give the title compound as yellow oil.
Yield: 365 mg (95%).
$^1$H-NMR (400 MHz, $CDCl_3$) δ: 1.44 (s, 9H), 1.95 (br, 1H), 2.09 (br, 1H), 2.87 (s, 3H), 3.20-3.31 (m, 2H), 3.53 (br, 2H), 4.58 (br, 1H), 7.65 (br, 1H), 7.71 (br, 2H), 8.04 (br, 1H).

Referential Example 12

Synthesis of (3S)-3-[N-methyl-N-(2-nitrobenzenesulfonyl)amino]pyrrolidine :

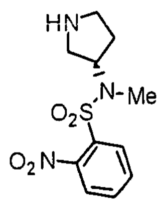

To a solution of (3S)-1-(*tert*-butoxycarbonyl)-3-[N-methyl-N-(2-nitrobenzenesulfonyl)amino]pyrrolidine (365 mg) in dichloromethane (25 mL) was added trifluoroacetic acid (1 mL) at 0°C. The mixture was stirred at room temperature for 3 hours, concentrated under reduced pressure and incorporated with chloroform. The solution was washed with saturated aqueous sodium hydrogen carbonate and saturated brine, dried over anhydrous sodium sulfate and concentrated under reduced pressure to give the title compound as yellow oil.
Yield: 135 mg (50%).
$^1$H-NMR (400 MHz, $CDCl_3$) δ: 1.69-1.74 (m, 1H), 1.87 (br, 1H), 1.95-2.02 (m, 1H), 2.80 (dd, 1H, J=11.7 Hz, 5.7 Hz), 2.84-2.91 (m, 4H), 2.96-3.05 (m, 1H), 3.10 (dd, 1H, J=11.7 Hz, 8.2 Hz), 4.48-4.56 (m, 1H), 7.61-7.63 (m, 1H), 7.66-7.73 (m, 2H), 8.01-8.04 (m, 1H).

Referential Example 13

Synthesis of (3S)-3-[N-methyl-N-(2-nitrobenzenesulfonyl)amino]-1-[[2-(3,4,5-trimethoxyphenyl)pyridin-4-yl]methyl] pyrrolidine :

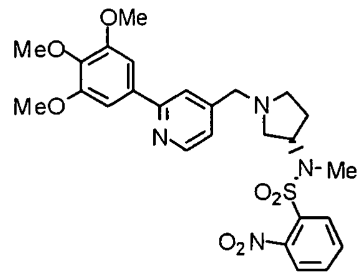

(3S)-3-[N-methyl-N-(2-nitrobenzenesulfonyl)amino]pyrrolidine (135 mg) and 4-chloromethyl-2-(3,4,5-trimethoxyphenyl)pyridine (139 mg) were treated in the same manner as described in Preparation Example 2 to give the title compound as yellow amorphous substance.
Yield: 247 mg (96%).
$^1$H-NMR (400 MHz, CDC13) δ: 1.80-1.87 (m, 1H), 2.15-2.30 (m, 2H), 2.52 (dd, 1H, J=10.5 Hz, 8.2 Hz), 2.71 (dd, 1H, J=10.5 Hz, 8.2 Hz), 2.90 (dt, 1H, J=8.8 Hz, 2.9 Hz), 2.96 (s, 3H), 3.53 (d, 1H, J=13.9 Hz), 3.58 (d, 1H, J=13.9 Hz), 3.90 (s, 3H), 3.96 (s, 6H), 4.61-4.68 (m, 1H), 7.16 (dd, 1H, J=4.9 Hz, 1.2 Hz), 7.21 (s, 2H), 7.58-7.60 (m, 2H), 7.64-7.69 (m, 2H), 7.99-8.02 (m, 1H), 8.58 (d, 1H, J=4.9 Hz,).

Referential Example 14

Synthesis of (3S)-3-methylamino-1-[[2-(3,4,5-trimethoxyphenyl)-pyridin-4-yl]methyl]pyrrolidine:

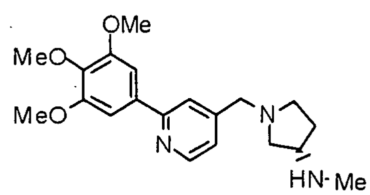

To a solution of (3S)-3-[N-methyl-N-(2-nitrobenzenesulfonyl)amino]-1-[[2-(3,4,5-trimethoxyphenyl)pyridin-4-yl]methyl]pyrrolidine (242 mg) in acetonitrile (5 mL) was added potassium carbonate (94 mg) and thiophenol (75 mg). The mixture was stirred at 80°C for 1 hour. After cooling, ethyl acetate was added to the mixture, and the solution was washed with saturated aqueous sodium hydrogen carbonate, water and saturated brine, dried over anhydrous sodium sulfate and concentrated under reduced pressure. The residue was purified by preparative TLC using chloroform-methanol (20:1) as an eluent to give the title compound as yellow oil.
Yield: 104 mg (64%).
$^1$H-NMR (400 MHz, $CDCl_3$) δ: 1.32 (br, 1H), 1.56-1.64 (m, 1H), 2.11-2.17 (m, 1H), 2.38 (s, 3H), 2.44 (dd, 1H, J=7.4 Hz, 4.5 Hz), 2.50-2.55 (m, 1H), 2.66-2.75 (m, 2H), 3.20-3.26 (m, 1H), 3.66 (s, 2H), 3.90 (s, 3H), 3.97 (s, 6H), 7.21 (d, 1H, J=4.1 Hz), 7.25 (s, 2H), 7.64 (s, 1H), 8.59 (d, 1H, J=4.9 Hz).

Preparation Example 3

Synthesis of (3S)-3-[N-methyl-N-[[2-(3,4,5-trimethoxyphenyl)pyridin-4-yl] methyl]amino]-1-[[2-(3,4,5-trimethoxyphenyl)pyridin-4-yl]methyl]pirrolidine tertrahydrochloride.

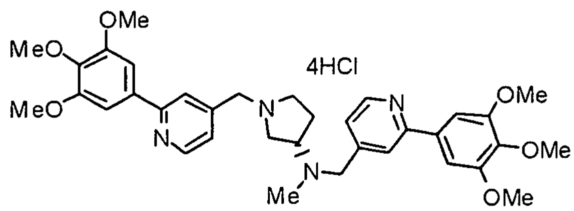

(3S)-3-Methylamino-1-[[2-(3,4,5-trimethoxyphenyl)pyridin-4-yl]methyl] pyrrolidine (104 mg) and 4-chloromethyl-2-(3,4,5-trimethoxyphenyl)pyridine (85 mg) were reacted in the same manner as described in Preparation Example 2. The resulting product was converted to a tetrahydrochloride to give the title compound as yellow powder.
Yield: 151 mg (68%).
$^1$H-NMR (400 MHz, measured as a free base, $CDCl_3$) δ: 1.89-1.92 (m, 1H), 2.04-2.08 (m, 1H), 2.18 (s, 3H), 2.60-2.76 (m, 4H), 3.25-3.29 (m, 1H), 3.53 (d, 1H, J=14.3 Hz), 3.62 (d, 1H, J=14.3 Hz), 3.64 (d, 1H, J=13.9 Hz), 3.73 (d, 1H, J=13.9 Hz), 3.89 (s, 6H), 3.95 (s, 6H), 3.96 (s, 6H), 7.20-7.21 (m, 2H), 7.23 (s, 2H), 7.24 (s, 2H), 7.61 (s, 1H), 7.65 (s, 1H), 8.59 (d, 1H, J=5.7 Hz), 8.60 (d, 1H, J=5.3 Hz).

Referential Example 15

Synthesis of 1-[[2-(3,4,5-trimethoxyphenyl)pyridin-4-yl]methyl]piperidine-4-carboxamide:

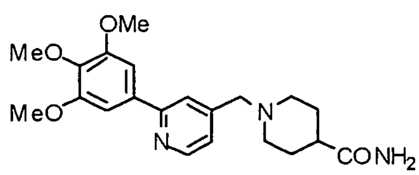

Piperidine-4-carboxamide (385 mg) and 4-chloromethyl-2-(3,4,5-trimethoxyphenyl)pyridine (881 mg) were reacted by the same method as described in Preparation Example 2 to give the title compound as white needles.
Yield: 1.01 g (87%).
$^1$H-NMR (400 MHz, $CDCl_3$) δ: 1.70-1.88 (m, 4H), 2.01-2.23 (m, 3H), 2.95 (d, 2H, J=11.0 Hz), 3.56 (s, 2H), 3.90 (s, 3H), 3.98 (s, 6H), 5.46 (d, 2H, J=16.3 Hz), 7.21 (d, 1H, J=5.0 Hz), 7.24 (s, 2H), 7.64 (s, 1H), 8.59 (d, 1H, J=5.0 Hz).

Referential Example 16

Synthesis of 4-amino-1-[[2-(3,4,5-trimethoxyphenyl)pyridin-4-yl]methyl]piperidine trihydrochloride:

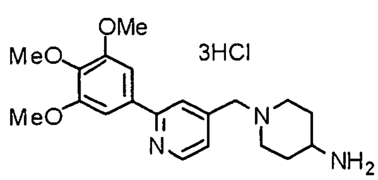

To a solution of 1-[[2-(3,4,5-trimethoxypheyl)pyridin-4-yl]methyl]piperidine-4-carboxamide (192 mg) in a mixed solvent of water (50 mL) and acetonitrile (50 mL) was added [bis(trifluoroacetoxy)iodo]benzene (323 mg). The mixture was stirred at room temperature overnight and concentrated under reduced prepare. Saturated aqueous sodium hydrogen carbonate was added to the residue to alkalinize the residue and the residue was extracted with chloroform. The chloroform layer was washed with saturated brine, dried over anhydrous magnesium sulfate and concentrated under reduced pressure. Yellow oil thus obtained was then converted to a hydrochloride which gave yellow powder. The title compound was used for next step without further purification.
Yield: 201 mg (theoretical amount).

Referential Example 17

Synthesis of 2-(3,4,5-trimethoxyphenyl)isonicotinic acid:

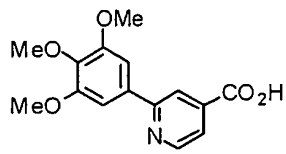

To a solution of ethyl 2-(3,4,5-trimethoxyphenyl)isonicotinate (3.17 g) in ethanol (40 mL) was added 10% potassium hydroxide (2.42 g). The mixture was stirred at room temperature for 5 hours and concentrated under reduced pressure.
Water was added to the residue and the pH thereof was adjusted to 7. The resulting white precipitates were filtered. The resulting title compound was used for next step without further purification.
Yield: 2.60 g (90%).

Preparation Example 4

Synthiesis of 1-[[2-(3,4,5-trimethoxyphenyl)pyridin-4-yl]methyl] -4-[2-(3,4,5-trimethoxyphenyl)pyridin-4-yl]-carbonylamino]piperidine maleate:

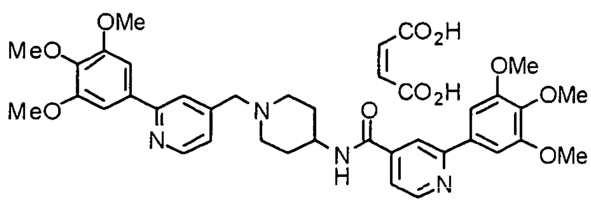

2-(3,4,5-Trimethoxyphenyl)pyridin-4-carboxylic acid (72 mg) and 4-amino-1- [[2-(3,4,5-trimethoxyphenyl)pyridin-4-yl]methyl]piperidine (117 mg) were reacted with each other in the same manner as described in Preparation Example 1.
The resulting product was converted to a maleate to give the title compound.
Yield: 173 mg (93%).
$^1$H-NMR (400 MHz, measured as a maleate, DMSO-$d_6$) δ: 1.82-1.94 (m, 2H), 2.03-2.08 (m, 2H), 2.77-2.83 (m, 2H), 3.20-3.27 (m, 2H), 3.79 (s, 6H), 3.90 (s, 12H), 4.00 (br, 1H), 4.06 (s, 2H), 6.15 (s, 2H), 7.36-7.38 (m, 1H), 7.39 (s, 2H), 7.41 (s, 2H), 7.61-7.63 (m, 1H), 7.90 (s, 1H), 8.12 (s, 1H), 8.27-8.32 (m, 1H), 8.67 (d, 1H, J=4.9 Hz), 8.74 (d, 1H, J=5.1 Hz).

Referential Example 18

Synthesis of 4-[(2-nitrobenzenesulfonyl)amino]-1-[[2-(3,4,5-trimethoxyphenyl) pyridin-4-yl]methyl]piperidine:

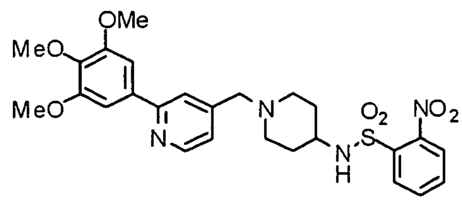

4-Amino-1-[[2-(3,4,5-trimethoxyphenyl)pyridin-4-yl]methyl]piperidine (467 mg) and 2-nitrobenzenesulfonyl chloride (244 mg) were reacted with each other in the same manner as described in Referential Example 10 to give the title compound.
Yield: 494 mg (91%).

Referential Example 19

Synthesis of 4-[N-(2-nitrobenzenesulfonyl)-N-[[2-(3,4,5-trimethoxyphenyl)-pyridin-4-yl]methyl]amino]-1-[[2-(3,4,5-trimethoxyphneyl)pyridin-4-yl]methyl]piperidine:

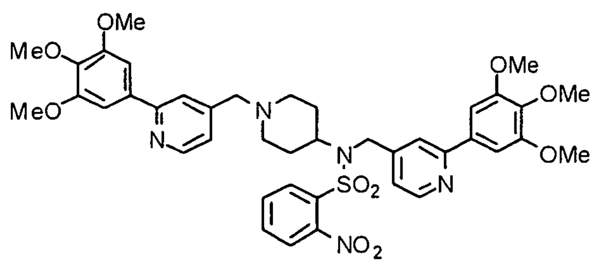

4-[(2-Nitrobenzene)sulfonylamino]-1-[[2-(3,4,5-trimethoxyphenyl)pyridin-4-yl]methyl]piperidine (494 mg) and 4-chloromethyl-2-(3,4,5-trimethoxyphenyl)pyridine (267 mg) were reacted with each other in the same manner as described in Preparation Example 2 to give the title compound.
Yield: 443 mg (61%).

Preparation Example 5

Synthesis of 1-[[2-(3,4,5-trimethoxyphenyl)pyridin-4-yl]methyl]-4-[[2-(3,4,5-trimethoxyphenyl)pyridin-4-yl] methylamino]piperidine difumalate:

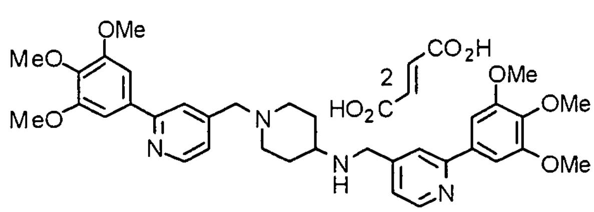

4-[N-(2-Nitrobenzenesulfonyl)-N-[[2-(3,4,5-trimethoxyphenyl)pyridin-4-yl] methyl]amino]-1-[[2-(3,4,5-trimethoxyphneyl)pyridin-4-yl]methyl]piperidine (443 mg) was treated in the same manner as described in Referential Example

14. The title compound was obtained as a difumalate.
Yiled: 103 mg (24%).
$^1$H-NMR (400 MHz, measured as a free base, DMSO-$d_6$) δ: 1.44-1.53 (m, 2H), 1.87-1.91 (m, 2H), 2.15 (t, 2H, J=1.1 Hz), 2.57-2.64 (m, 1H), 2.82-2.85 (m, 2H), 3.59 (s, 2H), 3.78 (s, 6H), 3.89 (s, 12H), 3.90 (s, 2H), 6.63 (s, 4H), 7.24 (d, 1H, J=4.9 Hz), 7.29 (d, 1H, J=4.9 Hz), 7.35 (s, 2H), 7.37 (s, 2H), 7.76 (s, 1H), 7.85 (s, 1H), 8.53-8.56 (m, 2H).

Referential Example 20

Synthesis of 4-(ethoxycarbonylamino)-1-[[2-(3,4,5-trimethoxyphenyl)pyridin-4-yl]methyl]piperidine:

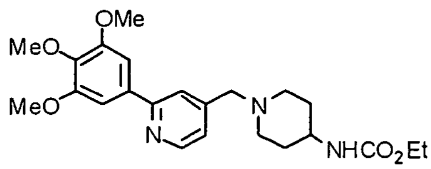

To a solution of 1-[[2-(3,4,5-trimethoxyphenyl)pyridin-4-yl]methyl] piperidine-4-carboxamide (528 mg) in a mixed solvent of ethanol (10 mL) and acetonitrile (10 mL) was added [bis(trifluoroacetoxy)iodo]benzene (884 mg). The mixture was stirred at room temperature overnight. After concentrating under reduce pressure, the mixture was incorporated with saturated aqueous sodium hydrogen carbonate and extracted with chloroform. The organic layer was washed with saturated brine, dried over anhydrous magnesium sulfate and concentrated under reduced pressure. The residue was purified by column chromatography on silica gel using chloroform-methanol (20:1) as an eluent to give the title compound.
Yield: 566 mg (96%).
$^1$H-NMR (400 MHz, $CDCl_3$) δ: 1.21 (t, 3H, J=7.0 Hz), 1.40-1.51 (m, 2H), 1.92 (d, 2H, J=10.9 Hz), 2.15 (t, 2H, J=10.9 Hz), 2.78 (d, 2H, J=11.6 Hz), 3.52 (br, 3H), 3.87 (s, 3H), 3.94 (s, 6H), 4.07 (q, 2H, J=7.0 Hz), 4.56 (br, 1H), 7.17 (d, 1H, J=4.9 Hz), 7.21 (s, 2H), 7.59 (s, 1H), 8.56 (d, 1H, J=5.1 Hz).

Referential Example 21

Synthesis of 4-(methylamino)-1-[[2-(3,4,5-trimethoxyphenyl)pyridin-4-yl]methyl] piperidine:

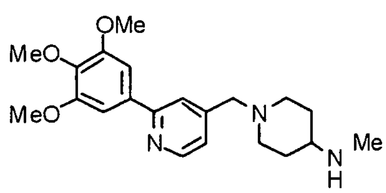

To a suspension of lithium aluminum hydride (100 mg) in dry THF (50 mL) was added gradually a solution of 4-(ethoxycarbonylamino)-1-[[2-(3,4,5-trimethoxyphenyl) pyridin-4-yl]methyl]piperidine (566 mg) in dry THF (50 mL) under an argon atmosphere. The mixture was then refluxed overnight, then cooled down. Saturated aqueous ammonium chloride was added to the mixture and the resulting mixture was extracted with ethyl acetate after bubbling ceased. The organic layer was washed with brine, dried over anhydrous sodium sulfate and concentrated under reduced pressure. The residue was purified by silica gel column chromatography using chloroform-ammonia saturated methanol (9:1) to give the title compound as yellow oil.
Yiled: 379 mg (78%).
$^1$H-NMR (400 MHz, $CDCl_3$) δ: 1.36-1.46 (m, 2H), 1.89 (d, 2H, J=12.5 Hz), 2.10 (dt, 2H, J=11.5 Hz, 1.1 Hz), 2.35-2.43 (m, 1H), 2.43 (s, 3H), 2.86 (d, 2H, J=11.6 Hz), 3.56 (s, 2H), 3.90 (s, 3H), 3.97 (s, 6H), 7.21 (d, 1H, J=5.1 Hz), 7.24 (s, 2H), 7.64 (s, 1H), 8.59(d, 1H, J=4.9 Hz).

Referential Example 22

Synthesis of 1-[[2-(3,4,5-trimethoxyphenyl)pyridin-4-yl]methyl]-4-piperidone ethylene ketal:

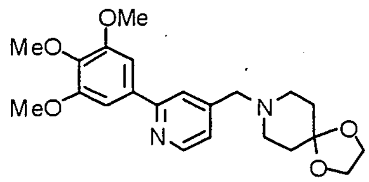

4-Piperidone ethylene ketal (12.0 g) and 4-chloromethyl-2-(3,4,5-trimethoxyphenyl)pyridine (12.3 g) were reacted with each other in the same manner as described in Example 2 to give the title compound.
Yield: 19.0 g (theoretical amount).
$^1$H-NMR (400MHz, $CDCl_3$) δ: 1.68 (t, 4H, J=5.6 Hz), 2.48 (br, 4H), 3.50 (s, 2H), 3.82 (s, 3H), 3.86 (s, 4H), 3.88 (s, 6H), 7.13 (d, 1H, J=4.9 Hz), 7.17 (s, 2H), 7.57 (s, 1H), 8.51 (d, 1H, J=4.9 Hz).

Referential Example 23

Synthesis of 1-[[2-(3,4,5-trimethoxyphenyl)pyridin-4-yl]methyl]-4-piperidone:

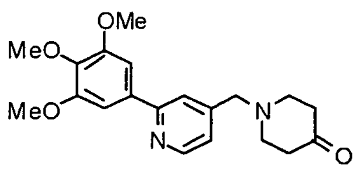

To a solution of 1-[[2-(3,4,5-trimethoxyphenyl)pyridin-4-yl]methyl]- 4-piperidone ethylene ketal (19.0 g) in THF (200 mL) was added 1 M hydrochloric acid (200 mL). The mixture was stirred at 90°C overnight, then neutralized with 2 M sodium hydroxide and extracted with ethyl acetate. The organic layer was washed with saturated brine, dried over anhydrous sodium sulfate and concentrated under reduced pressure. The residue was purified by column chromatography on silica gel using chloroform-methanol (40:1) as an eluent to give the title compound.
Yield: 15.0 g (75%).
$^1$H-NMR (400MHz, $CDCl_3$) δ : 2.48 (t, 4H, J=6.1 Hz), 2.79 (t, 4H, J=6.0 Hz), 3.69 (s, 2H), 3.89 (s, 3H), 3.96 (s, 6H), 7.24 (s, 2H), 7.26 (d, 1H, J=4.9 Hz), 7.66 (s, 1H), 8.62 (d, 1H, J=4.9 Hz).

Referential Example 24

Synthesis of 1-[[2-(3,4,5-trimethoxyphenyl)pyridin-4-yl]methyl]-4-piperidone:

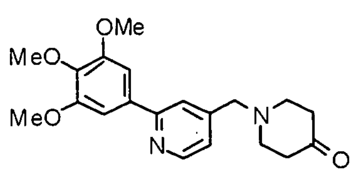

4-Piperidone hydrochloride monohydrate (3.07 g) and 4-chloromethyl-2-(3,4,5-trimethoxyphenyl)pyridine (2.94 g) were reacted with each other in by the same manner as described in Example 2 to give the title compound . Yield: 3.55 g (99%).

Referential Example 25

Synthesis of 4-(methylamino)-1-[[2-(3,4,5-trimethoxyphenyl)pyridin-4-yl]methyl]piperidine:

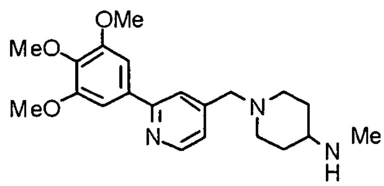

To a solution of 1-[[2-(3,4,5-trimethoxyphenyl)pyridin-4-yl]methyl]-4-piperidone (1.00 g) in 1,2-dichloroethane (60 mL) were added 30% solution of methylamine in ethanol (750 mg) and sodium triacetoxyborohydride (1.66 g). The mixture was stirred at room temperature for 3 hours, incorporated with water and concentrated under reduced pressure. After adding water, the residue was extracted with chloroform. The organic layer was washed with saturated brine, dried over anhydrous sodium sulfate and concentrated under reduced pressure. The residue was purified by silica gel column chromatography using chloroform-methanol (40:1) as an eluent to give the title compound.
Yield: 640 mg (62%).

Referential Example 26

Synthesis of ethyl 3-(3,4,5-trimethoxyphenyl)benzoate:

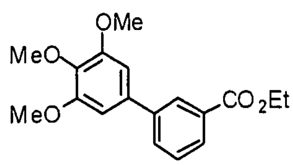

3,4,5-Trimethoyphenylboronic acid (3.7 g) and ethyl 3-bromobenzoate (4.02 g) were reacted with each other in the same manner as described in Referential Example 1 to give the title compound.
Yield: 5.09 g (92%).
$^1$H-NMR (400MHz, $CDCl_3$) δ: 1.42 (t, 3H, J=7.1 Hz), 3.90 (s, 3H), 3.94 (s, 6H), 4.41 (q, 2H, J=7.1 Hz), 6.79 (s, 2H), 7.50 (t, 1H, J=7.8 Hz), 7.73 (dt, 1H, J=7.1 Hz, 1.5 Hz), 8.01 (dt, 1H, J=7.8 Hz, 1.4 Hz), 8.23 (t, 1H, J=1.8 Hz).

Referential Example 27

Synthesis of 3-(3,4,5-trimethoxyphenyl)benzoic acid:

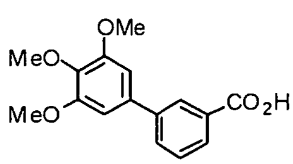

Ethyl 3-(3,4,5-trimethoxyphenyl)benzoate (1.19 g) was treated in the same manner as described in Referential Example 17 to give the title compound.
Yield: 986 mg (91%).

Preparation Example 6

Synthesis of 4- [N-methyl-N-[3-(3,4,5-trimethoxyphenyl)benzoyl]amino]-1-[[2-(3,4,5-trimethoxyphenyl)pyridin-4-yl]methyl]piperidine dihydrochloride:

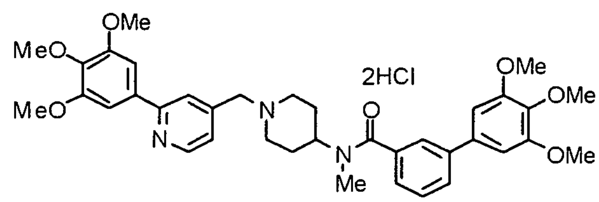

3-(3,4,5-Trimethoxyphenyl)benzoic acid (1.03 g) and 4-(methylamino)-1-[[2-(3,4,5-trimethoxyphenyl)pyridin-4-yl]methyl]piperidine (1.32 g) were reacted with each other in the same method as described in Preparation Example 1 to give the title compound as a dihydrochloride.
Yield: 1.44 g (57%).
$^1$H-NMR (400 MHz, measured as a dihydrochloride, DMSO-$d_6$) δ: 1.89 (d, 2H, J=11.7 Hz), 2.54-2.62 (m, 2H), 2.89 (s, 3H), 3.09 (t, 2H, J=12.7 Hz), 3.43 (d, 2H, J=14.4 Hz), 3.76 (s, 3H), 3.78 (s, 3H), 3.88 (s, 6H), 3.91 (s, 6H), 4.34 (br, 3H), 6.91 (s, 2H), 7.33 (d, 1H, J=7.6 Hz), 7.47-7.51 (m, 2H), 7.54 (s, 2H), 7.60 (s, 1H), 7.71 (d, 1H, J=7.8 Hz), 8.55 (s, 1H), 8.68 (d, 1H, J=5.1 Hz).

Preparation Example 7

Synthesis of 4-[N-methyl-N-[[3-(3,4,5-trimethoxyphenyl)pyridin-5-yl]methyl]amino]-1-[[2-(3,4,5-trimethoxyphenyl) pyridin-4-yl]methyl]piperidine difumarate:

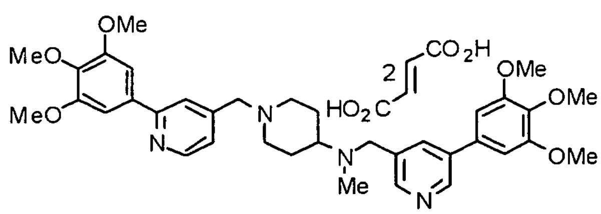

4-Methylamino-1-[[2-(3,4,5-trimethoxyphenyl)pyridin-4-yl]methyl]piperidine (135 mg) and 5-chloromethyl-3-(3,4,5-trimethoxypyenyl)pyridine (107 mg) were reacted with each other similarly to the method as described in Preparation Example 2 to obtain the title compound as a difumarate.
Yield: 180 mg (58%).
$^1$H-NMR (400 MHz, measured as a free base, $CDCl_3$) δ: 1.69-1.73 (m, 2H), 1.82-1.85 (m, 2H), 2.03-2.08 (m, 2H), 2.25 (s, 3H), 2.48-2.51 (m, 1H), 2.97-2.99 (m, 2H), 3.56 (s, 2H), 3.67 (s, 2H), 3.90 (s, 3H), 3.91 (s, 3H), 3.94 (s, 6H), 3.98 (s, 6H), 6.76 (s, 2H), 7.22 (d, 1H, J=5.1 Hz), 7.24 (s, 2H), 7.62 (s, 1H), 7.80 (s, 1H), 8.50 (d, 1H, J=2.0 Hz), 8.60 (d, 1H, J=4.3 Hz), 8.69 (d, 1H, J=5.1 Hz).

Referential Example 28

Synthesis of 1-bromo-4-chloro-3,5-dimethoxybenzene:

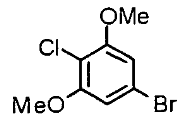

A solution of sodium nitrite (97 mg) in water (2.0 mL) was added dropwise to an ice-cold suspension of 4-bromo-2,6-dimethoxyaniline (232 mg) in 6.0 M hydrochloric acid (2.5 mL) after adding thereto crushed ice. After stirring the mixture in ice bath for 30 minutes, a solution of cupric chloride (495 mg) in concentrated hydrochloric acid (2.0 mL) was added thereto. The reaction mixture was stirred at room temperature for 30 minutes, then at 100°C for 2 hours, and extracted with ethyl acetate. The organic layer was washed with a saturated aqueous sodium hydrogencarbonate and water, dried over anhydrous sodium sulfate, and concentrated under reduced pressure. The residue was purified by column chromatography on silica gel using hexane-ethyl acetate (10:1) as an eluent to give the title compound as white powder.
Yield: 230 mg (92%).

Referential Example 29

Synthesis of 4-chloro-3,5-dimethoxyphenylboronic acid:

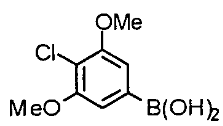

Under an argon atomsphere, to dry THF (2 mL) cooled in a dry ice-methanol bath was gradually added a 1.57 M solution of n-butyllithium in hexane (0.8 mL), followed by the dropwise addition of a solution of 1-bromo-4-chloro-3,5-dimethoxybenzene (160 mg) in dry THF (2 mL). After the mixture was stirred for 20 minutes in the dry ice-methanol bath, triisopropyl borate (0.18 mL) was added and the mixture was additionally stirred for 20 minutes. The reaction mixture was then stirred at room temperature for 1 hour and pH of the mixture was adjusted at 3 using 4 M hydrochloric acid. The mixture was stirred at 0°C for 1 hour and extracted with ethyl acetate. The organic layer was washed with saturated brine, dried over anhydrous sodium sulfate and concentrated under reduced pressure. The residue was recrystallized from ethyl acetate-hexane to give the title compound as white powder.
Yield: 90 mg (66%).

Referential Example 30

Synthesis of ethyl 2-(4-chloro-3,5-dimethoxyphenyl)isonicotinate:

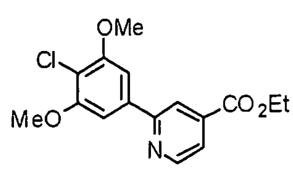

4-Chloro-3,5-dimethoxyphenylboronic acid (7.45 g) and ethyl 2-chloroisonicotinate (6.39 g) were treated in

the same manner as described in Referential Example 1 to give the title compound.
Yield: 8.55 g (77%).
$^1$H-NMR (400 MHz, $CDCl_3$) δ : 1.45 (t, 3H, J=7.3 Hz), 4.03 (s, 6H), 4.45 (q, 2H, J=7.3 Hz), 7.32 (s, 2H), 7.80 (d, 1H, J=5.1 Hz), 8.27 (s, 1H), 8.83 (d, 1H, J=5.0 Hz).

Referential Example 31

Synthesis of 2-(4-chloro-3,5-dimethoxyphenyl)isonicotinic acid:

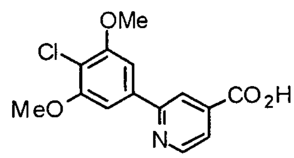

To a solution of ethyl 2-(4-chloro-3,5-dimethoxyphenyl)isonicotinate (8.55 g) in ethanol (80 mL) was added 2 M sodium hydroxide (100 mL). The mixture was stirred under reflux for 30 min and ethanol was distilled away under reduced pressure. The mixture was neutralized by addition of 1 M hydrochloric acid. The resulting precipitates were dissolved in a mixed solvent of ethyl acetate-THF (3:1), dried over anhydrous sodium sulfate, and concentrated under reduced pressure to give the title compound.
Yield: 7.20 g (92%).
$^1$H-NMR (400 MHz, $CDCl_3$) δ: 4.02 (s, 6H), 7.34 (s, 2H), 7.83 (d, 1H, J=4.9 Hz), 7.84 (s, 1H), 8.82 (d, 1H, J=4.9 Hz).

Referential Example 32

Synthesis of 2-(4-chloro-3,5-dimethoxyphenyl)-4-hydroxymethylpyridine:

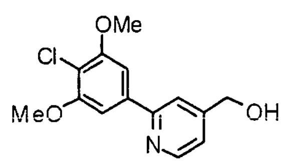

To a solution of 2-(4-chloro-3,5-dimethoxyphenyl)isonicotinic acid (7.20 g) and triethylamine (5.6 mL) in THF (70 mL) was added ethyl chloroformate (2.8 mL) at 0°C. The mixture was stirred at room temperature for 1 hour and insoluble matter was filtered off. To the filtrate was then added a solution of sodium borohydride (1.25 g) in water (4 mL). The mixture was stirred at room temperature for another hour and concentrated under reduced pressure. After adding water, the residue was extracted with chloroform. The organic layer was washed with saturated brine, dried over anhydrous sodium sulfate and concentrated under reduced pressure. The residue was purified by silica gel column chromatography using chloroform-methanol (20:1 ~ 15:1) to give the title compound.
Yeld: 4.10 g (60%).
$^1$H-NMR (400 MHz, $CDCl_3$+DMSO-$d_6$) δ: 4.01 (s, 6H), 4.76 (s, 2H), 7.20-7.35 (m, 3H), 7.78 (s, 1H), 8.62 (s,1H).

Referential Example 33

Synthesis of 2-(4-chloro-3,5-dimethoxyphenyl)-4-chloromethylpyridine:

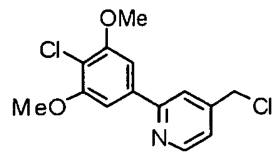

2-(4-Chloro-3,5-dimethoxyphenyl)-4-hydroxymethylpyridine (4.10 g) was dissolved in chloroform (20 mL), incorporated with thionyl chloride (5.5 mL) and stirred at 70°C for 1 hour. After concentrating the mixture under reduced pressure, the resulting residue was neutralized with a saturated aqueous solution of sodium hydrogen carbonate and extracted with ethyl acetate. The organic layer was washed with water and saturated brine, dried over anhydrous magnesium sulfate and concentrated under reduced pressure to give the title compound.
Yield: 4.20 g (96%).
$^1$H-NMR (400 MHz, $CDCl_3$) δ: 4.02 (s, 6H), 4.63 (s, 2H), 7.26 (s, 2H), 7.29 (d, 1H, J=4.9 Hz), 7.72 (s, 1H), 8.69 (d, 1H, J=4.9 Hz).

Referential Example 34

Synthesis of 1-[[2-(4-chloro-3,5-dimethoxyphenyl)pyridin-4-yl]methyl]piperidine-4-carboxamide:

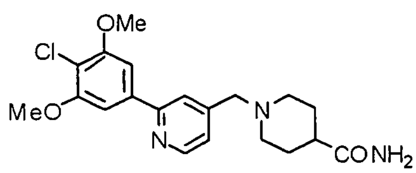

Piperidine-4-carboxamide (301 mg) and 2-(4-chloro-3,5-dimethoxyphenyl)-4-chloromethyl pyridine (600 mg) were reacted with each other in the same manner as described in Preparation Example 2 to give the title compound.
Yield: 743 mg (95%).
$^1$H-NMR (400 MHz, $CDCl_3$) δ: 1.75-1.90 (m, 4H), 2.07-2.25 (m, 3H), 2.94 (d, 2H, J=11.6 Hz), 3.57 (s, 2H), 4.02(s, 6H), 7.24-7.31 (m, 3H), 7.67 (s, 1H), 8.61 (d, 1H, J=5.1 Hz).

Referential Example 35

Synthesis of 1-[[2-(4-chloro-3,5-dimethoxyphenyl)pyridin-4-yl]methyl]-4-(ethoxycarbonylamino)piperidine:

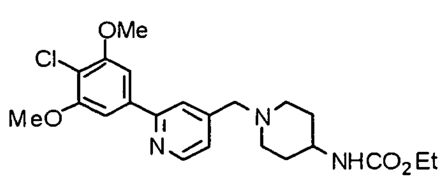

1-[[2-(4-Chloro-3,5-dimethoxyphenyl)pyridin-4-yl]methyl]piperidine-4-carboxamide (743 mg) was treated in the same manner as described in Referential Example 20 to give the title compound.

Yield: 887 mg (theoretical amount).
$^1$H-NMR (400 MHz, $CDCl_3$) δ: 1.24 (t, 3H, J=7.1 Hz), 1.43-1.59 (m, 2H), 1.96 (d, 2H, J=11.4 Hz), 2.19 (t, 2H, J=11.0 Hz), 2.82 (d, 2H, J=11.5 Hz), 3.56 (s, 2H), 4.02 (s, 6H), 4.10 (q, 2H, J=7.1 Hz), 7.26 (s, 2H), 7.66 (s, 1H), 7.71 (dd, 1H, J=5.6 Hz, 1.0 Hz), 8.6 (dd, 1H, J=4.9 Hz, 0.5 Hz).

Referential Example 36

Synthesis of 1-[[2-(4-chloro-3,5-dimethoxyphenyl)pyridin-4-yl]methyl]-4-methylaminopiperidine:

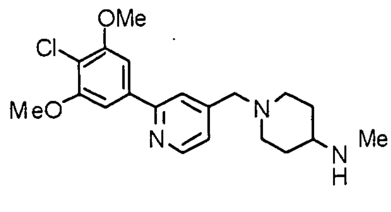

1-[[2-(4-Chloro-3,5-diemthoxyphenyl)pyridin-4-yl]methyl]-4-(ethoxycarbonylamino)piperidine (887 mg) was treated in the same manner as described in Referential Example 21 to give the title compound.
Yield: 195 mg (27%).
$^1$H-NMR (400 MHz, $CDCl_3$) δ: 1.35-1.49 (m, 2H), 1.89 (d, 2H, J=12.3 Hz), 2.11 (t, 2H, J=9.4 Hz), 2.38-2.45 (m, 1H), 2.44 (s, 3H), 2.87 (d, 2H, J=10.7 Hz), 3.57 (s, 2H), 4.02 (s, 6H), 7.23-7.29 (m, 3H), 7.68 (s, 1H), 8.61 (d, 1H, J=4.9 Hz).

Preparation Example 8

Synthesis of 1-[[2-(4-chloro-3,5-dimethoxyphenyl)pyridin-4-yl]methyl]-4-[N-[[2-(4-chloro-3,5-dimethoxyphenyl)pyridin-4-yl]methyl]-N-methylamino]piperidine tetrahydrochloride:

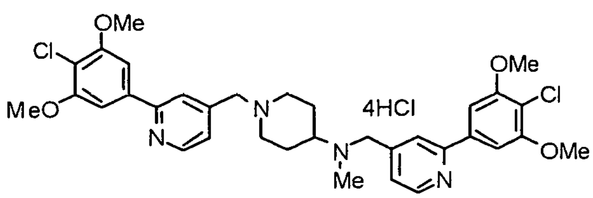

1-[[2-(4-Chloro-3,5-dimethoxyphenyl)pyridin-4-yl]methyl]-4-methylaminopiperidine (195 mg) and 2-(4-chloro-3,5-dimethoxyphenyl)-4-chloromethylpyridine (152 mg) were reacted with each other in the same manner as described in Preparation Example 2. The free base thus obtained was converted to a tetrahydrochloride to give the title compound as yellow powder.
Yield: 300 mg (75%).
$^1$H-NMR (400 MHz, measured as a free base, $CDCl_3$) δ: 1.60-1.90 (m, 4H), 2.06 (t, 2H, J=11.7 Hz), 2.26 (s, 3H), 2.45-2.55 (m, 1H), 2.97 (d, 2H, J=11.3 Hz), 3.57 (s, 2H), 3.67 (s, 2H), 4.01 (s, 6H), 4.02 (s, 6H), 7.24-7.28 (m, 6H), 7.65 (s, 1H), 7.67 (s, 1H), 8.61 (d, 1H, J=5.4 Hz), 8.62 (d, 1H, J=5.4 Hz).

Referential Example 37

Synthesis of 4-(*p*-anisidino)-1-[[2-(3,4,5-trimethoxyphenyl)pyridin-4-yl]methyl]-piperidine:

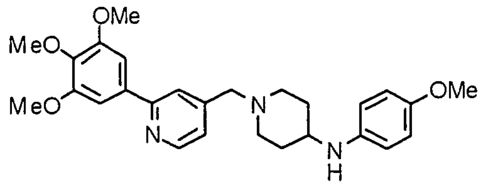

To a solution of 1-[[2-(3,4,5-trimethoxyphenyl)pyridin-4-yl]methyl-4-piperidone (2.17 g) in toluene (40 mL) was added *p*-anisidine (900 mg) and molecular sieves 4A (6.0 g). The mixture was refluxed overnight, then the molecular sieves were filtered off and the filtrate was evaporated. The residue was dissolved in ethanol (40 mL) and sodium borohydride (276 mg) was added. The mixture was stirred at room temperature for 2 hours before concentration in vacuo. The residue was incorporaetd with water and extracted with ethyl acetate. The organic layer was washed with saturated brine, dried over anhydrous sodium sulfate and concentrated under reduced pressure. The residue was purified by silica gel column chromatography using chloroform-methanol (50:1) to give the title compound.
Yield: 1.56 g (55%).
$^1$H-NMR (400MHz, $CDCl_3$) δ: 1.48 (br, 2H), 2.05 (br, 2H), 2.20 (br, 2H), 2.86 (br, 2H), 3.23 (s, 1H), 3.58 (s, 2H), 3.74 (s, 3H), 3.91 (s, 3H), 3.97 (s, 6H), 6.58 (d, 2H, J=8.8 Hz), 6.77 (d, 2H, J=9.0 Hz), 7.22 (d, 1H, J=5.1 Hz), 7.26 (s, 2H), 7.64 (s, 1H), 8.59 (d, 1H, J=4.9 Hz).

Referential Example 38

Synthesis of ethyl 2-(3,4,5-trimethoxyphenyl)nicotinate:

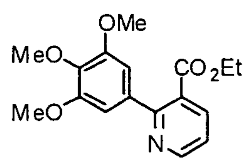

3,4,5-Trimethoxyphenylboronic acid (694 mg) and ethyl 2-chloronicotinate (608 mg) were reacted with each other in the same manner as described in Referential Example 1 to give the title compound.
Yield: 799 mg (77%).
$^1$H-NMR (400MHz, $CDCl_3$) δ: 1.10 (t, 3H, J=7.2 Hz), 3.89 (s, 9H), 4.19 (q, 2H, J=7.2 Hz), 6.79 (s, 2H), 7.34 (dd, 1H, J=7.8 Hz, 4.8 Hz), 8.06 (dd, 1H, J=7.8 Hz, 1.7 Hz), 8.75 (dd, 1H, J=4.8 Hz, 1.7 Hz).

Referential Example 39

Synthesis of 3-hydroxymethyl-2-(3,4,5-trimethoxyphenyl)pyridine:

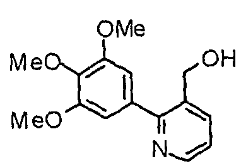

Ethyl 2-(3,4,5-trimethoxyphenyl)nicotinate (468 mg) was treated in the same manner as described in Referential Example 2 to give the title compound.
Yield: 293 mg (72%).
$^1$H-NMR (400MHz, $CDCl_3$) δ: 3.90 (s, 9H), 4.72 (s, 2H), 6.83 (s, 2H), 7.32 (dd, 1H, J=7.9 Hz, 4.8 Hz), 7.92 (dd, 1H, J=7.9 Hz, 1.7 Hz), 8.62 (dd, 1H, J=4.8 Hz, 1.7 Hz).

Referential Example 40

Synthesis of 3-chloromethyl-2-(3,4,5-trimethoxyphenyl)pyridine:

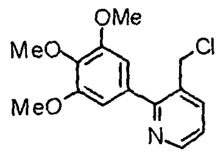

3-Hydroxymethyl-2-(3,4,5-trimethoxyphenyl)pyridine (293 mg) was treated in the same manner as described in the Referential Example 3 to give the title compound.
Yield: 311 mg (theoretical amount).

Preparation Example 9

Synthesis of 4-[N-(4-methoxyphenyl)-N-[[2-(3,4,5-trimethoxyphenyl)pyridin-3-yl]methyl]amino]-1-[[2-(3,4,5-trimethoxyphenyl)pyridin-4-yl]methyl]piperidine trihydrochloride:

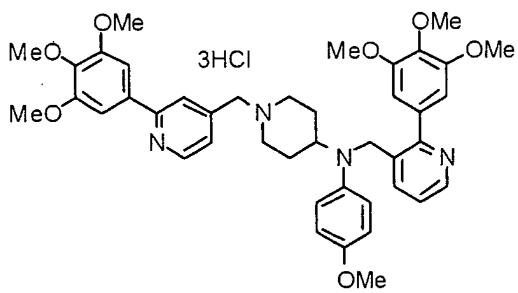

To a solution of 4-(*p*-anisidino)-1-[[2-(3,4,5-trimethoxyphenyl)pyridin-4-yl] methyl]piperidine (139 mg) and 3-chloromethyl-2-(3,4,5-trimethoxyphenyl)pyridine (114 mg) in acetonitrile (5 ml) was added potassium carbonate (83 mg) and potassium iodide (63 mg). The mixture was stirred at 70°C overnight and concentrated under reduced pressure. The residue was dissolved in chloroform, washed with water and saturated brine, dried over anhydrous magnesium sulfate and concentrated under reduced pressure. The residue was purified by column chromatography on silica gel using ether-metanol (20:1) as an eluent. The free base thus obtained was converted to a trihydrochloride to give the title compound as yellow powder.
Yield: 16 mg, (8%).
$^1$H-NMR (400MHz, measured as a free base, $CDCl_3$) δ: 1.60 (br, 2H), 1.77 (br, 2H), 2.09 (br, 2H), 2.93 (br, 2H), 3.45 (br, 1H), 3.54 (s, 2H), 3.73 (s, 3H), 3.90 (s, 6H), 3.91 (s, 6H), 3.96 (s, 6H), 4.34 (s, 2H), 6.65 (d, 2H, J=9.0 Hz), 6.71 (s, 2H), 6.74 (d, 2H, J=9.0 Hz), 7.16-7.19 (m, 2H), 7.22 (s, 2H), 7.55 (s, 1H), 7.79 (d, 1H, J=7.0 Hz), 8.50 (br, 1H), 8.58 (d, 1H, J=4.9 Hz).

Preparation Example 10

Synthesis of 4-[N-(4-methoxyphenyl)-N-[[2-(3,4,5-trimethoxyphenyl)pyridin-4-yl]methyl]amino]-1-[[2-(3,4,5-trimethoxyphenyl)pyridin-4-yl]methyl]piperidine trihydrochloride:

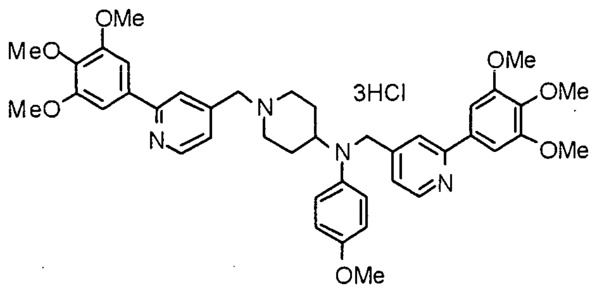

4-(*p*-Anisidino)-1-[[2-(3,4,5-trimethoxyphenyl)pyridin-4-yl]methyl]piperidine (1.56g) and 4-chloromethyl-2-(3,4,5-trimethoxyphenyl)pyridine (1.08g) were reacted with each other in the same manner as described in Preparation Example 9. The resulting free base was converted to a trihydrochloride which gave the title compound as yellow powder.
Yield: 1.17 g (40%).
$^1$H-NMR (400MHz, measured as a free base, $CDCl_3$) δ: 1.68-1.97 (m, 4H), 2.09-2.23 (m, 2H), 2.98 (br, 2H), 3.54-3.66 (m, 3H), 3.73 (s, 3H), 3.89 (s, 3H), 3.90 (s, 3H), 3.93 (s, 6H), 3.96 (s, 6H), 4.45 (s, 2H), 6.74 (d, 2H, J=9.2 Hz), 6.79 (d, 2H, J=9.2 Hz), 7.15 (s, 2H), 7.16-7.21 (m, 2H), 7.23 (s, 2H), 7.57 (s, 1H), 7.60 (s, 1H), 8.54 (d, 1H, J=5.1 Hz), 8.59 (d, 1H, J=4.9 Hz).

Referential Example 41

Synthesis of 3-(3,4,5-trimethoxyphenyl)benzyl alcohol:

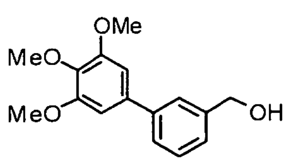

Ethyl 3-(3,4,5-trimethoxyphenyl)benzoate (5.09 g) was treated in the same manner as described in Referential Example 2 to give the title compound.
Yield: 4.25 g (97%).
$^1$H-NMR (400MHz, $CDCl_3$) δ: 1.87 (t, 1H, J=6.0 Hz), 3.89 (s, 3H), 3.92 (s, 6H), 4.76 (d, 1H, J=5.6 Hz), 6.77 (s, 2H), 7.34 (d, 1H, J=7.4 Hz), 7.42 (t, 1H, J=7.5 Hz), 7.48 (d, 1H, J=7.6 Hz), 7.55 (s, 1H).

Referential Example 42

Synthesis of 3-(3,4,5-trimethoxyphenyl)benzyl chloride:

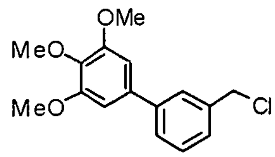

3-(3,4,5-Trimethoxyphenyl)benzyl alcohol (1.21 g) was treated in the same manner as described in Referential Example 3 to give the title compound.
Yield: 893 mg (69%).
$^1$H-NMR (400MHz, $CDCl_3$) δ: 3.87 (s, 3H), 3.90 (s, 6H), 4.62 (s, 2H), 6.75 (s, 2H), 7.33 (d, 1H, J=7.6 Hz), 7.39 (t, 1H, J=7.7 Hz), 7.48 (d, 1H, J=7.6 Hz), 7.54 (s, 1H).

Preparation Example 11

Synthesis of 4-[N-(4-methoxyphenyl)-N-[3-(3,4,5-trimethoxyphenyl)benzyl]amino]-1 -[[2-(3,4,5-trimethoxyphenyl) pyridin-4-yl]methyl]piperidine dihydrochloride:

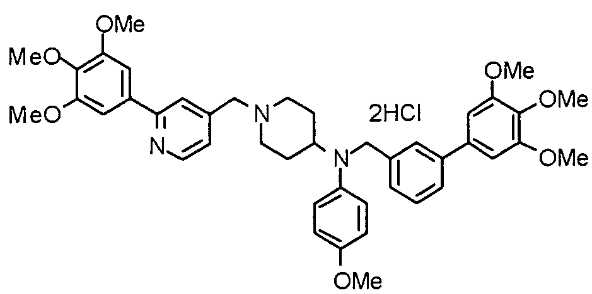

4-(*p*-Anisidino)-1-[[2-(3,4,5-trimethoxyphenyl)pyridin-4-yl]methyl]piperidine (139 mg) and 3-(3,4,5-trimethoxyphenyl)benzyl chloride (114 mg) were reacted with each other in the same manner as described in Preparation Example 9. The resulting free base was converted to a dihydrochloride which gave the title compound as yellow powder.
Yield: 52 mg (22%).
$^1$H-NMR (400MHz, measured as a free base, $CDCl_3$) δ: 1.77-1.92 (m, 5H), 2.14-2.20 (m, 2H), 2.95-3.00 (m, 5H), 2.14-2.20 (m, 2H), 2.95-3.00 (m, 2H), 3.58 (s, 2H), 3.72 (s, 3H), 3.88 (s, 3H), 3.89 (s, 6H), 3.90 (s, 3H), 3.96 (s, 6H), 4.47 (s, 2H), 6.70 (s, 2H), 6.74-6.83 (m, 4H), 7.20 (d, 1H, J=7.4 Hz), 7.23 (s, 2H), 7.25-7.27 (m, 1H), 7.33 (t, 1H, J=7.4 Hz), 7.38 (d, 1H, J=8.7 Hz), 7.43 (s, 1H), 7.62 (s, 1H), 8.59 (d, 1H, J=5.1 Hz).

Referential Example 43

Synthesis of ethyl 6-(3,4,5-trimethoxyphenyl)nicotinate:

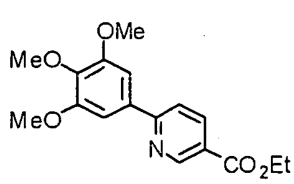

3,4,5-Trimethoxyphneylboronic acid (1.16 g) and ethyl 6-chloronicotinate (1.02 g) were reacted with each other in the same manner as described in the Referential Example 1 to give the title compound.
Yield: 1.42 g (82%)
$^1$H-NMR (400MHz, $CDCl_3$) δ: 1.43 (t, 3H, J=7.2 Hz), 3.92 (s, 3H), 3.98 (s, 6H), 4.44 (q, 2H, J=7.2 Hz), 7.32 (s, 2H), 7.76 (d, 1H, J=8.3 Hz), 8.33 (dd, 1H, J=8.2 Hz, 2.2 Hz), 9.26 (d, 1H, J=2.2 Hz).

Referential Example 44

Synthesis of 5-hydroxymethyl-2-(3,4,5-trimethoxyphenyl)pyridine:

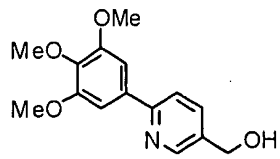

Ethyl 6-(3,4,5-trimethoxyphenyl)nicotinate (658 mg) was treated in the same manner as described in Referential Example 2 to give the title compound.
Yield: 482 mg (85%).
$^1$H-NMR (400MHz, $CDCl_3$) δ: 3.91 (s, 3H), 3.97 (s, 6H), 4.76 (s, 2H), 7.23 (s, 2H), 7.68 (d, 1H, J=7.4 Hz), 7.78 (dd, 1H, J=7.4 Hz, 2.3 Hz), 8.63 (d, 1H, J=2.3 Hz).

Referential Example 45

Synthesis of 5-chloromethyl-2-(3,4,5-trimethoxyphenyl)pyridine:

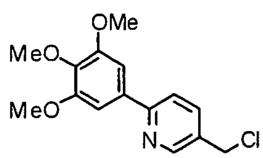

5-Hydroxymethyl-2-(3,4,5-trimethoxyphenyl)pyridine (685 mg) was treated in the same manner as described in Referential Example 3 to give the title compound. Yield: 717 mg (theoretical amount).

Preparation Example 12

Synthesis of 4-[N-(4-methoxyphenyl)-N-[[2-(3,4,5-trimethoxyphenyl)pyridin-5-yl] methyl]amino]-1-[[2-(3,4,5-trimethoxyphenyl)pyridin-4-yl]methyl]piperidine trihydrochloride:

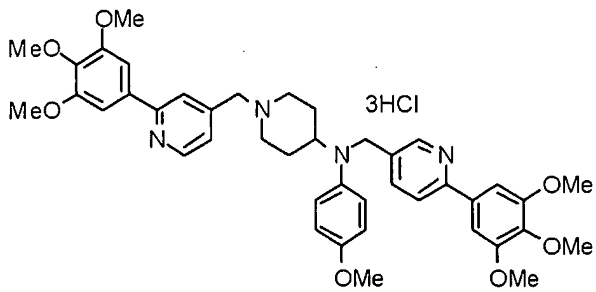

4-(*p*-Anisidino)-1-[[2-(3,4,5-trimethoxyphenyl)pyridin-4-yl]methyl]piperidine (139 mg) and 5-chloromethyl-2-(3,4,5-trimethoxyphenyl)pyridine (114 mg) were reacted with each other in the same manner as described in Preparation Example 9.
The resulting free base was converted to a trihydrochloride which gave the title compound as yellow powder.
Yield: 13 mg (5%).
$^1$H-NMR (400MHz, measured as a free base, $CDCl_3$) δ: 1.76 (br, 2H), 1.88 (br, 2H), 2.14 (br, 2H), 2.97 (br, 2H), 3.51 (br, 1H), 3.57 (s, 2H), 3.73 (s, 3H), 3.89 (s, 3H), 3.90 (s, 3H), 3.94 (s, 6H), 3.96 (s, 6H), 4.42 (s, 2H), 6.78 (br, 4H), 7.20 (br, 3H), 7.23 (s, 2H), 7.57-7.70 (m, 3H), 8.58-8.60 (m, 2H).

Referential Example 46

Synthesis of ethyl 5-(3,4,5-trimethoxyphenyl)nicotinate:

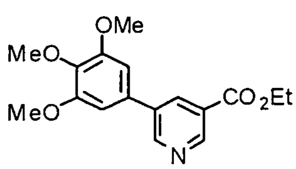

3,4,5-Trimethoxyphenylboronic acid (6.36 g) and ethyl 5-bromonicotinate (6.90 g) were reacted with each other in the same manner as described in Referential Example 1 to give the title compound.
Yield: 7.19 g (76%).
$^1$H-NMR (400MHz, $CDCl_3$) δ: 1.44 (t, 3H, J=7.1 Hz), 3.91 (s, 3H), 3.95 (s, 6H), 4.46 (q, 2H, J=7.1 Hz), 6.79 (s, 2H), 8.44 (t, 1H, J=2.1 Hz), 8.96 (d, 1H, J=2.1 Hz), 9.18 (d, 1H, J=1.8 Hz).

Referential Example 47

Synthesis of 3-hydroxymethyl-5-(3,4,5-trimethoxyphenyl)pyridine:

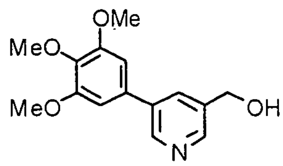

Ethyl 5-(3,4,5-trimethoxyphenyl)nicotinate (7.19 g) was treated in the same manner as described in the Referential Example 2 to give the title compound.
Yield; 3.83 g (61%).
$^1$H-NMR (400MHz, $CDCl_3$) δ: 3.88 (s, 3H), 3.89 (s, 6H), 4.39 (br, 1H), 4.80 (s, 2H), 6.72 (s, 2H), 7.89 (t, 1H, J=1.2 Hz), 8.47 (d, 1H, J=2.1 Hz), 8.63 (d, 1H, J=2.2 Hz).

Referential Example 48

Synthesis of 3-chloromethyl-5-(3,4,5-trimethoxyphenyl)pyridine:

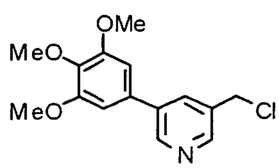

3-Hydroxymethyl-5-(3,4,5-trimethoxyphenyl)pyridine (2.85 g) was treated in the same manner as described in Referential Example 3 to give the title compound.
Yield: 1.97 g (65%).
$^1$H-NMR (400MHz, $CDCl_3$) δ: 3.90 (s, 3H), 3.94 (s, 6H), 4.67 (s, 2H), 6.75 (s, 2H), 7.87 (t, 1H, J=2.1 Hz), 8.59 (d, 1H, J=2.0 Hz), 8.76 (d, 1H, J=2.1 Hz).

Preparation Example 13

Synthesis of 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine trihydrochloride:

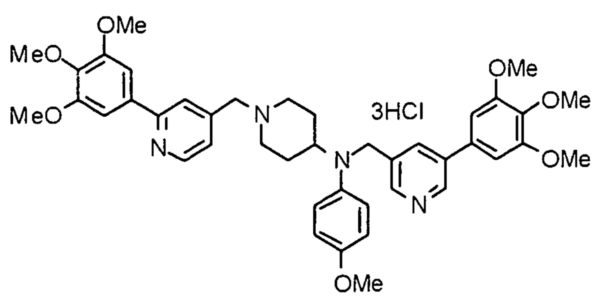

4-(*p*-Anisidino)-1-[[2-(3,4,5-trimethoxyphenyl)pyridin-4-yl]methyl]piperidine (139 mg) and 3-chloromethyl-5-(3,4,5-trimethoxyphenyl)pyridine (114 mg) were reacted with each other in the same manner as described in Preparation Example 9. The resulting free base was converted to a trihydrochloride which gave the title compound as yellow powder.
Yield: 14 mg (5%).
$^1$H-NMR (400 MHz, measured as a free base, $CDCl_3$) 5; 1.73-1.75 (m, 2H), 1.88 (d, 2H, J=11.3 Hz), 2.13 (t, 2H, J=11.3 Hz), 2.96 (d, 2H, J=11.5 Hz), 3.50 (br, 1H), 3.55 (s, 2H), 3.72 (s, 3H), 3.88 (s, 3H), 3.89 (s, 9H), 3.96 (s, 6H), 4.45 (s, 2H), 6.65 (s, 2H), 6.76 (d, 2H, J=9.6 Hz), 6.80 (d, 2H, J=9.4 Hz), 7.20 (d, 1H, J=5.3 Hz), 7.22 (s, 2H), 7.59 (s, 1H), 7.67 (s, 1H), 8.50 (s, 1H), 8.59 (d, 1H, J=4.7 Hz), 8.62 (s, 1H).

Referential Example 49

Synthesis of 4-iodo-2,6-dimethoxyphenol:

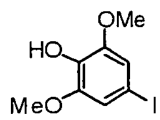

To a solution of 5-iodo-1,2,3-trimethoxybenzene (3.2 g) in 1,2-dichloroethane (40 mL) was added aluminum chloride (1.6 g). The mixture was stirred at 60°C for 4 hours and concentrated under reduced pressure. The residue was dissolved in 1 M aqueous sodium hydroxide solution and washed with ether. The aqueous layer was then acidified and extracted with chloroform. The organic layer was washed with brine, dried over anhydrous magnesium sulfate and concentrated under reduced pressure to give the title compound as white crystalline powder.
Yield: 1.0 g (31%)

Referential Example 50

Synthesis of 1,3-dimethoxy-5-iodo-2-isopropoxybenzene:

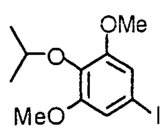

To a suspension of 2,6-dimethoxy-4-iodophenol (1.0 g) and potassium carbonate (938 mg) in DMF (10 mL) was added 2-iodopropane (507 mL). The mixture was stirred at 60°C for 3 hours and concentrated under reduced pressure. Ethyl acetate and water were added to the residue, the organic layer was separated, washed with brine, dried over anhydrous sodium sulfate and concentrated under reduced pressure. The residue was purified by column chromatography on silica gel using hexane-ethyl acetate (5:1) as an eluent to give the title compound.
Yeld: 788 mg (72%).

Referential Example 51

Synthesis of 3,5-dimethoxy-4-isopropoxyphenylboronic acid:

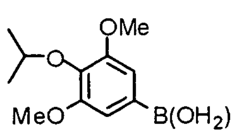

1,3-Dimethoxy-5-iodo-2-isopropoxybenzene (2.25 g) was treated in the same manner as described in Referential Example 29 to give the title compound.
Yield: 1.23 g (74%).

Referential Example 52

Synthesis of ethyl 2-(3,5-dimethoxy-4-isopropoxyphenyl)isonicotinate:

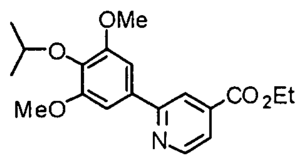

3,5-Dimethoxy-4-isopropoxyphenylboronic acid (1.23 g) and ethyl 2-chloroisonicotinate (0.95 g) were reacted with each other in the same manner as described in Referential Example 1 to give the title compound.
Yield: 1.57 g(89%).
$^1$H-NMR (400 MHz, $CDCl_3$) δ: 1.33 (d, 6H, J=4.9 Hz), 1.44 (t, 3H, J=7.1 Hz), 3.95 (s, 6H), 4.42-4.49 (m, 3H), 7.29 (s, 2H), 7.75 (dd, 1H, J=4.9 Hz, 1.4 Hz), 8.24 (s, 1H), 8.80 (d, 1H, J=4.9 Hz).

Referential Example 53

Synthesis of 2-(3,5-dimethoxy-4-isopropoxyphenyl)-4-hydroxymethylpyridine:

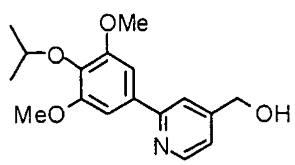

To a suspension of lithium aluminium hydride (190 mg) in THF (20 mL) was added dropwise a solution of 2-(4-isopropoxy-3,5-dimethoxyphenyl)isonicotinate (1.57 g) in THF (30 mL) under ice cooling in the atmosphere of argon. The mixture was stirred at 0°C for 30 minutes and a saturated aqueous ammonium chloride was added thereto. After the mixture was extracted with ethyl acetate, the organic layer was washed with saturated brine, dried over anhydrous sodium sulfate and concentrated under reduced pressure. The residue was purified by column chromatography on silica gel eluting with hexane-ethyl acetate (3:1) and then chloroform-methanol (15:1) to give the title compound.
Yield: 1.31 g (95%).
$^1$H-NMR (400 MHz, $CDCl_3$) δ: 1.32 (d, 6H, J=6.1 Hz), 3.93 (s, 6H), 4.45 (quint, 1H, J=6.1 Hz), 4.81 (s, 2H), 7.20 (d, 1H, J=5.1 Hz), 7.23 (s, 2H), 7.68 (s, 1H), 8.62 (d, 1H, J=5.1 Hz).

Referential Example 54

Synthesis of 4-chloromethyl-2-(3,5-dimethoxy-4-isopropoxyphenyl)pyridine:

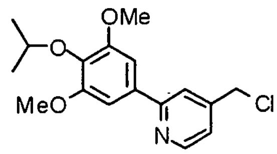

2-(3,5-Dimethoxy-4-isopropoxyphenyl)-4-hydroxymethylpyridine (1.49 g) was treated in the same manner as described in Referential Example 3 to give the title compound.
Yield: 1.33 g (84%).
$^1$H-NMR (400 MHz, $CDCl_3$) δ: 1.32 (d, 6H, J=6.2 Hz), 3.94 (s, 6H), 4.45 (quint, 1H, J=6.1 Hz), 4.61 (s, 2H), 7.23-7.26 (m, 3H), 7.69 (s, 1H), 8.66 (d, 1H, J=5.1 Hz).

Referential Example 55

Synthesis of 1-[[2-(3,5-dimethoxy-4-isopropoxyphenyl)pyridin-4-yl]methyl] -4-piperidone ethylene ketal:

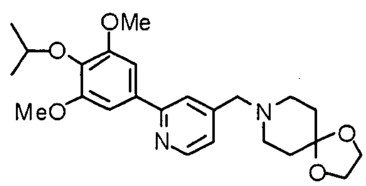

4-Chloromethyl-2-(3, 5-dimethoxy-4-isopropoxyphenyl)pyridine (643 mg) and 4-piperidone ethylene ketal (287 mg) were reacted with each other in the same manner as described in Preparation Example 2 to give the title compound.
Yield: 818 mg (95%).
$^1$H-NMR (400 MHz, $CDCl_3$) δ: 1.32 (d, 6H, J=6.1 Hz), 1.78 (t, 4H, J=5.7 Hz), 2.57 (br, 4H), 3.49 (s, 4H), 3.59 (s, 2H), 3.94 (s, 6H), 4.44 (quint, 1H, J=6.1 Hz), 7.21 (d, 1H, J=5.1 Hz), 7.23 (s, 2H), 7.65 (s, 1H), 8.59 (d, 1H, J=5.1 Hz).

Referential Example 56

Synthesis of 1-[[2-(3,5-dimethoxy-4-isopropoxyphenyl)pyridin-4-yl]methyl] -4-piperidone:

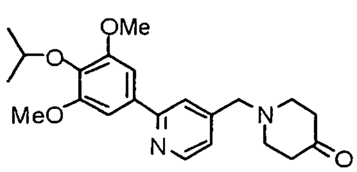

1-[[2-(3,5-Dimethoxy-4-isopropoxyphenyl)pyridin-4-yl]methyl]-4-piperidone ethylene ketal (818 mg) was treated in the same manner as described in Referential Example 23 to give the title compound.
Yield: 717 mg (98%).
$^1$H-NMR (400 MHz, $CDCl_3$) δ: 1.32 (d, 6H, J=6.2 Hz), 2.50 (t, 4H, J=6.1 Hz), 2.81 (t, 4H, J=6.1 Hz), 3.69 (s, 2H), 3.95

(s, 6H), 4.45 (quint, 1H, J=6.2 Hz), 7.24 (s, 2H), 7.25-7.27 (m, 1H), 7.68 (s, 1H), 8.63 (d, 1H, J=5.1 Hz).

Referential Example 57

Synthesis of 4-(*p*-anisidino)-1-[[2-(3,5-dimethoxy-4-isopropoxyphenyl)pyridin -4-yl] methyl]piperidine:

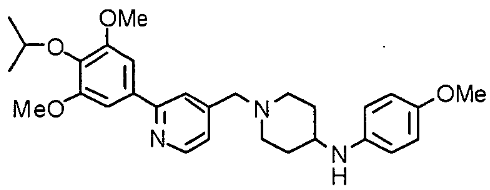

1-[[2-(3,5-Dimethoxy-4-isopropoxyphenyl)pyridin-4-yl]methyl]-4-piperidon e (350 mg) and *p*-anisidine (123 mg) were treated in the same manner as described in Referential Example 37 to give the title compound.
Yield: 307 mg (69%).
$^{1}$H-NMR (400 MHz, $CDCl_3$) δ: 1.32 (d, 6H, J=6.3 Hz), 1.46-1.52 (m, 2H), 2.00-2.24 (m, 2H), 2.22 (t, 2H, J=11.1 Hz), 2.86 (d, 2H, J=12.1 Hz), 3.18-3.28 (m, 1H), 3.58 (s, 2H), 3.74 (s, 3H), 3.94 (s, 6H), 4.40 (quint, 1H, J=6.3 Hz), 6.58 (d, 2H, J=6.6 Hz), 6.78 (d, 2H, J=6.6 Hz), 7.20 (d, 1H, J=5.1 Hz), 7.24 (s, 2H), 7.64 (s, 1H), 8.59 (d, 1H, J=5.1 Hz).

Preparation Example 14

Synthesis of 1-[[2-(4-isopropoxy-3,5-dimethoxyphenyl)pyridin-4-yl]methyl]-4-[N-[[2-(4-isopropoxy-3,5-dimethoxyphenyl)pyridin-4-yl]methyl]-N-(4-methoxyphenyl) amino]piperidine trihydrochloride:

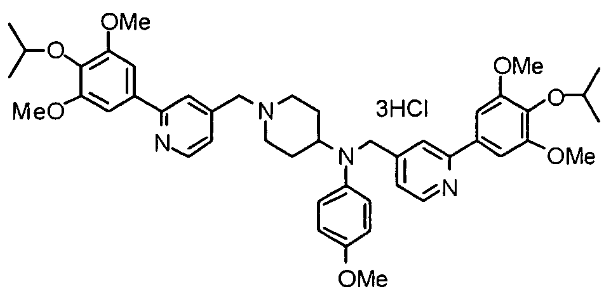

4-(*p*-Anisidino)-1-[[2-(3,5-dimethoxy-4-isopropoxyphenyl)pyridin-4-yl]methyl] piperidine (307 mg) and 4-chloromethyl-2-(3,5-dimethoxy-4-isopropoxyphenyl) pyridine (201 mg) were reacted with each other in the same manner as described in Preparation Example 9. A free base obtained was converted to a trihydrochloride giving the title compound as yellow powder.
Yield: 230 mg (46%).
$^{1}$H-NMR (400 MHz, measured as a free base, $CDCl_3$) δ: 1.31 (d, 6H, J=3.3 Hz), 1.32 (d, 6H, J=6.8 Hz), 1.70-1.92 (m, 4H), 2.10-2.20 (m, 2H), 2.92-3.01 (m, 2H), 3.56 (s, 2H), 3.73 (s, 3H), 3.85-3.95 (m, 1H), 3.90 (s, 6H), 3.93 (s, 6H), 4.39-4.49 (m, 4H), 6.73 (d, 2H, J=4.8 Hz), 6.78 (d, 2H, J=4.8 Hz), 7.14 (s, 2H), 7.15-7.20 (m, 2H), 7.23 (s, 2H), 7.58 (s, 1H), 7.60 (s, 1H), 8.53 (d, 1H, J=5.1 Hz), 8.58 (d, 1H, J=5.1 Hz).

Referential Example 58

Synthesis of 4-benzylamino-1-[[2-(3,4,5-trimethoxyphenyl)pyridin-4-yl]methyl] piperidine:

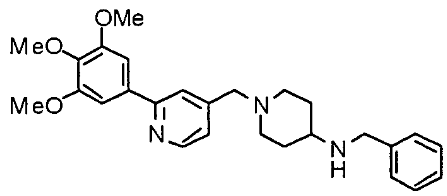

1-[[2-(3,4,5-Trimethoxyphenyl)pyridin-4-yl]methyl-4-piperidone (1.40 g) and benzylamine (0.51 g) were reacted with each other in the same manner as described in Referential Example 37 to give the title compound as yellow amorphous.
Yield: 1.20 g (68%).
$^1$H-NMR (400MHz, $CDCl_3$) δ: 1.40-1.60 (m, 2H), 1.88-2.09 (m, 5H), 2.54 (br, 1H), 2.82-2.85 (m, 2H), 3.52 (s, 2H), 3.80 (s, 2H), 3.89 (s, 2H), 3.95 (s, 6H), 7.18-7.31 (m, 8H), 7.64 (s, 1H), 8.57 (d, 1H, J=5.1 Hz).

Preparation Example 15

Synthesis of 4-[N-benzyl-N-[[2-(3,4,5-trimethoxypheny)pyridin-3-yl]methyl]amino]-1-[[2-(3,4,5-trimethoxyphenyl) pyridin-4-yl]methyl]piperidine tetrahydrochloride:

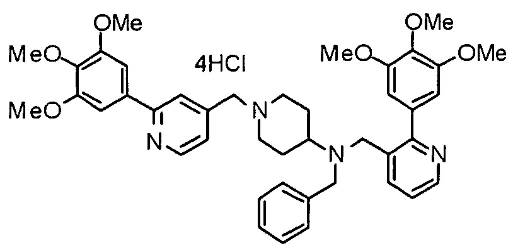

4-Benzylamino-1-[[2-(3,4,5-trimethoxyphenyl)pyridin-4-yl]methyl]piperidine (134 mg) and 3-chloromethyl-2-(3,4,5-trimethoxyphenyl)pyridine (114 mg) were reacted with each other in the same manner as described in Preparation Example 9. A free base obtained was converted to a tetrahydrochloride to give the title compound as yellow powder.
Yield: 43 mg, (17%).
$^1$H-NMR (400MHz, measured as a free base, $CDCl_3$) δ: 1.63 (br, 4H), 1.87 (br, 2H), 2.39 (br, 1H), 2.88 (br, 2H), 3.49 (s, 2H), 3.57 (s, 2H), 3.68 (s, 2H), 3.86 (s, 6H), 3.88 (s, 3H), 3.90 (s, 3H), 3.96 (s, 6H), 6.60 (s, 2H), 7.17 (d, 1H, J=5.1 Hz), 7.22-7.29 (m, 8H), 7.56 (s, 1H), 8.02 (d, 1H, J=8.0 Hz), 8.50 (d, 1H, J=6.4 Hz), 8.58 (d, 1H, J=5.1 Hz).

Preparation Example 16

Synthesis of 4-[N-benzyl-N-[[2-(3,4,5-trimethoxyphenyl)pyridin-4-yl]methyl]amino]-1-[[2-(3,4,5-trimethoxyphenyl) pyridin-4-yl]methyl]piperidine tetrahydrochloride:

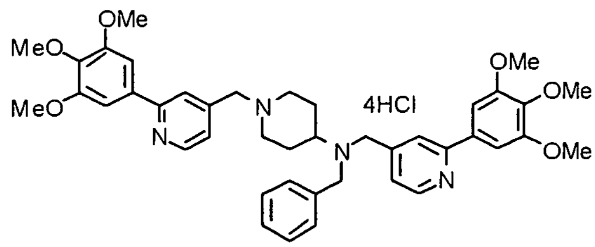

4-Benzylamino-1-[[2-(3,4,5-trimethoxyphenyl)pyridin-4-yl]methyl]piperidine (230 mg) and 4-chloromethyl-2-(3,4,5-trimethoxyphenyl)pyridine (158 mg) were reacted with each other in the same manner as described in Preparation Example 9.
The resulting free base was converted to a tetrahydrochloride which gave the title compound as yellow powder.
Yield: 172 mg (47%).
$^1$H-NMR (400MHz, measured as a free base, $CDCl_3$) δ: 1.69-1.85 (m, 4H), 1.93-1.99 (m, 2H), 2.56 (br, 1H), 2.93-3.00 (m, 2H), 3.51 (s, 2H), 3.71 (s, 2H), 3.74 (s, 2H), 3.90 (s, 6H), 3.96 (s, 6H), 3.96 (s, 6H), 7.18-7.32 (m, 9H), 7.38 (d, 2H, J=7.1 Hz), 7.59 (s, 1H), 7.68 (s, 1H), 8.56 (d, 1H, J=5.1 Hz), 8.60 (d, 1H, J=5.1 Hz).

Preparation Example 17

Synthesis of 4-[N-benzyl-N-[3-(3,4,5-trimethoxyphenyl)benzyl]amino]-1-[[2 - (3,4,5-trimethoxyphenyl)pyridin-4-yl] methyl]piperidine trihydrochloride:

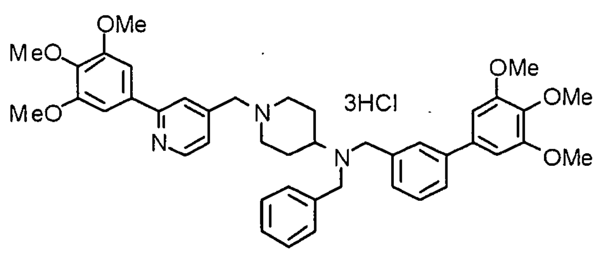

4-Benzylamino-1-[[2-(3,4,5-trimethoxyphenyl)pyridin-4-yl]methyl]piperidine (134 mg) and 3-(3,4,5-trimethoxyphenyl)benzyl chloride (114 mg) were reacted with each other in the same manner as described in Preparation Example 9. The resulting free base was converted to a trihydrochloride which gave the title compound as yellow powder.
Yield: 47 mg (18%).
$^1$H-NMR (400MHz, measured as a free base, $CDCl_3$) δ: 1.70-1.86 (m, 4H), 1.96 (br, 2H), 2.59 (br, 1H), 2.94 (br, 2H), 3.51 (s, 2H), 3.70 (s, 2H), 3.74 (s, 2H), 3.89 (s, 3H), 3.90 (s, 3H), 3.92 (s, 6H), 3.96 (s, 6H), 6.75 (s, 2H), 7.18-7.30 (m, 6H), 7.35-7.40 (m, 5H), 7.56 (s, 1H), 7.60 (s, 1H), 8.58 (d, 1H, J=5.1 Hz).

Preparation Example 18

Synthesis of 4-[N-benzyl-N-[[2-(3,4,5-trimethoxyphenyl)pyridin-5-yl]methyl]amino]-1- [[2-(3,4,5-trimethoxyphenyl) pyridin-4-yl]methyl]piperidine tetrahydrochloride:

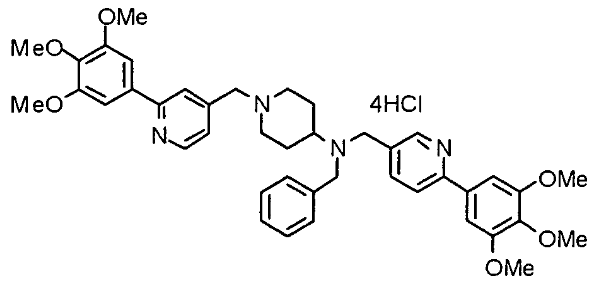

4-Benzylamino-1-[[2-(3,4,5-trimethoxyphenyl)pyridin-4-yl]methyl]piperidine (134 mg) and 5-chloromethyl-2-(3,4,5-trimethoxyphenyl)pyridine (114 mg) were reacted with each other in the same manner as described in Preparation Example 9.
The resulting free base was converted to a tetrahydrochloride which gave the title compound as yellow powder.
Yield: 44 mg (17%).
$^1$H-NMR (400MHz, measured as a free base, $CDCl_3$) δ: 1.81 (br, 4H), 1.96 (br, 2H), 2.55 (br, 1H), 2.96 (br, 2H), 3.52 (s, 2H), 3.69 (s, 4H), 3.89 (s, 6H), 3.95 (s, 6H), 3.96 (s, 6H), 7.19-7.32 (m, 8H), 7.36-7.38 (m, 2H), 7.61 (d, 2H, J=7.6 Hz), 7.69-7.73 (m, 1H), 8.59 (d, 1H, J=4.9 Hz), 8.63 (s, 1H).

Preparation Example 19

Synthesis of 4-[N-benzyl-N-[[5-(3,4,5-trimethoxyphenyl)pyridin-3-yl]methyl] amino]-1-[[2-(3,4,5-trimethoxyphenyl) pyridin-4-yl]methyl]piperidine tetrahydrochloride:

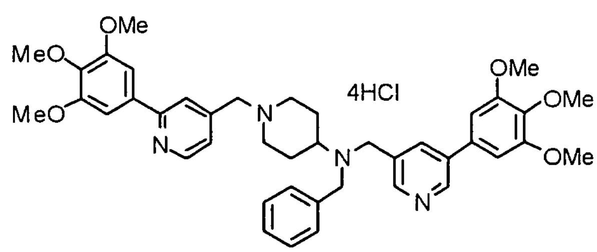

4-Benzylamino-1-[[2-(3,4,5-trimethoxyphenyl)pyridin-4-yl]methyl]piperidine (134 mg) and 3-chloromethyl-5-(3,4,5-trimethoxyphenyl)pyridine (114mg) were reacted with each other in the same manner as described in Preparation Example 9. The resulting free base was converted to a tetrahydrochloride which gave the title compound as yellow powder.
Yield: 26 mg (10%).
$^1$H-NMR (400 MHz, measured as a free base, $CDCl_3$) δ: 1.83 (br, 4H), 1.97 (br, 2H), 2.58 (br, 1H), 2.95 (br, 2H), 3.53 (s, 2H), 3.71 (s, 2H), 3.75 (s, 2H), 3.90 (s, 6H), 3.93 (s, 6H), 3.96 (s, 6H), 6.74 (s, 2H), 7.19-7.30 (m, 6H), 7.36 (d, 2H, J=6.8 Hz), 7.60 (s, 1H), 7.79 (s, 1H), 8.54 (s, 1H), 8.59 (d, 1H, J=5.1 Hz), 8.64 (s, 1H).

Referential Example 59

Synthesis of 1-(*tert*-butoxycarbonyl)-4-[N-[[2-(3,4,5-trimethoxyphenyl)pyridin-4-yl]methyl]aminomethyl]piperidine:

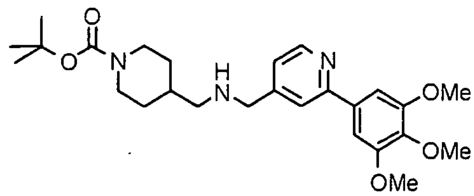

1-(*tert*-Butoxycarbonyl)-4-aminomethylpiperidine (200mg) and 4-chloromethyl-2-(3,4,5-trimethoxyphenyl)pyridine (183mg) were reacted with each other in the same manner as described in Preparation Example 2 to give the title compound as yellow oil.
Yield: 264 mg (90%).
$^1$H-NMR (400MHz, $CDCl_3$) δ: 1.12-1.27 (m, 3H), 1.45 (s, 9H), 1.60 (br, 1H), 1.74 (d, 2H, J=12.9 Hz), 2.54 (d, 2H, J=6.6 Hz), 2.69 (br, 2H), 3.87 (s, 2H), 3.90 (s, 3H), 3.97 (s, 6H), 4.03-4.14 (m, 2H), 7.20 (d, 1H, J=3.9 Hz), 7.24 (s, 2H), 7.65 (s, 1H), 8.60 (d, 1H, J=4.9 Hz).

Referential Example 60

Synthesis of 1-(*tert*-butoxycarbonyl)-4-[N-methyl-N-[[2-(3,4,5-trimethoxyphenyl) pyridin-4-yl]methyl]aminomethyl] piperidine:

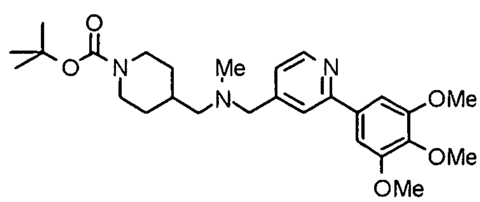

1-(*tert*-Butoxycarbonyl)-4-[N-[[2-(3,4,5-trimethoxyphenyl)pyridin-4-yl] methyl]aminomethyl]piperidine (264 mg) was treated in the same manner as described in Referential Example 11 to give the title compound as yellow oil.
Yield: 157 mg (58%).
$^1$H-NMR (400MHz, $CDCl_3$) δ: 1.00-1.09 (m, 2H), 1.43 (s, 9H), 1.65-1.70 (m, 1H), 1.79 (d, 2H, J=12.7 Hz), 2.21 (d, 2H, J=7.4 Hz), 2.23 (s, 3H), 2.69 (br, 2H), 3.52 (s, 2H), 3.89 (s, 3H), 3.96 (s, 6H), 4.07-4.13 (m, 2H), 7.20 (d, 1H, J=4.9 Hz), 7.24 (s, 2H), 7.64 (s, 1H), 8.58 (d, 1H, J=5.1 Hz).

Referential Example 61

Synthesis of 4-[N-methyl-N-[[2-(3,4,5-trimethoxyphenyl)pyridin-4-yl]methyl] aminomethyl] piperidine:

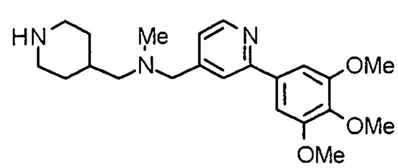

1-(*tert*-Butoxycarbonyl)-4-[N-methyl-N-[[2-(3,4,5-trimethoxyphenyl)pyridin-4-yl]methyl]aminomethyl]piperidine (152 mg) was treated in the same manner as described in Referential Example 12 to give the title compound as yellow crystals.
Yield: 105 mg (88%).
$^1$H-NMR (400MHz, $CDCl_3$) δ: 1.00-1.10 (m, 2H), 1.60-1.68 (m, 1H), 1.80 (d, 2H, J=12.5 Hz), 2.03 (br, 1H), 2.20 (d, 2H, J=8.4 Hz), 2.21 (s, 3H), 2.58 (dt, 2H, J=12.1 Hz, 2.1 Hz), 3.05 (d, 2H, J=12.1 Hz), 3.51 (s, 2H), 3.89 (s, 3H), 3.95 (s, 6H), 7.20 (d, 1H, J=5.1 Hz), 7.24 (s, 2H), 7.65 (s, 1H), 8.57 (d, 1H, J=5.9 Hz).

Preparation Example 20

Synthesis of 4-[N-methyl-N-[[2-(3,4,5-trimethoxyphenyl)pyridin-4-yl]methyl] aminomethyl]-1-[[2-(3,4,5-trimethoxyphenyl)pyridin-4-yl]methyl]piperidine dioxalate:

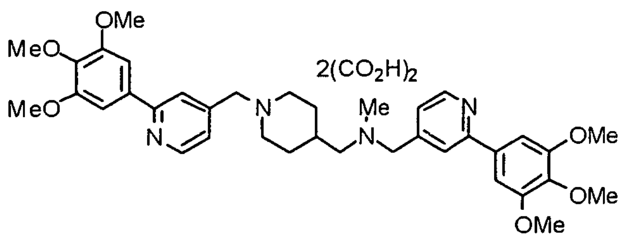

4-[N-Methyl-N-[[2-(3,4,5-trimethoxyphenyl)pyridin-4-yl]methyl]aminomethyl] piperidine (96 mg) and 4-chloromethyl-2-(3,4,5-trimethoxyphenyl)pyridine (73 mg) were reacted with each other in the same manner as described in Preparation Example 2. The title compound was obtained as white powder after converting the resulting product to a dioxalate.
Yield: 109 mg (40%).
$^1$H-NMR (400MHz, measured as a free base, $CDCl_3$) δ: 1.19-1.27 (m, 2H), 1.56 (br, 1H), 1.81 (d, 2H, J=11.1 Hz), 1.99-2.04 (m, 2H), 2.23 (s, 5H), 2.88 (d, 2H, J=11.1 Hz), 3.53 (s, 4H), 3.89 (s, 3H), 3.90 (s, 3H), 3.94 (s, 6H), 3.96 (s, 6H), 7.20 (br, 2H), 7.23 (s, 4H), 7.61 (s, 1H), 7.64 (s, 1H), 8.58 (d, 2H, J=4.9 Hz).

Referential Example 62

Synthesis of 4-(3,5-dimethoxyphenylamino)-1-[[2-(3,4,5-trimethoxyphenyl)pyridin-4-yl]methyl]piperidine:

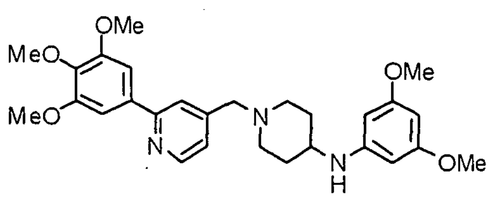

1-[[2-(3,4,5-Trimethoxyphenyl)pyridin-4-yl]methyl-4-piperidone (1.40 g) and 3,5-dimethoxyaniline (722 mg) were reacted with each other in the same manner as described in Referential Example 37 to give the title compound.
Yield: 800 mg (41%).
$^1$H-NMR (400MHz, $CDCl_3$) δ: 1.40-1.90 (m, 2H), 1.95-2.50 (m, 4H), 2.93 (br, 2H), 3.31 (br, 1H), 3.65 (br, 2H), 3.72 (s, 6H), 3.88 (s, 3H), 3.96 (s, 6H), 5.76 (s, 2H), 5.85 (s, 1H), 7.20-7.35 (m, 3H), 7.73 (br, 1H), 8.60 (d, 1H, J=4.9 Hz).

Preparation Example 21

Synthesis of 4-[N-(3,5-dimethoxyphenyl)-N-[[2-(3,4,5-trimethoxyphenyl)pyridin-3-yl]methyl]amino]-1-[[2-(3,4,5-trimethoxyphenyl)pyridin-4-yl]methyl]piperidine trihydrochloride:

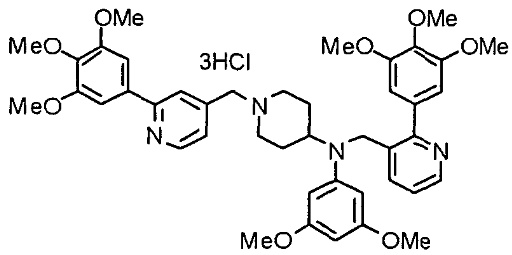

4-(3,5-Dimethoxyphenylamino)-1-[[2-(3,4,5-trimethoxyphenyl)pyridin-4-yl] methyl]piperidine (148 mg) and 3-chloromethyl-2-(3,4,5-trimethoxyphenyl)pyridine (114 mg) were reacted with each other in the same manner as described in Preparation Example 9. The resulting free base was converted to a trihydrochloroide to give the title compound as yellow powder.
Yield: 29 mg, (11%).
$^1$H-NMR (400MHz, measured as a free base, $CDCl_3$) δ: 1.60-1.63 (m, 2H), 1.79 (d, 2H, J=11.7 Hz), 2.13 (t, 2H, J=11.4 Hz), 2.94 (d, 2H, J=11.3 Hz), 3.54 (s, 2H), 3.71 (s, 6H), 3.78-3.84 (m, 1H), 3.90 (s, 3H), 3.91 (s, 6H), 3.92 (s, 3H), 3.96 (s, 6H), 4.41 (s, 2H), 5.84 (s, 2H), 6.72 (s, 2H), 7.09-7.24 (m, 5H), 7.53 (s, 1H), 7.71 (d, 1H, J=6.6 Hz), 8.51 (dd, 1H, J=4.7 Hz, 1.6 Hz), 8.59 (d, 1H, J=4.9 Hz).

Referential Example 63

Synthesis of ethyl 2-(3,4,5-trimethoxyphenyl)benzoate:

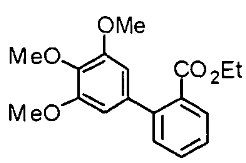

3,4,5-Trimethoxyphenylboronic acid (649 mg) and ethyl 2-bromobenzoate (479 mg) were reacted with each other in the same manner as described in Referential Example 1 to give the title compound.
Yield: 655 mg (69%).
$^1$H-NMR (400MHz, $CDCl_3$) δ: 1.04 (t, 3H, J=7.2 Hz), 3.86 (s, 6H), 3.89 (s, 3H), 4.12 (q, 2H, J=7.2 Hz), 6.54 (s, 2H), 7.40-7.42 (m, 2H), 7.51 (t, 1H, J=7.8 Hz), 7.77 (d, 1H, J=6.8 Hz).

Referential Example 64

Synthesis of 2-(3,4,5-trimethoxyphenyl)benzyl alcohol:

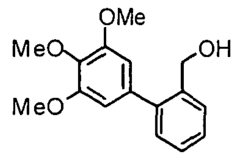

Ethyl 2-(3,4,5-trimethoxyphenyl)benzoate (655 mg) was treated in the same manner as described in Referential Example 2 to give the title compound.
Yield: 630 mg (theoretical amount).
$^1$H-NMR (400MHz, $CDCl_3$) δ: 3.85 (s, 6H), 3.90 (s, 3H), 4.61 (s, 2H), 6.61 (s, 2H), 7.26-7.39 (m, 3H), 7.53 (d, 1H, J=6.8 Hz).

Referential Example 65

Synthesis of 2-(3,4,5-trimethoxyphneyl)benzyl chloride:

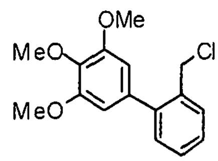

2-(3,4,5-Trimethoxyphenyl)benzyl alcohol (630 mg) was treated in the same manner as described in Referential Example 3 to give the title compound.
Yield: 615 mg (theoretical amount).
$^1$H-NMR (400MHz, $CDCl_3$) δ: 3.87 (s, 6H), 3.90 (s, 3H), 4.53 (s, 2H), 6.66 (s, 2H), 7.29-7.32 (m, 1H), 7.34-7.39 (m, 2H), 7.50-7.52 (m, 1H).

Preparation Example 22

Synthesis of 4-[N-(3,5-dimethoxyphenyl)-N-[2-(3,4,5-trimethoxyphenyl)benzyl] amino]-1-[[2-(3,4,5-trimethoxyphenyl) pyridin-4-yl]methyl]piperidine dihydrochloride:

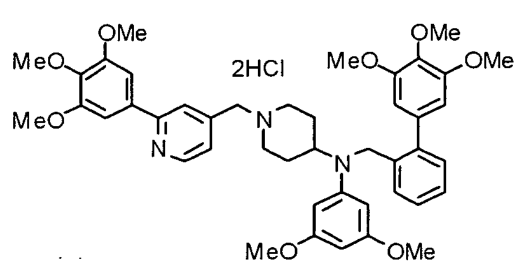

4-(3,5-Dimethoxyphenylamino)-1-[[2-(3,4,5-trimethoxyphenyl)pyridin-4-yl] methyl]piperidine (148 mg) and 2-(3,4,5-trimethoxyphenyl)benzyl chloride (114 mg) were reacted with each other in the same manner as described in Preparation Example 9. A free base obtained was converted to a dihydrochloroide to give the title compound as yellow

powder.
Yield: 20 mg, (8%).
$^1$H-NMR (400MHz, measured as a free base, $CDCl_3$) δ: 1.50-1.90 (m, 4H), 2.05-2.20 (m, 2H), 2.92 (br, 2H), 3.52 (br, 3H), 3.68 (s, 6H), 3.85 (s, 6H), 3.88 (s, 3H), 3.89 (s, 3H), 3.94 (s, 6H), 4.31 (s, 2H), 5.85 (br, 3H), 6.52 (s, 2H), 7.05-7.27 (m, 6H), 7.34 (s, 1H), 7.51(s, 1H), 8.56(s, 1H).

Preparation Example 23

Synthesis of 4-[N-(3,5-dimethoxyphenyl)-N-[[2-(3,4,5-trimethoxyphenyl)pyridin-4-yl]methyl]amino]-1-[[2-(3,4,5-trimethoxyphenyl)pyridin-4-yl]methyl]piperidine trihydrochloride:

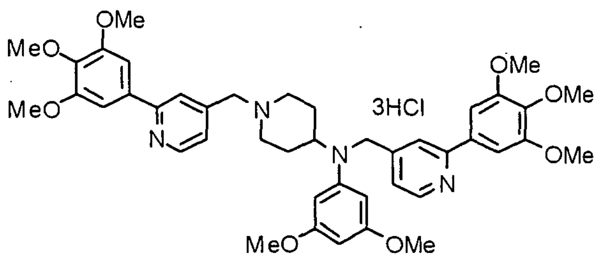

4-(3,5-Dimethoxyphenylamino)-1-[[2-(3,4,5-trimethoxyphenyl)pyridin-4-yl] methyl]piperidine (148 mg) and 4-chloromethyl-2-(3,4,5-trimethoxyphenyl)pyridine (114 mg) were reacted with each other in the same manner as described in Preparation Example 9. The resulting free base was converted to a trihydrochloride which gave the title compound as yellow powder.
Yield: 40 mg (18%).
$^1$H-NMR (400MHz, measured as a free base, $CDCl_3$) δ: 1.68-1.90 (m, 4H), 2.12-2.22 (m, 2H), 2.94-3.02 (m, 2H), 3.57 (s, 2H), 3.71 (s, 6H), 3.81-3.83 (m, 1H), 3.89 (s, 3H), 3.90 (s, 3H), 3.93 (s, 6H), 3.96 (s, 6H), 4.52 (s, 2H), 5.89-5.94 (m, 3H), 7.14 (d, 1H, J=5.3 Hz), 7.16 (s, 2H), 7.20 (d, 1H, J=3.7 Hz), 7.22 (s, 2H), 7.54-7.60 (m, 2H), 8.55 (d, 1H, J=5.1 Hz), 8.59 (d, 1H, J=5.1 Hz).

Preparation Example 24

Synthesis of 4-[N-(3,5-dimethoxyphenyl)-N-[3-(3,4,5-trimethoxyphenyl)benzyl] amino]-1-[[2-(3,4,5-trimethoxyphenyl) pyridin-4-yl]methyl]piperidine dihydrochloride:

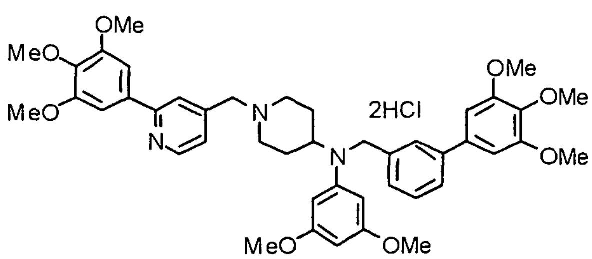

4-(3,5-Dimethoxyphenylamino)-1-[[2-(3,4,5-trimethoxyphenyl)pyridin-4-yl] methyl]piperidine (148 mg) and 3-(3,4,5-trimethoxyphenyl)benzyl chloride (114 mg) were reacted with each other in the same manner as described in Preparation Example 9. The resulting free base was converted to a dihydrochloride which gave the title compound as yellow powder.
Yield: 41 mg (16%).
$^1$H-NMR (400MHz, measured as a free base, $CDCl_3$) δ: 1.78-1.88 (m, 4H), 2.16 (t, 2H, J=10.7 Hz), 2.96 (d, 2H, J=11.3 Hz), 3.56 (s, 2H), 3.70 (s, 6H), 3.73-3.84 (m, 1H), 3.87 (s, 3H), 3.89 (s, 6H), 3.90 (s, 3H), 3.95 (s, 6H), 4.54 (s, 2H),

5.95 (s, 2H), 6.71 (s, 2H), 7.19-7.26 (m, 4H), 7.31-7.39 (m, 3H), 7.42 (s, 1H), 7.59 (s, 1H), 8.58 (d, 1H, J=4.9 Hz).

Preparation Example 25

Synthesis of 4-[N-(3,5-dimethoxyphenyl)-N-[[2-(3,4,5-trimethoxyphenyl)pyridin-5-yl]methyl]amino]-1-[[2-(3,4,5-trimethoxyphenyl)pyridin-4-yl]methyl]piperidine trihydrochloride:

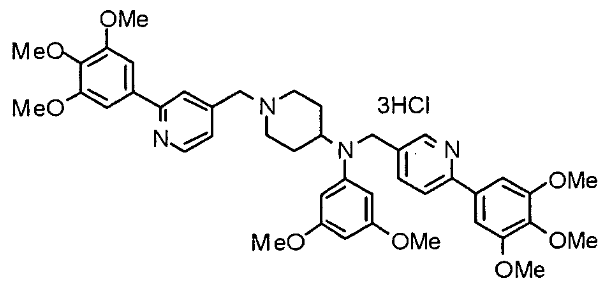

4-(3,5-Dimethoxyphenylamino)-1-[[2-(3,4,5-trimethoxyphenyl)pyridin-4-yl] methyl]piperidine (148 mg) and 5-chloromethyl-2-(3,4,5-trimethoxyphenyl)pyridine (114 mg) were reacted with each other in the same manner as described in Preparation Example 9. The resulting free base was converted to a trihydrochloride which gave the title compound as yellow powder.
Yield: 23 mg (10%).
$^1$H-NMR (400MHz, measured as a free base, $CDCl_3$) δ: 1.64 (br, 2H), 1.82 (br, 2H), 2.10 (br, 2H), 2.94 (br, 2H), 3.48-3.60 (m, 3H), 3.64 (s, 6H), 3.82 (s, 3H), 3.83 (s, 3H), 3.87 (s, 6H), 3.90 (s, 6H), 4.46 (s, 2H), 5.85 (br, 3H), 7.05-7.24 (m, 6H), 7.53-7.54 (m, 2H), 8.51 (s, 1H), 8.54 (br, 1H).

Referential Example 66

Synthesis of ethyl 4-(3,4,5-trimethoxyphenyl)benzoate:

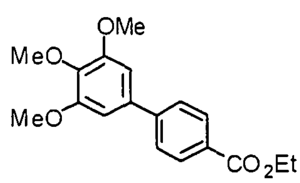

3,4,5-Trimethoxyphenylboronic acid (2.01 g) and ethyl 4-bromobenzoate (2.29 g) were reacted with each other in the same manner as described in Referential Example 1 to give the title compound.
Yield: 2.99 g (95%).
$^1$H-NMR (400MHz, $CDCl_3$) δ: 1.42 (t, 3H, J=7.2 Hz), 3.90 (s, 3H), 3.94 (s, 6H), 4.38 (q, 2H, J=7.2 Hz), 6.81 (s, 2H), 7.62 (d, 2H, J=8.2 Hz), 8.10 (d, 2H, J=8.2 Hz).

Referential Example 67

Synthesis of 4-(3,4,5-trimethoxyphenyl)benzyl alcohol:

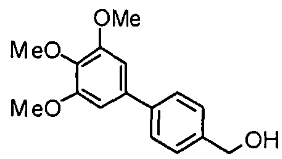

Ethyl 4-(3,4,5-trimethoxyphenyl)benzoate (2.99 g) was treated in the same manner as described in Referential Example 2 to give the title compound.
Yield: 1.83 g (71%)

Referential Example 68

Synthesis of 4-(3,4,5-trimethoxyphenyl)benzyl chloride:

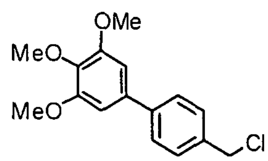

4-(3,4,5-Trimethoxyphenyl)benzyl alcohol (1.83 g) was treated in the same manner as describe in Referential Example 3 to give the title compound.
Yield: 1.65 g (84%)
$^1$H-NMR (400MHz, $CDCl_3$) δ: 3.90 (s, 3H), 3.93 (s, 6H), 4.65 (s, 2H), 6.77 (s, 2H), 7.46 (d, 2H, J=8.0 Hz), 7.55 (d, 2H, J=8.0 Hz).

Preparation Example 26

Synthesis of 4-[N-(3,5-dimethoxyphenyl)-N-[[4-(3,4,5-trimethoxypheny)benzyl] amino]-1-[[2-(3,4,5-trimethoxyphenyl) pyridin-4-yl]methyl]piperidine dihydrochloride:

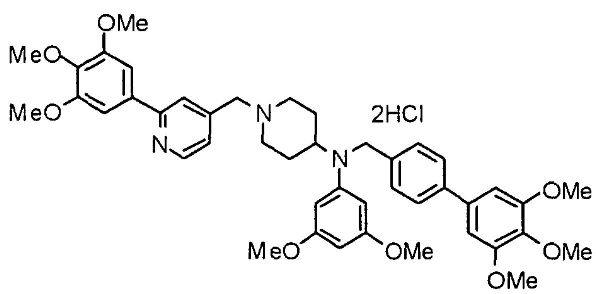

4-(3,5-Dimethoxyphenylamino)-1-[[2-(3,4,5-trimethoxyphenyl)pyridin-4-yl] methyl]piperidine (148 mg) and

4-(3,4,5-trimethoxyphenyl)benzyl chloride (114 mg) were reacted with each other in by the same manner as described in Preparation Example 9. The resulting free base was converted to a dihydrochloride which gave yellow powder of the title compound.
Yield: 35 mg (14%).
$^1$H-NMR (400 MHz, measured as a free base, $CDCl_3$) δ; 1.80-1.89 (m, 4H), 2.17 (br, 2H), 2.97 (d, 2H, J=10.5 Hz), 3.57 (s, 2H), 3.70 (s, 6H), 3.77-3.84 (m, 1H), 3.87 (s, 3H), 3.90 (s, 3H), 3.91 (s, 6H), 3.96 (s, 6H), 4.52 (s, 2H), 5.93 (s, 2H), 6.74 (s, 2H), 7.19-7.22 (m, 4H), 7.31 (d, 2H, J=8.2 Hz), 7.46 (d, 2H, J=8.2 Hz), 7.60 (s, 1H), 8.59 (d, 1H, J=5.1 Hz).

Referential Example 69

Synthesis of 4-(3,4-methylenedioxyphenylamino)-1-[[2-(3,4,5-trimethoxyphenyl) pyridin-4-yl]methyl]piperidine:

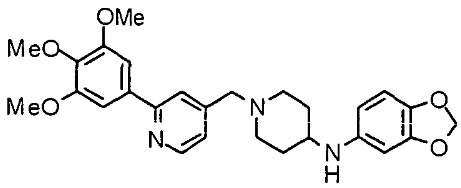

1-[[2-(3,4,5-Trimethoxyphenyl)pyridin-4-yl]methyl-4-piperidone (1.40 g) and 3,4-methylenedioxyaniline (646 mg) were reacted with each other in the same manner as described in Referential Example 37 to give the title compound.
Yield: 810 mg (43%).
$^1$H-NMR (400MHz, $CDCl_3$) δ: 1.63 (br, 2H), 2.02-2.60 (m, 4H), 2.80-3.15 (m, 2H), 3.25 (br, 1H), 3.70 (br, 2H), 3.88 (s, 3H), 3.96 (s, 6H), 5.83 (s, 2H), 6.02 (d, 1H, J=8.3 Hz), 6.22 (s, 1H), 6.61 (d, 1H, J=8.3 Hz), 7.18-7.28 (m, 3H), 7.64 (br, 1H), 8.60 (d, 1H, J=4.9 Hz).

Preparation Example 27

Synthesis of 4-[N-(3,4-methylenedioxyphenyl)-N-[[2-(3,4,5-trimethoxypheny) pyridin-3-yl]methyl]amino]-1-[[2-(3,4,5-trimethoxyphenyl)pyridin-4-yl]methyl] piperidine trihydrochloride:

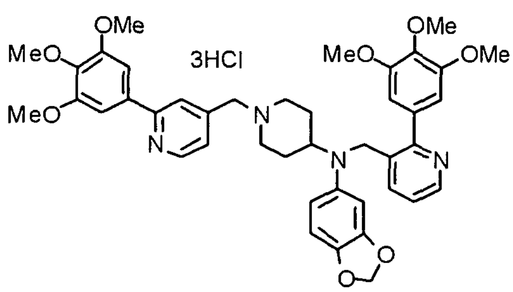

4-(3,4-Methylenedioxyphenylamino)-1-[[2-(3,4,5-trimethoxyphenyl)pyridin-4-yl]methyl]piperidine (119 mg) and 3-chloromethyl-2-(3,4,5-trimethoxyphenyl)pyridine (114 mg) were reacted with each other in the same manner as described in Preparation Example 9. The resulting free base was converted to a trihydrochloroide to give the title compound as yellow powder.
Yield: 30 mg (14%).
$^1$H-NMR (400MHz, measured as a free base, $CDCl_3$) δ: 1.45-2.25 (m, 6H), 2.90 (br, 2H), 3.40 (br, 1H), 3.55 (br, 2H), 3.87 (s, 3H), 3.88 (s, 9H), 3.93 (s, 6H), 4.28 (s, 2H), . 5.82 (s, 2H), 6.10 (br, 1H), 6.28 (s, 1H), 6.58 (d, 1H, J=8.4 Hz), 6.67 (s, 2H), 7.12-7.30 (m, 4H), 7.52 (br, 1H), 7.75 (br, 1H), 8.51 (br, 1H), 8.57 (br, 1H).

Preparation Example 28

Synthesis of 4-[N-(3,4-methylenedioxyphenyl)-N-[2-(3,4,5-trimethoxyphenyl) benzyl]amino] -1-[[2-(3,4,5-trimethoxyphenyl)pyridin-4-yl]metyl]piperidine dihydrochloride:

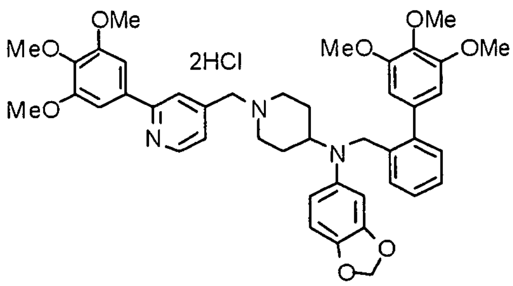

4-(3,4-Methylenedioxyphenylamino)-1-[[2-(3,4,5-trimethoxyphenyl)pyridin-4-yl]methyl]piperidine (119 mg) and 2-(3,4,5-trimethoxyphenyl)benzyl chloride (114 mg) were reacted with each other in the same manner as described in Preparation Example 9. A free base obtained was converted to a dihydrochloroide to give the title compound as yellow powder.
Yield: 13 mg (6%).
$^1$H-NMR (400MHz, measured as a free base, $CDCl_3$) δ: 1.61 (br, 2H), 1.78 (br, 2H), 2.10 (br, 2H), 2.91 (br, 2H), 3.50-3.54 (m, 3H), 3.87 (s, 6H), 3.90 (s, 3H), 3.92 (s, 3H), 3.99 (s, 6H), 4.26 (s, 2H), 5.82 (s, 2H), 6.12 (d, 1H, J=8.6 Hz), 6.32 (s, 1H), 6.53 (s, 2H), 6.62 (d, 1H, J=8.6 Hz), 7.17-7.26 (m, 6H), 7.42 (br, 1H), 7.55 (s, 1H), 8.58 (d, 1H, J=4.9 Hz).

Preparation Example 29

Synthesis of 4-[N-(3,4-methylenedioxyphenyl)-N-[[2-(3,4,5-trimethoxyphenyl) pyridin-4-yl]methyl]amino]-1-[[2-(3,4,5-trimethoxyphenyl)pyridin-4-yl]methyl] piperidine trihydrochloride:

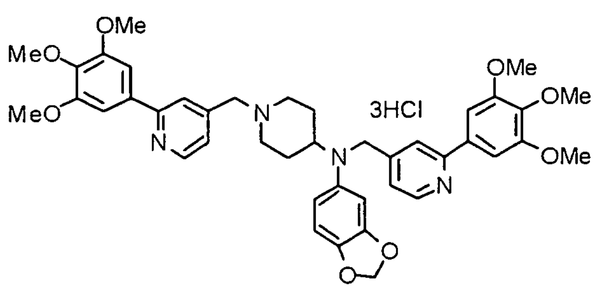

4-(3,4-Methylenedioxyphenylamino)-1-[[2-(3,4,5-trimethoxyphenyl)pyridin-4-yl]methyl]piperidine (119 mg) and 4-chloromethyl-2-(3,4,5-trimethoxyphenyl) pyridine (114 mg) were reacted with each other in the same manner as described in Preparation Example 9. The resulting free base was converted to a trihydrochloride which gave the title compound as yellow powder.
Yield: 52 mg (25%).
$^1$H-NMR (400MHz, measured as a free base, $CDCl_3$) δ: 1.60-1.95 (m, 4H), 2.20 (br, 2H), 3.00 (br, 2H), 3.58 (br, 3H), 3.86 (s, 3H), 3.87 (s, 3H), 3.91 (s, 6H), 3.94 (s, 6H), 4.41 (s, 2H), 5.82 (s, 2H), 6.17 (d, 1H, J=8.4 Hz), 6.39 (s, 1H), 6.62 (d, 1H, J=8.4 Hz), 7.12-7.13 (m, 3H), 7.18 (d, 1H, J=4.1 Hz), 7.23 (br, 2H), 7.54 (br, 2H), 8.51 (d, 1H, J=5.1 Hz), 8.57 (d, 1H, J=4.9 Hz).

Preparation Example 30

Synthesis of 4-[N-(3,4-methylenedioxyphenyl)-N-[3-(3,4,5-trimethoxyphenyl) benzyl]amino]-1-[[2-(3,4,5-trimethoxyphenyl)pyridin-4-yl]methyl]piperidine dihydrochloride:

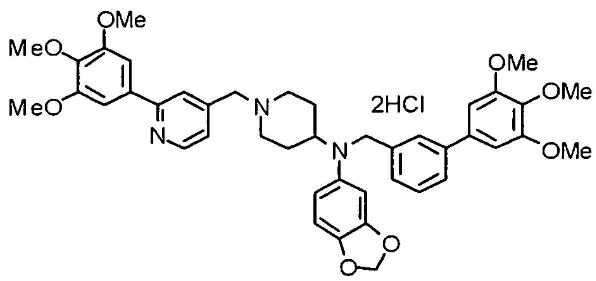

4-(3,4-Methylenedioxyphenylamino)-1-[[2-(3,4,5-trimethoxyphenyl)pyridin-4-yl]methyl]piperidine (119 mg) and 3-(3,4,5-trimethoxyphenyl)benzyl chloride (114 mg) were reacted with each other in the same manner as described in Preparation Example 9. The resulting free base was converted to a dihydrochloride which gave the title compound as yellow powder.
Yield: 58 mg (29%).
$^1$H-NMR (400MHz, measured as a free base, $CDCl_3$) δ: 1.60-1.97 (m, 4H), 2.15 (br, 2H), 3.00 (br, 2H), 3.58 (br, 3H), 3.86 (s, 3H), 3.88 (s, 9H), 3.94 (s, 6H), 4.43 (s, 2H), 5.81 (s, 2H), 6.21 (br, 1H), 6.42 (s, 1H), 6.62 (d, 1H, J=8.4 Hz), 6.69 (s, 2H), 7.18 (d, 1H, J=4.9 Hz), 7.22-7.39 (m, 6H), 7.60 (br, 1H), 8.57 (d, 1H, J=4.9 Hz).

Preparation Example 31

Synthesis of 4-[N-(3,4-methylenedioxyphenyl)-N-[[2-(3,4,5-trimethoxyphenyl) pyridin-5-yl]methyl]amino]-1-[[2-(3,4,5-trimethoxyphenyl)pyridin-4-yl]methyl] piperidine trihydrochloride:

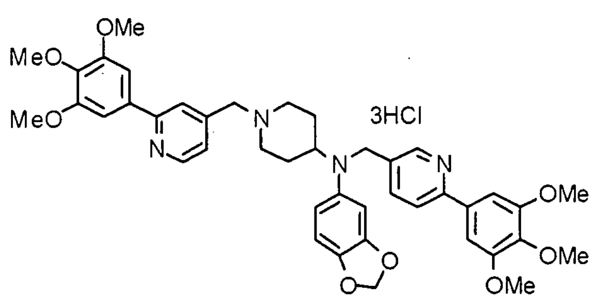

4-(3,4-Methylenedioxyphenylamino)-1-[[2-(3,4,5-trimethoxyphenyl)pyridin-4-yl]methyl]piperidine (119 mg) and 5-chloromethyl-2-(3,4,5-trimethoxyphenyl) pyridine (114 mg) were reacted with each other in the same manner as described in Preparation Example 9. The resulting free base was converted to a trihydrochloride which gave the title compound as yellow powder.
Yield: 69 mg (27%).
$^1$H-NMR (400MHz, measured as a free base, $CDCl_3$) δ: 1.71-1.88 (m, 4H), 2.14 (d, 2H, J=11.2 Hz), 2.97 (d, 2H, J=11.5 Hz), 3.45-3.52 (m, 1H), 3.56 (s, 2H), 3.89 (s, 3H), 3.90 (s, 3H), 3.94 (s, 6H), 3.96 (s, 6H), 4.12 (s, 2H), 5.85 (s, 2H), 6.24 (dd, 1H, J=8.5 Hz, 2.5 Hz), 6.45 (d, 1H, J=2.4 Hz), 6.64 (d, 1H, J=8.5 Hz), 7.20-7.21 (m, 1H), 7.21 (s, 2H), 7.23 (s, 2H), 7.58-7.65 (m, 3H), 8.57 (d, 1H, J=1.5 Hz), 8.59 (d, 1H, J=4.9 Hz).

Preparation Example 32

Synthesis of 4-[N-(3,4-methylenedioxyphenyl)-N-[4-(3,4,5-trimethoxyphenyl) benzyl]amino]-1-[[2-(3,4,5-trimethoxyphenyl)pyridin-4-yl]methyl]piperidine dihydrochloride:

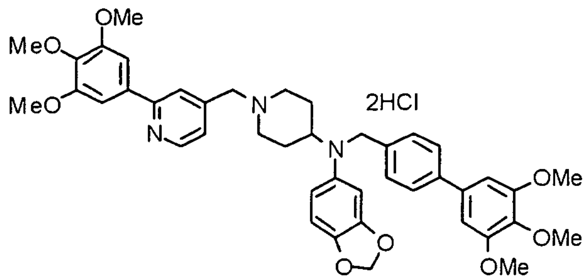

4-(3,4-Methylenedioxyphenylamino)-1-[[2-(3,4,5-trimethoxyphenyl)pyridin-4-yl]methyl]piperidine (119 mg) and 4-(3,4,5-trimethoxyphenyl)benzyl chloride (114 mg) were reacted with each other in the same manner as described in Preparation Example 9. The resulting free base was converted to a dihydrochloride which gave the title compound as yellow powder.
Yield: 29 mg (14%).
$^1$H-NMR (400 MHz, measured as a free base, $CDCl_3$) δ; 1.62-2.00 (m, 4H), 2.20 (br, 2H), 2.99 (br, 2H), 3.58 (br, 3H), 3.86 (s, 3H), 3.87 (s, 3H), 3.88 (s, 6H), 3.89 (s, 6H), 4.41 (s, 2H), 5.82 (s, 2H), 6.19 (d, 1H, J=8.6 Hz), 6.39 (s, 1H), 6.63 (d, 1H, J=8.4 Hz), 6.72 (s, 2H), 7.18 (d, 1H, J=5.1 Hz), 7.23 (s, 2H), 7.29 (d, 2H, J=8.0 Hz), 7.43 (d, 2H, J=8.2 Hz), 7.60 (br, 1H), 8.57 (d, 1H, J=4.9 Hz).

Referential Example 70

Synthesis of 4-[N-methyl-N-[(2-nitrobenzene)sulfonyl]aminomethyl]-2-(3,4,5-trimethoxyphenyl)pyridine:

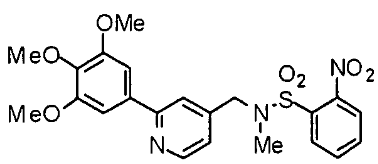

4-Chloromethyl-2-(3,4,5-trimethoxyphenyl)pyridine (232 mg), N-methyl-2-nitrobenzenesulfonamide (171mg) and potassium carbonate (138 mg) were suspended in acetonitrile (10 mL). The mixture was stirred at room temperature overnight and concentrated under reduced pressure. The residue was dissolved in chloroform, washed with saturated aqueous sodium hydrogen carbonate and saturated brine, dried over anhydrous magnesium sulfate and concentrated under reduced pressure to give the title compound.
Yield: 362 mg (97.0%).

Referential Example 71

Synthesis of 4-(methylaminomethyl)-2-(3,4,5-trimethoxyphenyl)pyridine:

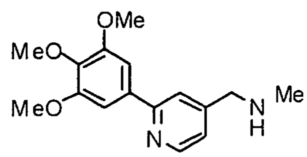

To a suspension of 4-[N-methyl-N-[(2-nitrobenzene)sulfonyl]aminomethyl]-2-(3,4,5-trimethoxyphenyl) pyridine (691 mg) and potassium carbonate (203 mg) in acetonitrile (20 mL) was added thiophenol (228μL). The mixture was stirred at 50°C overnight and concentrated under reduced pressure. The residue was dissolved in chloroform, washed with saturated aqueous sodium hydrogen carbonate and saturated brine, dried over anhydrous magnesium sulfate and concentrated under reduced pressure. The residue was purified by column chromatography on silica gel using chloroform-methanol (40:1 ~ 10:1) as eluents to give the title compound.
Yield: 356 mg (84%).

Preparation Example 33

Synthesis of 4-[N-methyl-N-[[2-(3,4,5-trimethoxyphenyl)pyridin-4-yl]methyl]aminocarbonyl]-1-[[2-(3,4,5-trimethoxyphenyl)pyridin-4-yl]methyl]piperidine maleate:

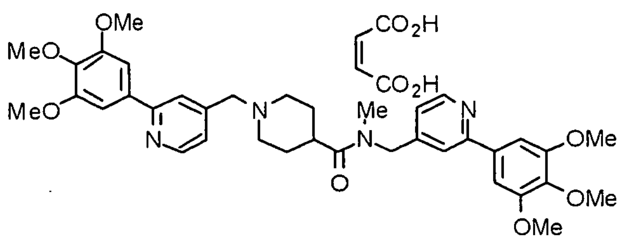

1-[[2-(3,4,5-Trimethoxyphenyl)pyridin-4-yl]methyl]piperidine-4-caroxylic acid (98 mg) and 4-(methylaminomethyl)-2-(3,4,5-trimethoxyphenyl)pyridine (73 mg) were treated in the same manner as described in Preparation Example 1 giving a maleate of the title compound.
Yield: 145 mg (75%).
$^1$H-NMR (400 MHz, measured as a maleate, DMSO-$d_6$)δ: 1.89-1.97 (m, 4H), 2.75-2.96 (m, 3H), 3.03 (s, 3H), 3.27 (d, 2H, J=12.0 Hz), 3.78 (s, 3H), 3.79 (s, 3H), 3.87 (s, 6H), 3.90 (s, 6H), 4.09 (s, 2H), 4.64 (s, 2H), 6.14 (s, 2H), 7.09 (d, 1H, J=5.0 Hz), 7.33 (s, 2H), 7.37 (d, 1H, J=5.0 Hz), 7.38 (s, 2H), 7.65 (s, 1H), 7.90 (s, 1H), 8.57 (d, 1H, J=5.0 Hz), 8.67 (d, 1H, J=5.0 Hz).

Referential Example 72

Synthesis of (3S)-1-(tert-butoxycarbonyl)-3-[N-[(2-nitrobenzene)sulfonyl]-N-[[2-(3,4,5-trimethoxyphenyl)pyridin-4-yl] methyl]amino]pyrrolidine:

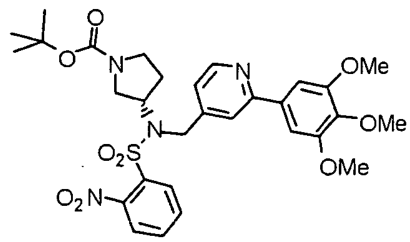

(3 S)-1-(tert-Butoxycarbonyl)-3-[(2-nitrobenzene)sulfonylamino]pyrrolidine (72 mg) and 4-chloromethyl-2-(3,4,5-trimethoxyphenyl)pyridine (57 mg) were reacted with each other in the same manner as described in Example 2 to give a colorless amorphous substance of the title compound.
Yield:103 mg (85%).

Referential Example 73

Synthesis of (3S)-3-[N-[(2-nitrobenzene)sulfonyl]-N-[[2-(3,4,5-trimethoxyphenyl)pyridin-4-yl]methyl]amino]pyrrolidine:

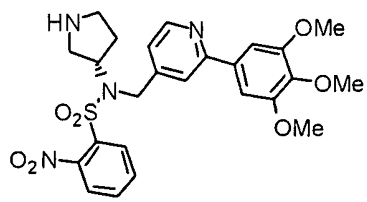

(3 S)-1-(tert-Butoxycarbonyl)-3-[N-[(2-nitrobenzene)sulfonyl]-N-[[2-(3,4, 5-trimethoxyphenyl)pyridin-4-yl]methyl]amino]pyrrolidine (103 mg) was treated in the same manner as described in Referential Example 12 to give a yellow amorphous substance of the title compound.
Yield: 72 mg (84%).
$^1$H-NMR (400 MHz, $CDCl_3$)δ: 1.66-1.75 (m, 1H), 2.03-2.05 (m, 1H), 2.78-2.85 (m, 2H), 3.00-3.10 (m, 2H), 3.39 (br, 1H), 3.90 (s, 3H), 3.96 (s, 6H), 4.59-4.67 (m, 1H), 4.70 (s, 2H), 7.13-7.18 (m, 1H), 7.20 (s, 2H), 7.52-7.64 (m, 4H), 7.95 (dd, 1H, J=7.9 Hz, 1.1 Hz), 8.52 (d, 1H, J=5.1 Hz).

Referential Example 74

Synthesis of (3S)-3-[N-[(2-nitrobenzene)sulfonyl]-N-[[2-(3,4,5-trimethoxyphenyl)pyridin-4-yl]methyl]amino]-1-[[2-(3,4,5-trimethoxyphenyl)pyridin-4-yl]methyl]pyrrolidine:

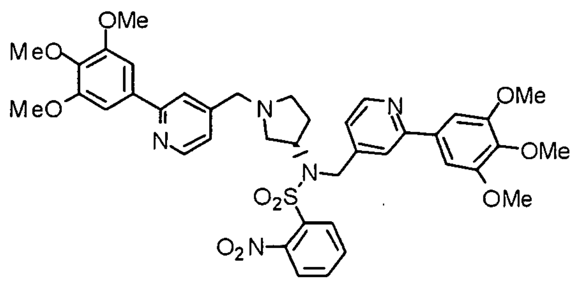

(3 S)-3-[N-[(2-Nitrobenzene)sulfonyl]-N-[[2-(3,4,5-trimethoxyphenyl)pyridin-4-yl]methyl]amino]pyrrolidine (72 mg) and 4-chloromethyl-2-(3,4,5-trimethoxyphenyl)pyridine (40 mg) were reacted with each other in the same manner as described in Preparation Example 2 to give a yellow amorphous substance of the title compound.
Yield: 97 mg (91%).
$^1$H-NMR (400 MHz, $CDCl_3$)δ: 1.59 (br, 1H), 1.80-1.90 (m, 1H), 2.20-2.30 (m, 2H), 2.55 (dd, 1H, J=10.5 Hz, 8.2 Hz), 2.78 (dd, 1H, J=10.6 Hz, 3.2 Hz), 2.87 (t, 1H, J=7.2 Hz), 3.50 (d, 1H, J=13.7 Hz), 3.64 (d, 1H, J=13.7 Hz), 3.89 (s, 3H), 3.90 (s, 3H), 3.92 (s, 6H), 3.93 (s, 6H), 4.83 (d, 2H, J=4.5 Hz), 7.07 (d, 1H, J=5.1 Hz), 7.10 (d, 1H, J=4.9 Hz), 7.15 (s, 2H), 7.17 (s, 2H), 7.41-7.45 (m, 1H), 7.50-7.55 (m, 3H), 7.61 (s, 1H), 7.81 (d, 1H, J=7.4 Hz), 8.45 (d, 1H, J=4.9 Hz), 8.51 (d, 1H, J=5.1 Hz).

Preparation Example 34

Synthesis of (3S)-1-[[2-(3,4,5-trimethoxyphenyl)pyridin-4-yl]methyl]-3-[[2-(3,4,5-trimethoxyphenyl)pyridin-4-y] methylamino]pyrrolidine trihydrochloride:

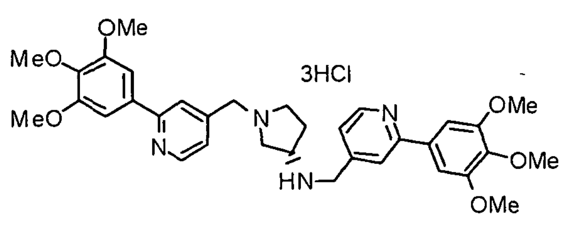

(3S)-3-[N-(2-Nitrobenzenesulfonyl-N-[[2-(3,4,5-trimethoxyphenyl)pyridin-4-yl]methyl]amino]-1-[[2-(3,4,5-trimethoxyphenyl)pyridin-4-yl]methyl]pyrrolidine (97 mg) was treated in the same manner as described in Referential Example 14. The product was converted to a trihydrochloride according to a conventional method to give yellow powder of the title compound.
Yield: 80mg (89%).
$^1$H-NMR (400 MHz, measured as a free base, $CDCl_3$)δ: 1.71 (br, 2H), 2.19-2.21 (m, 1H), 2.52-2.55 (m, 2H), 2.73-2.77 (m, 2H), 3.39 (br, 1H), 3.66 (d, 1H, J=13.7 Hz), 3.71 (d, 1H, J=13.7 Hz), 3.82 (s, 2H), 3.90 (s, 6H), 3.95 (s, 12H), 7.18-7.21 (m, 2H), 7.23 (s, 2H), 7.24 (s, 2H), 7.63 (s, 2H), 8.59 (d, 1H, J=4.3 Hz), 8.60 (d, 1H, J=4.3 Hz).

Preparation Example 35

Synthesis of 4-[3-(3,4,5-trimethoxyphenyl)benzoylamino]-1-[[2-(3,4,5-trimethoxyphenyl)pyridin-4-yl]methyl]piperidine maleate:

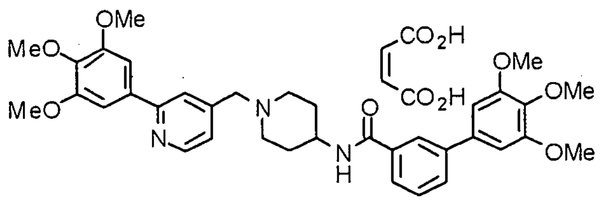

3-(3,4,5-Trimethoxyphenyl)benzoic acid (69 mg) and 4-amino-1-[[2-(3,4,5-trimethoxyphenyl)pyridin-4-yl]methyl]piperidine (114 mg) were reacted with each other in the same manner as described in Preparation Example 1. The title compound was obtained after converting the product to a maleate.
Yield: 100 mg (56%)
$^1$H-NMR (400 MHz, measured as a maleate, DMSO-$d_6$)δ: 1.85-2.10 (m, 4H), 2.77-2.93 (m, 2H), 3.20-3.31 (m, 2H), 3.77 (s, 3H, 3.79 (s, 3H), 3.89 (s, 6H), 3.91 (s, 6H), 3.98-4.07 (m, 1H), 4.13 (s, 2H), 6.15 (s, 2H), 6.94 (s, 2H), 7.40-7.52 (m, 4H), 7.73-7.80 (m, 2H), 8.02-8.10 (m, 3H), 8.67-8.68 (m, 1H).

Preparation Example 36

Synthesis of 4-[N-methyl-N-[[2-(3,4,5-trimethoxyphenyl)pyridin-4-yl]methyl]amino]-1-[[2-(3,4,5-trimethoxyphenyl) pyridin-4-yl]methyl]piperidine tetrahydrochloride:

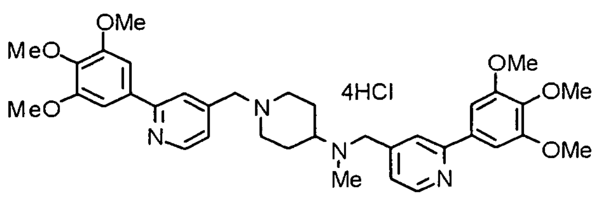

4-(Methylamino)-1-[[2-(3,4,5-trimethoxyphenyl)pyridin-4-yl]methyl]piperidine (2.67 g) and 4-chloromethyl-2-(3,4,5-trimethoxyphenyl)pyridine (2.12 g) were reacted with each other in the same manner as described in Preparation Example 2. The title compound was obtained after converting the product to a tetrahydrochloride.
Yield: 2.55 g (46%).
$^1$H-NMR (400 MHz, measured as a free base, $CDCl_3$)δ: 1.66-1.74 (m, 2H), 1.82 (d, 2H, J=10.7 Hz), 2.04 (t, 2H, J=11.0 Hz), 2.25 (s, 3H), 2.45-2.51 (m, 1H), 2.98 (d, 2H, J=11.7 Hz), 3.55 (s, 2H), 3.66 (s, 2H), 3.90 (s, 3H), 3.91 (s, 3H), 3.96 (s, 6H), 3.97 (s, 6H), 7.21-7.23 (m, 2H), 7.24 (s, 2H), 7.25 (s, 2H), 7.62 (s, 1H), 7.63 (s, 1H), 8.59 (d, 1H, J=5.1 Hz), 8.60 (d, 1H, J=5.3 Hz).

Referential Example 75

Synthesis of 1-(ethoxycarbonyl)-4-[[2-(3,4,5-trimethoxyphenyl)pyridin-4-yl]methylamino]piperidine:

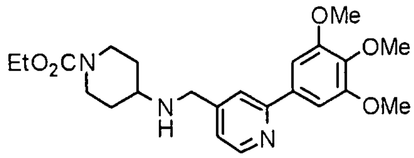

1-(Ethoxycarbonyl)piperidine (341 mg) and 4-chloromethyl-2-(3,4,5-trimethoxyphenyl)pyridine (300 mg) were reacted with each other in the same manner as described in Preparation Example 2 to give the title compound.
Yield: 438 mg (theoretical yield).
$^1$H-NMR (400 MHz, $CDCl_3$)δ: 1.25 (t, 3H, J=7.1 Hz), 1.27-1.34 (m, 2H), 1.60 (br, 1H), 1.90 (d, 2H, J=10.9 Hz), 2.67-2.72 (m, 1H), 2.87 (t, 2H, J=11.5 Hz), 3.90 (s, 3H), 3.91 (br, 2H), 3.96 (s, 6H), 4.09 (br, 2H), 4.12 (q, 2H, J=7.0 Hz), 7.21 (d, 1H, J=3.5 Hz), 7.24 (s, 2H), 7.65 (s, 1H), 8.59 (d, 1H, J=4.9 Hz).

Referential Example 76

Synthesis of 1-(ethoxycarbonyl)-4-[N-methyl-N-[[2-(3,4,5-trimethoxyphenyl)pyridin-4-yl]methyl]amino]piperidine:

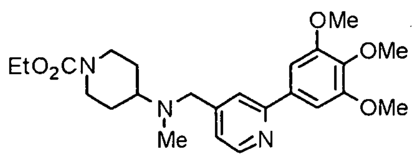

1-(Ethoxycarbonyl)-4-[[2-(3,4,5-trimethoxyphenyl)pyridin-4-yl]methylamino]piperidine (438 mg) was treated in the same manner as described in Referential Example 11 to give the title compound as a yellow oil.
Yeld: 235mg (52%).
$^1$H-NMR (400 MHz, $CDCl_3$)δ: 1.26 (t, 3H, J=7.1 Hz), 1.42-1.57 (m, 2H), 1.82 (d, 2H, J=11.9 Hz), 2.24 (s, 3H), 2.59-2.65 (m, 1H), 2.75 (t, 2H, J=12.0 Hz), 3.65 (s, 2H), 3.90 (s, 3H), 3.97 (s, 6H), 4.13 (q, 2H, J=7.0 Hz), 4.23 (br, 2H), 7,22 (dd, 1H, J=5.0 Hz, 1.3 Hz), 7.24 (s, 2H), 7.63 (s, 1H), 8.59 (d, 1H, J=4.5 Hz).

Referential Example 77

Synthesis of 4-[N-methyl-N-[[2-(3,4,5-trimethoxyphenyl)pyridin-4-yl]methyl]amino]piperidine:

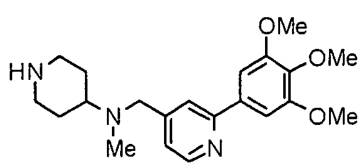

To a solution of 1-(ethoxycarbonyl)-4-[N-methyl-N-[[2-(3,4,5-trimethoxyphenyl)pyridin-4-yl]methyl]amino]piperidine (100 mg) in ethanol (2 mL) was added 4 M sodium hydroxide (8 mL). The mixture was stirred at 110°C overnight and extracted with chloroform. The extract was washed with water and saturated brine, dried over anhydrous sodium sulfate and concentrated under reduced pressure. The residue was purified by column chromatography on silica gel

using chloroform-methanol (20:1) to give the title compound as a yellow oil.
Yeld: 73 mg (88%).
$^1$H-NMR (400 MHz, $CDCl_3$)δ: 1.50-1.55 (m, 2H), 1.84 (d, 2H, J=12.0 Hz), 1.99 (br, 1H), 2.25 (s, 3H), 2.55-2.63 (m, 3H), 3.16 (d, 2H, J=12.2 Hz), 3.65 (s, 2H), 3.90 (s, 3H), 3.97 (s, 6H), 7.22 (d, 1H, J=6.1 Hz), 7.24 (s, 2H), 7.64 (s, 1H), 8.58 (d, 1H, J=5.1 Hz).

Preparation Example 37

Synthesis of 4-[N-methyl-N-[[2-(3,4,5-trimethoxyphenyl)pyridin-4-yl]methyl]amino]-1-[[2-(3,4,5-trimethoxyphenyl) pyridin-4-yl]methyl]piperidine tetrahydrochloride:

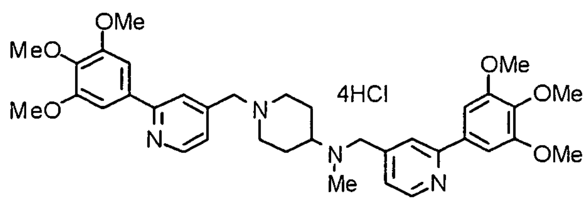

4-[N-Methyl-N-[[2-(3,4,5-trimethoxyphenyl)pyridin-4-yl]methyl]amino]piperidine (73 mg) and 4-chloromethyl-2-(3,4,5-trimethoxyphenyl)pyridine (58 mg) were reacted with each other in the same manner as described in Preparation Example 2. The title compound was obtained after converting the product to a tetrahydrochloride.
Yield: 126 mg (84%).

Preparation Example 38

Synthesis of 4-[N-methyl-N-[3-(3,4,5-trimethoxyphenyl)benzyl]amino]-1-[[2-(3,4,5-trimethoxyphenyl)pyridin-4-yl] methyl]piperidine difumarate:

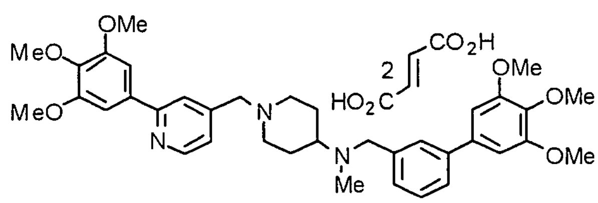

4-(Methylamino)-1-[[2-(3,4,5-trimethoxyphenyl)pyridin-4-yl]methyl]piperidine (111 mg) and 3-(3,4,5-trimethoxyphenyl)benzyl chloride (88 mg) were reacted with each other in the same manner as described in Preparation Example 2. The title compound was obtained as white powder after converting the product to a difumarate.
Yield: 59 mg (23%).
$^1$H-NMR (400 MHz, measured as a free base, $CDCl_3$)δ: 1.70-1.77 (m, 2H), 1.85-1.87 (m, 2H), 2.03-2.08 (m, 2H), 2.27 (s, 3H), 2.55-2.59 (m, 1H), 2.98 (d, 2H, J=11.3 Hz), 3.56 (s, 2H), 3.69 (s, 2H), 3.89 (s, 3H), 3.90 (s, 3H), 3.93 (s, 6H), 3.98 (s, 6H), 6.79 (s, 2H), 7.22 (d, 1H, J=4.9 Hz), 7.28 (s, 2H), 7.31 (d, 1H, J=7.6 Hz), 7.38 (t, 1H, J=7.4 Hz), 7.45 (d, 1H, J=7.6 Hz), 7.51 (s, 1H), 7.63 (s, 1H), 8.60 (d, 1H, J=5.1 Hz).

Preparation Example 39

Synthesis of 1-[[2-(4-hydroxy-3,5-dimethoxyphenyl)pyridin-4-yl]methyl]-4-[N-[[2-(4-hydroxy-3,5-dimethoxyphenyl) pyridin-4-yl]methyl]-N-methylamino]piperidine tetrahydrochloride:

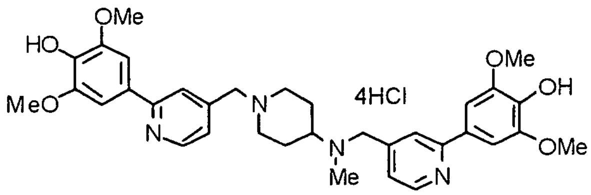

To a solution of 4-[N-methyl-N-[[2-(3,4,5-trimethoxyphenyl)pyridin-4-yl]methyl]amino]-N-[[2-(3,4,5-trimethoxyphenyl)pyridin-4 -yl]methyl]piperidine (100 mg) in dichloromethane (5 mL) was added iodotrimethylsilane (173 μL) at 0°C. The mixture was stirred at 0°C for 2 hours and then at room temperature overnight. A small amount of water, ethyl acetate and saturated aqueous sodium hydrogencarbonate were added to the mixture at 0°C and the organic layer was separated. The organic layer was washed with saturated brine, dried over magnesium sulfate and concentrated under reduced pressure. The residue was purified by preparative TLC on silica gel using chloroform-ammonia saturated methanol (15:1) and was converted to a tetrahydrochloride by the conventional method to give the title compound.
Yield: 50 mg (52.3%).
$^1$H-NMR (400 MHz, measured as a free base, $CDCl_3$)δ: 1.68-1.89 (m, 4H), 2.03-2.12 (m, 2H), 2.26 (s, 3H), 2.48-2.60 (m, 1H), 2.98-3.05 (m, 2H), 3.57 (s, 2H), 3.65 (s, 2H), 3.94 (s, 6H), 3.95 (s, 6H), 7.16-7.19 (m, 2H), 7.26 (s, 2H), 7.27 (s, 2H), 7.62-7.68 (m, 2H), 8.56 (d, 1H, J=5.3 Hz), 8.58 (d, 1H, J=5.2 Hz).

Referential Example 78

Synthesis of 1-(ethoxycarbonyl)-4-[N-ethyl-N-[[2-(3,4,5-trimethoxyphenyl)pyridin-4-yl]methyl]amino]piperidine:

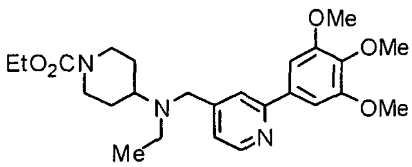

To a solution of 1-(ethoxycarbonyl)-4-[[2-(3,4,5-trimethoxyphenyl)pyridin-4-yl]methylamino]piperidine (400 mg) in acetonitrile (5 mL) was added potassium carbonate (13 mg) and iodoethane (145 mg). The mixture was placed in a sealed vessel and stirred at 80°C for 2 hours. After concentrating the reaction liquid in vacuo, the residue was incorporated with ethyl acetate washed with water and saturated brine, dried over sodium sulfate and concentrated under reduced pressure. The residue was subjected to column chromatography on silica gel using chloroform-methanol (30:1) as an eluent to give the title compound as a yellow oil.
Yield: 242 mg (57%).
$^1$H-NMR (400 MHz, $CDCl_3$)δ: 1.04 (t, 3H, J=7.1 Hz), 1.25 (t, 3H, J=7.1 Hz), 1.43-1.52 (m, 2H), 1.79 (d, 2H, J=11.5 Hz), 2.60 (q, 2H, J=7.0 Hz), 2.66-2.76 (m, 3H), 3.70 (s, 2H), 3.90 (s, 3H), 3.97 (s, 6H), 4.12 (q, 2H, J=7.0 Hz), 4.20 (br, 2H), 7.23 (s, 2H), 7.26 (d, 1H, J=5.7 Hz), 7.67 (s, 1H), 8.58 (d, 1H, J= 4.9 Hz).

Referential Example 79

Synthesis of 4-[N-ethyl-N-[[2-(3,4,5-trimethoxyphenyl)pyridin-4-yl]methyl]amino]piperidine:

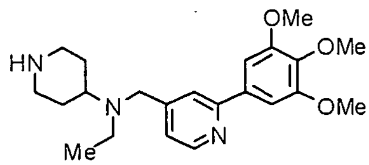

1-(Ethoxycarbonyl)-4-[N-ethyl-N-[[2-(3,4,5-trimethoxyphenyl)pyridin-4-yl] methyl]amino]piperidine (242 mg) was treated in the same manner as described in Referential Example 77 to give the title compound as a yellow oil.
Yield: 150 mg (74%).
$^1$H-NMR (400 MHz, $CDCl_3$)δ: 1.02 (t, 3H, J=7.0 Hz), 1.43-1.52 (m, 2H), 1.70 (br, 1H), 1.79 (d, 2H, J=12.3 Hz), 2.53-2.67 (m, 5H), 3.13 (d, 2H, J=11.9 Hz), 3.71 (s, 2H), 3.90 (s, 3H), 3.97 (s, 6H), 7.24 (s, 2H), 7.27 (d, 1H, J=5.1 Hz), 7.68 (s, 1H), 8.57 (d, 1H, J= 4.3 Hz).

Preparation Example 40

Synthesis of 4-[N-ethyl-N-[[2-(3,4,5-trimethoxyphenyl)pyridin-4-yl]methyl]amino]-1-[[2-(3,4,5-trimethoxyphenyl) pyridin-4-yl]methyl]piperidine tetrahydrochloride:

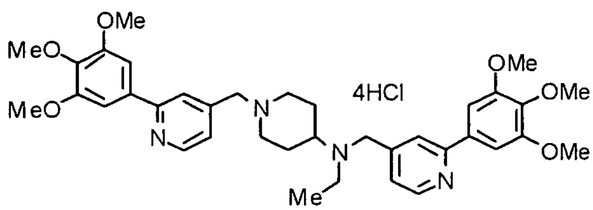

4-[N-Ethyl-N-[[2-(3,4,5-trimethoxyphenyl)pyridin-4-yl]methyl]amino]piperidine (65 mg) was reacted with 4-chloromethyl-2-(3,4,5-trimethoxyphenyl)pyridine (50 mg) in the same manner as described in Preparation Example 2. The title compound was obtained after converting the product to a tetrahydrochloride.
Yield: 121 mg (90%).
$^1$H-NMR (400 MHz, measured as a free base, $CDCl_3$)δ: 1.03 (t, 3H, J=7.1 Hz), 1.64-1.69 (m, 2H), 1.77 (d, 2H, J=10.7 Hz), 2.01 (t, 2H, J=10.8 Hz), 2.55-2.64 (m, 3H), 2.95 (d, 2H, J=11.1 Hz), 3.53 (s, 2H), 3.71 (s, 2H), 3.90 (s, 6H), 3.97 (s, 12H), 7.20-7.27 (m, 6H), 7.60 (s, 1H), 7.68 (s, 1H), 8.57 (d, 1H, J= 4.9 Hz), 8.59 (d, 1H, J= 5.1 Hz).

Referential Example 80

Synthesis of 4-(cyclohexylamino)-1-[[2-(3,4,5-trimethoxyphenyl)pyridin-4-yl]methyl]piperidine:

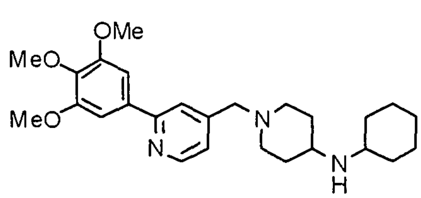

1-[[2-(3,4,5-Trimethoxyphenyl)pyridin-4-yl]methyl]-4-pieridone (400 mg) and cyclohexylamine (134 mg) were reacted with each other in the same manner as described in Referential Example 37 to give the title compound.
Yield: 342 mg (69%).
$^1$H-NMR (400 MHz, $CDCl_3$) δ: 1.05-1.30 (m, 6H), 1.38-1.52 (m, 2H), 1.53-1.80 (m, 3H), 1.87 (br, 4H), 2.07 (t, 2H, J=10.7 Hz), 2.59(br, 2H), 2.86 (br, 2H), 3.54 (s, 2H), 3.90 (s, 3H), 3.97 (s, 6H), 7.19 (d, 1H, J=4.9 Hz), 7.24 (s, 2H), 7.64 (s, 1H), 8.58 (d, 1H, J=4.9 Hz).

Preparation Example 41

Synthesis of 4-[N-cyclohexyl-N-[[2-(3,4,5-trimethoxyphenyl)pyridin-4-yl]methyl]amino]-1-[[2-(3,4,5-trimethoxyphenyl) pyridin-4-yl]methyl]piperidine tetrahydrochloride:

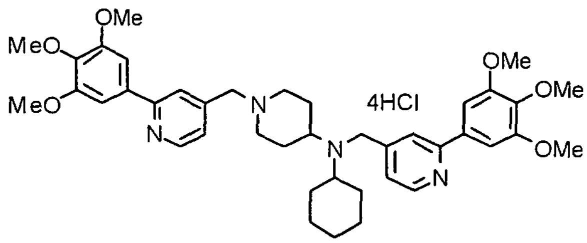

4-(Cyclohexylamino)-1-[[2-(3,4,5-trimethoxyphenyl)pyridin-4-yl]methyl] piperidine (342 mg) and 4-chloromethyl-2-(3,4,5-trimethoxyphenyl)pyridine (252 mg) were reacted with each otherin the same manner as described in Preparation Example 9. The title compound was obtained after converting the product to a tetrahydrochloride.
Yield: 55 mg (8%).
$^1$H-NMR (400 MHz, measured as a free base, $CDCl_3$)δ: 1.00-1.39 (m, 6H), 1.58-1.88 (m, 8H), 2.07 (br, 2H), 2.61 (br, 2H), 2.96 (br, 2H), 3.57 (br, 2H), 3.85 (s, 2H), 3.90 (s, 3H), 3.91 (s, 3H), 3.97 (s, 12H), 7.19-7.28 (m, 6H), 7.70 (br, 2H), 8.56 (d, 1H, J=5.1 Hz), 8.60 (d, 1H, J=5.1 Hz).

Referential Example 81

Synthesis of 4-anilino-1-[[2-(3,4,5-trimethoxyphenyl)pyridin-4-yl]methyl]piperidine:

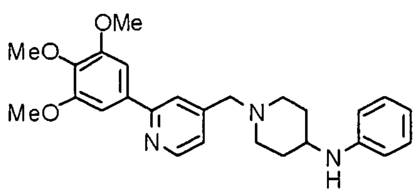

1-[[2-(3,4,5-Trimethoxyphenyl)pyridin-4-yl]methyl]-4-pieridone (1.1 g) and aniline (344 mg) were reacted with each other in the same manner as described in Referential Example 37 to give the title compound.
Yield: 1.09 g (81%).
$^1$H-NMR (400 MHz, $CDCl_3$)δ: 1.53 (br, 2H), 2.02-2.13 (m, 2H), 2.16-2.32 (m, 2H), 2.86 (br, 2H), 3.32 (br, 1H), 3.59 (s, 2H), 3.88 (s, 3H), 3.95 (s, 6H), 6.57 (d, 2H, J=8.6 Hz), 6.66 (t, 1H, J=7.3 Hz), 7.14 (t, 2H, J=7.9 Hz), 7.20-7.24 (m, 5H), 7.65 (br, 1H), 8.59 (d, 1H, J=5.1 Hz).

Preparation Example 42

Synthesis of 4-[N-phenyl-N-[[2-(3,4,5-trimethoxyphenyl)pyridin-4-yl]methyl]amino]-1-[[2-(3,4,5-trimethoxyphenyl) pyridin-4-yl]methyl]piperidine trihydrochloride:

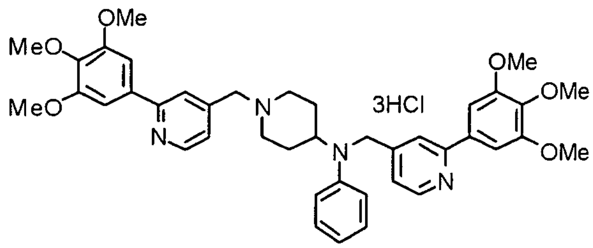

4-Anilino-1-[[2-(3,4,5-trimethoxyphenyl)pyridin-4-yl]methyl]piperidine (1.64 g) and 4-chloromethyl-2-(3,4,5-trimethoxyphenyl)pyridine (1.33 g) were reacted with each other in the same manner as described in Preparation Example 9. The title compound was obtained after converting the product to a trihydrochloride.
Yield: 635 mg (20%).
$^1$H-NMR (400 MHz, measured as a free base, $CDCl_3$)δ: 1.60-2.00 (m, 4H), 2.10-2.35 (m, 2H), 2.99 (br, 2H), 3.58 (br, 3H), 3.86 (s, 3H), 3.88 (s, 3H), 3.90 (s, 6H), 3.94 (s, 6H), 4.52 (s, 2H), 6.66-6.78 (m, 3H), 7.13-7.28 (m, 8H), 7.54 (br, 2H), 8.53 (d, 1H, J=5.1 Hz), 8.58 (d, 1H, J=4.9 Hz).

Referential Example 82

Synthesis of 1-[[2-(4-chloro-3,5-dimethoxyphenyl)pyridin-4-yl]methyl]-4-piperidone ethylene ketal:

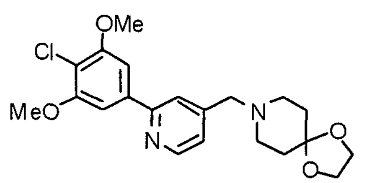

4-Piperidone ethylene ketal (573 mg) was reacted with 2-(4-chloro-3,5-dimetoxyphenyl)-4-chloromethylpyridine (1.19 g) in the same manner as described in Preparation Example 2 to give the title compound.
Yield: 1.67 g (theoretical amount).
$^1$H-NMR (400 MHz, $CDCl_3$)δ: 1.78 (t, 4H, J=5.6 Hz), 2.58 (br, 4H), 3.61 (s, 2H), 3.67 (s, 4H), 4.02 (s, 6H), 7.25-7.29 (m, 3H), 7.68 (s, 1H), 8.61 (d, 1H, J=4.9 Hz).

Referential Example 83

Synthesis of 1-[[2-(4-chloro-3,5-dimethoxyphenyl)pyridin-4-yl]methyl]-4-piperidone:

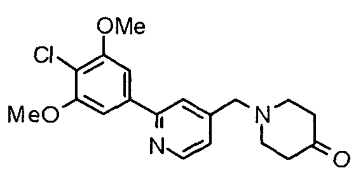

1-[[2-(4-Chloro-3,5-dimethoxyphenyl)pyridin-4-yl]methyl]-4-piperidone ethylene ketal (1.67 g) was treated in the same manner as described in Referential Example 23 to give the title compound.
Yield: 1.29 g (89%).
$^1$H-NMR (400 MHz, $CDCl_3$) δ: 2.50 (t, 4H, J=5.8 Hz), 2.81 (t, 4H, J=5.8 Hz), 3.71 (s, 2H), 4.02 (s, 6H), 7.26 (s, 2H), 7.33 (d, 1H, J=4.3 Hz), 7.70 (s, 1H), 8.66 (d, 1H, J=4.9 Hz).

Referential Example 84

Synthesis of 4-anilino-1-[[2-(4-chloro-3,5-dimethoxyphenyl)pyridin-4-yl]methyl]piperidine:

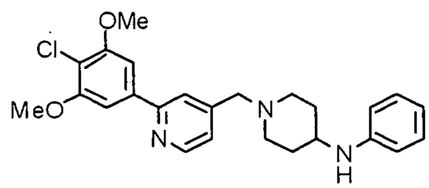

1-[[2-(4-Chloro-3,5-dimethoxyphenyl)pyridin-4-yl]methyl]-4-piperidone (600 mg) and aniline (0.18 mL) were treated in the same manner as described in Referential Example 37 to give the title compound.
Yield: 465 mg (63%).
$^1$H-NMR (400 MHz, $CDCl_3$)δ: 1.49-1.69 (m, 2H), 2.08 (d, 2H, J=7.8 Hz), 2.23 (t, 2H, J=9.3 Hz), 2.87 (d, 2H, J=7.8 Hz), 3.34 (br, 1H), 3.60 (s, 2H), 4.02 (s, 6H), 6.60 (d, 2H, J=7.6 Hz), 6.69 (t, 1H, J=7.3 Hz), 7.10-7.20 (m, 2H), 7.20-7.30 (m, 3H), 7.67 (s, 1H), 8.62 (d, 1H, J=5.2 Hz).

Preparation Example 43

Synthesis of 1-[[2-(4-chloro-3,5-dimethoxyphenyl)pyridin-4-yl]methyl]-4-[N-[[2-(4-chloro-3,5-dimethoxyphenyl)pyridin-4-yl]methyl]-N-phenylamino] piperidine trihydrochloride:

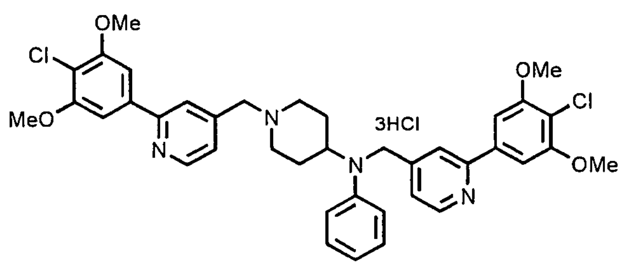

4-Anilino-1-[[2-(4-chloro-3,5-dimethoxyphenyl)pyridin-4-yl]methyl]piperidine (230 mg) was reacted with 2-(4-chloro-3,5-dimethoxyphenyl)-4-chloromethylpyridine (157 mg) in the same manner as described in Preparation Example 9. The title compound was obtained as a yellow powder after converting the product to a trihydrochloride.
Yield: 104 mg (24%).
$^1$H-NMR (400 MHz, measured as a free base, $CDCl_3$)δ: 1.70-1.85 (m, 4H), 2.20 (t, 2H, J=2.3 Hz), 3.00 (d, 2H, J=1.3 Hz), 3.59 (s, 2H), 3.96 (s, 6H), 4.00 (s, 6H), 4.56 (s, 2H), 6.65-6.78 (m, 3H), 7.16 (s, 2H), 7.18-7.28 (m, 6H), 7.59 (s, 1H), 7.62 (s,1H), 8.57 (d, 1H, J=5.1 Hz), 8.57 (d, 1H, J=4.8 Hz).

Referential Example 85

Synthesis of 4-(*p*-anisidino)-1-[[2-(4-chloro-3,5-dimethoxyphenyl)pyridin-4-yl]methyl]piperidine:

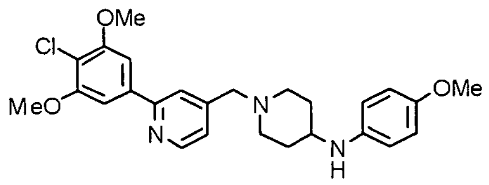

1-[[2-(4-Chloro-3,5-dimethoxyphenyl)pyridin-4-yl]methyl]-4-piperidone (690 mg) and *p*-anisidine (283 mg) were treated in the same manner as described in Referential Example 37 to give the title compound.
Yield: 646 mg (72%).
$^1$H-NMR (400 MHz, $CDCl_3$)δ: 1.45-1.55 (m, 2H), 2.05 (d, 2H, J=11.7 Hz), 2.20 (t, 2H, J=11.2 Hz), 2.87 (d, 2H, J=11.7 Hz), 3.20-3.35 (m, 1H), 3.59 (s, 2H), 3.74 (s, 3H), 4.02 (s, 6H), 6.58 (d, 2H, J=8.7 Hz), 6.77 (d, 2H, J=8.7 Hz), 7.25-7.28 (m, 3H), 7.67 (s, 1H), 8.62 (d, 1H, J=4.9 Hz).

Preparation Example 44

Synthesis of 1-[[2-(4-chloro-3,5-dimethoxyphenyl)pyridin-4-yl]methyl]-4-[N-[[2-(4-chloro-3,5-dimethoxyphenyl)pyridin-4-yl]methyl]-N-(4-methoxyphenyl)amino] piperidine trihydrochloride:

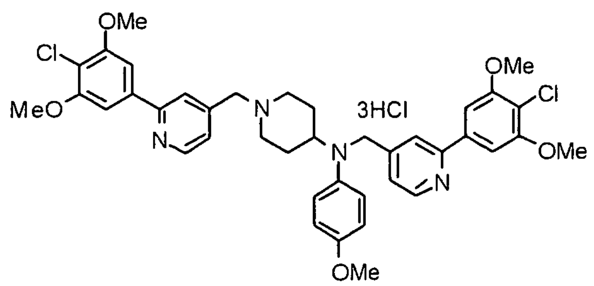

4-(*p*-Anisidino)-1-[[2-(4-chloro-3,5-dimethoxyphenyl)pyridin-4-yl]methyl]piperidine (271 mg) was reacted with 2-(4-chloro-3,5-dimethoxyphenyl)-4-chloromethylpyridine (173 mg) in the same manner as described in Preparation Example 9. The title compound was obtained as a yellow powder after converting the product to a trihydrochloride.
Yield: 324 mg (67%).
$^1$H-NMR (400 MHz, measured as a free base, $CDCl_3$)δ: 1.65-1.90 (m, 4H), 2.16 (t, 2H, J=10.4 Hz), 2.97 (d, 2H, J=7.5 Hz), 3.54-3.60 (m, 1H), 3.58 (s, 2H), 3.73 (s, 3H), 3.97 (s, 6H), 4.00 (s, 6H), 4.46 (s, 2H), 6.74 (d, 2H, J=9.4 Hz), 6.79 (d, 2H, J=9.4 Hz), 7.16 (s, 2H), 7.20-7.29 (m, 4H), 7.59 (s, 1H), 7.62 (s, 1H), 8.56 (d, 1H, J=4.8 Hz), 8.60 (d, 1H, J=4.8 Hz).

Referential Example 86

Synthesis of 4-(3-methylthioanilino)-1-[[2-(3,4,5-trimethoxyphenyl)pyridin-4-yl]methyl]piperidine:

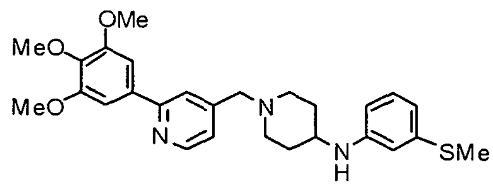

1-[[2-(3,4,5-Trimethoxyphenyl)pyridin-4-yl]methyl]-4-piperidone (1.40 g) and 3-methylthioaniline (655 mg) were treated in the same manner as described in Referential Example 37 to give the title compound.
Yield: 1.01 g (54%).
$^1$H-NMR (400 MHz, $CDCl_3$)δ: 1.44-1.60 (m, 2H), 1.98-2.10 (m, 2H), 2.23 (br, 2H), 2.42 (s, 3H), 2.88 (br, 2H), 3.30 (br, 1H), 3.59 (s, 2H), 3.88 (s, 3H), 3.95 (s, 6H), 6.35 (d, 1H, J=7.6 Hz), 6.47 (s, 1H), 6.55 (d, 1H, J=8.6 Hz), 7.05 (t, 1H, J=7.9 Hz), 7.20 (d, 1H, J=4.9 Hz), 7.24 (s, 2H), 7.68 (br, 1H), 8.58 (d, 1H, J=4.9 Hz).

Preparation Example 45

Synthesis of 4-[N-(3-methylthiophenyl)-N-[[2-(3,4,5-trimethoxyphenyl)pyridin-3-yl]methyl]amino]-1-[[2-(3,4,5-trimethoxyphenyl)pyridin-4-yl]methyl]piperidine trihydrochloride:

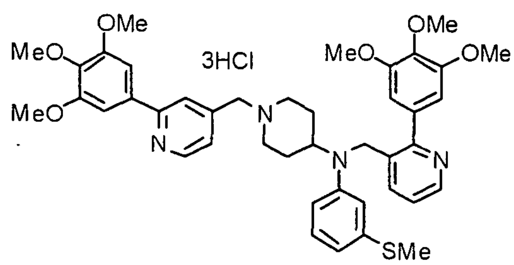

4-(3-Methylthioanilino)-1-[[2-(3,4,5-trimethoxyphenyl)pyridin-4-yl]methyl]piperidine (143 mg) was reacted with 3-chloromethyl-2-(3,4,5-trimethoxyphenyl)pyridine (114 mg) in the same manner as described in Preparation Example 9. The title compound was obtained as a yellow powder after converting the product to a trihydrochloride.
Yield: 45 mg (18%).
$^1$H-NMR (400 MHz, measured as a free base, $CDCl_3$)δ: 1.58-1.71 (s, 2H), 1.79 (d, 2H, J=10.7 Hz), 2.16 (t, 2H, J=11.2 Hz), 2.38 (s, 3H), 2.96 (d, 2H, J=11.2 Hz), 3.56 (s, 3H), 3.68-3.97 (m, 1H), 3.90 (s, 3H), 3.92 (s, 9H), 3.96 (s, 9H), 4.42 (s, 2H), 6.45 (d, 1H, J=8.3 Hz), 6.52 (s, 1H), 6.61 (d, 1H, J=7.3 Hz), 6.74 (s, 2H), 7.11 (t, 1H, J=8.1 Hz), 7.15-7.26 (m, 4H), 7.54 (s, 1H), 7.68 (d, 1H, J=7.8 Hz), 8.53 (d, 1H, J=3.2 Hz), 8.59 (d, 1H, J=4.8 Hz).

Preparation Example 46

Synthesis of 4-[N-(3-methylthiophenyl)-N-[2-(3,4,5-trimethoxyphenyl)benzyl]amino]-1-[[2-(3,4,5-trimethoxyphenyl) pyridin-4-yl]methyl]piperidine dihydrochloride:

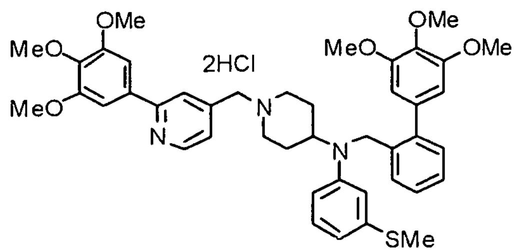

4-(3-Methylthioanilino)-1-[[2-(3,4,5-trimethoxyphenyl)pyridin-4-yl]methyl]piperidine (143 mg) was reacted with 2-(3,4,5-trimethoxyphenyl)benzyl chloride (114 mg) in the same manner as described in Preparation Example 9. The title compound was obtained as a yellow powder after converting the product to a dihydrochloride.
Yield: 51 mg (23%).
$^1$H-NMR (400 MHz, measured as a free base, $CDCl_3$)δ: 1.56-1.73 (m, 2H), 1.78-1.87 (m, 2H), 2.10-2.20 (m, 2H), 2.38 (s, 3H), 2.91-2.98 (m, 2H), 3.55 (s, 2H), 3.70-3.80 (m, 1H), 3.88 (s, 6H), 3.90 (s, 3H), 3.92 (s, 3H), 3.96 (s, 6H), 4.35 (s, 2H), 6.47 (d, 1H, J=8.2 Hz), 6.53-6.62 (m, 5H), 7.09 (t, 1H, J=8.0 Hz), 7.18-7.40 (m, 6H), 7.54 (s, 1H), 8.58 (d, 1H, J=4.7 Hz).

Preparation Example 47

Synthesis of 4-[N-(3-methylthiophenyl)-N-[[2-(3,4,5-trimethoxyphenyl)pyridin-4-yl]methyl]amino]-1-[[2-(3,4,5-trimethoxyphenyl)pyridin-4-yl]methyl]piperidine fumarate:

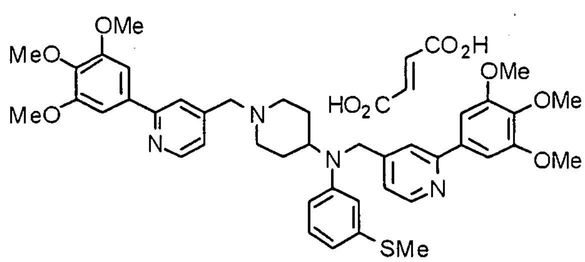

4-(3-Methylthioanilino)-1-[[2-(3,4,5-trimethoxyphenyl)pyridin-4-yl]methyl]piperidine (143 mg) was reacted with 4-chloromethyl-2-(3,4,5-trimethoxyphenyl)pyridine (114 mg) in the same manner as described in Preparation Example 9. The title compound was obtained as a white powder after converting the product to a fumarate.
Yield: 14 mg (5%).
$^1$H-NMR (400 MHz, measured as a free base, $CDCl_3$)δ: 1.76-1.86 (m, 5H), 2.17-2.23 (m, 2H), 2.39 (s, 3H), 2.97-3.00 (m, 2H), 3.58 (s, 2H), 3.89 (s, 3H), 3.90 (s, 3H), 3.93 (s, 6H), 3.96 (s, 6H), 4.54 (s, 2H), 6.47-6.50 (m, 1H), 6.63 (s, 1H), 6.64 (s, 1H), 7.10-7.15 (m, 2H), 7.15 (s, 2H), 7.20-7.21 (m, 1H), 7.22 (s, 2H), 7.55 (s, 1H), 7.59 (s, 1H), 8.56 (d, 1H, J=5.1 Hz), 8.59 (d, 1H, J=5.1 Hz).

Preparation Example 48

Synthesis of 4-[N-(3-methylthiophenyl)-N-[3-(3,4,5-trimethoxyphenyl)benzyl]amino]-1-[[2-(3,4,5-trimethoxyphenyl) pyridin-4-yl]methyl]piperidine dihydrochloride:

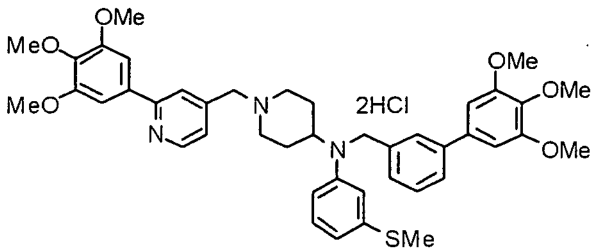

4-(3-Methylthioanilino)-1-[[2-(3,4,5-trimethoxyphenyl)pyridin-4-yl]methyl]piperidine (143 mg) was reacted with 3-(3,4,5-trimethcxyphenyl)benzyl chloride (114 mg) in the same manner as described in Preparation Example 9. The title compound was obtained as a yellow powder after converting the product to a dihydrochloride.
Yield: 60 mg (24%).
$^1$H-NMR (400 MHz, measured as a free base, $CDCl_3$)δ: 1.65-1.91 (m, 4H), 2.18 (t, 2H, J=10.5 Hz), 2.38 (s, 3H), 2.97 (d, 2H, J=10.9 Hz), 3.58 (s, 2H), 3.70-3.85 (m, 1H), 3.88 (s, 3H), 3.89 (s, 6H), 3.90 (s, 3H), 3.96 (s, 6H), 4.56 (s, 2H), 6.52 (d, 1H, J=8.4 Hz), 6.59 (d, 1H, J=7.6 Hz), 6.65 (s, 1H), 6.72 (s, 2H), 7.10 (t, 2H, J=8.0 Hz), 7.19-7.25 (m, 4H), 7.31-7.42 (m, 3H), 7.60 (s, 1H), 8.59 (d, 1H, J=7.8 Hz).

Preparation Example 49

Synthesis of 4-[N-(3-methylthiophenyl)-N-[[2-(3,4,5-trimethoxyphenyl)pyridin-5-yl]methyl]amino]-1-[[2-(3,4,5-trimethoxyphenyl)pyridin-4-yl]methyl]piperidine trihydrochloride:

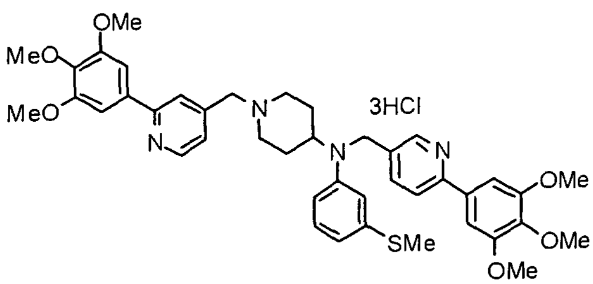

4-(3-Methylthioanilino)-1-[[2-(3,4,5-trimethoxyphenyl)pyridin-4-yl]methyl]piperidine (143 mg) was reacted with 5-chloromethyl-2-(3,4,5-trimethoxyphenyl)pyridine (114 mg) in the same manner as described in Preparation Example 9. The title compound was obtained as a yellow powder after converting the product to a trihydrochloride.
Yield: 22 mg (9%).
$^1$H-NMR (400 MHz, measured as a free base, $CDCl_3$)δ: 1.50-2.05 (m, 4H), 2.20 (br, 2H), 2.37 (s, 3H), 3.05 (br, 2H), 3.50-3.70 (br, 3H), 3.86 (s, 3H), 3.87 (s, 3H), 3.92 (s, 6H), 3.95 (s, 6H), 4.52 (s, 2H), 6.49 (d, 1H, J=8.3 Hz), 6.62 (br, 2H), 7.09 (t, 1H, J=8.2 Hz), 7.18-7.30 (m, 6H), 7.58 (s, 2H), 8.54 (br, 1H), 8.60 (br, 1H).

Preparation Example 50

Synthesis of 4-[N-(3-methylthiophenyl)-N-[4-(3,4,5-trimethoxyphenyl)benzyl]amino]-1-[[2-(3,4,5-trimethoxyphenyl) pyridin-4-yl]methyl]piperidine dihydrochloride:

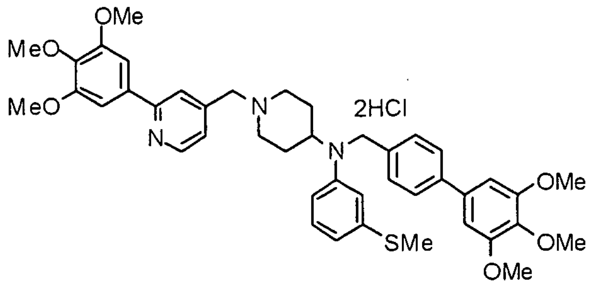

4-(3-Methylthioanilino)-1-[[2-(3,4,5-trimethoxyphenyl)pyridin-4-yl]methyl]piperidine (143 mg) was reacted with 4-(3,4,5-trimethoxyphenyl)benzyl chloride (114 mg) in the same manner as described in Preparation Example 9. The title compound was obtained as a yellow powder after converting the product to a dihydrochloride.
Yield: 57 mg (22%).
$^1$H-NMR (400 MHz, measured as a free base, $CDCl_3$)δ: 1.58-1.83 (m, 4H), 2.20 (t, 2H, J=11.3 Hz), 2.39 (s, 3H), 2.98 (d, 2H, J=11.1 Hz), 3.58 (s, 2H), 3.88 (s, 3H), 3.90 (s, 3H), 3.91 (s, 6H), 3.96 (s, 6H), 4.53 (s, 2H), 6.51 (dd, 1H, J=8.4 Hz, 2.4 Hz), 6.60 (d, 1H, J=8.0 Hz), 6.64 (s, 1H), 6.75 (s, 2H), 7.10 (t, 1H, J=8.1 Hz), 7.24-7.33 (m, 4H), 7.47 (d, 2H, J=8.0 Hz), 7.61 (s, 1H), 8.59 (d, 1H, J=5.0 Hz).

Referential Example 87

Synthesis of 4-propargylamino-1-[[2-(3,4,5-trimethoxyphenyl)pyridin-4-yl]methyl]piperidine:

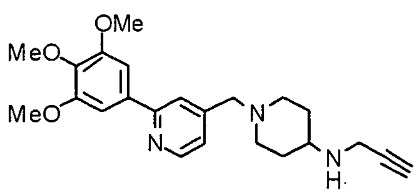

1-[[2-(3,4,5-Trimethoxyphenyl)pyridin-4-yl]methyl]-4-piperidone (400 mg) and propargylamine (80 mg) were treated in the same manner as described in Referential Example 25 to give the title compound.
Yield: 227 mg (63%).
$^1$H-NMR (400 MHz, $CDCl_3$)δ: 1.38-1.51 (m, 2H), 1.83-1.86 (m, 3H), 2.10-2.15 (m, 2H), 2.21 (s, 1H), 2.74 (br, 1H), 2.83-2.87 (m, 2H), 3.45 (s, 2H), 3.56 (s, 2H), 3.89 (s, 3H), 3.96 (s, 6H), 7.19 (d, 1H, J=4.9 Hz), 7.24 (s, 2H), 7.65 (s, 1H), 8.58 (d, 1H, J=4.9 Hz).

Preparation Example 51

Synthesis of 4-[N-propargyl-N-[[2-(3,4,5-trimethoxyphenyl)pyridin-4-yl]methyl]amino]-1-[[2-(3,4,5-trimethoxyphenyl)pyridin-4-yl]methyl]piperidine tetrahydrochloride:

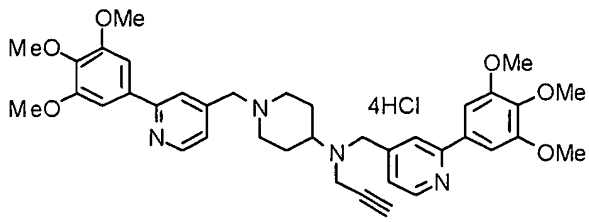

4-Propargylamino-1-[[2-(3,4,5-trimethoxyphenyl)pyridin-4-yl]methyl] piperidine (227 mg) was reacted with 4-chloromethyl-2-(3,4,5-trimethoxyphenyl)pyridine (226 mg) in the same manner as described in Preparation Example 9. The title compound was obtained as a yellow powder after converting the product to a tetrahydrochloride.
Yield: 128 mg (23%).
$^1$H-NMR (400 MHz, measured as a free base, $CDCl_3$)δ: 1.48-2.40 (m, 7H), 2.72 (br, 1H), 3.02 (br, 2H), 3.39 (s, 2H), 3.64 (br, 2H), 3.84 (s, 2H), 3.91 (s, 6H), 3.98 (s, 6H), 3.99 (s, 6H), 7.22-7.29 (m, 6H), 7.66 (br, 2H), 8.60 (d, 1H, J=4.9 Hz), 8.62 (d, 1H, J=4.9 Hz).

Referential Example 88

Synthesis of 4-(5-indanylamino)-1-[[2-(3,4,5-trimethoxyphenyl)pyridin-4-yl]methyl]piperidine:

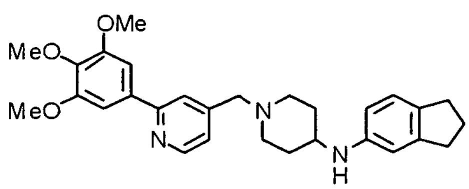

1-[[2-(3,4,5-Trimethoxyphenyl)pyridin-4-yl]methyl]-4-piperidone (1.40 g) and 5-aminoindan (680 mg) were treated in the same manner as described in Referential Example 37 to give the title compound.
Yield: 1.22 g (59%).
$^1$H-NMR (400 MHz, $CDCl_3$)δ: 1.40-1.57 (m, 2H), 2.00-2.15 (m, 5H), 2.19-2.25 (m, 2H), 2.77-2.93 (m, 6H), 3.30 (br, 1H), 3.58 (s, 2H), 3.91 (s, 3H), 3.97 (s, 6H), 6.41 (d, 1H, J=8.0 Hz), 6.52 (s, 1H), 7.01 (d, 1H, J=8.0 Hz), 7.21-7.26 (m, 3H), 7.64 (s, 1H), 8.60 (d, 1H, J=4.9 Hz).

Preparation Example 52

Synthesis of 4-[N-(indan-5-yl)-N-[[2-(3,4,5-trimethoxyphenyl)pyridin-3-yl]methyl]amino]-1-[[2-(3,4,5-trimethoxyphenyl) pyridin-4-yl]methyl]piperidine trihydrochloride:

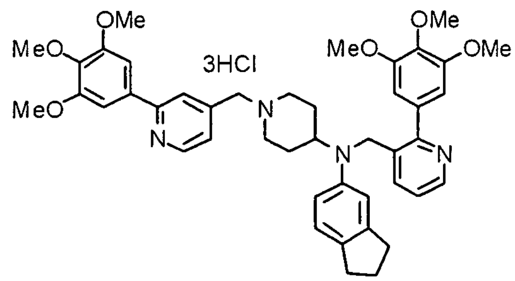

4-(5-Indanylamino)-1-[[2-(3,4,5-trimethoxyphenyl)pyridin-4-yl]methyl]piperidine (142 mg) was reacted with 3-chloromethyl-2-(3,4,5-trimethoxyphenyl)pyridine (114 mg) in the same manner as described in Preparation Example 9. The title compound was obtained as a yellow powder after converting the product to a trihydrochloride.
Yield: 90 mg (41 %).
$^1$H-NMR (400 MHz, measured as a free base, $CDCl_3$)δ: 1.54-1.67 (m, 2H), 1.74-1.83 (m, 2H), 1.98-2.07 (m, 2H), 2.09-2.98 (m, 2H), 3.55 (s, 2H), 3.64-3.74 (m, 1H), 3.90 (s, 3H), 3.91 (s, 6H), 3.92 (s, 3H), 3.96 (s, 6H), 4.41 (s, 2H), 6.49 (dd, 1H, J=8.2 Hz, 2.4 Hz), 6.59 (s, 1H), 6.74 (s, 2H), 7.04 (d, 1H, J=8.2 Hz), 7.15-7.20 (m, 2H), 7.22 (s, 2H), 7.54 (s, 1H), 7.77 (dd, 1H, J=7.8 Hz, 1.4 Hz), 8.52 (dd, 1H, J=4.7 Hz, 1.8 Hz), 8.59 (d, 1H, J=5.1 Hz).

Preparation Example 53

Synthesis of 4-[N-(indan-5-yl)-N-[2-(3,4,5-trimethoxyphenyl)benzyl]amino]-1-[[2-(3,4,5-trimethoxyphenyl)pyridin-4-yl] methyl]plperldine dihydrochloride:

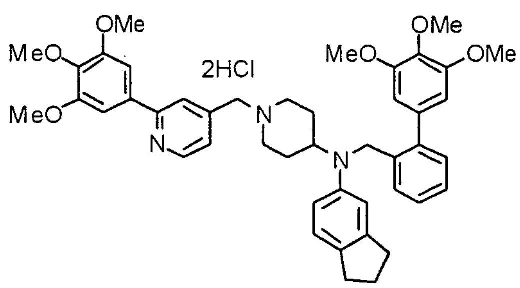

4-(5-Indanylamino)-1-[[2-(3,4,5-trimethoxyphenyl)pyridin-4-yl]methyl]piperidine (142 mg) was reacted with 2-(3,4,5-trimethoxyphenyl)benzyl chloride (114 mg) in the same manner as described in Preparation Example 9. The title compound was obtained as a yellow powder after converting the product to a dihydrochloride.
Yield: 115 mg (47%).
$^1$H-NMR (400 MHz, measured as a free base, $CDCl_3$)δ: 1.56-1.66 (m, 2H), 1.80-1.83 (m, 2H), 2.00-2.05 (m, 2H), 2.11-2.18 (m, 2H), 2.77-2.83 (m, 4H), 2.92-2.95 (m, 2H), 3.55 (s, 2H), 3.72 (br, 1H), 3.87 (s, 6H), 3.90 (s, 3H), 3.92 (s, 3H), 3.96 (s, 6H), 4.34 (s, 2H), 6.49 (d, 1H, J=8.3 Hz), 6.56 (s, 2H), 6.60 (s, 1H), 7.02 (d, 1H, J=8.3 Hz), 7.17-7.27 (m, 5H), 7.42-7.45 (m, 1H), 7.54 (s, 1H), 8.58 (d, 1H, J=4.9 Hz).

Preparation Example 54

Synthesis of 4-[N-(indan-5-yl)-N-[[2-(3,4,5-trimethoxyphenyl)pyridin-4-yl]methyl]amino]-1-[[2-(3,4,5-trimethoxyphenyl)pyridin-4-yl]methyl]piperidine trihydrochloride:

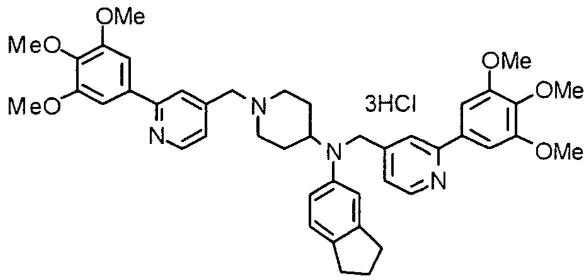

4-(5-Indanylamino)-1-[[2-(3,4,5-trimethoxyphenyl)pyridin-4-yl]methyl]piperidine (142 mg) was reacted with 4-chloromethyl-2-(3,4,5-trimethoxyphenyl)pyridine (114 mg) in the same manner as described in Preparation Example 9. The title compound was obtained as a white powder after converting the product to a trihydrochloride.
Yield: 23 mg (9%).
$^1$H-NMR (400 MHz, measured as a free base, $CDCl_3$)δ: 1.60-1.95 (m, 4H), 2.00 (quint, 2H, J=7.3 Hz), 2.20 (br, 2H), 2.75-2.81 (m, 4H), 2.99 (br, 2H), 3.58 (br, 2H), 3.77(s, 1H), 3.86 (s, 3H), 3.87 (s, 3H), 3.91 (s, 6H), 3.94 (s, 6H), 4.49 (s, 2H), 6.51 (d, 1H, J=8.3 Hz), 6.62 (s, 1H), 7.02 (d, 1H, J=8.0 Hz), 7.16 (s, 2H), 7.18-7.22 (m, 4H), 7.57 (br, 2H), 8.52 (d, 1H, J=4.9 Hz), 8.57 (d, 1H, J=4.9 Hz).

Preparation Example 55

Synthesis of 4-[N-(indan-5-yl)-N-[3-(3,4,5-trimethoxyphenyl)benzyl]amino]-1-[[2-(3,4,5-trimethoxyphenyl)pyridin-4-yl]methyl]piperidine dihydrochloride:

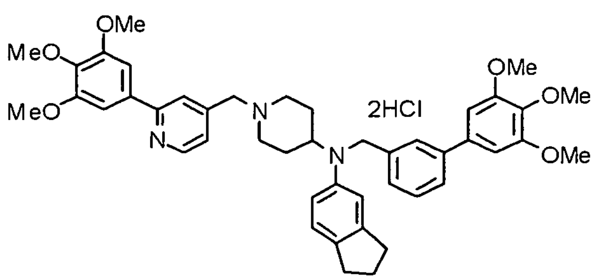

4-(5-Indanylamino)-1-[[2-(3,4, 5-trimethoxyphenyl)pyridin-4-yl]methyl]piperidine (60 mg) was reacted with 3-(3,4,5-trimethoxyphenyl)benzyl chloride (114 mg) in the same manner as described in Preparation Example 9. The title compound was obtained as a yellow powder after converting the product to a dihydrochloride.
Yield: 18 mg (19%).
$^1$H-NMR (400 MHz, measured as a free base, $CDCl_3$)δ: 1.60-1.95 (m, 4H), 2.00 (quint, 2H, J=7.2 Hz), 2.20 (br, 2H), 2.75-2.81 (m, 4H), 2.95 (br, 2H), 3.60 (br, 2H), 3.85 (br, 1H), 3.86 (s, 3H), 3.87 (s, 6H), 3.88 (s, 3H), 3.94 (s, 6H), 4.51 (s, 2H), 6.54 (d, 1H, J=8.2 Hz), 6.66 (s, 1H), 6.70 (s, 2H), 7.01 (d, 1H, J=8.4 Hz), 7.19 (d, 1H, J=4.9 Hz), 7.19-7.42 (m, 6H), 7.60 (br, 1H), 8.59 (br, 1H).

Preparation Example 56

Synthesis of 4-[N-(indan-5-yl)-N-[[2-(3,4,5-trimethoxyphenyl)pyridin-5-yl]methyl]amino]-1-[[2-(3,4,5-trimethoxyphenyl)pyridin-4-yl]methyl]piperidine trihydrochloride:

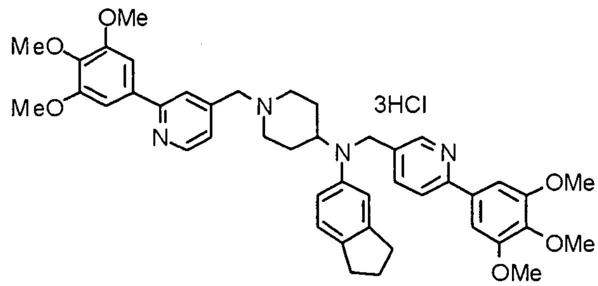

4-(5-Indanylamino)-1-[[2-(3,4,5-trimethoxyphenyl)pyridin-4-yl]methyl]piperidine (143 mg) was reacted with 5-chloromethyl-2-(3,4,5-trimethoxyphenyl)pyridine (114 mg) in the same manner as described in Preparation Example 9. The title compound was obtained as a yellow powder after converting the product to a trihydrochloride.
Yield: 138 mg (63%).
$^1$H-NMR (400 MHz, measured as a free base, $CDCl_3$)δ: 1.71-1.91 (m, 4H), 1.98-2.06 (m, 2H), 2.13-2.22 (m, 2H), 2.76-2.84 (m, 4H), 2.94-3.05 (m, 2H), 3.57 (s, 2H), 3.69-3.78 (m, 1H), 3.89 (s, 3H), 3.90 (s, 3H), 3.94 (s, 6H), 3.96 (s, 6H), 4.50 (s, 2H), 6.57 (dd, 1H, J=8.2 Hz, 2.3 Hz), 6.67 (s, 1H), 7.04 (d, 1H, J=8.4 Hz), 7.20-7.22 (m, 1H), 7.22 (s, 2H), 7.23 (s, 2H), 7.57-7.62 (m, 1H), 7.60 (s, 1H), 7.65 (dd, 1H, J=8.2 Hz, 2.2 Hz), 8.58-8.62 (m, 2H).

Preparation Example 57

Synthesis of 4-[N-(indan-5-yl)-N-[4-(3,4,5-trimethoxyphenyl)benzyl]amino]-1-[[2-(3,4,5-trimethoxyphenyl)pyridin-4-yl]methyl]piperidine dihydrochloride:

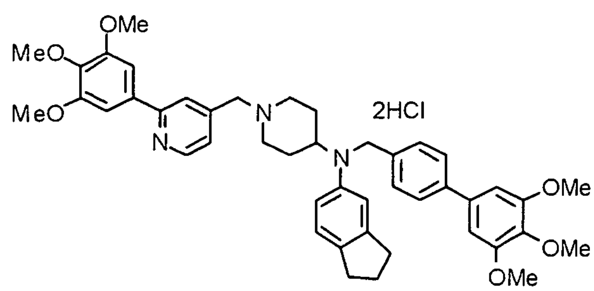

4-(5-Indanylamino)-1-[[2-(3,4,5-trimethoxyphenyl)pyridin-4-yl]methyl]piperidine (143 mg) was reacted with 4-(3,4,5-trimethoxyphenyl)benzyl chloride (114 mg) in the same manner as described in Preparation Example 9. The title compound was obtained as a yellow powder after converting the product to a dihydrochloride.
Yield: 95 mg (39%).
$^1$H-NMR (400 MHz, measured as a free base, $CDCl_3$)δ: 1.74-1.90 (m, 4H), 2.01-2.06 (m, 2H), 2.16-2.22 (m, 2H), 2.78-2.84 (m, 4H), 2.96-2.99 (m, 2H), 3.58 (s, 2H), 3.72 (br, 1H), 3.88 (s, 3H), 3.90 (s, 3H), 3.91 (s, 6H), 3.96 (s, 6H), 4.51 (s, 2H), 6.55 (d, 1H, J=8.3 Hz), 6.67 (s, 1H), 6.72 (s, 2H), 7.04 (d, 1H, J=8.3 Hz), 7.20 (d, 1H, J=5.1 Hz), 7.23 (s, 2H), 7.35 (d, 2H, J=8.1 Hz), 7.47 (d, 2H, J=8.1 Hz), 7.61 (s, 1H), 8.59 (d, 1H, J=4.9 Hz).

Referential Example 89

Synthesis of 4-(4-butylanilino)-1-[[2-(3,4,5-trimethoxyphenyl)pyridin-4-yl]methyl]piperidine:

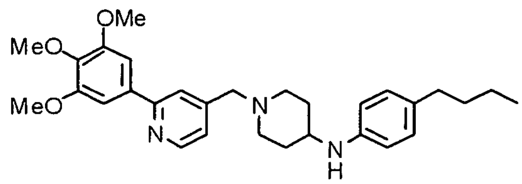

1-[[2-(3,4,5-Trimethoxyphenyl)pyridin-4-yl]methyl]-4-piperidone (1.24 g) and 4-butylaniline (149 mg) were treated in the same manner as described in Referential Example 37 to give the title compound.
Yield: 1.23 g (72%).
$^1$H-NMR (400 MHz, $CDCl_3$)δ: 0.82 (t, 3H, J=7.3 Hz), 1.20-1.30 (m, 2H), 1.38-1.50 (m, 4H), 1.92-2.25 (m, 4H), 2.40 (t, 2H, J=7.7 Hz), 2.77 (br, 2H), 3.21 (br, 1H), 3.50 (s, 2H), 3.82 (s, 3H), 3.89 (s, 6H), 6.45 (d, 2H, J=7.8 Hz), 6.89 (d, 2H, J=8.0 Hz), 7.13 (d, 1H, J=4.9 Hz), 7.18 (s, 2H), 7.58 (s, 1H), 8.52 (d, 1H, J=4.9 Hz).

Preparation Example 58

Synthesis of 4-[N-(4-butylphenyl)-N-[[2-(3,4,5-trimethoxyphenyl)pyridin-3-yl]methyl]amino]-1-[[2-(3,4,5-trimethoxyphenyl)pyridin-4-yl]methyl]piperidine trihydrochloride:

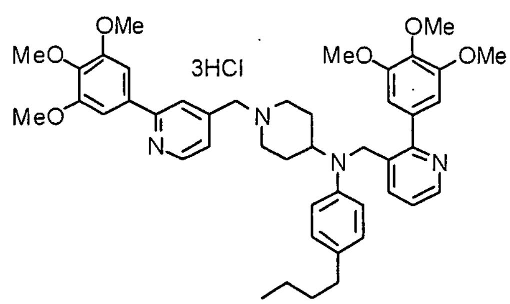

4-(4-Butylanilino)-1-[[2-(3,4,5-trimethoxyphenyl)pyridin-4-yl]methyl]piperidine (147 mg) was reacted with 3-chloromethyl-2-(3,4,5-trimethoxyphenyl)pyridine (114 mg) in the same manner as described in Preparation Example 9. The title compound was obtained as a yellow powder after converting the product to a trihydrochloride.
Yield: 58 mg (27%).
$^1$H-NMR (400 MHz, measured as a free base, $CDCl_3$)δ: 0.91 (t, 3H, J=7.3 Hz), 1.32-1.35 (m, 2H), 1.50-1.70 (m, 4H), 1.75 (br, 2H), 2.10-2.20 (m, 2H), 2.49 (t, 2H, J=7.6 Hz), 2.95 (br, 2H), 3.55 (s, 2H), 3.70 (br, 1H), 3.90 (s, 3H), 3.91 (s, 6H), 3.92 (s, 3H), 3.96 (s, 6H), 4.41 (s, 2H), 6.59 (d, 2H, J=8.8 Hz), 6.74 (s, 2H), 7.00 (d, 2H, J=8.6 Hz), 7.16-7.17 (m, 1H), 7.19 (d, 1H, J=4.9 Hz), 7.22 (s, 2H), 7.54 (s, 1H), 8.59 (d, 1H, J=7.5 Hz), 8.52 (br, 1H), 8.59 (d, 1H, J=4.9 Hz).

Preparation Example 59

Synthesis of 4-[N-(4-butylphenyl)-N-[2-(3,4,5-trimethoxyphenyl)benzyl]amino]-1-[[2-(3,4,5-trimethoxyphenyl)pyridin-4-yl]methyl]piperidine dihydrochloride:

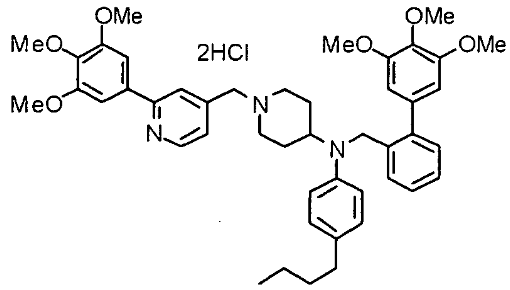

4-(4-Butylanilino)-1-[[2-(3,4,5-trimethoxyphenyl)pyridin-4-yl]methyl]piperidine (147 mg) was reaction with 2-(3,4,5-trimethoxyphenyl)benzyl chloride (114 mg) in the same manner as described in Preparation Example 9. The title compound was obtained as a yellow powder after converting the product to a dihydrochloride.
Yield: 59 mg (24%).
$^1$H-NMR (400 MHz, measured as a free base, $CDCl_3$)δ: 0.90 (t, 3H, J=7.4 Hz), 1.25-1.41 (m, 2H), 1.48-1.75 (m, 4H), 1.81 (d, 2H, J=11.7 Hz), 2.13 (t, 2H, J=11.2 Hz), 2.48 (t, 2H, J=7.5 Hz), 2.93 (d, 2H, J=11.2 Hz), 3.55 (s, 2H), 3.65-3.80 (m, 1H), 3.87 (s, 6H), 3.90 (s, 3H), 3.92 (s, 1H), 3.96 (s, 6H), 4.33 (s, 2H), 6.56 (s, 2H), 6.60 (d, 2H, J=8.5 Hz), 6.98 (d, 2H, J=8.5 Hz), 7.18 (d, 1H, J=4.9 Hz), 7.21 (s, 2H), 7.20-7.37 (m, 3H), 7.41 (br, 1H), 7.54 (s, 1H), 8.58 (d, 1H, J=4.9 Hz).

Preparation Example 60

Synthesis of 4-[N-(4-buthylphenyl)-N-[[2-(3,4,5-trimethoxyphenyl)pyridin-4-yl]methyl]amino]-1-[[2-(3,4,5-trimethoxyphenyl)pyridin-4-yl]methyl]piperidine trihydrochloride:

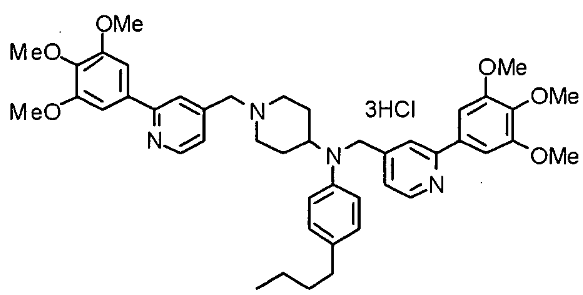

4-(4-Butylanilino)-1-[[2-(3,4,5-trimethoxyphenyl)pyridin-4-yl]methyl]piperidine (196 mg) was reacted with 4-chloromethyl-2-(3,4,5-trimethoxyphenyl)pyridine (129 mg) in the same manner as described in Preparation Example 9. The title compound was obtained as a white powder after converting the product to a trihydrochloride.
Yeld: 20 mg (6%).
$^1$H-NMR (400 MHz, measured as a free base, $CDCl_3$)δ: 0.88 (t, 3H, J=7.3 Hz), 1.20-1.35 (m, 2H), 1.49-1.60 (m, 2H), 1.62-2.02 (m, 4H), 2.20 (br, 2H), 2.46 (t, 2H, J=7.3 Hz), 3.05 (br, 2H), 3.60 (br, 3H), 3.87 (s, 3H), 3.88 (s, 3H), 3.90 (s, 6H), 3.94 (s, 6H), 4.49 (s, 2H), 6.62 (d, 2H, J=8.3 Hz), 6.98 (d, 2H, J=8.3 Hz), 7.13 (s, 2H), 7.15-7.40 (m, 4H), 7.55 (br, 2H), 8.52 (d, 1H, J=4.9 Hz), 8.60 (br, 1H).

Preparation Example 61

Synthesis of 4-[N-(4-butylphenyl)-N-[3-(3,4,5-trimethoxyphenyl)benzyl]amino]-1-[[2-(3,4,5-trimethoxyphenyl)pyridin-4-yl]methyl]piperidine dihydrochloride:

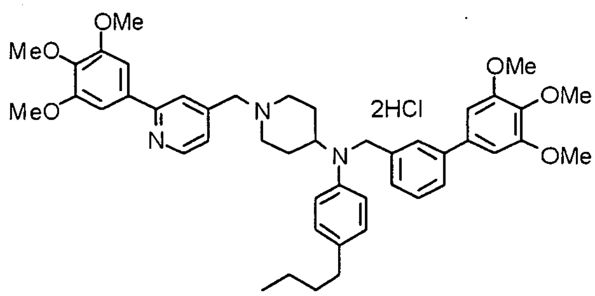

4-(4-Butylanilino)-1-[[2-(3,4,5-trimethoxyphenyl)pyridin-4-yl]methyl]piperidine (147 mg) was reacted with 3-(3,4,5-trimethoxyphenyl)benzyl chloride (114 mg) in the same manner as described in Preparation Example 9. The title compound was obtained as a yellow powder after converting the product to a dihydrochloride.
Yield: 102 mg (42%).
$^1$H-NMR (400 MHz, measured as a free base, $CDCl_3$)δ: 0.90 (t, 3H, J=7.4 Hz), 1.30-1.36 (m, 2H), 1.48-1.56 (m, 2H), 1.76-1.89 (m, 4H), 2.19 (br, 2H), 2.48 (t, 2H, J=7.8 Hz), 2.97 (br, 2H), 3.58 (s, 2H), 3.86 (br, 1H), 3.88 (s, 3H), 3.58 (s, 2H), 3.86 (br, 1H), 3.88 (s, 3H), 3.90 (s, 3H), 3.95 (s, 6H), 4.54 (s, 2H), 6.68 (d, 2H, J=8.6 Hz), 6.72 (s, 2H), 7.00 (d, 2H, J=8.6 Hz), 7.20-7.27 (m, 2H), 7.23 (s, 2H), 7.32-7.40 (m, 2H), 7.44 (s, 1H), 7.62 (s, 1H), 8.59 (d, 1H, J=5.1 Hz).

Preparation Example 62

Synthesis of 4-[N-(4-butylphenyl)-N-[[2-(3,4,5-trimethoxyphenyl)pyridin-5-yl]methyl]amino]-1-[[2-(3,4,5-trimethoxyphenyl)pyridin-4-yl]methyl]piperidine trihydrochloride:

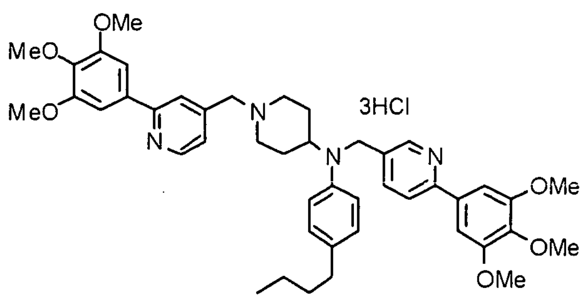

4-(4-Butylanilino)-1-[[2-(3,4,5-trimethoxyphenyl)pyridin-4-yl]methyl]piperidine (147 mg) was reacted with 5-chloromethyl-2-(3,4,5-trimethoxyphenyl)pyridine (114 mg) in the same manner as described in Preparation Example 9. The title compound was obtained as a yellow powder after converting the product to a trihydrochloride.
Yield: 65 mg (21%).
$^1$H-NMR (400 MHz, measured as a free base, $CDCl_3$)δ: 0.90 (t, 3H, J=7.3 Hz), 1.32-1.36 (m, 2H), 1.50-1.54 (m, 2H), 1.70-1.95 (m, 4H), 2.17 (br, 2H), 2.49 (t, 2H, J=7.7 Hz), 2.96 (br, 2H), 3.58 (s, 2H), 3.75 (br, 1H), 3.89 (s, 3H), 3.90 (s, 3H), 3.94 (s, 6H), 3.96 (s, 6H), 4.50 (s, 2H), 6.68 (d, 2H, J=8.6 Hz), 7.00 (d, 2H, J=8.6 Hz), 7.20-7.22 (m, 3H), 7.23 (s, 2H), 7.58-7.66 (m, 3H), 8.59 (br, 1H), 8.60 (br, 1H).

Preparation Example 63

Synthesis of 4-[N-(4-butylphenyl)-N-[4-(3,4,5-trimethoxyphenyl)benzyl]amino]-1-[[2-(3,4,5-trimethoxyphenyl)pyridin-4-yl]methyl]piperidine dihydrochloride:

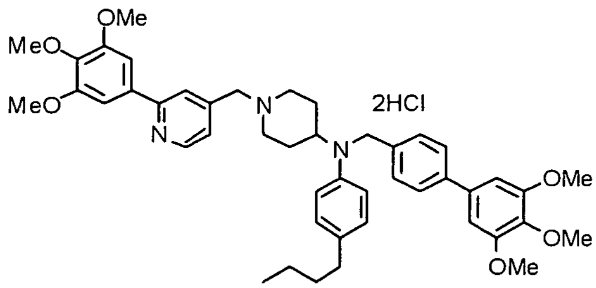

4-(4-Butylanilino)-1-[[2-(3,4,5-trimethoxyphenyl)pyridin-4-yl]methyl]piperidine (147 mg) was reacted with 4-(3,4,5-trimethoxyphenyl)benzyl chloride (114 mg) in the same manner as described in Preparation Example 9. The title compound was obtained as a yellow powder after converting the product to a dihydrochloride.
Yield: 82 mg (33%).
$^1$H-NMR (400 MHz, measured as a free base, $CDCl_3$)δ: 0.90 (t, 3H, J=7.3 Hz), 1.30-1.36 (m, 2H), 1.51-1.55 (m, 2H), 1.79-1.90 (m, 4H), 2.18 (br, 2H), 2.48 (t, 2H, J=7.7 Hz), 2.98 (d, 2H, J=10.7 Hz), 3.57 (s, 2H), 3.72-3.85 (m, 1H), 3.88 (s, 3H), 3.90 (s, 3H), 3.91 (s, 6H), 3.96 (s, 6H), 4.50 (s, 2H), 6.66 (d, 2H, J=8.8 Hz), 6.75 (s, 2H), 7.00 (d, 2H, J=8.8 Hz), 7.20 (d, 1H, J=4.9 Hz), 7.22 (s, 2H), 7.33 (d, 2H, J=8.2 Hz), 7.47 (d, 2H, J=8.2 Hz), 7.61 (s, 1H), 8.59 (d, 1H, J=5.1 Hz).

Referential Example 90

Synthesis of 1-(4-pycolyl)-4-piperidone:

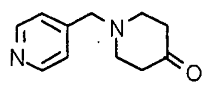

4-Piperidone hydrochloride monohydrate (922 mg) and 4-picolyl chloride hydrochloride (820 mg) were reacted with each other in the same manner as described in Preparation Example 2 to give the title compound.
Yield: 870 mg (92%).
$^1$H-NMR (400 MHz, $CDCl_3$)δ: 2.46 (t, 4H, J=5.9 Hz), 2.74 (t, 4H, J=6.2 Hz), 3.61 (s, 2H), 7.29 (d, 2H, J=6.2 Hz), 8.55 (dd, 2H, J=6.2 Hz, 1.1 Hz).

Referential Example 91

Synthesis of 1-(4-pycolyl)-4-(4-pycolylamino)piperidine tetrahydrochloride:

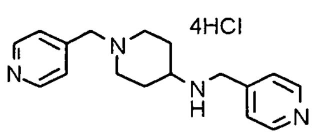

1-(4-Pycolyl)-4-piperidone (870 mg) was reacted with 4-picolylamine (497 mg) in the same manner as described in Referential Example 37. The aimed compound was obtained as a pale brown tetrahydrochloride.
Yield: 363 mg (19%).
$^1$H-NMR (400 MHz, measured as a free base, $CDCl_3$)δ: 1.37-1.51 (m, 2H), 1.82-1.90 (m, 2H), 2.04 (dt, 2H, J=11.6 Hz, 2.7 Hz), 2.44-2.55 (m, 1H), 2.76-2.82 (m, 2H), 3.47 (s, 2H), 3.82 (s, 2H), 7.23-7.26 (m, 4H), 8.50-8.53 (m, 4H).

Referential Example 92

Synthesis of 4-(*p*-anisidino)-1-(tert-butoxycarbonyl)piperidine:

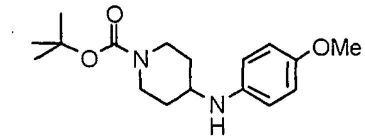

1-(tert-Butoxycarbonyl)-4-piperidone (116 g) was reacted with *p*-anisidine (68.3 g) in the same manner as described in Referential Example 37 to give the title compound.
Yield: 125 g (74%).
$^1$H-NMR (400 MHz, $CDCl_3$) δ: 1.23-1.35 (m, 2H), 1.46 (s, 9H), 1.96-2.06 (m, 2H), 2.83-2.96 (m, 2H), 3.27-3.38 (m, 1H), 3.74 (s, 9H), 3.94-4.12 (m, 2H), 6.58 (d, 2H, J=9.0 Hz), 6.77 (d, 2H, J=9.0 Hz).

Referential Example 93

Synthesis of 1-(tert-butoxycarbonyl)-4-[N-(4-methoxyphenyl)-N-[3-(3,4,5-trimethoxyphenyl) benzoyl]amino]piperidine:

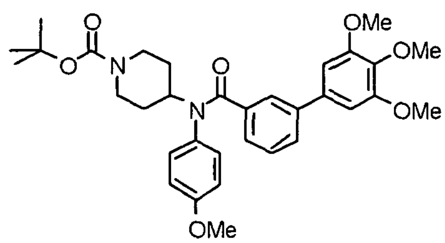

4-(*p*-Anisidino)-1-(tert-butoxycarbonyl)piperidine (613 mg) was reacted with 3-(3,4,5-trimethoxyphenyl)benzoic acid (577 mg) in the same manner as described in Preparation Example 1 to give the title compound.
Yield: 416 mg (36%).

Referential Example 94

Synthesis of 4-[N-(4-methoxyphenyl)-N-[3-(3,4,5-trimethoxyphenyl)benzoyl]amino]piperidine hydrochloride:

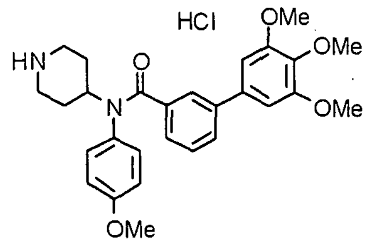

To a solution of 1-(tert-butoxycarbonyl)-4-[N-(4-methoxyphenyl)-N-[3-(3,4, 5-trimethoxyphenyl) benzoylamino]piperidine (416 mg) in ethyl acetate (5 mL) was added 4 M solution of hydrogen chloride in ethyl acetate (5 mL). The mixture was stirred at room temperature for 4 hr, and the resulting precipitates were filtered, washed with ethyl acetate on a funnel and driedto give the title compound.
Yield: 315 mg (85%)

Preparation Examples 64 to 66

These compounds were prepared by the reaction of 4-[N-(4-methoxyphenyl)-N-[3-(3,4,5-trimethoxyphenyl) benzoyl]amino] piperidine hydrochloride with chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 64 | 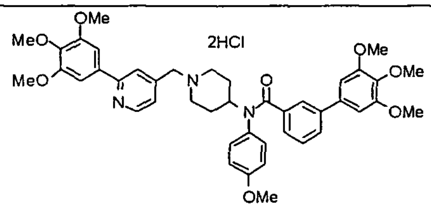 | 68% | 1.53-1.55 (m, 2H), 1.89 (d, 2H, J=12.0 Hz), 2.23 (t, 2H, J=12.0 Hz), 2.91 (d, 2H, J=11.0 Hz), 3.51 (s, 2H), 3.70 (s, 3H), 3.84 (s, 3H), 3.87 (s, 9H), 3.92 (s, 6H), 4.78 (br, 1H), 6.54 (s, 2H), 6.72 (d, 2H, J=8.5 Hz), 6.94 (d, 2H, J=8.5 Hz), 7.13-7.20 (m, 4H), 7.18 (s, 2H), 7.32 (d, 1H, J=5.3 Hz), 7.45 (s, 1H), 8.19 (d, 1H, J=4.9 Hz). |
| 65 | 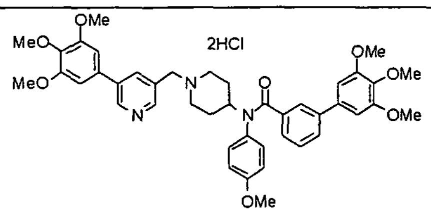 | 52% | 1.66-1.89 (m, 4H), 2.05-2.17 (m, 2H), 2.97 (d, 2H, J=10.3 Hz), 3.43-3.60 (m, 1H), 3.57 (s, 2H), 3.86 (s, 3H), 3.87 (s, 6H), 3.87 (s, 6H), 3.91 (s, 6H), 4.42 (s, 2H), 6.63 (s, 2H), 6.72-6.79 (m, 6H), |

| | | | |
|---|---|---|---|
| | | | 7.64 (s, 1H), 7.78 (br, 1H), 8.46 (d, 2H, J=1.6 Hz), 8.59 (d, 1H, J=2.4 Hz), 8.68 (d, 1H, J=2.2 Hz). |
| 66 | 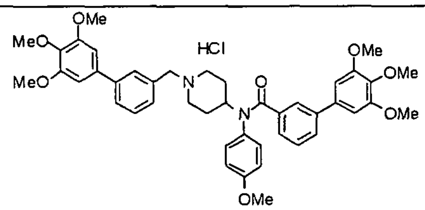 | 75% | 1.42-1.58 (m, 2H), 1.85-1.92 (m, 2H), 2.14-2.23 (m, 2H), 2.93-3.03 (m, 2H), 3.56 (s, 2H), 3.70 (s, 3H), 3.85 (s, 3H), 3.87 (s, 3H), 3.87 (s, 6H), 3.90 (s, 6H), 4.79 (br, 1H), 6.54 (s, 2H), 6.70 (d, 2H, J=8.9 Hz), 6.74 (s, 2H), 6.93 (d, 2H, J=8.9 Hz), 7.17-7.23 (m, 3H), 7.31-7.43 (m, 5H). |

Referential Example 95

Synthesis of 1-(tert-butoxycarbonyl)-4-[N-(4-methoxyphenyl)-N-[[2-(3,4,5-trimethoxyphenyl) pyridin-4-yl]methyl] amino]piperidine:

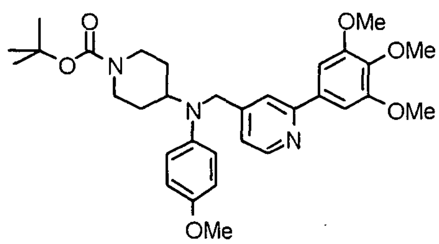

4-(*p*-Anisidino)-1-(tert-butoxycarbonyl)piperidine (2.21 g) was reacted with 4-chloromethyl-2-(3,4,5-trimethoxyphenyl)pyridine (2.12 g) in the same manner as described in Preparation Example 9 to give the title compound.
Yield: 3.76 g (93%)
$^1$H-NMR (400 MHz, $CDCl_3$) δ: 1.40-1.64 (m, 2H), 1.44 (s, 9H), 1.82-1.91 (m, 2H), 2.71-2.84 (m, 2H), 3.62-3.73 (m, 1H), 3.74 (s, 3H), 3.89 (s, 3H), 3.94 (s, 6H), 4.10-4.30 (m, 2H), 4.40 (s, 2H), 6.76 (d, 2H, J=9.4 Hz), 6.79 (d, 2H, J=9.8 Hz), 7.14-7.19 (m, 3H), 7.56 (s, 1H), 8.55 (d, 1H, J=5.1 Hz).

Referential Example 96

Synthesis of 4-[N-(4-methoxyphenyl)-N-[[2-(3,4,5-trimethoxyphenyl)pyridin-4-yl]methyl]amino] piperidine dihydrochloride:

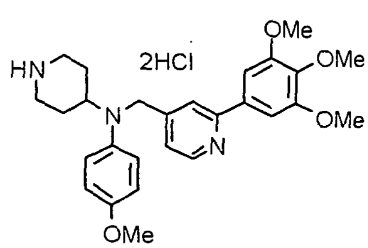

1-(tert-Butoxycarbonyl)-4-[N-(4-methoxyphenyl)-N-[[2-(3,4,5-trimethoxyphe nyl)pyridin-4-yl]methyl]amino] piperidine (3.76 g) was treated in the same manner as described in Referential Example 94 to give the title compound. Yield: 3.77 g (theoretical yield).

Referential Example 97

Synthesis of 1-(tert-butoxycarbonyl)-4-[N-(4-methoxyphenyl)-N-[[3-(3,4,5-trimethoxyphenyl) pyridin-5-yl]methyl] amino]piperidine :

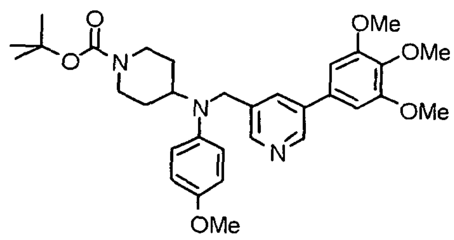

4-(*p*-Anisidino)-1-(tert-butoxycarbonyl)piperidine (613 mg) was reacted with 5-chloromethyl-3-(3,4,5-trimethoxyphenyl)pyridine (588 mg) in the same manner as described in Referential Example 9 to give yellow amorphous substance of the title compound.
Yield: 159 mg (14%).
$^1$H-NMR (400 MHz, $CDCl_3$) δ: 1.44 (s, 9H), 1.50-1.65 (m, 2H), 1.83-1.91 (m, 2H), 2.70-2.84 (m, 2H), 3.53-3.62 (m, 1H), 3.73 (s, 3H), 3.89 (s, 3H), 3.91 (s, 6H), 4.10-4.29 (m, 2H), 4.41 (s, 2H), 6.66 (s, 2H), 6.76-6.84 (m, 4H), 7.70 (s, 1H), 8.49 (s, 1H), 8.63 (d, 1H, J=2.1 Hz).

Referential Example 98

Synthesis of 4-[N-(4-methoxyphenyl)-N-[[3-(3,4,5-trimethoxyphenyl)pyridin-5-yl]methyl]amino] piperidine dihydrochloride:

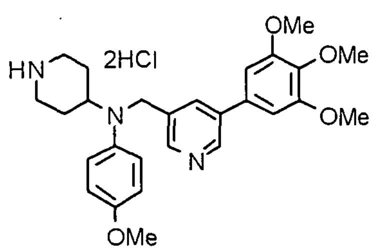

1-(tert-Butoxycarbonyl)-4-[N-(4-methoxyphenyl)-N-[[3-(3,4,5-trimethoxyphenyl)pyridin-5-yl]methyl]amino] piperidine (159 mg) was treated in the same manner as described in Referential Example 94 to give yellow powder of the title compound.
Yield: 142 mg (94%).

Referential Example 99

Synthesis of 1-(tert-butoxycarbonyl)-4-[N-(4-methoxyphenyl)-N-[3-(3,4,5-trimethoxyphenyl)benzyl] amino]piperidine :

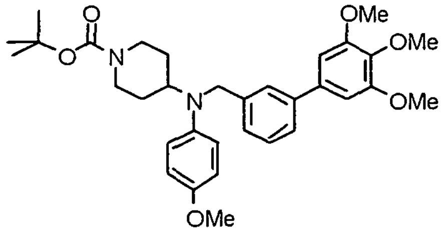

4-(p-Anisidino)-1-(tert-butoxycarbonyl)piperidine (613 mg) and 3-(3,4,5-trimethoxyphenyl)benzyl chloride (586 mg) were treated in the same manner as described in Preparation Example 9 to give a yellow amorphous substance of the title compound.
Yield: 1.12 g (90%).
$^1$H-NMR (400 MHz, $CDCl_3$) δ: 1.44 (s, 9H), 1.50-1.63 (m, 2H), 1.82-1.91 (m, 2H), 2.71-2.83 (m, 2H), 3.69 (tt, 1H, J=11.5 Hz, 3.5 Hz), 3.73 (s, 3H), 3.88 (s, 3H), 3.90 (s, 6H), 4.10-4.28 (m, 2H), 4.42 (s, 2H), 6.71 (s, 2H), 6.78 (s, 4H), 7.24-7.28 (m, 1H), 7.31-7.40 (m, 2H), 7.42 (s, 1H).

Referential Example 100

Synthesis of 4-[N-(4-methoxyphenyl)-N-[3-(3,4,5-trimethoxyphenyl)benzyl]amino]piperidine hydrochloride:

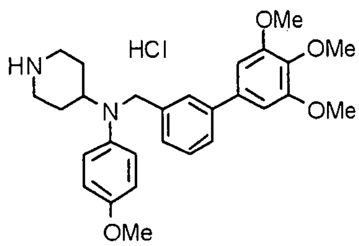

1-(tert-Butoxycarbonyl)-4-[N-(4-methoxyphenyl)-N-[3-(3,4,5-trimethoxyphen yl)benzyl]amino]piperidine (1.12 g) was treated in the same manner as described in Referential Example 94 to give a yellow powder of the title compound.
Yield: 980 mg (99%).

Preparation Examples 67 to 71.

These compounds were obtained by the reaction of amines obtained in Referential Examples 96, 98 and 100 with chloride derivatives obtained in Referential Examples 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 67 | 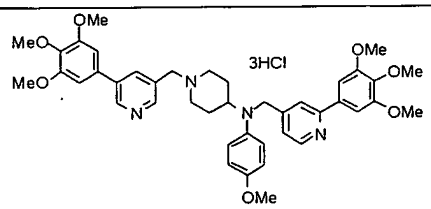 | 62% | 1.60-1.92 (m, 4H), 2.08-2.22 (m, 2H), 2.92-3.06 (m, 2H), 3.54-3.64 (m, 3H), 3.73 (s, 3H), 3.89 (s, 3H), 3.90 (s, 3H), 3.93 (s, 12H), 4.43 (s, 2H), 6.70-6.81 (m, 6H), 7.12-7.17 (m, 3H), 7.56 (s, 1H), 7.76 (s, 1H), 8.49 (d, 1H, J=1.8 Hz), 8.53 (d, 1H, J=5.1 Hz), 8.70 (s, 1H). |
| 68 | 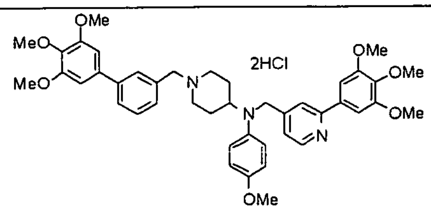 | 54% | 1.65-1.79 (m, 2H), 1.81-1.90 (m, 2H), 2.04-2.18 (m, 2H), 2.94-3.06 (m, 2H), 3.52-3.66 (m, 3H), 3.72 (s, 3H), 3.89 (s, 6H), 3.92 (s, 6H), 3.93 (s, 6H), 4.44 (s, 2H), 6.70-6.80 (m, 6H), 7.13-7.17 (m, 3H), 7.24-7.50 (m, 4H), 7.55 (s, 1H), 8.53 (d, 1H, J=4.9 Hz). |

| | | | |
|---|---|---|---|
| 69 | 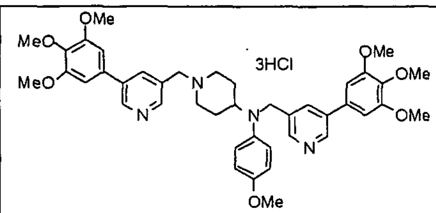 | 52% | 1.66-1.89 (m, 4H), 2.05-2.17 (m, 2H), 2.97 (d, 2H, J=10.3 Hz), 3.43-3.60 (m, 1H), 3.57 (s, 2H), 3.86 (s, 3H), 3.87 (s, 6H), 3.87 (s, 6H), 3.91(s, 6H), 4.42 (s, 2H), 6.63 (s, 2H), 6.72-6.79 (m, 6H), 7.64 (s, 1H), 7.78 (br, 1H), 8.46 (d, 2H, J=1.6 Hz), 8.59 (d, 1H, J=2.4 Hz), 8.68 (d, 1H, J=2.2 Hz). |
| 70 | 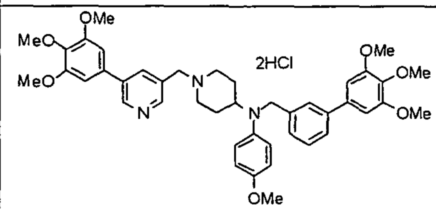 | 69% | 1.55-1.97 (m, 4H), 2.06-2.21 (m, 2H), 2.92-3.07 (m, 2H), 3.53-3.68 (m, 3H), 3.72 (s, 3H), 3.87 (s, 3H), 3.89 (s, 6H), 3.89 (s, 3H), 3.94 (s, 3H), 4.46 (s, 2H), 6.69 (s, 2H), 6.73-6.82 (m, 6H), 7.22-7.29 (m, 1H), 7.32 (t, 1H, J=7.4 Hz), 7.36 (d, 1H, J=7.8 Hz), 7.41 (s, 1H), 7.79(br, 1H), 8.48 (s, 1H), 8.71(br, 1H). |
| 71 | 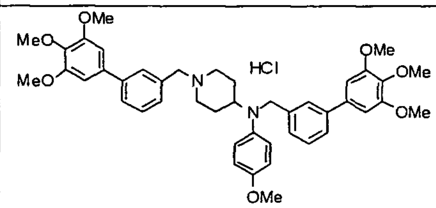 | 75% | 1.69-1.89 (m, 4H), 2.06-2.15 (m, 2H), 2.96-3.04 (m, 2H), 3.56-3.66 (m, 1H), 3.57 (s, 2H), 3.72 (s, 3H), 3.87 (s, 3H), 3.89 (s, 9H), 3.92 (s, 6H), 3.92 (s, 6H), 4.46 (s, 2H), 6.70 (s, 2H), 6.71-6.79 (m, 6H), 7.23-7.47 (m, 8H). |

Referential Example 101

Synthesis of 1-(tert-butoxycarbonyl)-4-(4-ethoxyphenylamino)piperidine:

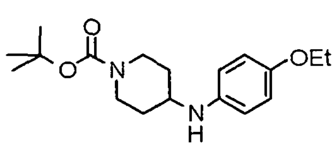

1-(tert-Butoxycarbonyl)-4-piperidinone (5.00 g) was reacted with *p*-phenetidine (3.28 g) in the same manner as described in Referential Example 37 to give a brown powder of the title compound.
Yield: 7.00 g (91%).
$^1$H-NMR (400 MHz, $CDCl_3$) δ: 1.21-1.31 (m, 2H), 1.37 (t, 3H, J=7.0 Hz), 1.46 (s, 9H), 1.97-2.05 (m, 2H), 2.84-2.95 (m, 2H), 3.28-3.37 (m, 1H), 3.96 (q, 2H, J=7.0 Hz), 3.99-4.10 (m, 2H), 6.57 (d, 2H, J=8.8 Hz), 6.77 (d, 2H, J=9.0 Hz).

Referential Example 102

Synthesis of 1-(tert-butoxycarbonyl)-4-[N-(4-ethoxyphenyl)-N-[[2-(3,4,5-trimethoxyphenyl)pyridin-4-yl]methyl]amino] piperidine:

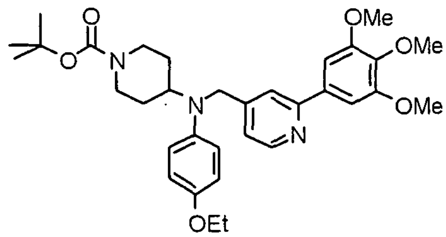

1-(tert-Butoxycarbonyl)-4-[(4-ethoxyphenyl)amino]piperidine (641 mg) was reacted with 4-chloromethyl-2-(3,4,5-trimethoxyphenyl)pyridine (588 mg) in the same manner as described in Preparation Example 9 to give a yellow amorphous substance of the title compound.
Yield: 2.08 g (94%).
$^1$H-NMR (400 MHz, $CDCl_3$) δ: 1.36 (t, 3H, J=7.9 Hz), 1.44 (s, 9H), 1.49-1.58 (m, 2H), 1.82-1.92 (m, 2H), 2.70-2.85 (m, 2H), 3.62-3.72 (m, 1H), 3.89 (s, 3H), 3.94 (s, 6H), 4.12-4.29 (m, 2H), 4.39 (s, 2H), 6.75 (d, 2H, J=9.2 Hz), 6.78 (d, 2H, J=9.6 Hz), 7.14-7.18 (m, 3H), 7.55 (s, 1H), 8.54 (d, 1H, J=5.1 Hz).

Referential Example 103

Synthesis of 4-[N-(4-ethoxyphenyl)-N-[[2-(3,4,5-trimethoxyphenyl)pyridin-4-yl]methyl]amino] piperidine dihydrochloride:

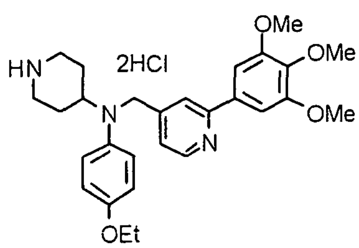

1-(tert-Butoxycarbonyl)-4-[N-(4-ethoxyphenyl)-N-[[2-(3,4,5-trimethoxypheny 1)pyridin-4-yl]methyl]amino]piperidine (1.08 g) was treated in the same manner as described in Referential Example 94 to give a light yellow powder of the title compound.
Yield: 1.01 g (98%).

Referential Example 104.

Synthesis of 1-(tert-butoxycarbonyl)-4-[N-(4-ethoxyphenyl)-N-[[3-(3,4,5-trimethoxyphenyl)pyridin-5-yl]methyl]amino] piperidine:

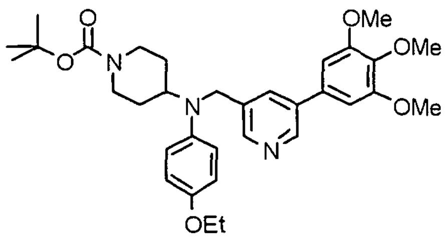

1-(tert-Butoxycarbonyl)-4-[(4-ethoxyphenyl)amino]piperidine (641 mg) was reacted with 5-chloromethyl-3-(3,4,5-trimethoxyphenyl)pyridine (588 mg) in the same manner as described in Preparation Example 9 to give a light yellow amorphous substance of the title compound.
Yield: 452 mg (39%).
$^1$H-NMR (400 MHz, $CDCl_3$) δ: 1.36 (t, 3H, J=6.8 Hz), 1.44 (s, 9H), 1.50-1.60 (m, 2H), 1.82-1.90 (m, 1H), 2.68-2.82 (m, 2H), 3.52-3.61 (m, 1H), 3.88 (s, 3H), 3.90 (s, 6H), 3.94 (q, 2H, J=7.0 Hz), 4.10-4.25 (m, 2H), 4.40 (s, 2H), 6.66 (s, 2H), 6.77 (d, 2H, J=9.2 Hz), 6.81 (d, 2H, J=9.2 Hz), 7.67 (s, 1H), 8.49 (d, 1H, J=2.0 Hz), 8.62 (d, 1H, J=2.1 Hz).

Referential Example 105

Synthesis of 4-[N-(4-ethoxyphenyl)-N-[[3-(3,4,5-trimethoxyphenyl)pyridin-5-yl]methyl]amino] piperidine dihydrochloride:

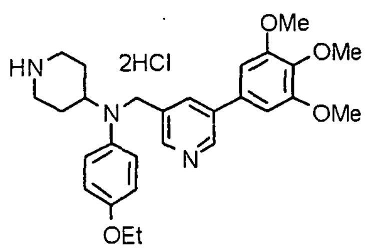

1-(tert-Butoxycarbonyl)-4-[N-(4-ethoxyphenyl)-N-[[3-(3,4,5-trimethoxyphenyl)pyridin-5-yl]methyl]amino]piperidine (452 mg) was treated in the same manner as described in Referential Example 94 to give a light yellow powder of the title compound.
Yield: 380 mg (88%).

Referential Example 106

Synthesis of 1-(tert-butoxycarbonyl)-4-[N-(4-ethoxyphenyl)-N-[3-(3,4,5-trimethoxyphenyl)benzyl] amino]piperidine:

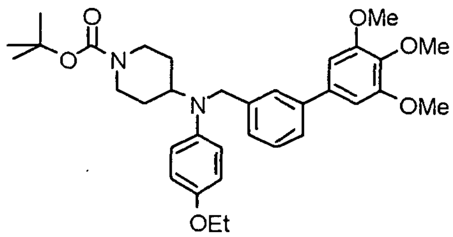

1-(tert-Butoxycarbonyl)-4-[(4-ethoxyphenyl)amino]piperidine (641 mg) was reacted with 3-(3,4,5-trimethoxyphenyl)benzyl chloride (586 mg) in the same manner as described in Preparation Example 9 to give a light yellow amorphous substance of the title compound.
Yield: 1.06 g (92%).
$^1$H-NMR (400 MHz, $CDCl_3$) δ: 1.36 (t, 3H, J=7.0 Hz), 1.44 (s, 9H), 1.53-1.59 (m, 2H), 1.83-1.91 (m, 2H), 2.70-2.83 (m, 2H), 3.64-3.73 (m, 1H), 3.88 (s, 3H), 3.90 (s, 6H), 3.94 (q, 2H, J=7.0 Hz), 4.10-4.29 (m, 2H), 4.41 (s, 2H), 6.71 (s, 2H), 6.76 (s, 4H), 7.26 (d, 1H, J=7.9 Hz), 7.33 (dd, 1H, J=7.4 Hz, 7.4 Hz), 7.38 (d, 1H, J=7.6 Hz), 7.42 (s, 1H).

Referential Example 107

Synthesis of 4-[N-(4-ethoxyphenyl)-N-[3-(3,4,5-trimethoxyphenyl)benzyl]amino]piperidine hydrochloride:

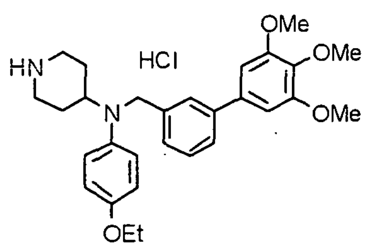

1-(tert-Butoxycarbonyl)-4-[N-(4-ethoxyphenyl)-N-[3-(3,4,5-trimethoxyphenyl)benzyl]amino]piperidine (1.06 g) was treated in the same manner as described in Referential Example 94 to give a light yellow powder of the title compound.
Yield: 913 mg (97%).

Preparation Examples 72 to 79

These compounds were obtained by the reaction of amines obtained in Referential Examples 103, 105 and 107 with chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 72 | 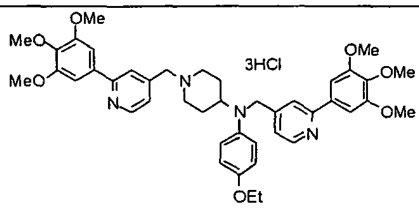 | 49% | 1.36 (t, 3H, J=7.1Hz), 1.68-1.94 (m, 4H), 2.10-2.24 (m, 2H), 2.93-3.04 (m, 2H), 3.54-3.65 (m, 3H), 3.89 (s, 3H), 3.90 (s, 3H), 3.93 (s, 6H), 3.96 (s, 6H), 4.45 (s, 2H), 6.72 (d, 2H, J=9.2 Hz), 6.78 (d, 2H, J=9.3 Hz), 7.15 (s, 2H), 7.17 (d, 1H, J=6.1 Hz), 7.20 (dd, 1H, J=4.9 Hz, 1.0 Hz), 7.23 (s, 2H), 7.57 (s, 1H), 7.61(br, 1H), 8.54 (d, 1H, J=5.2 Hz), 8.59 (d, 1H, J=4.9 Hz). |
| 73 | 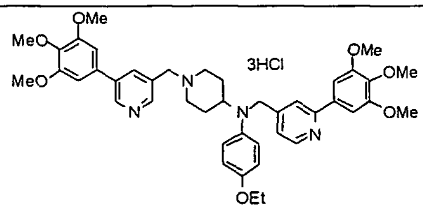 | 63% | 1.36 (t, 3H, J=7.0 Hz), 1.56-1.74 (m, 2H), 1.80-1.90 (m, 2H), 2.07-2.19 (m, 2H), 2.92-3.02 (m, 2H), 3.58 (s, 2H), 3.88-3.95 (m, 2H), 3.89 (s, 3H), 3.93 (s, 12H), 4.43 (s, 2H), 6.69-6.79 (m, 6H), 7.12-7.17 (m, 3H), 7.55 (s, 1H), 7.76 (s, 1H), 8.49 (d, 1H, J=1.8 Hz), 8.53 (d, 1H, J=5.1 Hz), 8.69 (s, 1H). |
| 74 | 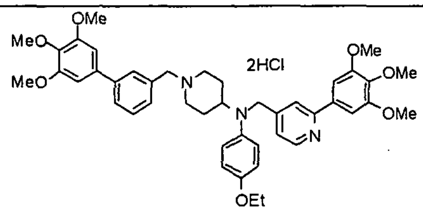 | 65% | 1.36 (t, 3H, J=7.0 Hz), 1.58-1.78 (m, 2H), 1.80-1.89 (m, 2H), 2.04-2.16 (m, 2H), 2.95-3.05 (m, 2H), 3.52-3.66 (m, 1H), 3.57 (s, 1H), 3.85-3.97 (m, 2H), 3.89 (s, 6H), 3.92 (s, 6H), 3.93 (s, 6H), 4.44 (s, 2H), 6.67-6.80 (m, 6H), |

| | | | |
|---|---|---|---|
| | | | 7.13-7.18 (m, 3H), 7.25-7.31 (m, 1H), 7.37 (dd, 1H, J=7.6 Hz, 7.6 Hz), 7.41-7.48 (m, 2H), 7.55 (s, 1H), 8.53 (d, 1H, J=4.9 Hz). |
| 75 | 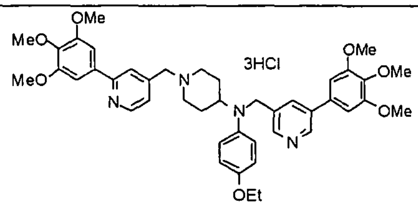 | 42% | 1.36 (t, 3H, J=7.0 Hz), 1.74-2.34 (m, 6H), 2.96-3.10 (m, 2H), 3.47-3.73 (m, 3H), 3.87-3.98 (m, 2H), 3.88 (s, 3H), 3.90 (s, 9H), 3.97 (s, 6H), 4.44 (s, 2H), 6.65 (s, 2H), 6.74-6.82 (m, 4H), 7.18-7.32 (m, 4H), 7.67 (s, 1H), 8.49 (d, 1H, J=1.6 Hz), 8.57-8.65 (m, 2H). |
| 76 | 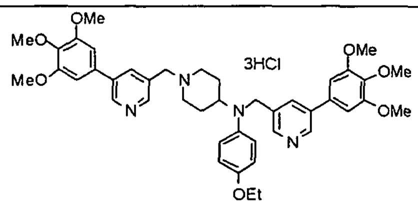 | 43% | 1.36 (t, 3H, J=6.8 Hz), 1.63-1.96 (m, 4H), 2.00-2.26 (m, 2H), 2.92-3.03 (m, 2H), 3.44-3.66 (m, 3H), 3.86-3.96 (m, 2H), 3.88 (s, 3H), 3.89 (s, 6H), 3.90 (s, 3H), 3.93 (s, 6H), 4.44 (s, 2H), 6.65 (s, 2H), 6.72-6.80 (m, 6H), 7.67 (s, 1H), 7.77(br, 1H), 8.47-8.53 (m, 2H), 8.62 (d, 1H, J=1.9 Hz), 8.70 (s, 1H). |
| 77 | 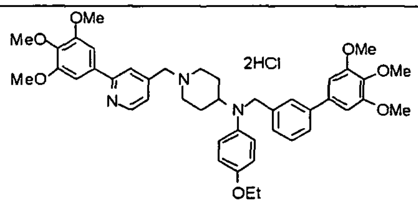 | 82% | 1.35 (t, 3H, J=6.8 Hz), 1.70-1.82 (m, 2H), 1.84-1.92 (m, 2H), 2.10-2.19 (m, 2H), 2.92-3.00 (m, 2H), 3.52-3.65 (m, 3H), 3.88 (s, 3H), 3.89 (s, 6H), 3.90 (s, 3H), 3.93 (q, 2H, J=7.1 Hz), 3.96 (s, 6H), 4.47 (s, 2H), 6.70 (s, 2H), 6.73 (d, 2H, J=9.3 Hz), 6.77 (d, 2H, J=9.3 Hz), 7.18-7.28 (m, 4H), 7.33 (dd, 1H, J=7.3 Hz, 7.3 Hz), 7.37 (d, 1H, J=7.6 Hz), 7.43 (s, 1H), 7.59 (s, 1H), 8.58 (d, 1H, J=4.9 Hz). |
| 78 | 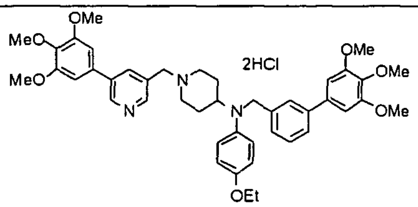 | 61% | 1.35 (t, 3H, J=6.9 Hz), 1.58-1.80 (m, 2H), 1.82-1.91 (m, 2H), 2.09-2.18 (m, 2H), 2.93-3.20 (m, 2H), 3.56-3.65 (m, 1H), 3.58 (s, 2H), 3.87 (s, 3H), 3.89 (s, 6H), 3.89 (s, 3H), 3.91-3.94 (m, 2H), 3.93 (s, 6H), 4.45 (s, 2H), 6.69 (s, 2H), 6.71-6.78 (m, 6H), 7.23-7.28 (m, 1H), 7.32 (t, 1H, J=7.5 Hz), 7.36 (d, 1H, J=7.6 Hz), 7.42 (s, 1H), 7.77 (s, 1H), 8.49 (d, 1H, J=1.8 Hz), 8.69 (d, 1H, J=1.8 Hz). |

| | | | |
|---|---|---|---|
| 79 | 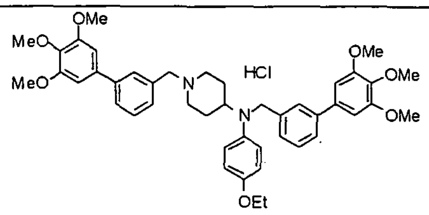 | 73% | 1.35 (t, 3H, J=6.8 Hz), 1.68-1.80 (m, 2H), 1.81-1.89 (m, 2H), 2.06-2.14 (m, 2H), 2.96-3.03 (m, 2H), 3.57 (s, 2H), 3.57-3.65 (m, 1H), 3.87 (s, 3H), 3.89 (s, 9H), 3.91-3.96 (m, 2H), 3.92 (s, 6H), 4.46 (s, 2H), 6.69-6.79 (m, 9H), 7.23-7.47 (m, 7H). |

Referential Example 108

Synthesis of 1-(tert-butoxycarbonyl)-4-(4-butoxyphenylamino)piperidine:

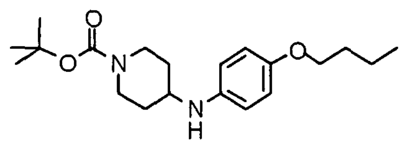

1-(tert-Butoxycarbonyl)-4-piperidone (5.00 g) was reacted with 4-butoxyaniline (3.95 g) in the same manner as described in Referential Example 37 to give a brown powder of the title compound.
Yield: 6.91 g (83%).
$^1$H-NMR (400 MHz, $CDCl_3$) δ: 0.96 (t, 3H, J=7.2 Hz), 1.23-1.35 (m, 2H), 1.42-1.53 (m, 2H), 1.46 (s, 9H), 1.68-1.76 (m, 2H), 1.97-2.05 (m, 2H), 2.84-2.95 (m, 2H), 3.28-3.37 (m, 1H), 3.88 (t, 2H, J=6.6 Hz), 3.96-4.12 (m, 2H), 6.57 (d, 2H, J=9.0 Hz), 6.77 (d, 2H, J=8.8 Hz).

Referential Example 109

Synthesis of 1-(tert-butoxycarbonyl)-4-[N-(4-butoxyphenyl)-N-[[2-(3,4,5-trimethoxyphenyl)pyridin-4-yl]methyl]amino] piperidine:

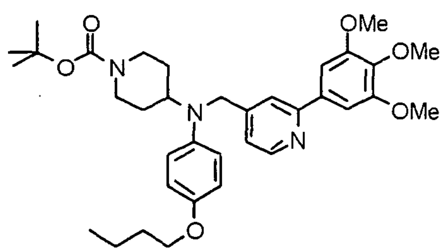

1-(tert-Butoxycarbonyl)-4-[(4-butoxyphenyl)amino]piperidine (696 mg) was reacted with 4-chloromethyl-2-(3,4,5-trimethoxyphenyl)pyridine (588 mg) in the same manner as described in Preparation Example 9 to give a light yellow amorphous substance of the title compound.
Yield: 980 mg (81%).
$^1$H-NMR (400 MHz, $CDCl_3$) δ: 0.95 (t, 3H, J=7.4 Hz), 1.40-1.50 (m, 2H), 1.44 (s, 9H), 1.67-1.76 (m, 2H), 1.82-1.90 (m, 2H), 1.82-1.90 (m, 2H), 2.70-2.82 (m, 2H), 3.61-3.71 (m, 1H), 3.84-3.90 (m, 5H), 3.94 (s, 6H), 4.10-4.28 (m, 2H), 4.39 (s, 2H), 6.74 (d, 2H, J=9.4 Hz), 6.78 (d, 2H, J=9.4 Hz), 7.14-7.18 (m, 3H), 7.56 (s, 1H), 8.54 (d, 1H, J=5.1 Hz).

Referential Example 110

Synthesis of 4-[N-(4-butoxyphenyl)-N-[[2-(3,4,5-trimethoxyphenyl)pyridin-4-yl]methyl]amino] piperidine dihydrochloride:

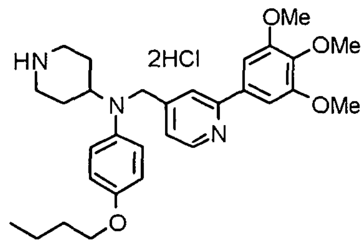

1-(tert-Butoxycarbonyl)-4-[N-(4-butoxyphenyl)-N-[[2-(3,4,5-trimethoxyphenyl)pyridin-4-yl]methyl]amino]piperidine (980 mg) was treated in the same manner as described in Referential Example 94 to give a yellow powder of the title compound.
Yield: 926 mg (99%).

Referential Example 111

Synthesis of 1-(tert-butoxycarbonyl)-4-[N-(4-butoxyphenyl)-N-[[3-(3,4,5-trimethoxyphenyl)pyridin-5-yl]methyl]amino] piperidine:

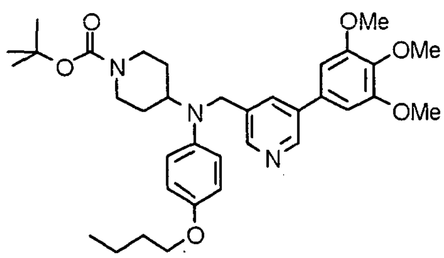

1-(tert-Butoxycarbonyl)-4-[(4-butoxyphenyl)amino]piperidine (697 mg) was reacted with 5-chloromethyl-3-(3,4,5-trimethoxyphenyl)pyridine (588 mg) in the same manner as described in Preparation Example 9 to give a light yellow amorphous substance of the title compound.
Yield: 485 mg (40%).
$^1$H-NMR (400 MHz, $CDCl_3$) δ: 0.95 (t, 3H, J=7.4 Hz), 1.40-1.57 (m, 2H), 1.44 (s, 9H), 1.67-1.75 (m, 2H), 1.82-1.90 (m, 2H), 2.69-2.81 (m, 2H), 3.51-3.60 (m, 1H), 3.87 (q, 2H, J=6.6 Hz), 3.88 (s, 3H), 3.90 (s, 6H), 4.06-4.23 (m, 2H), 4.39 (s, 2H), 6.66 (s, 2H), 6.77 (d, 2H, J=9.2 Hz), 6.81 (d, 2H, J=9.2 Hz), 6.81 (d, 2H, J=9.4 Hz), 7.67 (s, 1H), 8.49 (d, 1H, J=1.8 Hz), 8.62 (d, 1H, J=2.2 Hz).

Referential Example 112

Synthesis of 4-[N-(4-butoxyphenyl)-N-[[3-(3,4,5-trimethoxyphenyl)pyridin-5-yl]methyl]amino] piperidine dihydrochloride:

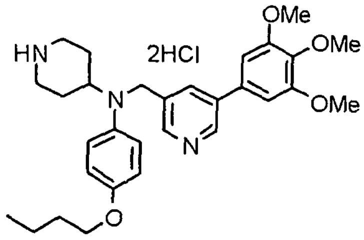

1-(tert-Butoxycarbonyl)-4-[N-(4-butoxyphenyl)-N-[[3-(3,4,5-trimethoxyphenyl)pyridin-5-yl]methyl]amino]piperidine (485 mg) was treated in the same manner as described in Referential Example 94 to give a light yellow powder of the title compound.
Yield: 456 mg (98%).

Referential Example 113

Synthesis of 1-(tert-butoxycarbonyl)-4-[N-(4-'outoxyphenyl)-N-[3-(3,4,5-trimethoxyphenyl)benzyl] amino]piperidine:

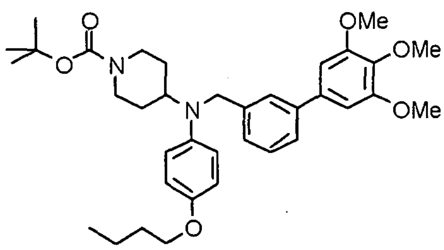

1-(tert-Butoxycarbonyl)-4-[(4-butoxyphenyl)amino]piperidine (697 mg) and 3-(3,4,5-trimethoxyphenyl)benzyl chloride (586 mg) were treated in the same manner as described in Preparation Example 9 to give a light yellow amorphous substance of the title compound.
Yield: 1.17 g (97%).
$^1$H-NMR (400 MHz, $CDCl_3$) δ: 0.95 (t, 3H, J=7.3 Hz), 1.40-1.61 (m, 4H), 1.44 (s, 9H), 1.67-1.75 (m, 2H), 1.83-1.90 (m, 2H), 2.70-2.83 (m, 2H), 3.63-3.72 (m, 2H), 3.87 (q, 2H, J=6.6 Hz), 3.88 (s, 3H), 3.90 (s, 6H), 4.09-4.28 (m, 2H), 4.41 (s, 2H), 6.70 (s, 2H), 6.76 (s, 4H), 7.26 (d, 2H, J=8.0 Hz), 7.33 (t, 1H, J=7.6 Hz), 7.38 (d, 1H, J=7.3 Hz), 7.42 (s, 1H).

Referential Example 114

Synthesis of 4-[N-(4-butoxyphenyl)-N-[3-(3,4,5-trimethoxyphenyl)benzyl]amino]piperidine hydrochloride:

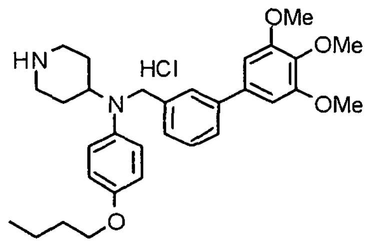

1-(tert-Butoxycarbonyl)-4-[N-(4-butoxyphenyl)-N-[3-(3,4,5-trimethoxyphenyl)benzyl]amino]piperidine (1.17 g) was treated in the same manner as described in Referential Example 94 to give light yellow powder of the title compound.
Yield: 1.02 g (98%).

Preparation Examples 80 to 87

These compounds were obtained by the reaction of the amines obtained in Referential Examples 110, 112 and 114 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 80 | 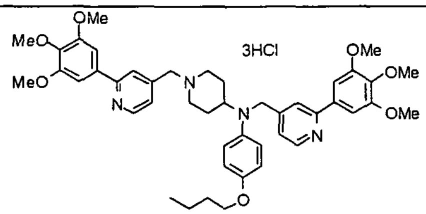 | 63% | 0.95 (t, 3H, J=7.3 Hz), 1.40-1.51 (m, 2H), 1.66-1.79 (m, 2H), 1.83-1.92 (m, 2H), 2.10-2.21 (m, 2H), 2.92-3.02 (m, 2H), 3.53-3.63 (m, 3H), 3.84-3.90 (m, 2H), 3.89 (s, 3H), 3.93 (s, 6H), 3.96 (s, 6H), 6.72 (d, 2H, J=9.3 Hz), 6.77 (d, 2H, J=9.3 Hz), 7.15 (s, 2H), 7.17 (d, 1H, J=5.1 Hz), 7.20 (d, 1H, J=6.1 Hz), 7.22 (s, 2H), 7.57 (s, 1H), 7.59 (s, 1H), 8.54 (d, 1H, J=4.9 Hz), 8.59 (d, 1H, J=5.1 Hz). |
| 81 | 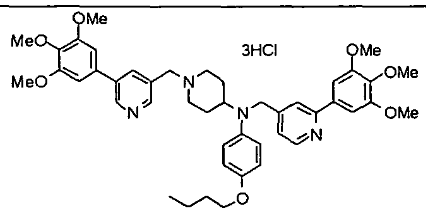 | 44% | 0.95 (t, 3H, J=7.4 Hz), 1.42-1.51 (m, 2H), 1.67-1.76 (m, 4H), 1.80-1.91 (m, 2H), 2.08-2.20 (m, 2H), 2.92-3.03 (m, 2H), 3.84-3.96 (m, 3H), 3.89 (s, 3H), 3.90 (s, 3H), 3.93 (s, 12H), 4.43 (s, 2H), 6.69-6.79 (m, 6H), 7.14 (s, 2H), 7.16 (d, 1H, J=5.2 Hz), 7.55 (s, 1H), 7.76 (s, 1H), 8.49 (d, 1H, J=1.8 Hz), 8.53 (d, 1H, J=5.0 Hz), 8.69 (s, 1H). |
| 82 | 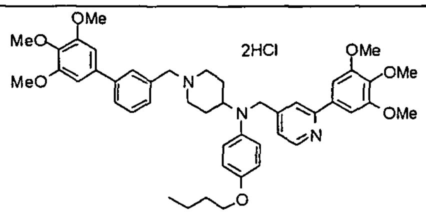 | 53% | 0.95 (t, 3H, J=7.2 Hz), 1.40-1.51 (m, 2H), 1.65-1.78 (m, 4H), 1.81-1.89 (m, 2H), 2.05-2.18 (m, 2H), 3.05-3.06 (m, 2H), 3.54-3.65 (m, 3H), 3.84-3.96 (m, 20H), 4.44 (s, 2H), 6.70 (d, 2H, J=9.2 Hz), 6.74-6.80 (m, 4H), 7.11-7.19 (m, 3H), 7.22-7.32 (m, 1H), 7.34-7.50 (m, 3H), 7.55 (s, 1H), 8.53 (d, 1H, J=5.1 Hz). |
| 83 | 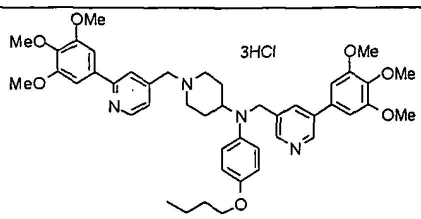 | 42% | 0.95 (t, 3H, 7.4Hz), 1.40-1.51 (m, 2H), 1.67-1.86 (m, 6H), 2.03-2.30 (m, 2H), 2.92-3.06 (m, 2H), 3.46-3.56 (m, 1H), 3.60 (s, 2H), 3.84-3.91 (m, 2H), 3.88 (s, 3H), 3.89 (s, 6H), 3.90 (s, 3H), 3.96 (s, |

| | | | |
|---|---|---|---|
| | | | 6H), 4.44 (s, 2H), 6.65 (s, 2H), 6.74-6.81 (m, 4H), 7.20 (d, 1H, J=4.9 Hz), 7.25 (s, 2H), 7.67(br, 2H), 8.50 (d, 1H, J=1.6 Hz), 8.60 (d, 1H, J=5.6 Hz). |
| 84 | 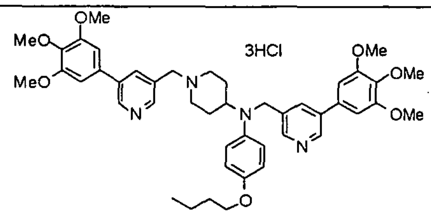 | 36% | 0.95 (t, 3H, J=7.4 Hz), 1.40-1.51 (m, 2H), 1.66-1.79 (m, 4H), 1.82-1.92 (m, 2H), 2.00-2.22 (m, 2H), 2.83-3.06 (m, 2H), 3.44-3.67 (m, 3H), 3.82-3.97 (m, 2H), 3.88 (s, 3H), 3.89 (s, 6H), 3.90 (s, 3H), 3.93 (s, 6H), 4.44 (s, 2H), 6.65 (s, 2H), 6.72-6.80 (m, 6H), 7.67 (s, 1H), 7.76(br, 1H), 8.47-8.53 (m, 2H), 8.62 (d, 1H, J=2.2 Hz), 8.70 (s, 1H). |
| 85 | 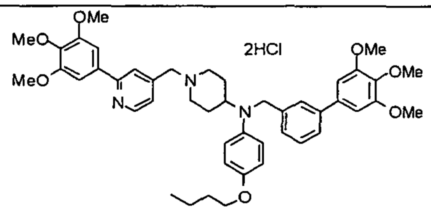 | 72% | 0.95 (t, 3H, J=7.3 Hz), 1.40-1.51 (m, 2H), 1.66-1.82 (m, 4H), 1.84-1.92 (m, 2H), 2.10-2.20 (m, 2H), 2.92-3.00 (m, 2H), 3.53-3.66 (m, 3H), 3.83-3.92 (m, 2H), 3.88 (s, 3H), 3.89 (s, 6H), 3.90 (s, 3H), 3.96 (s, 6H), 4.47 (s, 2H), 6.67 (s, 2H), 6.73 (d, 2H, J=9.2 Hz), 6.77 (d, 2H, J=9.5 Hz), 7.18-7.29 (m, 4H), 7.33 (dd, 1H, J=7.3 Hz, 7.3 Hz), 7.37 (d, 1H, J=7.6 Hz), 7.43 (s, 1H), 7.60 (s, 1H), 8.58 (d, 1H, J=4.9 Hz). |
| 86 | 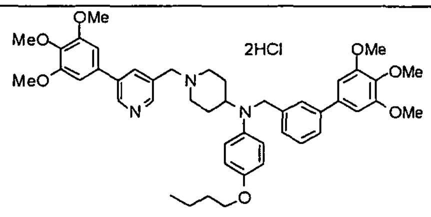 | 24% | 0.94 (t, 3H, J=7.4 Hz), 1.41-1.51 (m, 2H), 1.61-1.80 (m, 4H), 1.82-1.92 (m, 2H), 2.08-2.19 (m, 2H), 2.92-3.02 (m, 2H), 3.55-3.65 (m, 1H), 3.57 (s, 2H), 3.84-3.91 (m, 2H), 3.87 (s, 3H), 3.88 (s, 6H), 3.89 (s, 3H), 3.93 (s, 6H), 4.45 (s, 2H), 6.69 (s, 2H), 6.71-6.78 (m, 4H), 6.75 (s, 2H), 7.23-7.28 (m, 1H), 7.32 (t, 1H, J=7.4 Hz), 7.36 (d, 1H, J=7.6 Hz), 7.42 (s, 1H), 7.77 (s, 1H), 8.49 (d, 1H, J=1.6 Hz), 8.69 (s, 1H). |

| | | | |
|---|---|---|---|
| 87 | 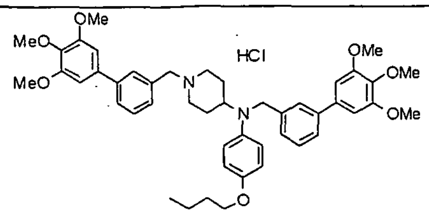 | 78% | 0.94 (t, 3H, J=7.3 Hz), 1.40-1.50 (m, 2H), 1.66-1.88 (m, 4H), 1.82-1.89 (m, 2H), 2.04-2.16 (m, 2H), 2.96-3.03 (m, 2H), 3.55-3.65 (m, 3H), 3.83-3.90 (m, 2H), 3.87 (s, 3H), 3.89 (s, 9H), 3.92 (s, 6H), 4.46 (s, 2H), 6.69-6.79 (m, 9H), 7.23-7.48 (m, 7H). |

Referential Example 115

Synthesis of 4-(*m*-anisidino)-1-(tert-butoxycarbonyl)piperidine:

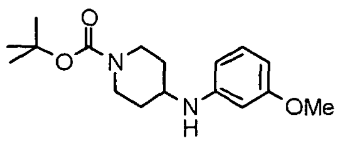

1-(tert-Butoxycarbonyl)-4-piperidone (4.78 g) was reacted with *m*-anisidine (2.96 g) in the same manner as described in Referential Example 37 to give the title compound.
Yield: 4.83 g (66%).
$^1$H-NMR (400 MHz, $CDCl_3$) δ: 1.20-1.39 (m, 2H), 1.44 (s, 9H), 1.99-2.05 (m, 2H), 2.89 (dt, 2H, J=13.5 Hz, 2.2 Hz), 3.33-3.44 (m, 1H), 3.75 (s, 3H), 3.96-4.07 (m, 2H), 6.14 (t, 1H, J=2.2 Hz), 6.18-6.29 (m, 2H), 7.05 (t, 1H, J=8.1 Hz).

Referential Example 116

Synthesis of 1-(tert-butoxycarbonyl)-4-[N-(3-methoxyphenyl)-N-[[2-(3,4,5-trimethoxyphenyl) pyridin-4-yl]methyl] amino]piperidine:

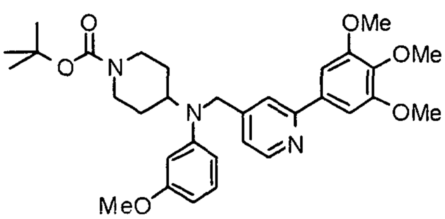

4-(*m*-Anisidino)-1-(tert-butoxycarbonyl)piperidine (613 mg) was reacted with 4-chloromethyl-2-(3,4,5-trimethoxyphenyl)pyridine (588 mg) in the same manner as described in Preparation Example 9 to give a light yellow amorphous substance of the title compound.
Yield: 789 mg (70%).
$^1$H-NMR (400 MHz, $CDCl_3$) δ: 1.45 (s, 9H), 1.50-1.67 (m, 2H), 1.82-1.91 (m, 2H), 2.74-2.87 (m, 2H), 3.74 (s, 3H), 3.88-3.98 (m, 1H), 3.89 (s, 3H), 3.94 (s, 6H), 4.14-4.32 (m, 2H), 4.48 (s, 2H), 6.28 (dd, 1H, J=2.2 Hz, 2.2 Hz), 6.31-6.37 (m, 2H), 7.10-7.15 (m, 2H), 7.16 (s, 2H), 7.55 (s, 1H), 8.56 (d, 1H, J=5.1 Hz).

Referential Example 117

Synthesis of 4-[N-(3-methoxyphenyl)-N-[[2-(3,4,5-trimethoxyphenyl)pyridin-4-yl]methyl]amino] piperidine dihydrochloride:

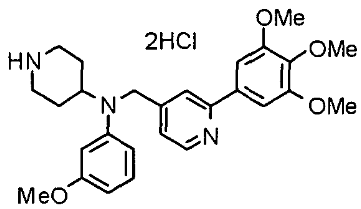

1-(tert-Butoxycarbonyl)-4-[N-(3-methoxyphenyl)-N-[[2-(3,4,5-trimethoxyphenyl)pyridin-4-yl]methyl]amino] piperidine (789 mg) was treated in the same manner as described in Referential Example 94 to give light yellow powder of the title compound.
Yield: 710 mg (95%).

Referential Example 118

Synthesis of 1-(tert-butoxycarbonyl)-4-[N-(3-methoxyphenyl)-N-[[3-(3,4,5-trimethoxyphenyl) pyridin-5-yl]methyl] amino]piperidine:

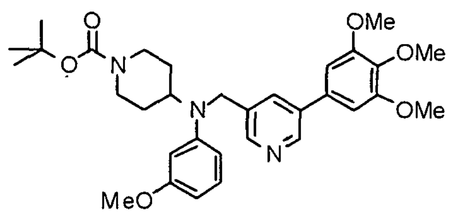

4-(*m*-Anisidino)-1-(tert-butoxycarbonyl)piperidine (613 mg) was reacted with 5-chloromethyl-3-(3,4,5-trimethoxyphenyl)pyridine (588 mg) in the same manner as described in Preparation Example 9 to give a light yellow amorphous substance of the title compound.
Yield: 396 mg (35%).
$^1$H-NMR (400 MHz, $CDCl_3$) δ: 1.45 (s, 9H), 1.54-1.66 (m, 2H), 1.81-1.91 (m, 2H), 2.73-2.87 (m, 2H), 3.74 (s, 3H), 3.87-3.93 (m, 1H), 3.88 (s, 3H), 3.90 (s, 6H), 4.14-4.29 (m, 2H), 4.51 (s, 2H), 6.30-6.35 (m, 2H), 6.38 (d, 1H, J=7.2 Hz), 6.68 (s, 2H), 7.12 (dd, 1H, J=8.8 Hz, 8.88 Hz), 7.66 (s, 1H), 8.49 (d, 1H, J=2.0 Hz), 8.66 (d, 1H, J=2.2 Hz).

Referential Example 119

Synthesis of 4-[N-(3-methoxyphenyl)-N-[[3-(3,4,5-trimethoxyphenyl)pyridin-5-yl]methyl]amino] piperidine dihydrochloride:

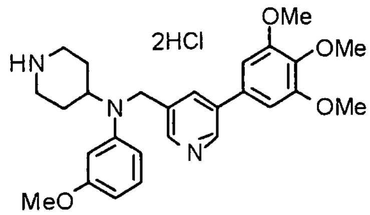

1-(tert-Butoxycarbonyl)-4-[N-(3-methoxyphenyl)-N-[[3-(3,4,5-trimethoxyphenyl)pyridin-5-yl]methyl]amino] piperidine (396 mg) was treated in the same manner as described in Referential Example 94 to give a light yellow powder of the title compound.
Yield: 348 mg (92%).

Referential Example 120

Synthesis of 1-(tert-butoxycarbonyl)-4-[N-(3-methoxyphenyl)-N-[3-(3,4,5-trimethoxyphenyl)benzyl] amino]piperidine:

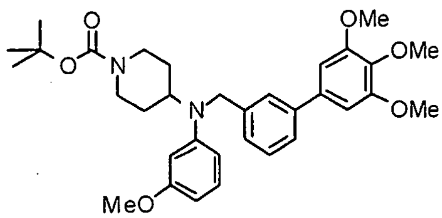

4-(*m*-Anisidino)-1-(tert-butoxycarbonyl)piperidine (613 mg) was reacted with 3-(3,4,5-trimethoxyphenyl)benzyl chloride (586 mg) in the same manner as described in Preparation Example 9 to give a light yellow amorphous substance of the title compound.
Yield: 1.01 g (90%).
$^1$H-NMR (400 MHz, $CDCl_3$) δ: 1.44 (s, 9H), 1.56-1.67 (m, 2H), 1.83-1.91 (m, 2H), 2.72-2.86 (m, 2H), 3.73 (s, 3H), 3.85-3.98 (m, 1H), 3.88 (s, 3H), 3.90 (s, 6H), 4.12-4.30 (m, 2H), 4.50 (s, 2H), 6.27-6.34 (m, 2H), 6.38 (dd, 1H, J=8.2 Hz, 2.4 Hz), 6.72 (s, 2H), 7.10 (dd, 1H, J=8.2 Hz, 8.2 Hz), 7.21-7.27 (m, 1H), 7.32-7.43 (m, 3H).

Referential Example 121

Synthesis of 4-[N-(3-methoxyphenyl)-N-[3-(3,4,5-trimethoxyphenyl)benzyl]amino]piperidine hydrochloride:

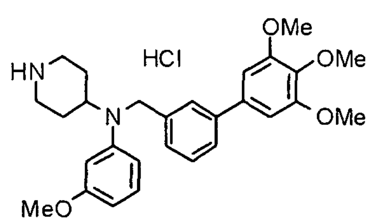

1-(tert-Butoxycarbonyl)-4-[N-(3-methoxyphenyl)-N-[3-(3,4,5-trimethoxyphenyl)benzyl]amino]piperidine (1.01 g) was treated in the same manner as described in Referential Example 94 to give light yellow powder of the title compound. Yield: 820 mg (92%).

Preparation Examples 88 to 95

These compounds were obtained by the reaction of the amines obtained in Referential Examples 117, 119 and 121 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 88 | 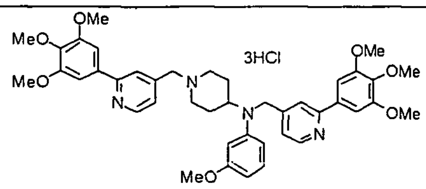 | 63% | 1.70-1.82 (m, 2H), 1.83-1.90 (m, 2H), 2.14-2.23 (m, 2H), 2.94-3.01 (m, 2H), 3.57 (s, 2H), 3.73 (s, 3H), 3.76-3.88 (m, 1H), 3.89 (s, 3H), 3.90 (s, 3H), 3.93 (s, 6H), 3.96 (s, 6H), 4.53 (s, 2H), 6.26-6.35 (m, 3H), 7.11 (dd, 1H, J=8.3 Hz, 8.3 Hz), 7.12-7.14 (m, 1H), 7.15 (s, 2H), 7.20 (d, 1H, J=5.1 Hz), 7.22 (s, 2H), 7.55 (s, 1H), 7.58 (s, 1H), 8.55 (d, 1H, J=4.9 Hz), 8.59 (d, 1H, J=4.9 Hz). |

| | | | |
|---|---|---|---|
| 89 | 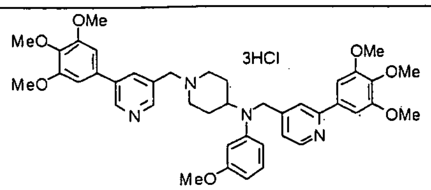 | 72% | 1.67-1.90 (m, 4H), 2.13-2.22 (m, 2H), 2.94-3.04 (m, 2H), 3.59 (s, 2H), 3.74 (s, 3H), 3.77-3.87 (m, 1H), 3.89 (s, 3H), 3.89 (s, 3H), 3.92 (s, 6H), 3.93 (s, 6H), 4.52 (s, 2H), 6.27 (dd, 1H, J=2.4 Hz, 2.4 Hz), 6.29-6.34 (m, 2H), 6.75 (s, 2H), 7.08-7.17 (m, 4H), 7.54 (s, 1H), 7.75 (s, 1H), 8.50 (d, 1H, J=1.8 Hz), 8.54 (d, 1H, J=5.1 Hz), 8.69 (d, 1H, J=2.0 Hz). |
| 90 | 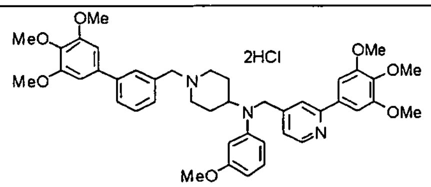 | 60% | 1.68-1.90 (m, 4H), 2.09-2.19 (m, 2H), 2.97-3.06 (m, 2H), 3.58 (s, 2H), 3.73 (s, 3H), 3.76-3.87 (m, 1H), 3.89 (s, 6H), 3.92 (s, 6H), 3.92 (s, 6H), 4.52 (s, 2H), 6.25-6.35 (m, 3H), 6.76 (s, 2H), 6.78-7.17 (m, 4H), 7.25-7.32 (m, 1H), 7.37 (dd, 1H, J=7.4 Hz, 7.4 Hz), 7.41-7.47 (m, 2H), 7.54 (s, 1H), 8.54 (d, 1H, J=5.1 Hz). |
| 91 | 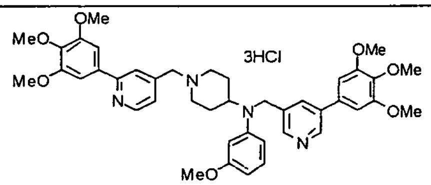 | 50% | 1.80-1.93 (m, 4H), 2.13-2.32 (m, 2H), 2.87-3.10 (m, 2H), 3.60 (s, 1H), 3.69-3.85 (m, 1H), 3.73 (s, 3H), 3.88 (s, 3H), 3.89 (s, 6H), 3.90 (s, 3H), 3.96 (s, 6H), 4.57 (s, 2H), 6.29-6.34 (m, 2H), 6.37 (dd, 1H, J=8.2 Hz, 8.1 Hz), 6.67 (s, 2H), 7.11 (dd, 1H, J=8.6 Hz, 8.6 Hz), 7.20-7.28 (m, 3H), 7.58-7.72 (m, 1H), 7.68 (s, 1H), 8.50 (d, 1H, J=1.8 Hz), 8.60 (d, 1H, J=4.7 Hz), 8.65 (d, 1H, J=2.0 Hz). |
| 92 | 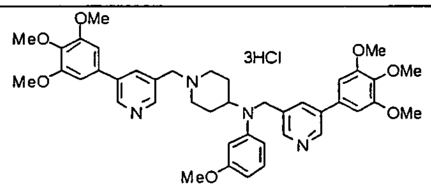 | 35% | 1.70-1.90 (m, 4H), 2.12-2.25 (m, 2H), 2.95-3.03 (m, 2H), 3.59 (s, 2H), 3.72-3.97 (m, 1H), 3.73 (s, 3H), 3.88 (s, 3H), 3.89 (s, 6H), 3.90 (s, 3H), 3.93 (s, 6H), 4.54 (s, 2H), 6.25-6.38 (m, 2H), 6.36 (d, 1H, J=8.4 Hz, 8.4 Hz), 6.67 (s, 2H), 6.75 (s, 2H), 7.11 (dd, 1H, J=8.4 Hz), 7.66 (s, 1H), 8.49 (s, 1H), 8.50 (d, 1H, J=1.8 Hz), 8.64 (d, 1H, J=2.0 Hz), 8.70 (d, 1H, J=1.9 Hz). |

| | | | |
|---|---|---|---|
| 93 | 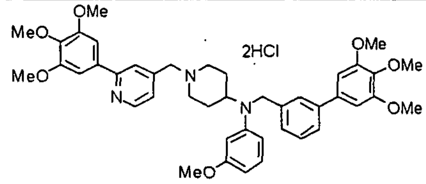 | 86% | 1.73-1.93 (m, 4H), 2.13-2.23 (m, 2H), 2.94-3.02 (m, 2H), 3.57 (s, 2H), 3.73 (s, 3H), 3.77-3.87 (m, 1H), 3.88 (s, 3H), 3.88 (s, 6H), 3.90 (s, 3H), 3.96 (s, 6H), 4.56 (s,2H), 6.27 (dd, 1H, J=8.0 Hz, 2.2 Hz), 6.31 (dd, 1H, J=2.2 Hz, 2.2 Hz), 6.36 (dd, 1H, J=8.2 Hz, 2.2 Hz), 6.71 (s, 2H), 7.09 (dd, 1H, J=8.1 Hz, 8.1 Hz), 7.18-7.28 (m, 4H), 7.34 (dd, 1H, J=7.4 Hz, 7.4 Hz), 7.38 (d, 1H, J=7.6 Hz), 7.42 (s, 1H), 7.59 (s, 1H), 8.59 (d, 1H, J=4.9 Hz). |
| 94 | 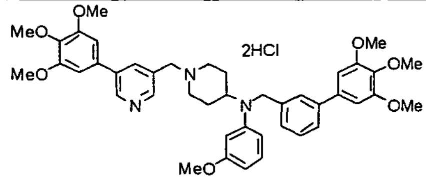 | 56% | 1.72-1.92 (m, 4H), 2.10-2.23 (m, 2H), 2.92-3.60 (m, 2H), 3.59 (s, 2H), 3.72 (s, 3H), 3.77-3.89 (m, 1H), 3.87 (s, 3H), 3.88 (s, 6H), 3.93 (s, 6H), 4.55 (s, 2H), 6.27 (dd, 1H, J=8.0 Hz, 2.2 Hz), 6.31 (dd, 1H, J=2.1 Hz, 2.1 Hz), 6.36 (dd, 1H, J=8.4 Hz, 2.4 Hz), 6.70 (s, 2H), 6.75 (s, 2H), 7.09 (dd, 1H, J=8.2 Hz, 8.2 Hz), 7.22 (d, 1H, J=7.4 Hz), 7.33 (dd, 1H, J=7.4 Hz, 7.4 Hz), 7.38 (d, 1H, J=7.8 Hz), 7.40 (s, 1H), 7.77 (s, 1H), 8.50 (d, 1H, J=1.8 Hz), 8.69 (d, 1H, J=1.8 Hz). |
| 95 | 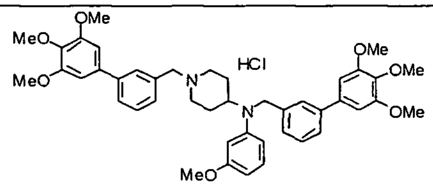 | 77% | 1.66-1.89 (m, 4H), 2.08-2.18 (m, 2H), 2.95-3.05 (m, 2H), 3.58 (s, 2H), 3.72 (s, 3H), 3.75-3.84 (m, 1H), 3.87 (s, 3H), 3.88 (s, 6H), 3.89 (s, 3H), 3.92 (s, 6H), 4.55 (s, 2H), 6.26 (dd, 1H, J=8.0 Hz, 2.2 Hz), 6.30 (dd, 1H, J=2.2 Hz, 2.2 Hz), 6.36 (dd, 1H, J=8.3 Hz, 2.2 Hz), 6.70 (s, 2H), 6.76 (s, 2H), 7.08 (dd, 1H, J=8.3 Hz, 8.3 Hz), 7.22 (d, 1H, J=7.3 Hz), 7.27-7.47 (m, 7H). |

Referential Example 122

Synthesis of 4-(*o*-anisidino)-1-(tert-butoxycarbonyl)piperidine:

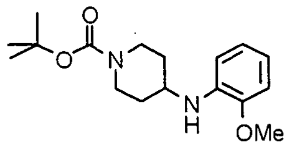

1-(tert-Butoxycarbonyl)-4-piperidone (4.78 g) was reacted with o-anisidine (2.96 g) in the same manner as described in Referential Example 37 to give the title compound.
Yield: 2.61 g (36%).
$^1$H-NMR (400 MHz, $CDCl_3$) 5: 1.31-1.41 (m, 2H), 1.47 (s, 9H), 2.00-2.08 (m, 2H), 2.90-3.01 (m, 2H), 3.38-3.47 (m, 1H), 3.83 (s, 3H), 4.00-4.21 (m, 2H), 6.60-6.69 (m, 2H), 6.76-6.89 (m, 2H).

Referential Example 123

Synthesis of 1-(tert-butoxycarbonyl)-4-[N-(2-methoxyphenyl)-N-[[2-(3,4,5-trimethoxyphenyl) pyridin-4-yl]methyl] amino]piperidine:

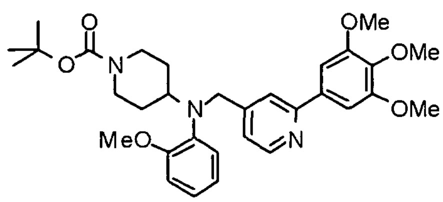

4-(*o*-Anisidino)-1-(tert-butoxycarbonyl)piperidine (613 mg) was reacted with 4-chloromethyl-2-(3,4,5-trimethoxyphenyl)pyridine (588 mg) in the same manner as described in Preparation Example 9 to give a light yellow amorphous substance of the title compound.
Yield: 763 mg (68%).
$^1$H-NMR (400 MHz, $CDCl_3$) δ: 1.41-1.58 (m, 2H), 1.44 (s, 9H), 1.81-1.91 (m, 2H), 2.62-2.78 (m, 2H), 3.29 (tt, 1H, J=7.6 Hz, 3.7 Hz), 3.86 (s, 3H), 3.89 (s, 3H), 3.95 (s, 6H), 4.06-4.16 (m, 2H), 4.37 (s, 2H), 6.80 (ddd, 1H, J=7.6 Hz, 7.6 Hz, 1.2 Hz), 6.87 (dd, 1H, J=8.5 Hz, 1.0 Hz), 7.00-7.06 (m, 2H), 7.14 (s, 2H), 7.20 (dd, 1H, J=4.9 Hz, 1.0 Hz), 7.61 (s, 1H), 8.49 (d, 1H, J=4.9 Hz).

Referential Example 124

Synthesis of 4-[N-(2-methoxyphenyl)-N-[[2-(3,4,5-trimethoxyphenyl)pyridin-4-yl]methyl]amino] piperidine dihydrochloride:

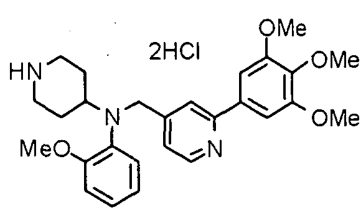

1-(tert-Butoxycarbonyl)-4-[N-(2-methoxyphenyl)-N-[[2-(3,4,5-trimethoxyphenyl)pyridin-4-yl]methyl]amino] piperidine (763 mg) was treated in the same manner as described in Referential Example 94 to give a light yellow powder of the title compound.
Yield: 701 mg (97%).

Referential Example 125

Synthesis of 1-(tert-butoxycarbonyl)-4-[N-(2-methoxyphenyl)-N-[[3-(3,4,5-trimethoxyphenyl) pyridin-5-yl]methyl] amino]piperidine:

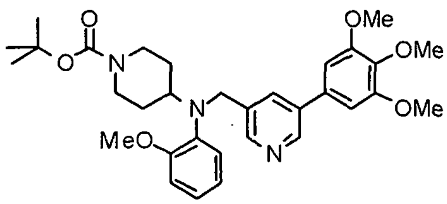

4-(*o*-Anisidino)-1-(tert-butoxycarbonyl)piperidine (613 mg) was reacted with 5-chloromethyl-3-(3,4,5-trimethoxyphenyl)pyridine (588 mg) in the same manner as described in Preparation Example 9 to give a light yellow amorphous substance of the title compound.
Yield: 353 mg (31%).
$^1$H-NMR (400 MHz, $CDCl_3$) δ: 1.44 (s, 9H), 1.46-1.53 (m, 2H), 1.82-1.91 (m, 2H), 2.62-2.78 (m, 2H), 3.24-3.33 (m, 1H), 3.83 (s, 3H), 3.89 (s, 3H), 3.91 (s, 6H), 4.03-4.16 (m, 2H), 4.37 (s, 2H), 6.64 (s, 2H), 6.79 (ddd, 1H, J=7.6 Hz, 7.6 Hz, 1.2 Hz), 6.84 (dd, 1H, J=7.0 Hz, 1.2 Hz), 6.97-7.06 (m, 2H), 7.68 (dd, 1H, J=1.3 Hz, 1.3 Hz), 8.49 (d, 1H, J=2.0 Hz), 8.56 (d, 1H, J=2.2 Hz).

Referential Example 126

Synthesis of 4-[N-(2-methoxyphenyl)-N-[[3-(3,4,5-trimethoxyphenyl)pyridin-5-yl]methyl]amino] piperidine dihydrochloride:

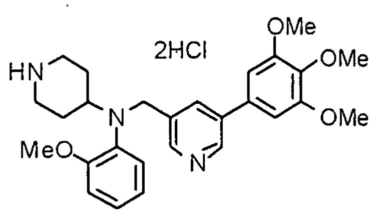

1-(tert-Butoxycarbonyl)-4-[N-(2-methoxyphenyl)-N-[[3-(3,4,5-trimethoxyphenyl)pyridin-5-yl]methyl]amino] piperidine (353 mg) was treated in the same manner as described in Referential Example 94 to give a light yellow powder of the title compound.
Yield: 312 mg (93%).

Referential Example 127

Synthesis of 1-(tert-butoxycarbonyl)-4-[N-(2-methoxyphenyl)-N-[3-(3,4,5-trimethoxyphenyl)benzyl] amino]piperidine:

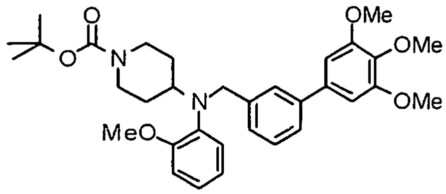

4-(*o*-Anisidino)-1-(tert-butoxycarbonyl)piperidine (613 mg) was reacted with 3-(3,4,5-trimethoxyphenyl)benzyl chloride (586 mg) in the same manner as described in Preparation Example 9 to give a light yellow amorphous substance of the title compound.
Yield: 1.12 g (100%).
$^1$H-NMR (400 MHz, $CDCl_3$) δ: 1.43 (s, 9H), 1.46-1.57 (m, 2H), 1.81-1.90 (m, 2H), 2.62-2.76 (m, 2H), 3.31 (tt, 1H, J=11.1 Hz, 3.3 Hz), 3.84 (s, 3H), 3.88 (s, 3H), 3.91 (s, 6H), 4.00-4.16 (m, 2H), 4.36 (s, 2H), 6.67 (s, 2H), 6.78 (t, 1H, J=7.3 Hz), 6.85 (d, 1H, J=7.9 Hz), 6.96-7.03 (m, 2H), 7.24-7.34 (m, 3H), 7.43 (s, 1H).

Referential Example 128

Synthesis of 4-[N-(2-methoxyphenyl)-N-[3-(3,4,5-trimethoxyphenyl)benzyl]amino]piperidine hydrochloride:

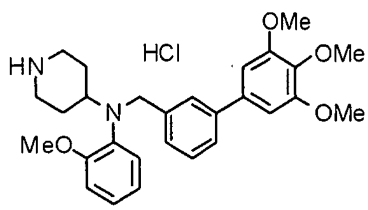

1-(tert-Butoxycarbonyl)-4-[N-(2-methoxyphenyl)-N-[3-(3,4,5-trimethoxyphenyl)benzyl]amino]piperidine (1.12 g) was treated in the same manner as described in Referential Example 94 to give a light yellow powder of the title compound.
Yield: 987 mg (99%).

Preparation Examples 96 to 101

These compounds were obtained by the reaction of the amines obtained in Referential Examples 124, 126 and 128 with the chloride derivatives obtained in Referential Examples 3 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 96 | 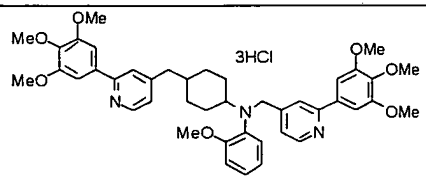 | 73% | 1.62-1.74 (m, 2H), 1.82-1.90 (m, 2H), 1.98-2.08 (m, 2H), 2.86-2.94 (m, 2H), 3.13-3.22 (m, 1H), 3.52 (s, 2H), 3.85 (s, 3H), 3.89 (s, 3H), 3.90 (s, 3H), 3.94 (s, 6H), 3.96 (s, 6H), 4.40 (s, 2H), 6.80 (ddd, 1H, J=7.6 Hz, 7.6 Hz, 1.2 Hz), 6.86 (dd, 1H, J=8.1 Hz,1.2 Hz), 6.98-7.05 (m, 1H), 7.14 (s, 2H), 7.18 (dd, 1H, J=4.9 Hz, 1.2 Hz), 7.20-7.24 (m, 1H), 7.22 (s, 2H), 7.58 (s, 1H), 7.62 (s, 1H), 8.49 (d, 1H, J=4.9 Hz), 8.57 (d, 1H, J=5.2 Hz). |
| 97 | 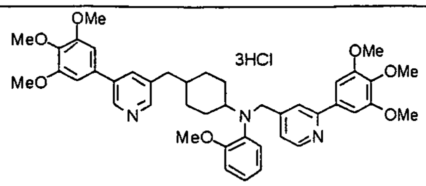 | 55% | 1.60-1.73 (m, 4H), 1.82-1.93 (m, 2H), 1.98-2.07 (m, 2H), 2.87-2.97 (m, 2H), 3.12-3.22 (m, 1H), 3.54 (s, 2H), 3.85 (s, 3H), 3.89 (s, 3H), 3.90 (s, 3H), 3.93 (s, 6H), 3.94 (s, 6H), 4.39 (s, 2H), 6.75 (s, 2H), |

| | | | |
|---|---|---|---|
| | | | 6.79 (dd, 1H, J=7.4 Hz, 7.4 Hz), 6.86 (d, 1H, J=7.8 Hz), 6.97-7.05 (m, 2H), 7.13 (s, 2H), 7.20 (d, 1H, J=4.7 Hz), 7.61 (s, 1H), 7.75 (s, 1H), 8.46-8.50 (m, 2H), 8.68 (d, 1H, J=2.0 Hz). |
| 98 | 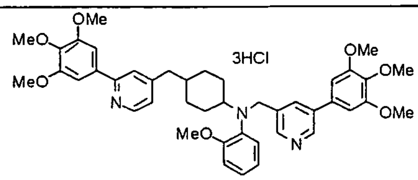 | 29% | 1.64-1.82 (m, 2H), 1.84-1.97 (m, 2H), 2.00-2.15 (m, 2H), 2.84-3.01 (m, 2H), 3.13-3.27 (m, 1H), 3.56 (s, 2H), 3.82 (s, 3H), 3.88 (s, 3H), 3.90 (s, 3H), 3.91 (s, 6H), 3.96 (s, 6H), 4.40 (s, 2H), 6.63 (s, 2H), 6.75-6.88 (m, 2H), 6.97-7.04 (m, 2H), 7.19 (d, 1H, J=4.3 Hz), 7.25 (s, 2H), 7.58-7.73 (m, 2H), 8.50 (d, 1H, J=1.6 Hz), 8.56 (d, 1H, J=2.2 Hz), 8.58 (d, 1H, J=4.9 Hz). |
| 99 | 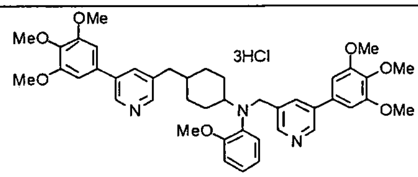 | 30% | 1.62-1.75 (m, 2H), 1.83-1.94 (m, 2H), 1.95-2.11 (m, 2H), 2.84-3.01 (m, 2H), 3.12-3.23 (m, 1H), 3.55 (s, 2H), 3.82 (s, 3H), 3.88 (s, 3H), 3.90 (s, 3H), 3.90 (s, 6H), 3.93 (s, 6H), 4.39 (s, 2H), 6.63 (s, 2H), 6.70-6.86 (m, 4H), 6.94-7.06 (m, 2H), 7.68 (s, 1H), 7.76 (s, 1H), 8.47 (d, 1H, J=1.7 Hz), 8.49 (d, 1H, J=1.7 Hz), 8.55 (d, 1H, J=2.2 Hz), 8.69 (s, 1H). |
| 100 | 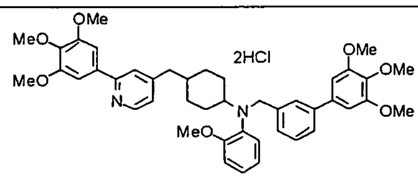 | 67% | 1.64-1.79 (m, 2H), 1.85-1.93 (m, 2H), 1.99-2.09 (m, 2H), 2.86-2.95 (m, 2H), 3.16-3.26 (m, 1H), 3.52 (s, 2H), 3.84 (s, 3H), 3.88 (s, 3H), 3.90 (s, 6H), 3.96 (s, 6H), 4.40 (s, 2H), 6.67 (s, 2H), 6.78 (dd, 1H, J=7.4 Hz, 7.4 Hz), 6.85 (d, 1H, J=8.2 Hz), 6.97 (dd, 1H, J=7.8 Hz, 7.8 Hz), 7.02 (dd, 1H, J=7.8, 1.6 Hz), 7.17-7.33 (m, 6H), 7.44 (s, 1H), 7.59 (s, 1H), 8.57 (d, 1H, J=5.1 Hz). |
| 101 | 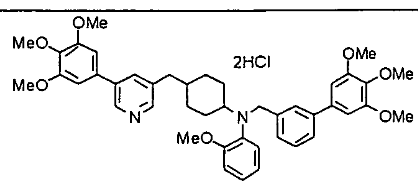 | 55% | 1.62-1.77 (m, 2H), 1.82-1.94 (m, 2H), 1.98-2.08 (m, 2H), 2.86-2.96 (m, 2H), 3.16-3.26 (m, 1H), 3.54 (s, 2H), 3.83 (s, 3H), 3.87 (s, 3H), 3.90 (s, 9H), 3.93 (s, 6H), 4.39 (s, 2H), 6.66 (s, 2H), 6.73-6.80 (m, 3H), 6.84 (d, 1H, J=7.8 Hz), 6.97 |

| | | | |
|---|---|---|---|
| | | | (dd, 1H, J=7.8 Hz, 7.8 Hz), 7.01 (d, 1H, J=7.8 Hz), 7.23-7.32 (m, 3H), 7.43 (s, 1H), 7.77 (s, 1H), 8.47 (d, 1H, J=1.4 Hz), 8.68 (d, 1H, J=1.8 Hz). |

Referential Example 129

Synthesis of 1-(tert-butoxycarbonyl)-4-(2,3-dimethoxyphenylamino)piperidine:

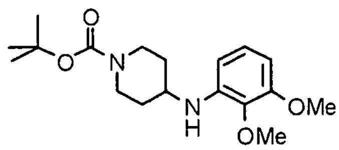

1-(tert-Butoxycarbonyl)-4-piperidone (4.78 g) was reacted with 2,3-dimethoxyaniline (3.68 g) in the same manner as described in Referential Example 37 to give the title compound.
Yield: 3.18 g (39%).
$^1$H-NMR (400 MHz, $CDCl_3$) δ: 1.29-1.42 (m, 2H), 1.45 (s, 9H), 1.97-2.03 (m, 2H), 2.92 (dt, 2H, J=13.5 Hz, 2.2 Hz), 3.38 (dt, 1H, J=13.8 Hz, 4.1 Hz), 3.77 (s, 3H), 3.82 (s, 3H), 3.99-4.03 (m, 2H), 4.17 (m, 1H), 6.27-6.32 (m, 2H), 6.88 (t, 1H, J=8.4 Hz).

Referential Example 130

Synthesis of 1-(tert-butoxycarbonyl)-4-[N-(2, 3-dimethoxyphenyl)-N-[[2-(3,4, 5-trimethoxyphenyl) pyridin-4-yl]methyl] amino]piperidine:

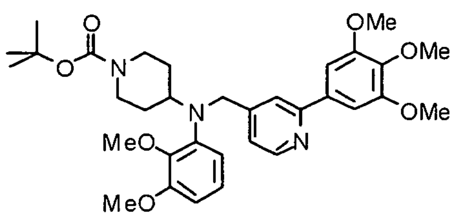

1-(tert-Butoxycarbonyl)-4-(2,3-dimethoxyphenylamino)piperidine (673 mg) was reacted with 4-chloromethyl-2-(3,4,5-trimethoxyphenyl)pyridine (588 mg) in the same manner as described in Preparation Example 9 to give a light yellow amorphous substance of the title compound.
Yield: 613 mg (52%).
$^1$H-NMR (400 MHz, $CDCl_3$) δ: 1.45 (s, 9H), 1.56-1.70 (m, 2H), 1.84-1.91 (m, 2H), 2.62-2.76 (m, 2H), 3.58 (tt, 1H, J=11.8 Hz, 3.6 Hz), 3.83 (s, 3H), 3.89 (s, 6H), 3.93 (s, 6H), 4.08-4.25 (m, 2H), 4.35 (s, 2H), 6.56-6.63 (m, 2H), 6.86 (t, 1H, J=8.3 Hz), 7.14 (s, 2H), 7.17 (dd, 1H, J=5.1 Hz, 1.2 Hz), 7.62 (s, 1H), 8.50 (d, 1H, J=5.1 Hz).

Referential Example 131

Synthesis of 4-[N-(2,3-dimethoxyphenyl)-N-[[2-(3,4,5-trimethoxyphenyl)pyridin-4-yl]methyl] amino]piperidine dihydrochloride:

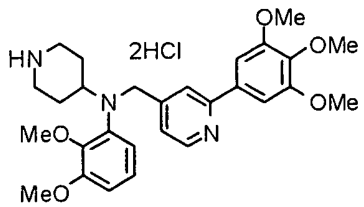

1-(tert-Butoxycarbonyl)-4-[N-(2,3-dimethoxyphenyl)-N-[[2-(3,4,5-trimethoxyphenyl)pyridin-4-yl]methyl]amino]piperidine (613 mg) was treated in the same manner as described in Referential Example 94 to give a light yellow powder of the title compound.
Yeld: 512 mg (88%).

Preparation Example 102

Synthesis of 4-[N-(2,3-dimethoxyphenyl)-N-[[2-(3,4,5-trimethoxyphenyl)pyridin-4-yl]methyl] amino]-1-[[2-(3,4,5-trimethoxyphenyl)pyridin-4-yl]methyl]piperidine trihydrochloride:

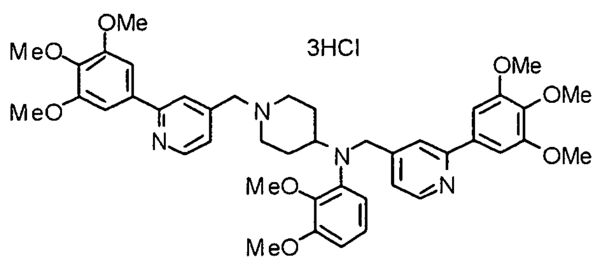

4-[N-(2,3-Dimethoxyphenyl)-N-[[2-(3,4,5-trimethoxyphenyl)pyridin-4-yl] methyl]]piperidine dihydrochloride (113 mg) was reacted with 4-chloromethyl-2-(3,4,5-trimethoxyphenyl)pyridine (59 mg) in the same manner as described in Preparation Example 2. The title compound was obtained as a light yellow powder after converting the product to a hydrochloride.
Yield: 21 mg (12%).
$^1$H-NMR (400 MHz, measured as a free base, $CDCl_3$) δ: 1.76-1.96 (m, 4H), 2.00-2.13 (m, 2H), 2.86-3.00 (m, 2H), 3.42-3.60 (m, 1H), 3.54 (s, 2H), 3.82 (s, 3H), 3.88 (s, 3H), 3.90 (s, 3H), 3.97 (s, 6H), 4.41 (s, 2H), 6.57 (d, 1H, J=8.0 Hz), 6.62 (d, 1H, J=8.2 Hz), 6.85 (dd, 1H, J=8.4 Hz, 8.4 Hz), 7.11-7.29 (m, 6H), 7.59 (s, 1H), 7.63 (s, 1H), 8.50 (d, 1H, J=4.9 Hz), 8.59 (d, 1H, J=4.9 Hz).

Referential Example 132

Synthesis of 1-(tert-butoxycarbonyl)-4-[4-[(trifluoromethoxy)phenyl]amino]piperidine:

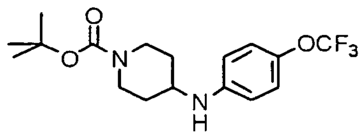

1-(tert-Butoxycarbonyl)-4-piperidone (5.00 g) was reacted with 4-(trifluoromethoxy)aniline (4.23 g) in the same manner as described in Referential Example 37 to give a white powder of the title compound.
Yield: 5.22 g (60%).
$^1$H-NMR (400 MHz, $CDCl_3$) δ: 1.25-1.40 (m, 2H), 1.47 (s, 9H), 1.98-2.08 (m, 2H), 2.83-2.98 (m, 2H), 3.34-3.43 (m, 1H), 3.97-4.12 (m, 2H), 6.58 (d, 2H, J=8.8 Hz), 7.03 (d, 2H, J=8.8 Hz).

Referential Example 133

Synthesis of 1-(tert-butoxycarbonyl)-4-[N-[4-(trifluoromethoxy)phenyl]-N-[[2-(3,4,5-trimethoxyphenyl)pyridin-4-yl] methyl]amino]piperidine:

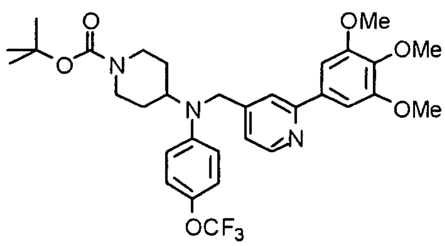

1-(tert-Butoxycarbonyl)-4-[4-[(trifluoromethoxy)phenyl]amino]piperidine (721 mg) was reacted with 4-chloromethyl-2-(3,4,5-trimethoxyphenyl)pyridine (588 mg) in the same manner as described in Preparation Example 9 to give a light yellow amorphous substance of the title compound.
Yield: 543 mg (44%).
$^1$H-NMR (400 MHz, $CDCl_3$) δ: 1.45 (s, 9H), 1.52-1.66 (m, 2H), 1.81-1.91 (m, 2H), 2.73-2.88 (m, 2H), 3.88-3.99 (m, 1H), 3.89 (s, 3H), 3.93 (s, 6H), 4.15-4.34 (m, 2H), 4.48 (s, 2H), 6.68 (d, 2H, J=9.2 Hz), 7.07 (d, 2H, J=8.6 Hz), 7.12 (dd, 1H, J=5.2 Hz, 1.3 Hz), 7.15 (s, 2H), 7.52 (s, 1H), 8.58 (d, 1H, J=5.2 Hz).

Referential Example 134

Synthesis of 4-[N-[4-(trifluoromethoxy)phenyl]-N-[[2-(3,4,5-trimethoxyphenyl)pyridin-4-yl]methyl] amino]piperidine dihydrochloride:

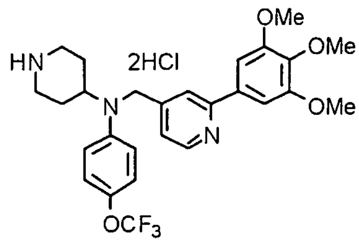

1-(tert-Butoxycarbonyl)-4-[N-[4-(trifluoromethoxy)phenyl]-N-[[2-(3,4,5-trifluoromethoxyphenyl)pyridin-4-yl] methyl]amino]piperidine (543 mg) was treated in the same manner as described in Referential Example 94 to give a light yellow powder of the title compound.
Yield: 481 mg (93%).

Referential Example 135

Synthesis of 1-(tert-butoxycarbonyl)-4-[N-[4-(trifluoromethoxy)phenyl]-N-[[3-(3,4,5-trimethoxyphenyl)pyridin-5-yl] methyl]amino]piperidine:

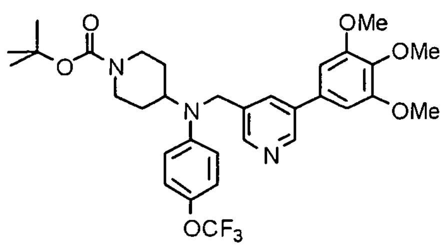

1-(tert-Butoxycarbonyl)-4-[4-[(trifluoromethoxy)phenyl]amino]piperidine (721 mg) and 5-chloromethyl-3-(3,4,5-trimethoxyphenyl)pyridine (588 mg) were treated in the same manner as described in Preparation Example 9 to give a light yellow amorphous substanceof the title compound.
Yield: 201 mg (16%).
$^1$H-NMR (400 MHz, $CDCl_3$) δ: 1.45 (s, 9H), 1.54-1.67 (m, 2H), 1.82-1.90 (m, 2H), 2.74-2.86 (m, 2H), 3.84-3.91 (m, 1H), 3.88 (s, 3H), 3.89 (s, 6H), 4.16-4.30 (m, 2H), 4.52 (s, 2H), 6.67 (s, 2H), 6.72 (d, 2H, J=9.4 Hz), 7.06 (d, 2H; J=8.4 Hz), 7.64 (t, 1H, J=2.1 Hz), 8.49 (d, 1H, J=2.2 Hz), 8.68 (d, 1H, J=2.1 Hz).

Referential Example 136

Synthesis of 4-[N-[4-(trifluoromethoxy)phenyl]-N-[[3-(3,4,5-trimethoxyphenyl)pyridin-5-yl]methyl] amino]piperidine dihydrochloride:

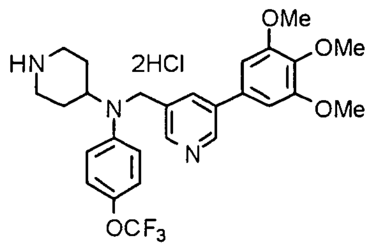

1-(tert-Butoxycarbonyl)-4-[N-[4-(trifluoromethoxy)phenyl]-N-[[3-(3,4,5-trimethoxyphenyl)pyridin-5-yl]methyl] amino]piperidine (201 mg) was treated in the same manner as described in Referential Example 94 to give a light yellow powder of the title compound.
Yield: 185 mg (96%).

Referential Example 137

Synthesis of 1-(tert-butoxycarbonyl)-4-[N-[(4-trifluoromethoxy)phenyl]-N-[3-(3,4,5-trimethoxyphenyl)benzyl]amino] piperidine:

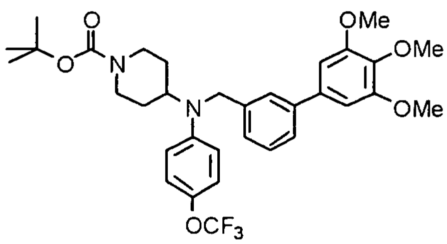

1-(tert-Butoxycarbonyl)-4-[4-[(trifluoromethoxy)phenyl]amino]piperidine (721 mg) was reacted with 3-(3,4,5-trimethoxyphenyl)benzyl chloride (586 mg) in the same manner as described in Preparation Example 9 to give a light yellow amorphous substanceof the title compound.
Yield: 1.06 mg (86%).
$^1$H-NMR (400 MHz, $CDCl_3$) δ: 1.45 (s, 9H), 1.56-1.68 (m, 2H), 1.83-1.90 (m, 2H), 2.71-2.86 (m, 2H), 3.87-3.90 (m, 1H), 3.88 (s, 3H), 3.89 (s, 6H), 4.16-4.29 (m, 2H), 4.51 (s, 2H), 6.70 (d, 2H, J=9.3 Hz), 6.70 (s; 2H), 7.04 (d, 2H, J=8.5 Hz), 7.22 (d, 1H, J=7.8 Hz), 7.34-7.44 (m, 3H).

Referential Example 138

Synthesis of 4-[N-[4-(trifluoromethoxy)phenyl]-N-[3-(3,4,5-trimethoxyphenyl)benzyl]amino] piperidine hydrochloride:

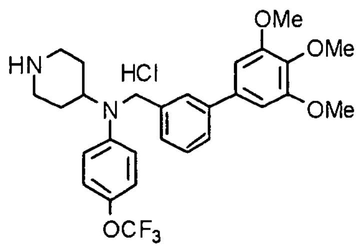

1-(tert-Butoxycarbonyl)-4-[N-[(4-trifluoromethoxy)phenyl]-N-[3-(3,4,5-trimethoxyphenyl)benzyl]amino]piperidine (1.06 g) was treated in the same manner as described in Referential Example 94 to give a light yellow powder of the title compound.
Yield: 795 mg (84%).

Preparation Examples 103 to 110

These compounds were obtained by the reaction of the amines obtained in Referential Examples 134, 136 and 138 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 103 | 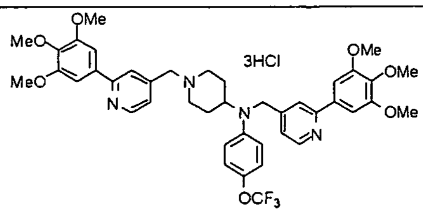 | 70% | 1.71-1.90 (m, 4H), 2.15-2.23 (m, 2H), 2.95-3.02 (m, 2H), 3.58 (s, 2H), 3.76-3.85 (m, 1H), 3.89 (s, 3H), 3.90 (s, 3H), 3.92 (s, 6H), 3.96 (s, 6H), 4.54 (s, 2H), 6.66 (d, 2H, J=9.3 Hz), 7.05 (d, 2H, J=8.5 Hz), 7.13 (dd, 1H, J=5.1 Hz, 1.2 |

| | | | |
|---|---|---|---|
| | | | Hz), 7.14 (s, 2H), 7.20 (dd, 1H, J=4.9 Hz, 1.2 Hz), 7.22 (s, 2H), 7.53 (s, 1H), 7.59 (s, 1H), 8.57 (d, 1H, J=4.9 Hz), 8.59 (d, 1H, J=5.2 Hz). |
| 104 | 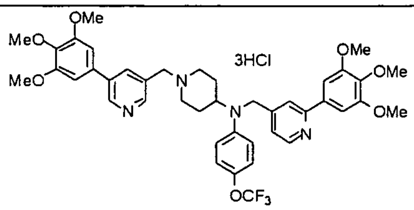 | 48% | 1.68-1.92 (m, 4H), 2.13-2.25 (m, 2H), 2.95-3.06 (m, 2H), 3.60 (s, 2H), 3.75-3.87 (m, 1H), 3.89 (s, 3H), 3.90 (s, 3H), 3.91 (s, 6H), 3.93 (s, 6H), 4.52 (s, 2H), 6.65 (d, 2H, J=9.4 Hz), 6.75 (s, 2H), 7.05 (d, 2H, J=9.2 Hz), 7.12 (d, 1H, J=5.1 Hz), 7.14 (s, 2H), 7.52 (s, 1H), 7.76 (s, 1H), 8.51 (d, 1H, J=1.8 Hz), 8.57 (d, 1H, J=5.1 Hz), 8.70 (d, 1H, J=2.1 Hz). |
| 105 | 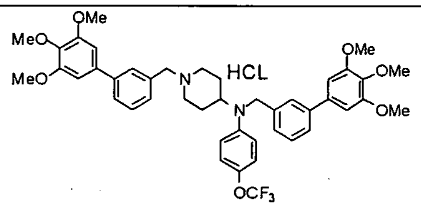 | 69% | 1.70-1.89 (m, 4H), 2.10-2.19 (m, 2H), 2.98-3.08 (m, 2H), 3.59 (s, 2H), 3.72-3.84 (m, 1H), 3.89 (s, 6H), 3.92 (s, 6H), 3.92 (s, 6H), 4.52 (s, 2H), 6.65 (d, 2H, J=9.4 Hz), 6.76 (s, 2H), 7.04 (d, 2H, J=8.6 Hz), 7.11 (d, 1H, J=5.1 Hz), 7.14 (s, 2H), 7.25-7.33 (m, 1H), 7.37 (dd, 1H, J=7.4 Hz, 7.4 Hz), 7.41-7.48 (m, 2H), 7.51 (s, 1H), 8.56 (d, 1H, J=5.1 Hz). |
| 106 | 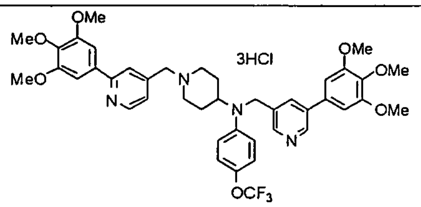 | 41% | 1.73-1.93 (m, 4H), 2.12-2.26 (m, 2H), 2.93-3.07 (m, 2H), 3.53-3.65 (m, 2H), 3.74-3.84 (m, 1H), 3.88 (s, 9H), 3.90 (s, 3H), 3.96 (s, 6H), 4.58 (s, 2H), 6.66 (s, 2H), 6.69 (d, 2H, J=9.2 Hz), 7.05 (d, 2H, J=8.8 Hz), 7.18-7.29 (m, 3H), 7.59(br, 1H), 7.64 (s, 1H), 8.49 (s, 1H), 8.60 (d, 1H, J=5.3 Hz), 8.67 (d, 1H, J=2.0 Hz). |
| 107 | 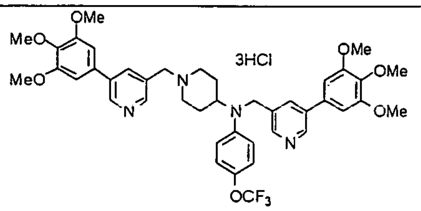 | 28% | 1.72-1.91 (m, 4H), 2.12-2.28 (m, 2H), 2.94-3.06 (m, 2H), 3.60 (s, 2H), 3.76-3.82 (m, 1H), 3.88 (s, 9H), 3.90 (s, 3H), 3.93 (s, 6H), 4.56 (s, 2H), 6.65 (s, 2H), 6.69 (d, 2H, J=9.2 Hz), 6.75 (s, 2H), 7.05 (d, 2H, J=8.8 Hz), 7.63 (s, 1H), 7.76 (s, 1H), 8.48 (d, 1H, J=1.8 Hz), 8.51 (d, 1H, J=1.8 Hz), 8.66 (d, 1H, J=2.2 Hz), 8.70 (d, 1H, |

| | | | |
|---|---|---|---|
| | | | J=2.2 Hz). |
| 108 | 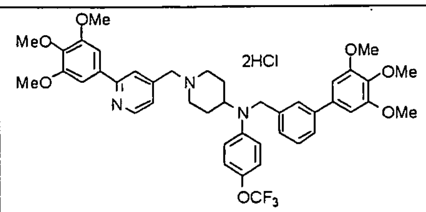 | 78% | 1.76-1.91 (m, 4H), 2.14-2.23 (m, 2H), 2.94-3.03 (m, 2H), 3.57 (s, 2H), 3.75-3.84 (m, 1H), 3.87 (s, 9H), 3.90 (s, 3H), 3.96 (s, 6H), 4.56 (s, 2H), 6.65-6.72 (m, 4H), 7.03 (d, 2H, J=8.8 Hz), 7.18-7.24 (m, 4H), 7.33-7.43 (m, 3H), 7.59 (s, 1H), 8.59 (d, 1H, J=4.9 Hz). |
| 109 | 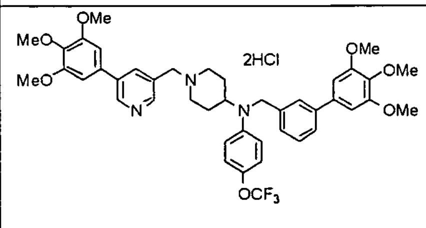 | 5% | 1.72-1.90 (m, 4H), 2.12-2.21 (m, 2H), 2.94-3.03 (m, 2H), 3.59 (s, 2H), 3.73-3.86 (m, 1H), 3.87 (s, 9H), 3.90 (s, 3H), 3.93 (s, 6H), 4.54 (s, 2H), 6.66-6.70 (m, 4H), 6.75 (s, 2H), 7.03 (d, 2H, J=9.0 Hz), 7.21 (d, 1H, J=7.2 Hz), 7.32-7.41 (m, 3H), 7.76 (s, 1H), 8.50 (d, 1H, J=1.6 Hz), 8.69 (d, 1H, J=1.6 Hz). |
| 110 | 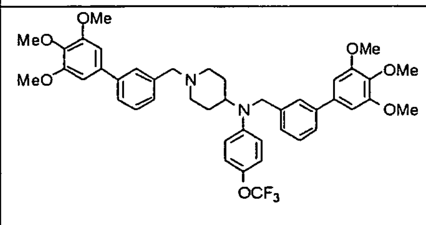 | 62% | 1.72-1.89 (m, 4H), 2.08-2.20 (m, 2H), 2.97-3.07 (m, 2H), 3.59 (s, 2H), 3.73-3.83 (m, 1H), 3.87 (s, 9H), 3.89 (s, 3H), 3.92 (s, 6H), 4.55 (s, 2H), 6.67 (d, 2H, J=9.3 Hz), 6.69 (s, 2H), 6.76 (s, 2H), 7.02 (d, 2H, J=8.6 Hz), 7.20 (d, 1H, J=7.6 Hz), 7.25-7.47 (m, 7H). |

Referential Example 139

Synthesis of 1-(tert-butoxycarbonyl)-4-[[4-(methylthio)phenyl]amino]piperidine:

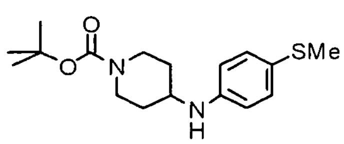

1-(tert-Butoxycarbonyl)-4-piperidone (5.00 g) was reacted with 4-(methylthio)aniline (3.33 g) in the same manner as described in Referential Example 37 to give a white powder of the title compound.
Yield: 3.80 g (49%).
$^1$H-NMR (400 MHz, $CDCl_3$) δ: 1.26-1.38 (m, 2H), 1.46 (s, 9H), 1.98-2.06 (m, 2H), 2.41 (s, 3H), 2.88-2.97 (m, 2H), 3.36-3.45 (m, 2H), 3.48-3.56 (br, 1H), 3.96-4.12 (m, 2H); 6.55 (d, 2H, J=8.8 Hz), 7.21 (d, 2H, J=8.8 Hz).

Referential Example 140

Synthesis of 1-(tert-butoxycarbonyl)-4-[N-[4-(methylthio)phenyl]-N-[[2-(3,4,5-trimethoxyphenyl) pyridin-4-yl]methyl] amino]piperidine:

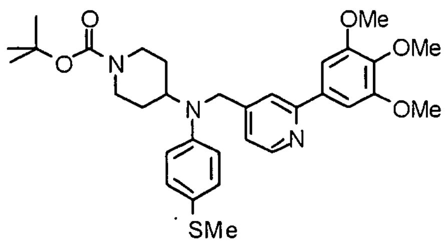

1-(tert-Butoxycarbonyl)-4-[[4-(methylthio)phenyl]amino]piperidine (644 mg) and 4-chloromethyl-2-(3,4,5-trimethoxyphenyl)pyridine (588 mg) were treated in the same manner as described in Preparation Example 9 to give a light yellow amorphous substanceof the title compound.
Yield: 671 mg (58%).
$^1$H-NMR (400 MHz, $CDCl_3$) δ: 1.45 (s, 9H), 1.50-1.66 (m, 2H), 1.81-1.89 (m, 2H), 2.40 (s, 3H), 2.74-2.87 (m, 2H), 3.88-3.94 (m, 1H), 3.90 (s, 3H), 3.94 (s, 6H), 4.15-4.29 (m, 2H), 4.48 (s, 2H), 6.67 (d, 2H, J=9.0 Hz), 7.11-7.18 (m, 1H), 7.16 (s, 2H), 7.22 (d, 2H, J=6.6 Hz), 7.54 (s, 1H), 8.57 (d, 1H, J=5.1 Hz).

Referential Example 141

Synthesis of 4-[N-[4-(methylthio)phenyl]-N-[[2-(3,4,5-trimethoxyphenyl)pyridin-4-yl]methyl] amino]piperidine dihydrochloride:

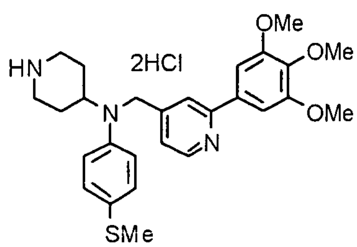

1-(tert-Butoxycarbonyl)-4-[N-[4-(methylthio)phenyl]-N-[[2-(3,4,5-trifluoromethoxyphenyl)pyridin-4-yl]methyl] amino]piperidine (671 mg) was treated in the same manner as described in Referential Example 94 to give a light yellow powder of the title compound.
Yeld: 602 mg (94%).

Referential Example 142

Synthesis of 1-(tert-butoxycarbonyl)-4-[N-[4-(methylthio)phenyl]-N-[[3-(3,4,5-trimethoxyphenyl) pyridin-5-yl]methyl] amino]piperidine:

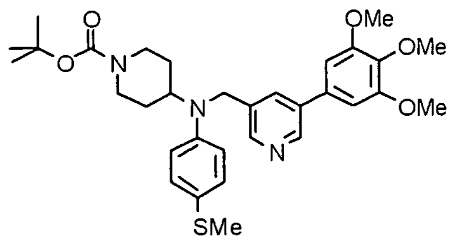

1-(tert-Butoxycarbonyl)-4-[[4-(methylthio)phenyl]amino]piperidine (645 mg) and 5-chloromethyl-3-(3,4,5-trimethoxyphenyl)pyridine (588 mg) were treated in the same manner as described in Preparation Example 9 to give a light yellow amorphous substance of the title compound.
Yield: 312 mg (27%).
$^1$H-NMR (400 MHz, $CDCl_3$) δ: 1.45 (s, 9H), 1.53-1.63 (m, 2H), 1.83-1.89 (m, 2H), 2.40 (s, 3H), 2.73-2.85 (m, 2H), 3.87-3.91 (m, 1H), 3.88 (s, 3H), 3.90 (s, 6H), 4.16-4.30 (m, 2H), 4.50 (s, 2H), 6.67 (s, 2H), 6.71 (d, 2H, J=9.0 Hz), 7.21 (d, 2H, J=9.0 Hz), 7.64 (s, 1H), 8.48 (d, 1H, J=2.2 Hz), 8.66 (d, 1H, J=2.1 Hz).

Referential Example 143

Synthesis of 4-[N-[4-(methylthio)phenyl]-N-[[3-(3,4,5-trimethoxyphenyl)pyridin-5-yl]methyl] amino]piperidine dihydrochloride:

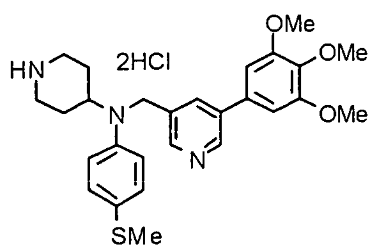

1-(tert-Butoxycarbonyl)-4-[N-[4-(methylthio)phenyl]-N-[[3-(3,4,5-trimethoxyphenyl)pyridin-5-yl]methyl]amino]piperidine (312 mg) was treated in the same manner as described in Referential Example 94 to give a light yellow powder of the title compound.
Yield: 251 mg (84%).

Referential Example 144

Synthesis of 1-(tert-butoxycarbonyl)-4-[N-[(4-methylthio)phenyl]-N-[3-(3,4,5-trimethoxyphenyl) benzyl]amino] piperidine:

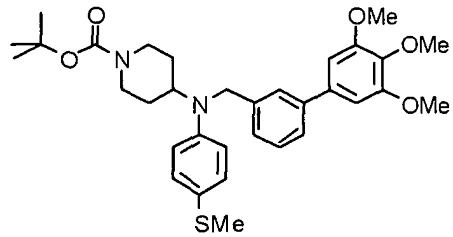

1-(tert-Butoxycarbonyl)-4-[N- [(methylthio)phenyl]amino]piperidine (645 mg) was reacted with 3-(3,4,5-trimethoxyphenyl)benzyl chloride (586 mg) in the same manner as described in Preparation Example 9 to give a light yellow amorphous substance of the title compound.
Yield: 1.10 g (95%).
$^1$H-NMR (400 MHz, $CDCl_3$) δ: 1.45 (s, 9H), 1.55-1.68 (m, 2H), 1.81-1.90 (m, 2H), 2.39 (s, 3H), 2.73-2.86 (m, 2H), 3.87-3.91 (m, 1H), 3.88 (s, 3H), 3.89 (s, 6H), 4.15-4.29(m, 2H), 4.50 (s, 2H), 6.68-6.73 (m, 4H), 7.19-7.24 (m, 3H), 7.33-7.43 (m, 3H).

Referential Example 145

Synthesis of 4-[N-[4-(methylthio)phenyl]-N-[3-(3,4,5-trimethoxyphenyl)benzyl]amino]piperidine hydrochloride:

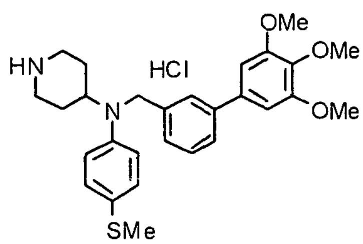

1-(tert-Butoxycarbonyl)-4-[N-[4-(methylthio)phenyl]-N-[3-(3,4,5-trimethoxyphenyl)benzyl]amino]piperidine (1.10 g) was treated in the same manner as described in Referential Example 94 to give light yellow powder of the title compound. Yield: 866 mg (89%).

Preparation Examples 111 to 118

These compounds were obtained by the reaction of the amines obtained in Referential Examples 141, 143 and 145 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 111 | 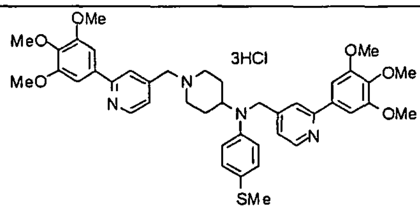 | 40% | 1.70-1.90 (m, 4H), 2.14-2.26 (m, 2H), 2.40 (s, 3H), 2.94-3.04 (m, 2H), 3.58 (s, 2H), 3.76-3.88 (m, 1H), 3.89 (s, 3H), 3.90 (s, 3H), 3.93 (s, 6H), 3.96 (s, 6H), 4.53 (s, 2H), 6.66 (d, 2H, J=9.0 Hz), 7.11-7.24 (m, 8H), 7.54 (s, 1H), 7.59 (s, 1H), 8.56 (d, 1H, J=5.1 Hz), 8.59 (d, 1H, J=5.1 Hz). |
| 112 | 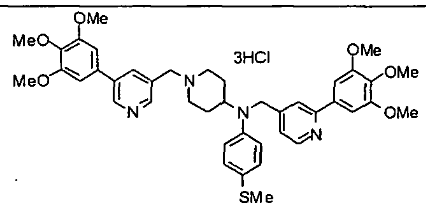 | 53% | 1.66-1.90 (m, 4H), 2.12-2.24 (m, 2H), 2.40 (s, 3H), 2.94-3.05 (m, 2H), 3.59 (s, 2H), 3.73-3.88 (m, 1H), 3.89 (s, 3H), 3.90 (s, 3H), 3.92 (s, 6H), 3.93 (s, 6H), 4.51 (s, 2H), 6.65 (d, 2H, J=8.8 Hz), 6.75 (s, 2H), 7.12 (d, 1H, J=4.9 Hz), 7.14 (s, 2H), 7.21 (d, 2H, J=8.8 Hz), 7.53 (s, 1H), 7.76 (s, 1H), 8.50 (d, 1H, J=1.9 Hz), 6.55 (d, 1H, J=4.9 Hz), 8.69 (d, 1H, J=1.4 Hz). |
| 113 | 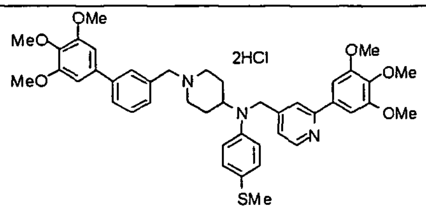 | 53% | 1.68-1.89 (m, 4H), 2.10-2.20 (m, 2H), 2.39 (s, 3H), 2.98-3.07 (m, 2H), 3.58 (s, 2H), 3.75-3.87 (m, 1H), 3.89 (s, 6H), 3.92 (s, 6H), 3.92 (s, 6H), 4.51 (s, 2H), 6.65 (d, 2H, J=9.0 Hz), 6.76 (s, 2H), 7.11 (d, 1H, J=5.1 Hz), 7.14 (s, 2H), 7.21 (d, 2H, J=8.8 Hz), 7.29 (d, 1H, J=7.4 Hz), 7.37 (dd, 1H, J=7.6 Hz, 7.6 Hz), 7.42-7.49 (m, 2H), 7.52 (s, 1H), 8.54 (d, 1H, J=4.9 Hz). |

| | | | |
|---|---|---|---|
| 114 | 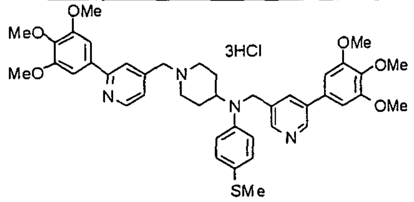 | 50% | 1.57-2.00 (m, 4H), 2.12-2.30 (m, 2H), 2.39 (s, 3H), 2.90-3.13 (m, 2H), 3.50-3.74 (m, 2H), 3.75-3.86 (m, 1H), 3.88 (s, 3H), 3.89 (s, 3H), 3.90 (s, 6H), 3.97 (s, 6H), 4.57 (s, 2H), 6.66 (s, 2H), 6.70 (d, 2H, J=9.0 Hz), 7.17-7.30 (m, 5H), 7.66(br, 2H), 8.48 (s, 1H), 8.58-8.70 (m, 2H). |
| 115 | 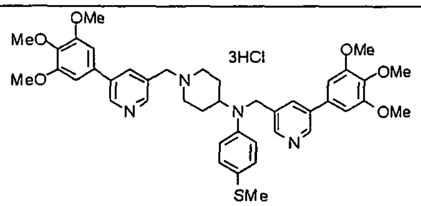 | 59% | 1.68-1.92 (m, 4H), 2.12-2.27 (m, 2H), 2.39 (s, 3H), 2.94-3.08 (m, 2H), 3.60 (s, 2H), 3.74-3.83 (m, 1H), 3.88 (s, 3H), 3.89 (s, 6H), 3.90 (s, 3H), 3.93 (s, 6H), 4.55 (s, 2H), 6.66 (s, 2H), 6.69 (d, 2H, J=8.8 Hz), 6.73-6.80 (m, 2H), 7.20 (d, 2H, J=8.8 Hz), 7.64 (s, 1H), 7.77(br, 1H), 8.48 (s, 1H), 8.50 (s, 1H), 8.65 (s, 1H), 8.71 (s, 1H). |
| 116 | 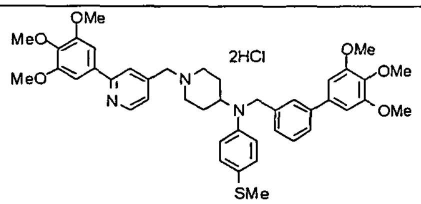 | 85% | 1.76-1.93 (m, 4H), 2.14-2.24 (m, 2H), 2.39 (s, 3H), 2.94-3.03 (m, 2H), 3.57 (s, 2H), 3.76-3.86 (m, 1H), 3.88 (s, 6H), 3.90 (s, 3H), 3.96 (s, 6H), 4.55 (s, 2H), 6.67-6.73 (m, 4H), 7.18-7.29 (m, 6H), 7.34 (dd, 1H, J=7.6 Hz, 7.6 Hz), 7.37-7.44 (m, 2H), 7.59 (s, 1H), 8.59 (d, 1H, J=4.9 Hz). |
| 117 | 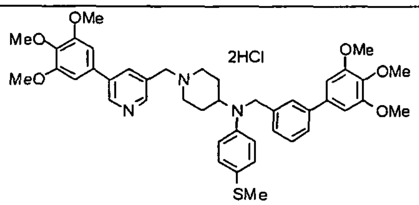 | 53% | 1.72-1.90 (m, 4H), 2.12-2.22 (m, 2H), 2.39 (s, 3H), 2.95-3.05 (m, 2H), 3.59 (s, 2H), 3.74-3.85 (m, 1H), 3.87 (s, 3H), 3.88 (s, 6H), 3.89 (s, 3H), 3.93 (s, 6H), 4.54 (s, 2H), 6.67-6.70 (m, 4H), 6.75 (s, 2H), 7.19-7.23 (m, 3H), 7.33 (dd, 1H, J=7.4 Hz, 7.4 Hz), 7.36-7.40 (m, 2H), 7.76 (s, 1H), 8.50 (d, 1H, J=1.8 Hz), 8.69 (s, 1H). |
| 118 | 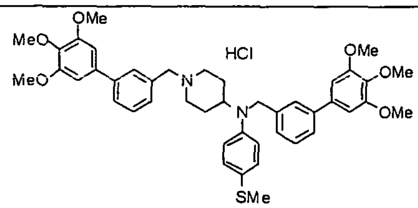 | 83% | 1.72-1.90 (m, 4H), 2.09-2.20 (m, 2H), 2.38 (s, 3H), 2.97-3.06 (m, 2H), 3.58 (s, 2H), 3.73-3.84 (m, 1H), 3.87 (s, 3H), 3.88 (s, 3H), 3.89 (s, 6H), 3.92 (s, 6H), 4.54 (s, 2H), 6.66-6.71 (m, 4H), 6.76 (s, 2H), 7.18-7.24 (m, 3H), 7.26-7.48 (m, 7H). |

Referential Example 146

Synthesis of 1-(tert-butoxycarbonyl)-4-[(4-methylphenyl)amino]piperidine:

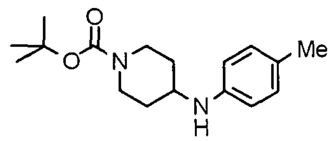

1-(tert-Butoxycarbonyl)-4-piperidone (5.00 g) and *p*-toluidine (2.56 g) was treated in the same manner as described in Referential Example 37 to give white powder of the title compound.
Yield: 5.79 g (83%).
$^1$H-NMR (400 MHz, $CDCl_3$) δ: 1.25-1.36 (m, 2H), 1.46 (s, 9H), 1.99-2.06 (m, 2H), 2.23 (s, 3H), 2.86-2.96 (m, 2H), 3.30-3.43 (m, 2H), 3.96-4.10 (m, 2H), 6.53 (d, 2H, J=8.4 Hz), 6.98 (d, 2H, J=8.0 Hz).

Referential Example 147

Synthesis of 1-(tert-butoxycarbonyl)-4-[N-(4-methylphenyl)-N-[[2-(3,4,5-trimethoxyphenyl)pyridin-4-yl]methyl]amino] piperidine:

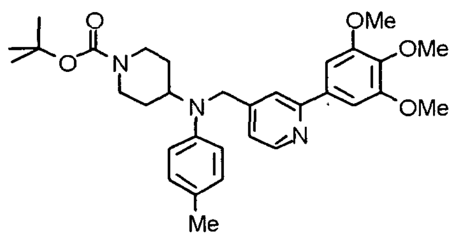

1-(tert-Butoxycarbonyl)-4-[N-(4-methylphenyl)amino]piperidine (581 mg) and 4-chloromethyl-2-(3,4,5-trimethoxyphenyl)pyridine (588 mg) were treated in the same manner as described in Preparation Example 9 to give a light yellow amorphous substance of the title compound.
Yield: 1.00 g (91%).
$^1$H-NMR (400 MHz, $CDCl_3$) δ: 1.45 (s, 9H), 1.55-1.59 (m, 2H), 1.81-1.90 (m, 2H), 2.23 (s, 3H), 2.72-2.86 (m, 2H), 3.81-3.94 (m, 1H), 3.89 (s, 3H), 3.93 (s, 6H), 4.14-4.30 (m, 2H), 4.45 (s, 2H), 6.66 (d, 2H, J=8.6 Hz), 7.02 (d, 2H, J=8.2 Hz), 7.13-7.16 (m, 3H), 7.55 (s, 1H), 8.55 (d, 1H, J=8.1 Hz).

Referential Example 148

Synthesis of 4-[N-(4-methylphenyl)-N-[[2-(3,4,5-trimethoxyphenyl)pyridin-4-yl]methyl]amino] piperidine dihydrochloride:

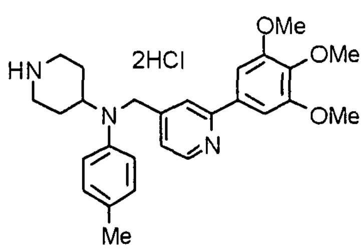

1-(tert-Butoxycarbonyl)-4-[N-(4-methylphenyl)-N-[[2-(3,4,5-trimethoxyphenyl)pyridin-4-yl]methyl]amino]piperidine (1.00 g) was treated in the same manner as described in Referential Example 94 to give light yellow powder of the title compound.
Yield: 924 mg (97%).

Referential Example 149

Synthesis of 1-(tert-butoxycarbonyl)-4-[N-(4-methylphenyl)-N-[[3-(3,4,5-trimethoxyphenyl)pyridin-5-yl]methyl]amino] piperidine:

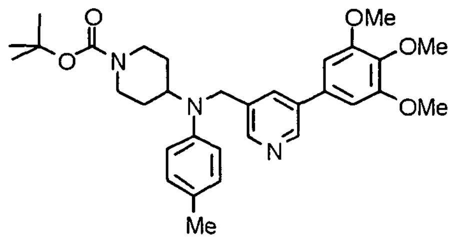

1-(tert-Butoxycarbonyl)-4-[(4-methylphenyl)amino]piperidine (581 mg) and 5-chloromethyl-3-(3,4,5-trimethoxyphenyl)pyridine (588 mg) were treated in the same manner as described in Preparation Example 9 to give a light yellow amorphous substance of the title compound.
Yield: 426 mg (39%).
$^1$H-NMR (400 MHz, $CDCl_3$) δ: 1.45 (s, 9H), 1.52-1.70 (m, 2H), 1.82-1.90 (m, 2H), 2.23 (s, 3H), 2.72-2.86 (m, 2H), 3.77-3.86 (m, 1H), 3.88 (s, 3H), 3.90 (s, 6H), 4.10-4.28 (m, 2H), 4.47 (s, 2H), 6.67 (s, 2H), 6.70 (d, 2H, J=8.6 Hz), 7.01 (d, 2H, J=8.2 Hz), 7.67 (dd, 1H, J=2.1 Hz, 2.1 Hz), 8.50 (d, 1H, J=2.0 Hz), 8.64 (d, 1H, J=2.2 Hz).

Referential Example 150

Synthesis of 4-[N-(4-methylphenyl)-N-[[3-(3,4,5-trimethoxyphenyl)pyridin-5-yl]methyl]amino] piperidine dihydrochloride:

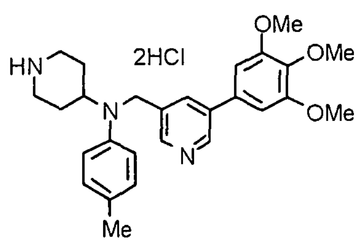

1-(tert-Butoxycarbonyl)-4-[N-(4-methylphenyl)-N-[[3-(3,4,5-trimethoxyphenyl)pyridin-5-yl]methyl]amino]piperidine (426 mg) was treated in the same manner as described in Referential Example 94 to give a light yellow powder of the title compound.
Yield: 400 mg (99%).

Referential Example 151

Synthesis of 1-(tert-butoxycarbonyl)-4-[N-(4-methylphenyl)-N-[3-(3,4,5-trimethoxyphenyl)benzyl] amino]piperidine:

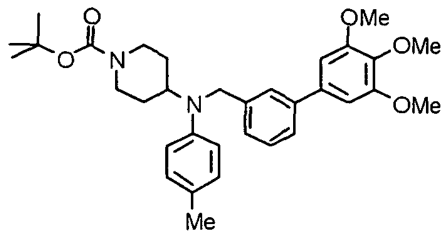

1-(tert-Butoxycarbonyl)-4-[(4-methylphenyl)amino]piperidine (581 mg) was reacted with 3-(3,4,5-trimethoxyphenyl)benzyl chloride (586 mg) in the same manner as described in Preparation Example 9 to give a light yellow amorphous substance of the title compound.
Yield: 1.03 g (94%).
$^1$H-NMR (400 MHz, $CDCl_3$) δ: 1.44 (s, 9H), 1.50-1.66 (m, 2H), 1.83-1.90 (m, 2H), 2.23 (s, 3H), 2.72-2.85 (m, 2H), 3.82-3.92 (m, 1H), 3.88 (s, 3H), 3.89 (s, 6H), 4.11-4.30 (m, 2H), 4.47 (s, 2H), 6.68 (d, 2H, J=8.6 Hz), 6.71 (s, 2H), 7.00 (d, 2H, J=8.8 Hz), 7.23-7.27 (m, 1H), 7.32-7.44 (m, 3H).

Referential Example 152

Synthesis of 4-[N-(4-methylphenyl)-N-[3-(3,4,5-trimethoxyphenyl)benzyl]amino]piperidine hydrochloride:

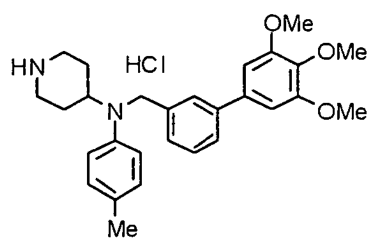

1-(tert-Butoxycarbonyl)-4-[N-(4-methylphenyl)-N-[3-(3,4,5-trimethoxyphenyl)benzyl]amino]piperidine (1.03 g) was treated in the same manner as described in Referential Example 94 to give light yellow powder of the title compound.
Yield: 882 mg (97%).

Preparation Examples 119 to 126

These compounds were obtained by the reaction of the amines obtained in Referential Examples148, 150 and 152 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 119 | 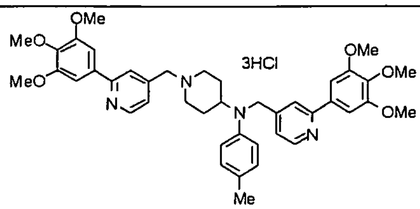 | 66% | 1.70-1.82 (m, 2H), 1.83-1.91 (m, 2H), 2.13-2.25 (m, 2H), 2.23 (s, 3H), 2.96-3.02 (m, 2H), 3.57 (s, 2H), 3.73-3.83 (m, 1H), 3.89 (s, 3H), 3.90 (s, 3H), 3.93 (s, 6H), 3.96 (s, 6H), 4.50 (s, 2H), 6.64 (d, 2H, J=8.8 Hz), 7.01 (d, 2H, J=8.5 Hz), 7.13-7.17 (m, 3H), 7.20 (d, 1H, J=4.9 Hz), 7.22 (s, 2H), 7.56 (s, 1H), 7.59 (s, 1H), 8.54 (d, 1H, J=5.1 Hz), 8.59 (d, 1H, J=4.9 Hz). |

| | | | |
|---|---|---|---|
| 120 | 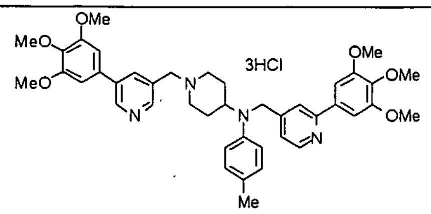 | 41% | 1.60-1.91 (m, 4H), 2.12-2.24 (m, 2H), 2.23 (s, 3H), 2.95-3.05 (m, 2H), 3.59 (s, 2H), 3.73-3.83 (m, 1H), 3.89 (s, 3H), 3.89 (s, 3H), 3.92 (s, 6H), 3.93 (s, 6H), 4.49 (s, 2H), 6.63 (d, 2H, J=8.6 Hz), 6.75 (s, 2H), 7.00 (d, 2H, J=8.6 Hz), 7.13-7.16 (m, 3H), 7.55 (s, 1H), 7.76 (s, 1H), 8.50 (d, 1H, J=1.8 Hz), 8.53 (d, 1H, J=5.1 Hz), 8.70 (s, 1H). |
| 121 | 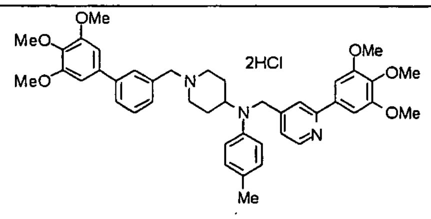 | 69% | 1.67-1.80 (m, 2H), 1.81-1.89 (m, 2H), 2.09-2.20 (m, 2H), 2.22 (s, 3H), 2.98-3.06 (m, 2H), 3.58 (s, 2H), 3.72-3.81 (m, 1H), 3.88 (s, 3H), 3.89 (s, 3H), 3.92 (s, 6H), 3.92 (s, 6H), 4.49 (s, 2H), 6.63 (d, 2H, J=8.4 Hz), 6.76 (s, 2H), 7.00 (d, 2H, J=8.6 Hz), 7.12-7.15 (m, 3H), 7.26-7.32 (m, 1H), 7.37 (dd, 1H, J=7.6 Hz, 7.6 Hz), 7.41-7.48 (m, 2H), 7.55 (s, 1H), 8.53 (d, 1H, J=5.0 Hz). |
| 122 | 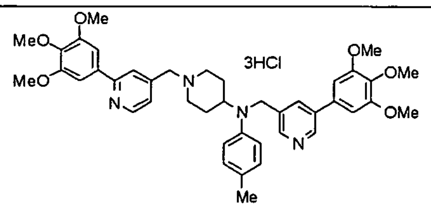 | 47% | 1.55-2.00 (m, 4H), 2.12-2.31 (m, 2H), 2.22 (s, 3H), 2.93-3.10 (m, 2H), 3.60(br, 2H), 3.69-3.80 (m, 1H), 3.88 (s, 3H), 3.89 (s, 6H), 3.90 (s, 3H), 3.96 (s, 6H), 4.53 (s, 2H), 6.66 (s, 2H), 6.69 (d, 2H, J=8.6 Hz), 7.00 (d, 2H, J=8.6 Hz), 7.19-7.27 (m, 4H), 7.68 (s, 1H), 8.50 (s, 1H), 8.60 (d, 1H, J=4.9 Hz), 8.64 (d, 1H, J=2.2 Hz). |
| 123 | 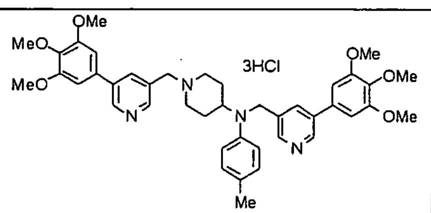 | 34% | 1.67-1.98 (m, 4H), 2.10-2.38 (m, 2H), 2.22 (s, 3H), 2.85-3.10 (m, 2H), 3.53-3.67 (s, 2H), 3.67-3.79 (m, 1H), 3.88 (s, 3H), 3.89 (s, 6H), 3.90 (s, 3H), 3.93 (s, 6H), 4.51 (s, 2H), 6.66 (s, 2H), 6.68 (d, 2H, J=8.8 Hz), 6.76 (s, 2H), 7.00 (d, 2H, J=8.2 Hz), 7.67 (s, 1H), 7.77(br, 1H), 8.47-8.53 (m, 2H), 8.63 (d, 1H, J=2.0 Hz), 8.70 (s, 1H). |

| | | | |
|---|---|---|---|
| 124 | 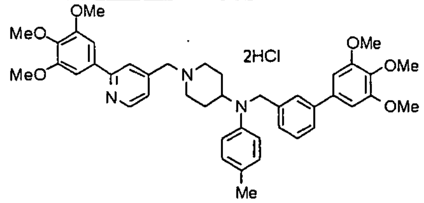 | 91% | 1.73-1.92 (m, 4H), 2.12-2.26 (m, 2H), 2.21 (s, 3H), 2.92-3.02 (m, 2H), 3.57 (s, 2H), 3.72-3.82 (m, 1H), 3.87 (s, 3H), 3.88 (s, 6H), 3.90 (s, 3H), 3.95 (s, 6H), 4.53 (s, 2H), 6.67 (d, 2H, J=7.8 Hz), 6.70 (s, 2H), 6.99 (d, 2H, J=8.0 Hz), 7.18-7.25 (m, 4H), 7.33 (dd, 1H, J=7.4 Hz, 7.4 Hz), 7.38 (d, 1H, J=7.2 Hz), 7.42 (s, 1H), 7.59 (s, 1H), 8.58 (d, 1H, J=4.7 Hz). |
| 125 | 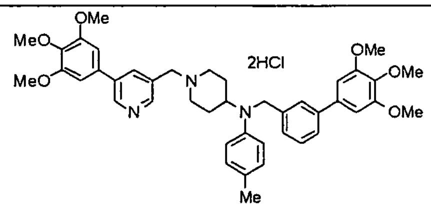 | 74% | 1.70-1.92 (m, 4H), 2.10-2.28 (m, 2H), 2.21 (s, 3H), 2.92-3.06 (m, 2H), 3.58 (s, 2H), 3.72-3.82 (m, 1H), 3.87 (s, 3H), 3.88 (s, 6H), 3.89 (s, 3H), 3.93 (s, 6H), 4.51 (s, 2H), 6.66 (d, 2H, J=8.6 Hz), 6.70 (s, 2H), 6.75 (s, 2H), 7.23 (d, 1H, J=7.0 Hz), 7.32 (dd, 1H, J=7.6 Hz, 7.6 Hz), 7.37 (d, 1H, J=7.8 Hz), 7.41 (s, 1H), 7.77 (s, 1H), 8.49 (s, 1H), 8.69 (s, 1H). |
| 126 | 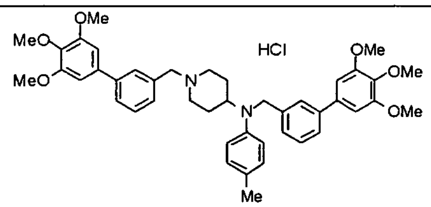 | 84% | 1.71-1.88 (m, 4H), 2.08-2.18 (m, 2H), 2.21 (s, 3H), 2.96-3.04 (m, 2H), 3.58 (s, 2H), 3.71-3.83 (m, 1H), 3.87 (s, 3H), 3.88 (s, 6H), 3.89 (s, 3H), 3.92 (s, 6H), 4.52 (s, 2H), 6.66 (d, 2H, J=8.6 Hz), 6.70 (s, 2H), 6.76 (s, 2H), 6.98 (d, 2H, J=8.3 Hz), 7.22-7.47 (m, 8H). |

Referential Example 153

Synthesis of 1-(tert-butoxycarbonyl)-4-[[4-(trifluoromethyl)phenyl]amino]piperidine:

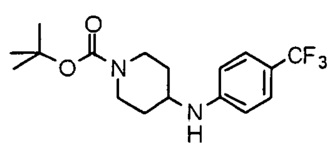

1-(tert-Butoxycarbonyl)-4-piperidone (5.00 g) was reacted with 4-(trifluoro)aniline (3.85 g) in the same manner as described in Referential Example 37 to give a white powder of the title compound.
Yield: 3.30 g (40%).
$^1$H-NMR (400 MHz, $CDCl_3$) δ: 1.30-1.41 (m, 2H), 1.47 (s, 9H), 2.00-2.07 (m, 2H), 2.88-2.99 (m, 2H), 3.32-3.52 (m, 1H), 3.83-3.89 (m, 1H), 4.00-4.14 (m, 2H), 6.59 (d, 2H, J=8.4 Hz), 7.39 (d, 2H, J=8.4Hz).

Referential Example 154

Synthesis of 1-(tert-butoxycarbonyl)-4-[N-[4-(trifluoromethyl)phenyl]-N-[[2-(3,4,5-trimethoxyphenyl)pyridin-4-yl]methyl]amino]piperidine:

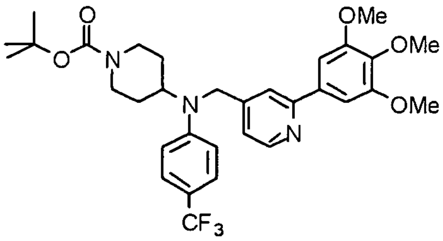

1-(tert-Butoxycarbonyl)-4-[[4-(trifluoromethyl)phenyl]amino]piperidine (688 mg) and 4-chloromethyl-2-(3,4,5-trimethoxyphenyl)pyridine (588 mg) were treated in the same manner as described in Preparation Example 9 to give a light yellow amorphous substance of the title compound.
Yield: 412 mg (34%).
$^1$H-NMR (400 MHz, $CDCl_3$) δ: 1.45 (s, 9H), 1.54-1.68 (m, 2H), 1.81-1.90 (m, 2H), 2.77-2.90 (m, 2H), 3.89 (s, 3H), 3.92 (s, 6H), 3.98-4.07 (m, 1H), 4.18-4.33 (m, 2H), 4.55 (s, 2H), 6.73 (d, 2H, J=8.8 Hz), 7.09 (d, 1H, J=3.7 Hz), 7.13 (s, 2H), 7.44 (d, 2H, J=8.8 Hz), 7.49 (s, 1H), 8.58 (d, 1H, J=5.1 Hz).

Referential Example 155

Synthesis of 4-[N-[4-(trifluoromethyl)phenyl]-N-[[2-(3,4,5-trimethoxyphenyl)pyridin-4-yl]methyl] amino]piperidine dihydrochloride:

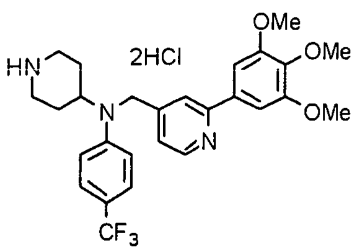

1-(tert-Butoxycarbonyl)-4-[N-[4-(trifluoromethyl)phenyl]-N-[[2-(3,4,5-trimethoxyphenyl)pyridin-4-yl]methyl] amino]piperidine (412 mg) was treated in the same manner as described in Referential Example 94 to give a light yellow powder of the title compound.
Yield: 359 mg (91%).

Referential Example 156

Synthesis of 1-(tert-butoxycarbonyl)-4-[N-[(4-trifluoromethyl)phenyl]-N-[3-(3,4,5-trimethoxyphenyl)benzyl]amino] piperidine:

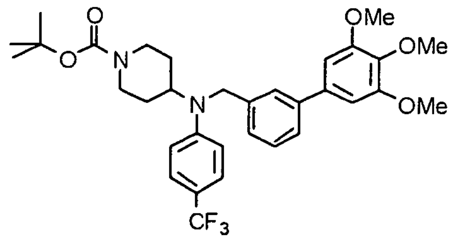

1-(tert-Butoxycarbonyl)-4-[[4-(trifluoromethyl)phenyl]amino]piperidine (689 mg) was reacted with 3-(3,4,5-trimethoxyphenyl)benzyl chloride (586 mg) in the same manner as described in Preparation Example 9 to give a light yellow amorphous substanceof the title compound.
Yield: 522 mg (44%).
$^1$H-NMR (400 MHz, $CDCl_3$) δ: 1.45 (s, 9H), 1.58-1.70 (m, 2H), 1.83-1.90 (m, 2H), 2.76-2.87 (m, 2H), 3.87 (s, 6H), 3.88 (s, 3H), 3.96-4.06 (m, 1H), 4.15-4.30 (m, 2H), 4.58 (s, 2H), 6.68 (s, 2H), 6.76 (d, 2H, J=8.8 Hz), 7.19 (s, 1H, J=7.4 Hz), 7.33-7.44 (m, 5H).

Referential Example 157

Synthesis of 4-[N-[4-(trifluoromethyl)phenyl]-N-[3-(3,4,5-trimethoxyphenyl)benzyl]amino] piperidine hydrochloride:

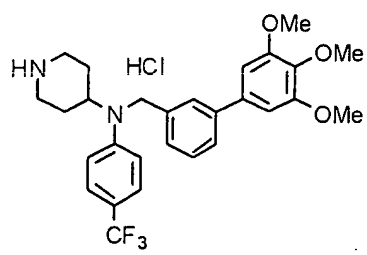

1-(tert-Butoxycarbonyl)-4-[N-[4-(trifluoromethyl)phenyl]-N-[3-(3,4,5-trimethoxyphenyl)benzyl]amino]piperidine (522 mg) was treated in the same manner as described in Referential Example 94 to give a light yellow powder of the title compound.
Yield: 460 mg (99%).

Preparation Examples 127 to 132

These compounds were obtained by the reaction of the amines obtained in Referential Examples 155 and 157 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 127 | 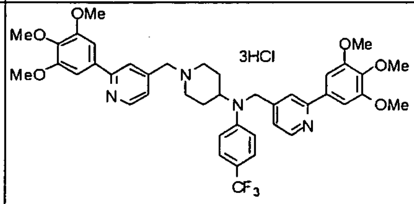 | 72% | 1.74-1.92 (m, 4H), 2.17-2.26 (m, 2H), 2.96-3.04 (m, 2H), 3.59 (s, 2H), 3.89 (s, 3H), 3.90 (s, 3H), 3.91 (s, 6H), 3.96 (s, 6H), 4.60 (s, 2H), 6.72 (d, 2H, J=8.8 Hz), 7.10 (d, 1H, J=4.9 Hz), 7.13 (s, 2H), 7.20 (d, 1H, J=5.1 Hz), 7.43 (d, 2H, J=8.8 Hz), 7.50 (s, 1H), 7.59 (s, 1H), 8.56 (d, 1H, J=4.9 Hz), 8.58 (d, 1H, J=5.1 Hz). |
| 128 | 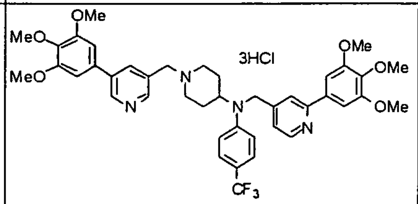 | 51% | 1.70-1.90 (m, 4H), 2.14-2.28 (m, 2H), 2.96-3.08 (m, 2H), 3.61 (s, 2H), 3.87-3.96 (m, 1H), 3.89 (s, 3H), 3.90 (s, 3H), 3.91 (s, 6H), 3.93 (s, 6H), 4.59 (s, 2H), 6.71 (d, 2H, J=8.8 Hz), 6.75 (s, 2H), 7.07-7.15 (m, 3H), 7.43 (d, 2H, J=8.8 Hz), 7.49 (s, 1H), 7.76 (s, 1H), 8.51 (d, 1H, J=1.8 Hz), 8.57 (d, 1H, J=5.1 Hz), 8.70 (s, 1H). |
| 129 | 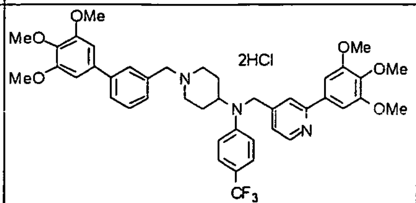 | 59% | 1.72-1.88 (m, 4H), 2.11-2.24 (m, 2H), 2.98-3.10 (m, 2H), 3.59 (s, 2H), 3.87-3.95 (m, 1H), 3.88 (s, 3H), 3.89 (s, 3H), 3.90 (s, 6H), 3.92 (s, 6H), 4.59 (s, 2H), 6.71 (d, 2H, J=9.0 Hz), 6.76 (s, 2H), 7.08 (d, 1H, J=5.1 Hz), 7.12 (s, 2H), 7.29 (d, 1H, J=7.4 Hz), 7.37 (dd, 1H, J=7.6 Hz, 7.6 Hz), 7.40-7.52 (m, 5H), 8.56 (d, 1H, J=5.1 Hz). |

| | | | |
|---|---|---|---|
| 130 | 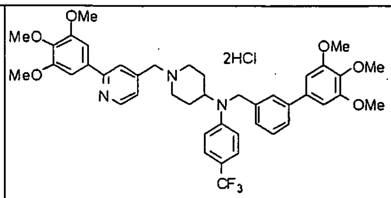 | 81% | 1.78-1.94 (m, 4H), 2.15-2.27 (m, 2H), 2.94-3.08 (m, 2H), 3.58 (s, 2H), 3.86 (s, 6H), 3.87 (s, 3H), 3.90 (s, 3H), 3.96 (s, 6H), 4.63 (s, 2H), 6.67 (s, 2H), 6.74 (d, 2H, J=8.8 Hz), 7.17-7.24 (m, 4H), 7.34-7.45 (m, 5H), 7.59 (s, 1H), 8.59 (d, 1H, J=5.1 Hz). |
| 131 | 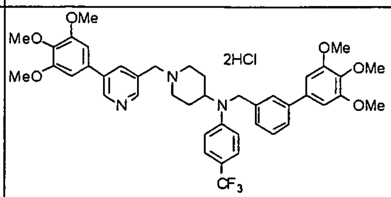 | 54% | 1.75-1.90 (m, 4H), 2.14-2.24 (m, 2H), 2.95-3.04 (m, 2H), 3.60 (s, 2H), 3.84-3.88 (m, 1H), 3.86 (m, 1H), 3.87 (s, 3H), 3.90 (s, 3H), 3.93 (s, 6H), 4.61 (s, 2H), 6.67 (s, 2H), 6.72-6.77 (m, 4H), 7.18 (d, 1H, J=7.4 Hz), 7.33-7.43 (m, 5H), 7.76 (s, 1H), 8.50 (d, 1H, J=1.9 Hz), 8.69 (d, 1H, J=1.9 Hz). |
| 132 | 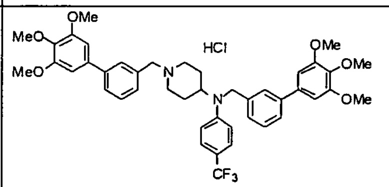 | 67% | 1.76-1.88 (m, 4H), 2.11-2.19 (m, 2H), 2.98-3.06 (m, 2H), 3.59 (s, 2H), 3.86 (s, 6H), 3.87 (s, 3H), 3.89 (s, 3H), 3.92 (s, 6H), 4.61 (s, 2H), 6.67 (s, 2H), 6.73 (d, 2H, J=8.8 Hz), 6.76 (s, 2H), 7.18 (d, 1H, J=7.3 Hz), 7.29 (d, 1H, J=7.6 Hz), 7.32-7.47 (m, 8H). |

Referential Example 158

Synthesis of 1-(tert-butoxycarbonyl)-4-(4-bromophenyl)amino-piperidine:

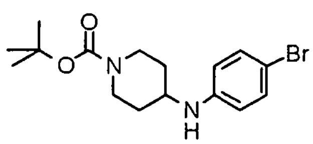

1-(tert-Butoxycarbonyl)-4-piperidone (5.00 g) was reacted with 4-bromoaniline (4.11 g) in the same manner as described in Referential Example 37 to give a white powder of the title compound.
Yield: 3.09 g (36%).
$^1$H-NMR (400 MHz, $CDCl_3$) δ: 1.25-1.37 (m, 2H), 1.46 (s, 9H), 1.97-2.05 (m, 2H), 2.86-2.96 (m, 2H), 3.33-3.42 (m, 2H), 3.47-3.57 (m, 1H), 3.96-4.12 (m, 2H), 6.47 (d, 2H, J=8.8 Hz), 7.24 (d, 2H, J=9.0 Hz).

Referential Example 159

Synthesis of 1-(tert-butoxycarboriyl)-4-[N-(4-bromophenyl)-N-[[2-(3,4,5-trimethoxyphenyl)pyridin-4-yl]methyl]amino]-piperidine:

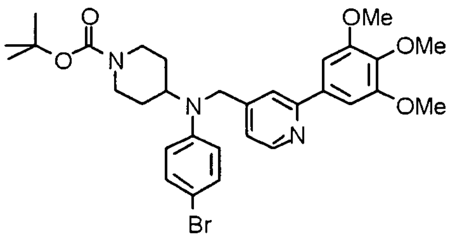

1-(tert-Butoxycarbonyl)-4-(4-bromophenyl)amino-piperidine (711 mg) and 4-chloromethyl-2-(3,4,5-trimethoxyphenyl)pyridine (588 mg) were treated in the same manner as described in Preparation Example 9 to give a light yellow amorphous substance of the title compound.
Yield: 607 mg (50%).
$^1$H-NMR (400 MHz, $CDCl_3$) δ: 1.45 (s, 9H), 1.50-1.64 (m, 2H), 1.81-1.88 (m, 2H), 2.74-2.88 (m, 2H), 3.86-3.94 (m, 1H), 3.89 (s, 3H), 3.93 (s, 6H), 4.14-4.32 (m, 2H), 4.46 (s, 2H), 6.59 (d, 2H, J=9.1 Hz), 7.10 (d, 1H, J=5.2 Hz), 7.14 (s, 2H), 7.28 (d, 2H, J=9.1 Hz), 7.50 (s, 1H), 8.57 (d, 1H, J=5.0 Hz).

Referential Example 160

Synthesis of 4-[N-(4-bromophenyl)-N-[[2-(3,4,5-trimethoxyphenyl)pyridin-4-yl]methyl]amino] piperidine dihydrochloride:

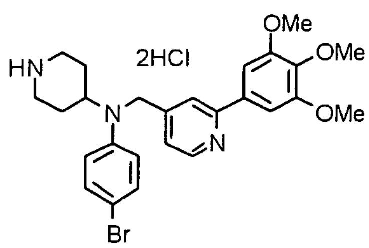

1-(tert-Butoxycarbonyl)-4-[N-(4-bromophenyl)-N-[[2-(3,4,5-trimethoxyphenyl)pyridin-4-yl]methyl]amino]- piperidine (607 mg) was treated in the same manner as described in Referential Example 94 to give a light yellow powder of the title compound.
Yeld: 541 mg (93%).

Referential Example 161

Synthesis of 1-(tert-butoxycarbonyl)-4-[N-(4-bromophenyl)-N-[[3-(3,4,5-trimethoxyphenyl)pyridin-5-yl]methyl]amino]-piperidine:

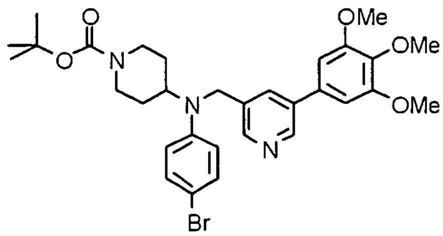

1-(tert-Butoxycarbonyl)-4-(4-bromophenyl)amino-piperidine (711 mg) and 5-chloromethyl-3-(3,4,5-trimethoxyphenyl)pyridine (588 mg) were treated in the same manner as described in Preparation Example 9 to give a light yellow amorphous substance of the title compound.
Yield: 347 mg (28%).
$^1$H-NMR (400 MHz, $CDCl_3$) δ: 1.45 (s, 9H), 1.52-1.67 (m, 2H), 1.80-1.89 (m, 2H), 2.72-2.87 (m, 2H), 3.82-3.92 (m, 1H), 3.89 (s, 3H), 3.90 (s, 6H), 4.14-4.33 (m, 2H), 4.50 (s, 2H), 6.63 (d, 2H, J=9.2 Hz), 6.65 (s, 2H), 7.28 (d, 2H, J=9.4 Hz), 7.61 (s, 1H), 8.47 (d, 1H, J=2.0 Hz), 8.67 (d, 1H, J=2.2 Hz).

Referential Example 162

Synthesis of 4-[N-(4-bromophenyl)-N-[[3-(3,4,5-trimethoxyphenyl)pyridin-5-yl]methyl]amino] piperidine dihydrochloride:

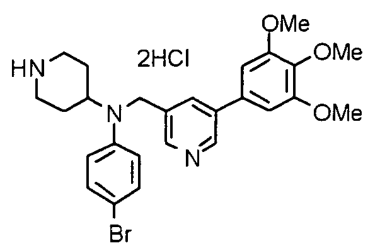

1-(tert-Butoxycarbonyl)-4-[N-(4-bromophenyl)-N-[[3-(3,4,5-trimethoxyphenyl)pyridin-5-yl]methyl]amino]- piperidine (347 mg) was treated in the same manner as described in Referential Example 94 to give a light yellow powder of the title compound.
Yield: 302 mg (91%).

Referential Example 163

Synthesis of 1-(tert-Butoxycarbonyl)-4-[N-(4-bromophenyl)-N-[3-(3,4, 5-trimethoxyphenyl)benzyl] amino]- piperidine:

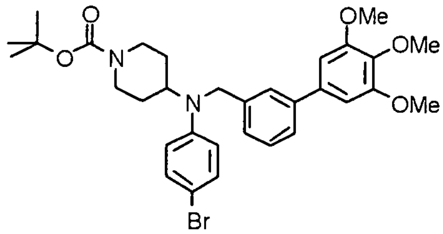

1-(tert-Butoxycarbonyl)-4-(4-bromophenyl)amino-piperidine (711 mg) was reacted with 3-(3,4,5-trimethoxyphenyl)benzyl chloride (586 mg) was treated in the same manner as described in Preparation Example 9 to give a light yellow amorphous substance of the title compound.
Yield: 1.14 g (93%).
$^1$H-NMR (400 MHz, $CDCl_3$) δ: 1.45 (s, 9H), 1.52-1.67 (m, 2H), 1.80-1.89 (m, 2H), 2.72-2.86 (m, 2H), 3.84-3.91 (m, 1H), 3.88 (s, 3H), 3.89 (s, 6H), 4.11-4.32 (m, 2H), 4.49 (s, 2H), 6.62 (d, 2H, J=9.2 Hz), 6.69 (s, 2H), 7.19 (d, 1H, J=7.6 Hz), 7.25 (d, 2H, J=5.5 Hz), 7.32-7.42 (m, 3H).

Referential Example 164

Synthesis of 4-[N-(4-bromophenyl)-N-[3-(3,4,5-trimethoxyphenyl)benzyl]amino]piperidine hydrochloride:

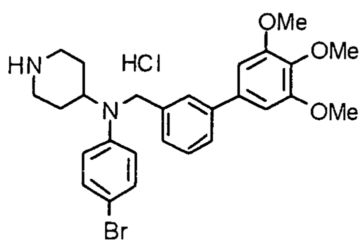

1-(tert-Butoxycarbonyl)-4-[N-(4-bromophenyl)-N-[3-(3,4,5-trimethoxyphenyl) benzyl]amino]- piperidine (1.03 g) was treated in the same manner as described in Referential Example 94 to give a light yellow powder of the title compound.
Yield: 973 mg (84%).

Preparation Examples 133 to 140

These compounds were obtained by the reaction of the amines obtained in Referential Examples 160, 162 and 164 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 133 | 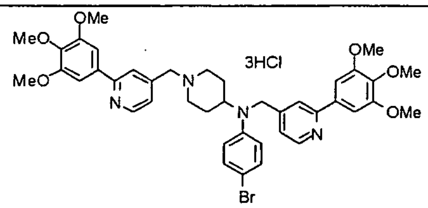 | 52% | 1.70-1.90 (m, 4H), 2.14-2.25 (m, 2H), 2.94-3.04 (m, 2H), 3.58 (s, 2H), 3.73-3.84 (m, 1H), 3.89 (s, 3H), 3.90 (s, 3H), 3.92 (s, 6H), 3.96 (s, 6H), 4.52 (s, 2H), 6.57 (d, 2H, J=8.8 Hz), 7.10 (d, 1H, J=4.9 Hz), 7.14 (s, 2H), 7.20 (d, 1H, J=4.9 Hz), 7.22 (s, 2H), 7.26 (d, 2H, J=8.5 Hz), 7.51 (s, 1H), 7.59 (s, 1H), 8.56 (d, 1H, J=4.9 Hz), 8.59 (d, 1H, J=4.9 Hz). |
| 134 | 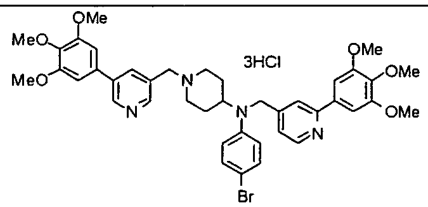 | 56% | 1.68-1.88 (m, 4H), 2.12-2.24 (m, 2H), 2.95-3.04 (m, 2H), 3.59 (s, 2H), 3.72-3.84 (m, 1H), 3.89 (s, 3H), 3.90 (s, 3H), 3.92 (s, 6H), 3.93 (s, 6H), 4.50 (s, 2H), 6.57 (d, 2H, J=9.2 Hz), 6.74 (s, 2H), 7.09 (d, 1H, J=3.9 Hz), 7.13 (s, 2H), 7.26 (d, 2H, J=8.8 Hz), 7.50 (s, 1H), 7.75 (s, 1H), 8.50 (d, 1H, J=2.0 Hz), 8.55 (d, 1H, J=5.0 Hz), 8.69 (d, 1H, J=2.0 Hz). |
| 135 | 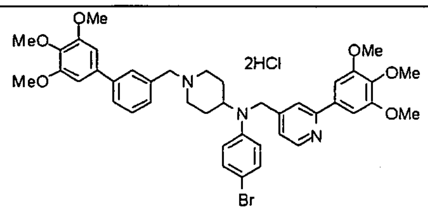 | 65% | 1.70-1.86 (m, 4H), 2.10-2.20 (m, 2H), 2.97-3.08 (m, 2H), 3.59 (s, 2H), 3.72-3.82 (m, 1H), 3.89 (s, 6H), 3.92 (s, 6H), 3.92 (s, 6H), 4.50 (s, 2H), 6.56 (d, 2H, J=9.2 Hz), 6.76 (s, 2H), 7.09 (d, 1H, J=5.1 Hz), 7.13 (s, 2H), 7.23-7.33 (m, 3H), 7.37 (dd, 1H, J=7.4 Hz), 7.41-7.48 (m, 2H), 7.49 (s, 1H), 8.54 (d, 1H, J=5.1 Hz). |

| | | | |
|---|---|---|---|
| 136 | 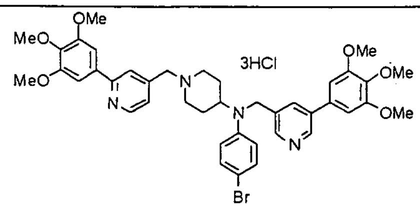 | 49% | 1.77-1.93 (m, 4H), 2.12-2.30 (m, 2H), 2.94-3.10 (m, 2H), 3.60 (s, 2H), 3.73-3.83 (m, 1H), 3.88 (s, 3H), 3.89 (s, 6H), 3.90 (s, 3H), 3.96 (s, 6H), 4.55 (s, 2H), 6.61 (d, 2H, J=9.2 Hz), 6.65 (s, 2H), 7.19-7.29 (m, 5H), 7.62(br, 2H), 8.47 (d, 1H, J=1.6 Hz), 8.60 (d, 1H, J=4.9 Hz), 8.66 (d, 1H, J=2.0 Hz). |
| 137 | 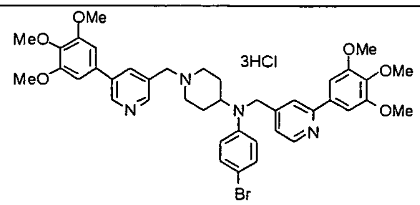 | 50% | 1.70-1.92 (m, 4H), 2.12-2.27 (m, 2H), 2.93-3.07 (m, 2H), 3.60 (s, 2H), 3.67-4.08 (m, 1H), 3.88 (s, 3H), 3.89 (s, 6H), 3.90 (s, 3H), 3.93 (s, 6H), 4.54 (s, 2H), 6.60 (d, 2H, J=9.0 Hz), 6.64 (s, 2H), 6.73-6.80 (m, 2H), 7.25 (s, 2H), 7.61 (s, 1H), 7.77(br, 1H), 8.45 (d, 1H, J=1.7 Hz), 8.50 (d, 1H, J=1.7 Hz), 8.65 (d, 1H, J=2.0 Hz). |
| 138 | 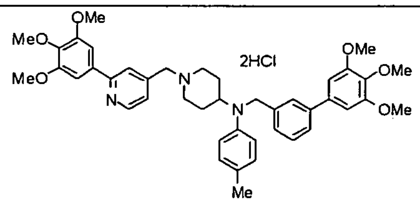 | 81% | 1.75-1.90 (m, 4H), 2.17-2.24 (m, 2H), 2.94-3.02 (m, 2H), 3.57 (s, 2H), 3.72-3.83 (m, 1H), 3.88 (s, 3H), 3.88 (s, 6H), 3.90 (s, 3H), 3.95 (s, 6H), 4.54 (s, 2H), 6.60 (d, 2H, J=9.2 Hz), 6.69 (s, 2H), 7.18-7.27 (m, 6H), 7.32-7.42 (m, 3H), 7.60 (s, 1H), 8.58 (d, 1H, J=4.9 Hz). |
| 139 | 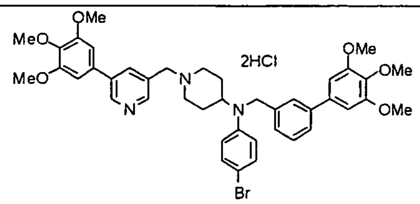 | 80% | 1.72-1.90 (m, 4H), 2.13-2.21 (m, 2H), 2.94-3.05 (m, 2H), 3.59 (s, 2H), 3.72-3.82 (m, 1H), 3.87 (s, 3H), 3.88 (s, 6H), 3.89 (s, 3H), 3.93 (s, 6H), 4.53 (s, 2H), 6.60 (d, 2H, J=9.0 Hz), 6.68 (s, 2H), 6.75 (s, 2H), 7.19 (d, 1H, J=7.2 Hz), 7.24 (d, 2H, J=9.0 Hz), 7.31-7.41 (m, 3H), 7.76 (s, 1H), 8.50 (d, 1H, J=1.8 Hz), 8.70 (s, 1H). |
| 140 | 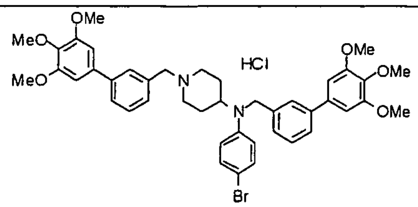 | 78% | 1.72-1.88 (m, 4H), 2.08-2.18 (m, 2H), 2.97-3.06 (m, 2H), 3.58 (s, 2H), 3.71-3.82 (m, 1H), 3.87 (s, 3H), 3.88 (s, 6H), 3.89 (s, 3H), 3.92 (s, 6H), 4.53 (s, 2H), 6.59 (d, 2H, J=9.3 Hz), 6.68 (s, 2H), 6.76 (s, 2H), 7.18 (d, 1H, J=7.3 Hz), 7.21-7.47 (m, 9H) |

Referential Example 165

Synthesis of 1-(tert-butoxycarbonyl)-4-[(4-chlorophenyl)amino]piperidine:

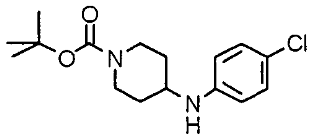

1-(tert-Butoxycarbonyl)-4-piperidone (5.00 g) was reacted with 4-chloroaniline (3.05 g) in the same manner as described in Referential Example 37 to give a white powder of the title compound.
Yield: 3.80 g (49%).
$^1$H-NMR (400 MHz, $CDCl_3$) δ: 1.24-1.38 (m, 2H), 1.46 (s, 9H), 1.97-2.05 (m, 2H), 2.86-2.96 (m, 2H), 3.32-3.42 (m, 2H), 3.51 (br, 1H), 6.52 (d, 2H, J=9.0 Hz), 7.11 (d, 2H, J=9.0 Hz).

Referential Example 166

Synthesis of 1-(tert-butoxycarbonyl)-4-[N-(4-chlorophenyl)-N-[[2-(3,4,5-trimethoxyphenyl)pyridin-4-yl]methyl]amino] piperidine:

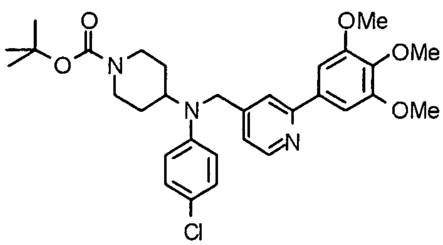

1-(tert-Butoxycarbonyl)-4-[(4-chlorophenyl)amino]piperidine (621 mg) and 4-chloromethyl-2-(3,4,5-trimethoxyphenyl)pyridine (588 mg) were treated in the same manner as described in Preparation Example 9 to give a light yellow amorphous substance of the title compound.
Yield: 789 mg (69%).
$^1$H-NMR (400 MHz, $CDCl_3$) δ: 1.45 (s, 9H), 1.51-1.68 (m, 2H), 1.80-1.89 (m, 2H), 2.72-2.86 (m, 2H), 3.87-3.90 (m, 1H), 3.89 (s, 3H), 3.93 (s, 6H), 4.64 (s, 2H), 6.64 (d, 2H, J=9.0 Hz), 7.14 (d, 1H, J=5.3 Hz), 7.15 (d, 2H, J=9.0 Hz), 7.51 (s, 2H), 8.57 (d, 2H, J=5.1 Hz).

Referential Example 167

Synthesis of 4-[N-(4-chlorophenyl)-N-[[2-(3,4,5-trimethoxyphenyl)pyridin-4-yl]methyl]amino] piperidine dihydrochloride:

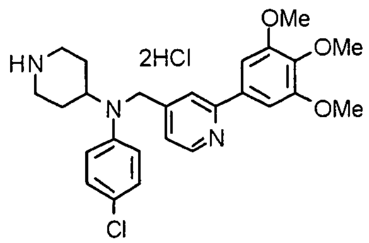

1-(tert-Butoxycarbonyl)-4-[N-(4-chlorophenyl)-N-[[2-(3,4,5-trimethoxyphenyl)pyridin-4-yl]methyl]amino]piperidine (789 mg) was treated in the same manner as described in Referential Example 94 to give a light yellow powder of the title compound.
Yield: 673 mg (90%).

Referential Example 168

Synthesis of 1-(tert-butoxycarbonyl)-4-[N-(4-chlorophenyl)-N-[[3-(3,4,5-trimethoxyphenyl)pyridin-5-yl]methyl]amino] piperidine:

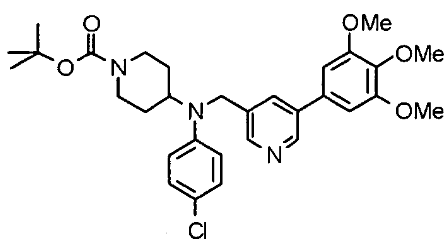

1-(tert-Butoxycarbonyl)-4-[(4-chlorophenyl)amino]piperidine (621 mg) and 5-chloromethyl-3-(3,4,5-trimethoxyphenyl)pyridine (588 mg) were treated in the same manner as described in Preparation Example 9 to give a light yellow amorphous substanceof the title compound.
Yield: 268 mg (24%).
$^1$H-NMR (400 MHz, $CDCl_3$) δ: 1.45 (s, 9H), 1.56-1.76 (m, 2H), 1.80-1.90 (m, 2H), 2.76-2.83 (m, 2H), 3.86-3.90 (m, 1H), 3.89 (s, 3H), 3.90 (s, 6H), 4.15-4.30 (m, 2H), 4.50 (s, 2H), 6.66 (s, 2H), 6.68 (d, 2H, J=9.2 Hz), 7.15 (d, 2H, J=9.0 Hz), 7.63 (s, 1H), 8.47 (d, 1H, J=2.0 Hz), 8.66 (d, 1H, J=2.0 Hz).

Referential Example 169

Synthesis of 4-[N-(4-chlorophenyl)-N-[[3-(3,4,5-trimethoxyphenyl)pyridin-5-yl]methyl]amino] piperidine dihydrochloride:

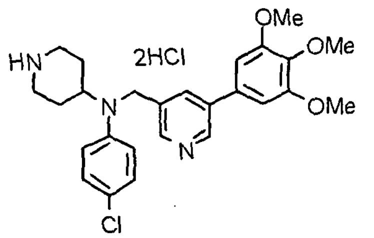

1-(tert-Butoxycarbonyl)-4-[N-(4-chlorophenyl)-N-[[3-(3,4,5-trimethoxyphenyl)pyridin-5-yl]methyl]amino]piperidine (268 mg) was treated in the same manner as described in Referential Example 94 to give a light yellow powder of the title compound.
Yield: 233 mg (91%).

Referential Example 170

Synthesis of 1-(tert-butoxycarbonyl)-4-[N-(4-chlorophenyl)-N-[3-(3,4, 5-trimethoxyphenyl)benzyl] amino]piperidine:

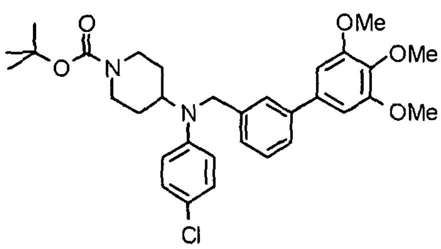

1-(tert-Butoxycarbonyl)-4-[4-(chlorophenyl)amino]piperidine (622 mg) was reacted with 3-(3,4,5-trimethoxyphenyl)benzyl chloride (586 mg) in the same manner as described in Preparation Example 9 to give a light yellow amorphous substance of the title compound.
Yield: 1.04 g (92%).
$^1$H-NMR (400 MHz, $CDCl_3$) δ: 1.45 (s, 9H), 1.58-1.67 (m, 2H), 1.82-1.91 (m, 2H), 2.74-2.86 (m, 2H), 3.85-3.92 (m, 1H), 3.88 (s, 3H), 3.89 (s, 6H), 4.35-4.31 (m, 2H), 4.49 (s, 2H), 6.66 (d, 2H, J=9.2 Hz), 6.70 (s, 2H), 7.12 (d, 2H, J=9.0 Hz), 7.20 (d, 2H, J=7.3 Hz), 7.33-7.43 (m, 3H).

Referential Example 171

Synthesis of 4-[N-(4-chlorophenyl)-N-[3-(3,4,5-trimethoxyphenyl)benzyl]amino]piperidine hydrochloride:

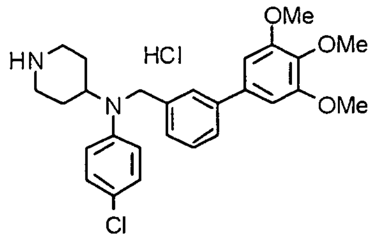

1-(tert-Butoxycarbonyl)-4-[N-(4-chlorophenyl)-N-[3-(3,4,5-trimethoxyphenyl) benzyl]amino]piperidine (1.04 g) was treated in the same manner as described in Referential Example 94 to give a light yellow powder of the title compound.
Yield: 899 mg (97%).

Preparation Examples 141 to 148

These compounds were obtained by the reaction of the amines obtained in Referential Examples 167, 169 and 171 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 141 | 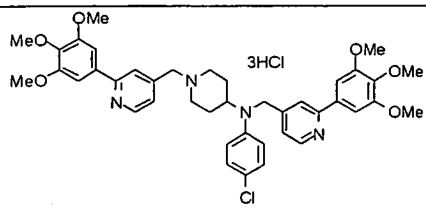 | 66% | 1.71-1.90 (m, 4H), 2.15-2.24 (m, 2H), 2.95-3.05 (m, 2H), 3.58 (s, 2H), 3.73-3.84 (m, 1H), 3.89 (s, 3H), 3.90 (s, 3H), 3.93 (s, 6H), 3.96 (s, 6H), 4.52 (s, 2H), 6.62 (d, 2H, J=9.0 Hz), 7.10-7.16 (m, 5H), 7.19-7.24 (m, 3H), 7.52 (s, 1H), 7.59 (s, 1H), 8.56 (d, 1H, J=4.9 Hz), 8.59 (d, 1H, J=4.9 Hz). |

| | | | |
|---|---|---|---|
| 142 | 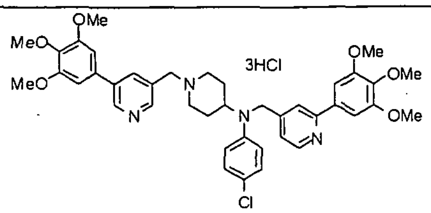 | 67% | 1.69-1.90 (m, 1H), 2.12-2.25 (m, 2H), 2.93-3.06 (m, 2H), 3.59 (s, 2H), 3.72-3.83 (m, 1H), 3.89 (s, 3H), 3.90 (s, 3H), 3.92 (s, 6H), 3.93 (s, 6H), 4.50 (s, 2H), 6.62 (d, 2H, J=9.2 Hz), 6.75 (s, 2H), 7.10 (d, 1H, J=5.3 Hz), 7.13 (s, 2H), 7.13 (d, 2H, J=9.0 Hz), 7.50 (s, 1H), 7.76 (s, 1H), 8.50 (d, 1H, J=1.8 Hz), 8.55 (d, 1H, J=5.1 Hz), 8.70 (d, 1H, J=1.8 Hz). |
| 143 | 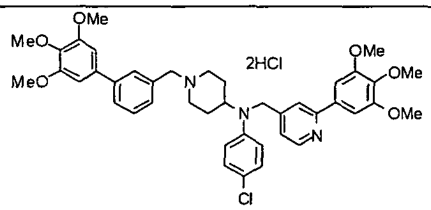 | 70% | 1.65-1.88 (m, 4H), 2.08-2.20 (m, 2H), 2.97-3.07 (m, 2H), 3.59 (s, 2H), 3.71-3.82 (m, 1H), 3.88 (s, 3H), 3.89 (s, 3H), 3.90-3.93 (m, 3H), 4.50 (s, 2H), 6.61 (d, 2H, J=8.2 Hz), 6.76 (s, 2H), 7.07-7.14 (m, 5H), 7.28 (d, 1H, J=6.6 Hz), 7.37 (dd, 1H, J=7.4 Hz), 7.40-7.47 (m, 2H), 7.50 (s, 1H), 8.54 (d, 1H, J=5.1 Hz). |
| 144 | 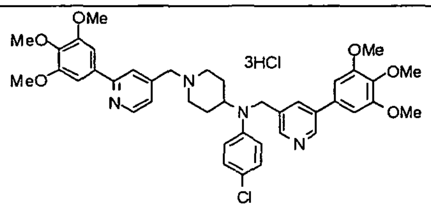 | 57% | 1.56-1.93 (m, 4H), 2.12-2.30 (m, 2H), 2.92-3.10 (m, 2H), 3.53-3.68 (m, 2H), 3.70-3.82 (m, 1H), 3.88 (s, 3H), 3.89 (s, 6H), 3.90 (s, 3H), 3.96 (s, 6H), 4.56 (s, 2H), 6.64-6.70 (m, 4H), 7.13 (d, 2H, J=9.0 Hz), 7.20-7.30 (m, 3H), 7.63(br, 2H), 8.48 (s, 1H), 8.60 (d, 1H, J=5.1 Hz), 8.66 (d, 1H, J=2.2 Hz). |
| 145 | 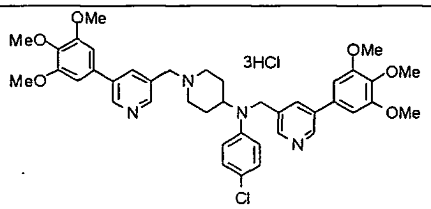 | 70% | 1.71-1.92 (m, 4H), 2.12-2.27 (m, 2H), 2.94-3.07 (m, 2H), 3.59 (s, 2H), 3.69-3.81 (s, 1H), 3.88 (s, 3H), 3.89 (s, 6H), 3.90 (s, 3H), 3.93 (s, 6H), 4.54 (s, 2H), 6.63-6.68 (m, 4H), 6.75 (s, 2H), 7.13 (d, 2H, J=9.0 Hz), 7.62 (s, 1H), 7.76 (s, 1H), 8.47 (d, 1H, J=1.8 Hz), 8.50 (d, 1H, J=1.8 Hz), 8.65 (d, 1H, J=2.0 Hz), 8.70 (s, 1H). |

| | | | |
|---|---|---|---|
| 146 | 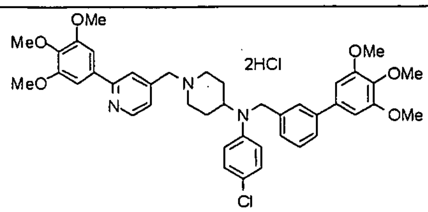 | 78% | 1.75-1.91 (m, 4H), 2.13-2.23 (m, 2H), 2.94-3.02 (m, 2H), 3.57 (s, 2H), 3.73-3.82 (m, 1H), 3.88 (s, 3H), 3.88 (s, 6H), 3.90 (s, 3H), 3.96 (s, 6H), 4.55 (s, 2H), 6.65 (d, 2H, J=9.0 Hz), 6.68 (s, 2H), 7.11 (d, 2H, J=8.5 Hz), 7.18-7.24 (m, 4H), 7.32-7.42 (m, 3H), 7.59 (s, 1H), 8.59 (d, 1H, J=4.9 Hz). |
| 147 | 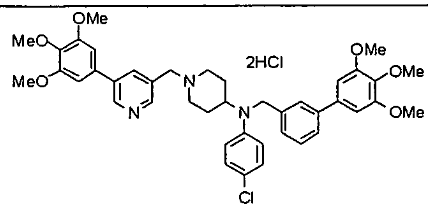 | 63% | 1.72-1.89 (m, 4H), 2.12-2.21 (m, 2H), 2.94-3.03 (m, 2H), 3.59 (s, 2H), 3.72-3.82 (m, 1H), 3.87 (s, 3H), 3.88 (s, 6H), 3.90 (s, 3H), 3.93 (s, 6H), 4.53 (s, 2H), 6.64 (d, 2H, J=9.2 Hz), 6.68 (s, 2H), 6.75 (s, 2H), 7.11 (d, 2H), 7.19 (d, 1H, J=7.6 Hz), 7.32-7.40 (m, 3H), 7.76 (s, 1H), 8.50 (d, 1H, J=1.8 Hz), 8.69 (d, 1H, J=2.2 Hz). |
| 148 | 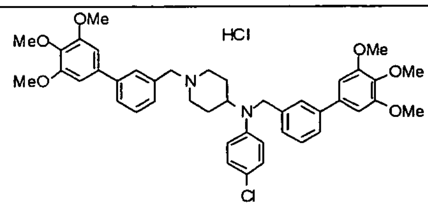 | 68% | 1.72-1.87 (m, 4H), 2.08-2.18 (m, 2H), 2.97-3.05 (m, 2H), 3.58 (s, 2H), 3.71-3.82 (m, 1H), 3.87 (s, 3H), 3.88 (s, 6H), 3.89 (s, 3H), 3.92 (s, 6H), 4.53 (s, 2H), 6.64 (dt, 2H, J=9.3 Hz, 2.9 Hz), 6.68 (s, 2H), 6.76 (s, 2H), 7.10 (dt, 2H, J=9.0 Hz, 2.8 Hz), 7.19 (d, 1H, J=7.6 Hz), 7.24-7.47 (m, 7H). |

Referential Example 172

Synthesis of 1-(tert-butoxycarbonyl)-4-[(3,4-difluorophenyl)amino]piperidine:

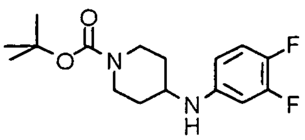

1-(tert-Butoxycarbonyl)-4-piperidone (5.00 g) was reacted with 3,4-difluoroaniline (3.09 g) in the same manner as described in Referential Example 37 to give a white powder of the title compound.
Yield: 4.66 g (62%).
$^1$H-NMR (400 MHz, $CDCl_3$) δ: 1.24-1.37 (m, 2H), 1.46 (s, 9H), 1.97-2.05 (m, 2H), 2.85-2.96 (m, 2H), 3.26-3.36 (m, 1H), 3.38-3.52 (m, 1H), 3.96-4.14 (m, 2H), 6.22-6.28 (m, 1H), 6.38 (ddd, 1H, J=12.7 Hz, 6.6 Hz, 2.9 Hz), 6.94 (dd, 1H, J=19.1 Hz, 9.0 Hz).

Referential Example 173

Synthesis of 1-(tert-butoxycarbonyl)-4-[N-(3,4-difluorophenyl)-N-[[2-(3,4,5-trimethoxyphenyl) pyridin-4-yl]methyl] amino]piperidine:

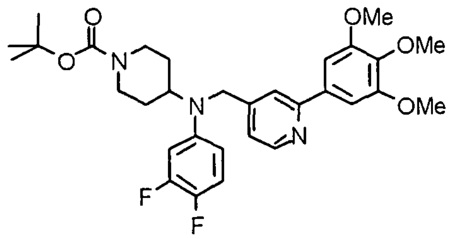

1-(tert-Butoxycarbonyl)-4-[(3,4-difluorophenyl)amino]piperidone (625 mg) and 4-chloromethyl-2-(3,4,5-trimethoxyphenyl)pyridine (588 mg) were treated in the same manner as described in Preparation Example 9 to give a light yellow amorphous substanceof the title compound.
Yield: 534 mg (47%).
$^1$H-NMR (400 MHz, $CDCl_3$) δ: 1.45 (s, 9H), 1.50-1.70 (m, 2H), 1.82-1.90 (m, 2H), 2.73-2.88 (m, 2H), 3.90 (s, 3H), 3.94 (s, 6H), 4.15-4.30 (m, 2H), 4.43 (s, 2H), 6.33-6.39 (m, 1H), 6.52 (ddd, 1H, J=13.6 Hz, 6.4 Hz, 3.1 Hz), 6.98 (dd, 1H, J=19.1 Hz, 9.2 Hz), 7.11 (dd, 1H, J=5.0 Hz, 1.3 Hz), 7.16 (s, 2H), 7.51 (s, 1H), 8.58 (d, 1H, J=5.1 Hz).

Referential Example 174

Synthesis of 4-[N-(3,4-difluorophenyl)-N-[[2-(3,4,5-trimethoxyphenyl)pyridin-4-yl]methyl]amino] piperidine dihydrochloride:

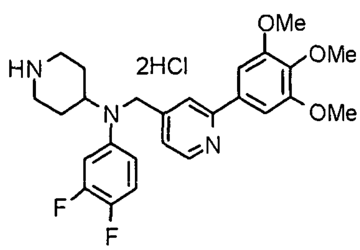

1-(tert-Butoxycarbonyl)-4-[N-(3,4-difluorophenyl)-N-[[2-(3,4,5-trimethoxyphenyl)pyridin-4-yl]methyl]amino] piperidine (534 mg) was treated in the same manner as described in Referential Example 94 to give a light yellow powder of the title compound.
Yield: 442 mg (87%).

Referential Example 175

Synthesis of 1-(tert-butoxycarbonyl)-4-[N-(3,4-difluorophenyl)-N-[[3-(3,4,5-trimethoxyphenyl) pyridin-5-yl]methyl] amino]piperidine:

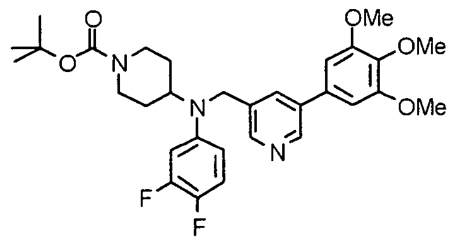

1-(tert-Butoxycarbonyl)-4-[(3,4-difluorophenyl)amino]piperidine (625 mg) and 5-chloromethyl-3-(3,4,5-trimethoxyphenyl)pyridine (588 mg) were treated in the same manner as described in Preparation Example 9 to give a light yellow amorphous substanceof the title compound.
Yield: 350 mg (31%).

Referential Example 176

Synthesis of 4-[N-(3,4-difluorophenyl)-N-[[3-(3,4,5-trimethoxyphenyl)pyridin-5-yl]methyl]amino] piperidine dihydrochloride:

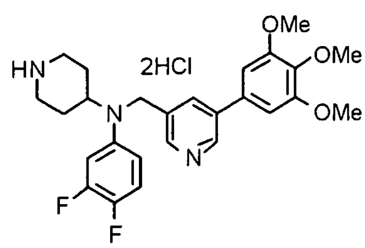

1-(tert-Butoxycarbonyl)-4-[N-(3,4-difluorophenyl)-N-[[3-(3,4,5-trimethoxyphenyl)pyridin-5-yl]methyl]amino] piperidine (350 mg) was treated in the same manner as described in Referential Example 94 to give a light yellow powder of the title compound.
Yield: 305 mg (92%).

Referential Example 177

Synthesis of 1-(tert-butoxycarbonyl)-4-[N-(3,4-difluorophenyl)-N-[3-(3,4,5-trimethoxyphenyl) benzyl]amino]piperidine:

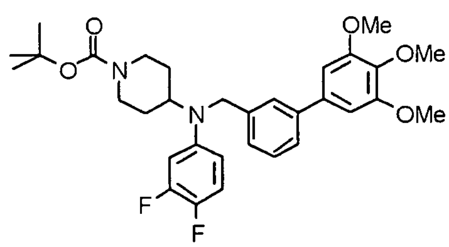

1-(tert-Butoxycarbonyl)-4-[(3,4-difluorophenyl)amino]piperidine (625 mg) was reacted with 3-(3,4,5-trimethoxyphenyl)benzyl chloride (586 mg) in the same manner as described in Preparation Example 9 to give a light yellow amorphous substance of the title compound.
Yield: 1.04 g (92%).
$^1$H-NMR (400 MHz, $CDCl_3$) δ: 1.45 (s, 9H), 1.52-1.66 (m, 2H), 1.81-1.89 (m, 2H), 2.72-2.85 (m, 2H), 3.78 (tt, 1H, J=11.8 Hz, 3.8 Hz), 3.88 (s, 3H), 3.90 (s, 6H), 4.12-4.30 (m, 2H), 4.45 (s, 2H), 6.36-6.42 (m, 1H), 6.54 (ddd, 1H, J=13.9 Hz, 6.8 Hz, 2.9 Hz), 6.71 (s, 2H), 6.95 (dd, 1H, J=19.2 Hz, 9.2 Hz), 7.20 (d, 1H, J=7.4 Hz), 7.36-7.43 (m, 3H).

Referential Example 178

Synthesis of 4-[N-(3,4-difluorophenyl)-N-[3-(3,4,5-trimethoxyphenyl)benzyl]amino]piperidine hydrochloride:

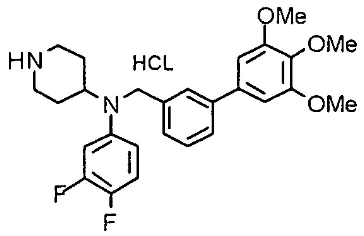

1-(tert-Butoxycarbonyl)-4-[ (3,4-difluorophenyl)-N-[3-(3,4,5-trimethoxyphenyl)benzyl]amino]piperidine (980 mg) was treated in the same manner as described in Referential Example 94 to give a light yellow powder of the title compound.
Yield: 819 mg (94%).

Preparation Examples 149 to 156

These compounds were obtained by the reaction of the amines obtained in Referential Examples 174, 176 and 178 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 149 | 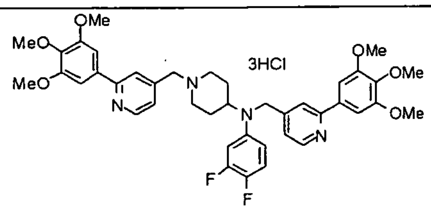 | 67% | 1.70-1.90 (m, 4H), 2.16-2.23 (m, 2H), 2.95-3.03 (m, 2H), 3.58 (s, 2H), 3.64-3.74 (m, 1H), 3.89 (s, 3H), 3.90 (s, 3H), 3.93 (s, 6H), 3.96 (s, 6H), 4.49 (s, 2H), 6.31-6.37 (m, 1H), 6.51 (ddd, 1H, J=13.9 Hz, 6.6 Hz, 3.1 Hz), 6.96 (dd, 1H, J=19.2 Hz, 9.8 Hz), 7.11 (d, 1H, J=5.1 Hz), 7.15 (s, 2H), 7.20 (d, 1H, J=5.1 Hz), 7.22 (s, 2H), 7.52 (s, 1H), 7.59 (s, 1H), 8.57 (d, 1H, J=5.1 Hz), 8.59 (d, 1H, J=5.1 Hz). |
| 150 | 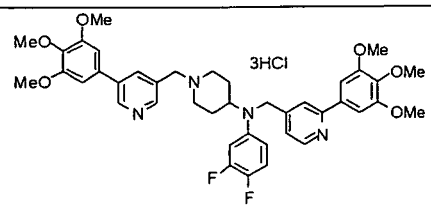 | 47% | 1.67-1.79 (m, 2H), 1.81-1.89 (m, 2H), 2.13-2.20 (m, 2H), 2.95-3.05 (m, 2H), 3.59 (s, 2H), 3.63-3.75 (m, 1H), 3.89 (s, 3H), 3.90 (s, 3H), 3.93 (s, 12H), 4.47 (s, 2H), 6.30-6.36 (m, 1H), 6.50 (ddd, 1H, J=13.9 Hz, 6.6 Hz, 3.1 Hz), 6.75 (s, 2H), 6.96 (d, 1H, J=19.0 Hz, 9.4 Hz), 7.10 (d, 1H, J=4.1 Hz), 7.15 (s, 2H), 7.51 (s, 1H), 7.75 (s, 1H), 8.50 (d, 1H, J=1.8 Hz), 8.56 (d, 1H, J=5.1 Hz), 8.70 (s, 1H). |
| 151 | 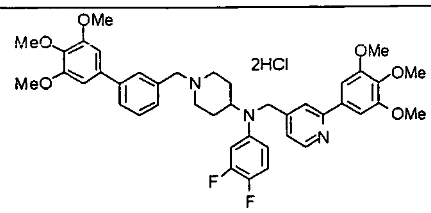 | 53% | 1.68-1.87 (m, 4H), 2.09-2.18 (m, 2H), 2.98-3.06 (m, 2H), 3.58 (s, 2H), 3.63-3.72 (m, 1H), 3.89 (s, 3H), 3.89 (s, 3H), 3.92 (s, 6H), 3.93 (s, 6H), 4.47 (s, 2H), 6.33-6.35 (m, 1H), 6.50 (ddd, 1H, J=13.9 Hz, 6.4 Hz, 2.9 Hz), 6.76 (s, 2H), 6.95 (dd, 1H, J=19.2 Hz, 9.4 |

| | | | |
|---|---|---|---|
| | | | Hz), 7.09 (d, 1H, J=5.1 Hz), 7.15 (s, 2H), 7.25-7.30 (m, 1H), 7.37 (dd, 1H, J=7.3 Hz, 7.3 Hz), 7.42-7.46 (m, 2H), 7.50 (s, 1H), 8.56 (d, 1H, J=5.1 Hz). |
| 152 | 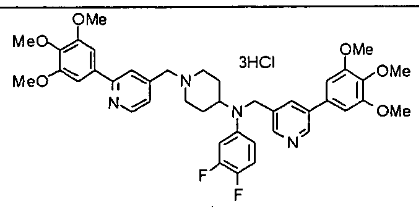 | 50% | 1.72-1.96 (m, 4H), 2.12-2.28 (m, 2H), 2.94-3.08 (m, 2H), 3.59 (s, 2H), 3.62-3.72 (m, 1H), 3.89 (s, 3H), 3.90 (s, 9H), 3.96 (s, 6H), 4.52 (s, 2H), 6.36-6.43 (m, 1H), 6.55 (ddd, 1H, J=13.7 Hz, 6.6 Hz, 2.9 Hz), 6.67 (s, 2H), 6.96 (dd, 1H, J=19.1 Hz, 9.2 Hz), 7.21 (dd, 1H, J=5.1 Hz, 1.2 Hz), 7.24 (s, 2H), 7.61(br, 1H), 7.64 (s, 1H), 8.47 (d, 1H, J=2.0 Hz), 8.60 (d, 1H, J=4.9 Hz), 8.67 (d, 1H, J=2.0 Hz). |
| 153 | 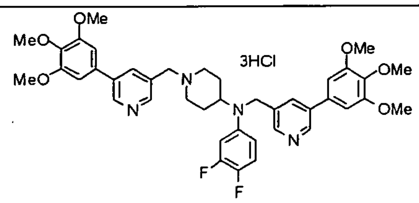 | 61% | 1.71-1.90 (m, 4H), 2.12-2.25 (m, 2H), 2.95-3.05 (m, 2H), 3.57-3.75 (m, 1H), 3.59 (s, 2H), 3.88 (s, 3H), 3.90 (s, 9H), 3.93 (s, 6H), 4.50 (s, 2H), 6.32-6.43 (m, 1H), 6.54 (ddd, 1H, J=13.6 Hz, 6.4 Hz, 2.7 Hz), 6.67 (s, 2H), 6.73-6.78 (m, 3H), 6.96 (dd, 1H, J=18.9 Hz, 9.6 Hz), 7.63 (s, 1H), 7.76 (s, 1H), 8.46 (s, 1H), 8.50 (d, 1H, J=1.6 Hz), 8.66 (d, 1H, J=1.8 Hz), 8.70 (d, 1H, J=2.0 Hz). |
| 154 | 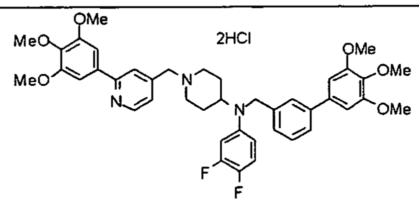 | 82% | 1.74-1.90 (m, 4H), 2.13-2.22 (m, 2H), 2.95-3.01 (m, 2H), 3.57 (s, 2H), 3.63-3.73 (m, 1H), 3.88 (s, 3H), 3.89 (s, 6H), 3.90 (s, 3H), 3.96 (s, 6H), 4.51 (s, 2H), 6.34-6.40 (m, 1H), 6.52 (ddd, 1H, J=14.1 Hz, 6.6 Hz, 3.1 Hz), 6.70 (s, 2H), 6.94 (dd, 1H, J=19.2 Hz, 9.4 Hz), 7.17-7.26 (m, 4H), 7.32-7.42 (m, 3H), 7.59 (s, 1H), 8.59 (d, 1H, J=5.1 Hz). |
| 155 | 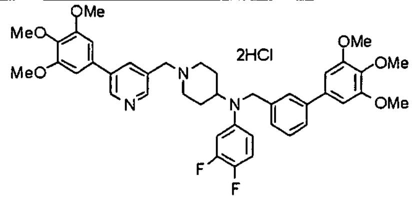 | 75% | 1.74-1.90 (m, 4H), 2.13-2.21 (m, 2H), 2.95-3.04 (m, 2H), 3.59 (s, 2H), 3.63-3.72 (m, 1H), 3.88 (s, 3H), 3.89 (s, 6H), 3.89 (s, 3H), 3.93 (s, 6H), 4.49 (s, 2H), 6.33-6.39 (m, 1H), 6.52 (ddd, 1H, J=14.3 Hz, 3.7 Hz, 2.9 Hz), 6.69 (s, |

| | | | |
|---|---|---|---|
| | | | 2H), 6.75 (s, 2H), 6.94 (dd, 1H, J=19.1 Hz, 9.8 Hz), 7.19 (d, 1H, J=7.8 Hz), 7.32-7.41 (m, 3H), 7.76 (s, 1H), 8.50 (d, 1H, J=1.5 Hz), 8.69 (s, 1H). |
| 156 | 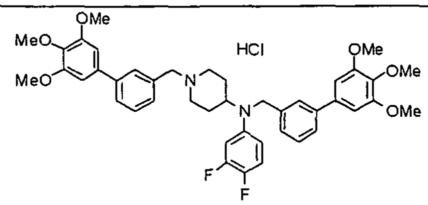 | 79% | 1.72-1.88 (m, 4H), 2.08-2.18 (m, 2H), 2.98-3.05 (m, 2H), 3.58 (s, 2H), 3.62-3.72 (m, 1H), 3.88 (s, 3H), 3.89 (s, 9H), 3.92 (s, 6H), 4.45 (s, 2H), 6.33-6.39 (m, 1H), 6.51 (ddd, 1H, J=13.9 Hz, 6.6 Hz, 3.0 Hz), 6.69 (s, 2H), 6.76 (s, 2H), 6.93 (dd, 1H, J=19.3 Hz, 9.5 Hz), 7.19 (d, 1H, J=7.6 Hz), 7.25-7.47 (m, 7H). |

Referential Example 179

Synthesis of 1-(tert-butoxycarbonyl)-4-[(4-fluorophenyl)amino]piperidine:

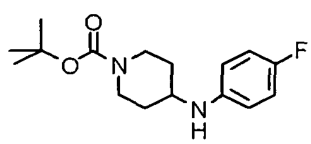

1-(tert-Butoxycarbonyl)-4-piperidone (5.00 g) was reacted with 4-fluoroaniline (2.66 g) in the same manner as described in Referential Example 37 to give a white powder of the title compound.
Yield: 4.99 g (71%).
$^1$H-NMR (400 MHz, $CDCl_3$) δ: 1.23-1.36 (m, 2H), 1.46 (s, 9H), 1.97-2.05 (m, 2H), 2.84-2.96 (m, 2H), 3.30-3.39 (m, 2H), 3.96-4.14 (m, 2H), 6.51-6.57 (m, 2H), 6.84-6.91 (m, 2H).

Referential Example 180

Synthesis of 1-(tert-butoxycarbonyl)-4-[N-(4-fluorophenyl)-N-[[2-(3,4,5-trimethoxyphenyl)pyridin-4-yl]methyl]amino] piperidine:

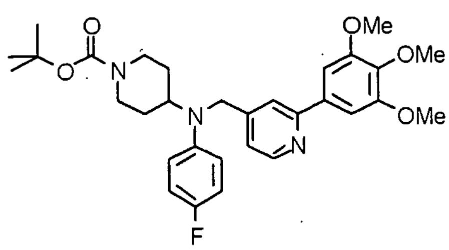

1-(tert-Butoxycarbonyl)-4-[(4-fluorophenyl)amino]piperidine (589 mg) and 4-chloromethyl-2-(3,4,5-trimethoxyphenyl)pyridine (588 mg) were treated in the same manner as described in Preparation Example 9 to give a light

yellow amorphous substance of the title compound.
Yield: 702 mg (64%).
$^1$H-NMR (400 MHz, $CDCl_3$) δ: 1.45 (s, 9H), 1.48-1.64 (m, 2H), 1.81-1.90 (m, 2H), 2.72-2.85 (m, 2H), 3.69-3.98 (m, 1H), 3.89 (m, 3H), 3.94 (m, 6H), 4.16-4.28 (m, 2H), 4.43 (s, 2H), 6.66-6.73 (m, 2H), 6.91 (dd, 2H, J=9.2 Hz, 9.2 Hz), 7.12-7.16 (m, 3H), 7.53 (s, 1H).

Referential Example 181

Synthesis of 4-[N-(4-fluorophenyl)-N-[[2-(3,4,5-trimethoxyphenyl)pyridin-4-yl]methyl]amino] piperidine dihydrochloride:

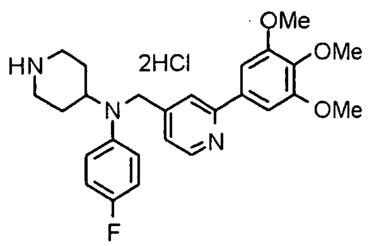

1-(tert-Butoxycarbonyl)-4-[N-(4-fluorophenyl)-N-[[2-(3,4,5-trimethoxyphenyl)pyridin-4-yl]methyl]amino]piperidine (702 mg) was treated in the same manner as described in Referential Example 94 to give a light yellow powder of the title compound.
Yield: 561 mg (84%).

Referential Example 182

Synthesis of 1-(tert-butoxycarbonyl)-4-[N-(4-fluorophenyl)-N-[[3-(3,4,5-trimethoxyphenyl)pyridin-5 -yl]methyl]amino] piperidine:

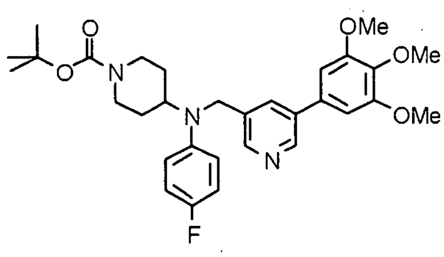

1-(tert-Butoxycarbonyl)-4-[(4-fluorophenyl)amino]piperidine (589 mg) and 5-chloromethyl-3-(3,4,5-trimethoxyphenyl)pyridine (588 mg) were treated in the same manner as described in Preparation Example 9 to give a light yellow amorphous substanceof the title compound.
Yield: 190 mg (17%).
$^1$H-NMR (400 MHz, $CDCl_3$) δ: 1.45 (s, 9H), 1.50-1.73 (m, 2H), 1.82-1.90 (m, 2H), 2.71-2.85 (m, 2H), 3.71 (tt, 1H, J=11.7 Hz, 3.1 Hz), 3.89 (s, 3H), 3.90 (s, 6H), 4.12-4.30 (m, 2H), 4.45 (s, 2H), 6.66 (s, 2H), 6.73-6.78 (m, 2H), 6.91 (dd, 2H, J=9.2 Hz, 8.2 Hz), 7.65 (s, 1H), 8.49 (d, 1H, J=2.0 Hz), 8.65 (d, 1H, J=2.0 Hz).

Referential Example 183

Synthesis of 4-[N-(4-fluorophenyl)-N-[[3-(3,4,5-trimethoxyphenyl)pyridin-5-yl]methyl]amino] piperidine dihydrochloride:

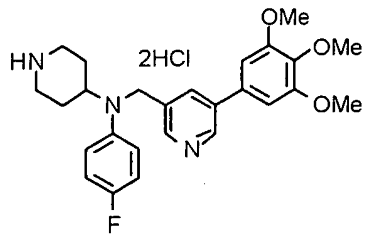

1-(tert-Butoxycarbonyl)-4-[N-(4-fluorophenyl)-N-[[3-(3,4,5-trimethoxyphenyl)pyridin-5-yl]methyl]amino]piperidine (190 mg) was treated in the same manner as described in Referential Example 94 to give a light yellow powder of the title compound.
Yield: 165 mg (91%).

Referential Example 184

Synthesis of 1-(tert-butoxycarbonyl)-4-[N-(4-fluorophenyl)-N-[3-(3,4,5-trimethoxyphenyl)benzyl] amino]piperidine:

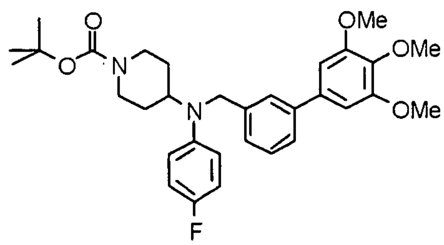

1-(tert-Butoxycarbonyl)-4-[(4-fluorophenyl)amino]piperidine (589 mg) was reacted with 3-(3,4,5-trimethoxyphenyl)benzyl chloride (586 mg) in the same manner as described in Preparation Example 9 to give a light yellow amorphous substance of the title compound.
Yield: 1.01 g (92%).
$^1$H-NMR (400 MHz, $CDCl_3$) δ: 1.44 (s, 9H), 1.51-1.65 (m, 2H), 1.82-1.90 (m, 2H), 2.82-2.84 (m, 2H), 3.78 (tt, 1H, J=11.7 Hz, 3.5 Hz), 3.88 (s, 3H), 3.90 (s, 6H), 4.10-4.30 (m, 2H), 4.45 (s, 2H), 6.68-6.73 (m, 4H), 6.89 (dd, 2H, J=9.2 Hz, 8.2 Hz), 7.21-7.25 (m, 1H), 7.32-7.41 (m, 3H).

Referential Example 185

Synthesis of 4-[N-(4-fluorophenyl)-N-[3-(3,4,5-trimethoxyphenyl)benzyl]amino]piperidine hydrochloride:

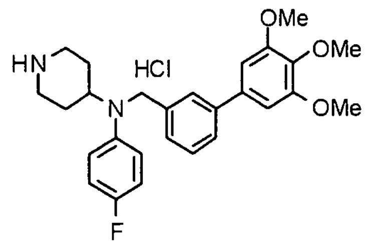

1-(tert-Butoxycarbonyl)-4-[N-(4-fluorophenyl)-N-[3-(3,4,5-trimethoxyphenyl) benzyl]amino]piperidine (1.01 g) was treated in the same manner as described in Referential Example 94 to give a light yellow powder of the title compound.
Yield: 790 mg (88%).

Preparation Examples 157 to 164

These compounds were obtained by the reaction of the amines obtained in Referential Examples 181, 183 and 185 with the chloride derivatives obtained in Referential Examples 3, 42 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 157 | 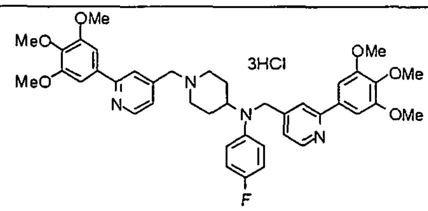 | 62% | 1.60-1.82 (m, 2H), 1.83-1.91 (m, 2H), 2.13-2.23 (m, 2H), 2.95-3.03 (m, 2H), 3.57 (s, 2H), 3.64-3.75 (m, 1H), 3.89 (s, 3H), 3.90 (s, 3H), 3.93 (s, 6H), 3.96 (s, 6H), 4.48 (s, 2H), 6.65-6.70 (m, 2H), 6.90 (dd, 2H, J=8.8 Hz, 8.8 Hz), 7.13-7.16 (m, 3H), 7.20 (d, 1H, J=5.1 Hz), 7.22 (s, 2H), 7.54 (s, 1H), 7.59 (s, 1H), 8.55 (d, 1H, J=5.1 Hz), 8.59 (d, 1H, J=4.9 Hz). |
| 158 | 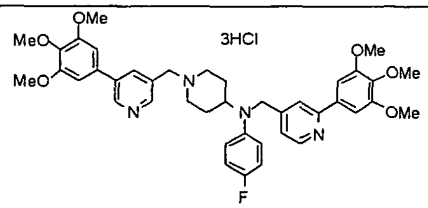 | 53% | 1.66-1.95 (m, 4H), 2.12-2.24 (m, 2H), 2.95-3.07 (m, 2H), 3.60 (s, 2H), 3.64-3.76 (m, 1H), 3.89 (s, 3H), 3.90 (s, 3H), 3.92 (s, 6H), 3.93 (s, 6H), 4.47 (s, 2H), 6.63-6.70 (m, 1H), 6.75 (s, 2H), 6.90 (dd, 1H, J=9.2 Hz, 9.2 Hz), 7.11-7.16 (m, 3H), 7.53 (s, 1H), 7.77 (s, 1H), 8.50 (d, 1H, J=2.0 Hz), 8.55 (d, 1H, J=4.9 Hz), 8.70 (d, 1H, J=5.9 Hz). |
| 159 | 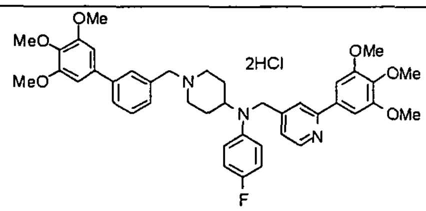 | 51% | 1.64-1.90 (m, 4H), 2.07-2.20 (m, 4H), 2.97-3.08 (m, 2H), 3.59 (s, 2H), 3.64-3.76 (m, 1H), 3.89 (s, 6H), 3.92 (s, 6H), 3.93 (s, 6H), 4.47 (s, 2H), 6.62-6.70 (m, 2H), 6.77 (s, 2H), 6.86-6.93 (m, 2H), 7.11-7.16 (m, 3H), 7.25-7.31 (m, 3H), 7.37 (dd, 1H, J=7.4 Hz, 7.4 Hz), 7.42-7.49 (m, 2H), 7.53 (s, 1H), 8.54 (d, 1H, J=5.1 Hz). |
| 160 | 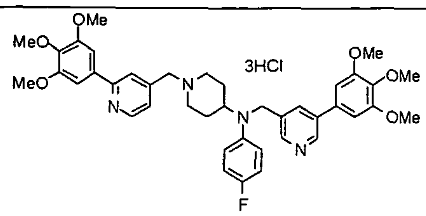 | 49% | 1.74-1.98 (m, 4H), 2.10-2.30 (m, 2H), 2.90-3.12 (m, 2H), 3.53-3.73 (m, 3H), 3.88 (s, 3H), 3.89 (s, 6H), 3.90 (s, 3H), 3.96 (s, 6H), 4.50 (s, 2H), 6.66 (s, 2H), 6.70-6.76 (m, 2H), 6.90 (dd, 2H, J=8.8 Hz, 8.8 |

| | | | |
|---|---|---|---|
| | | | Hz), 7.19-7.28 (m, 3H), 7.65 (br, 2H), 8.49 (d, 1H, J=1.8 Hz), 8.60 (d, 1H, J=4.9 Hz), 8.64 (d, 1H, J=2.2 Hz). |
| 161 | 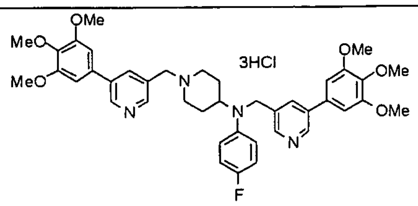 | 26% | 1.67-1.97 (m, 4H), 2.10-2.27 (m, 2H), 2.94-3.06 (m, 2H), 3.56-3.68 (m, 3H), 3.88 (s, 3H), 3.89 (s, 6H), 3.90 (s, 3H), 3.93 (s, 6H), 4.49 (s, 2H), 6.65 (s, 2H), 6.69-6.80 (m, 4H), 6.84-6.93 (m, 2H), 7.64 (s, 1H), 7.77 (br, 1H), 8.48 (d, 1H, J=1.7 Hz), 8.50 (d, 1H, J=1.7 Hz), 8.64 (d, 1H, J=1.9 Hz), 8.70 (s, 1H). |
| 162 | 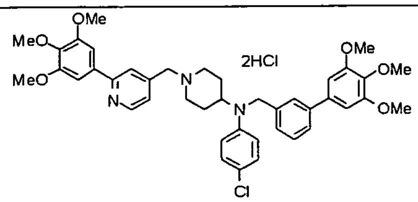 | 83% | 1.72-1.92 (m, 4H), 2.12-2.21 (m, 2H), 2.94-3.02 (m, 2H), 3.57 (s, 2H), 3.64-3.74 (m, 1H), 3.88 (s, 3H), 3.89 (s, 6H), 3.90 (s, 3H), 3.96 (s, 6H), 4.51 (s, 1H), 6.66-6.71 (m, 4H), 6.88 (dd, 2H, J=8.6 Hz, 8.6 Hz), 7.18-7.27 (m, 4H), 7.34 (dd, 1H, J=7.4 Hz, 7.4 Hz), 7.39 (d, 2H, J=5.4 Hz), 7.59 (s, 1H), 8.59 (d, 1H, J=5.1 Hz). |
| 163 | 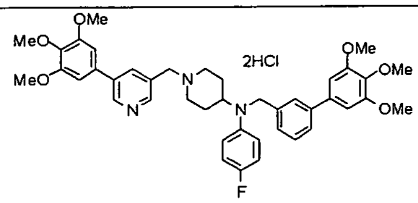 | 68% | 1.68-1.87 (m, 4H), 2.10-2.22 (m, 2H), 2.94-3.04 (m, 2H), 3.59 (s, 2H), 3.65-3.74 (m, 1H), 3.87 (s, 3H), 3.88 (s, 6H), 3.90 (s, 3H), 3.93 (s, 6H), 4.49 (s, 2H), 6.66-6.70 (m, 6H), 6.88 (dd, 2H, J=8.8 Hz, 8.8 Hz), 7.19-7.40 (m, 4H), 7.77 (s, 1H), 8.49 (d, 1H, J=1.8 Hz), 8.70 (s, 1H). |
| 164 | 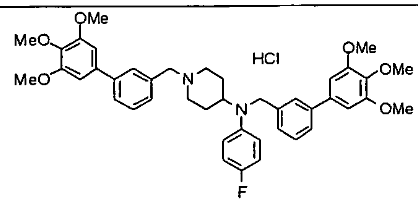 | 74% | 1.70-1.90 (m, 4H), 2.08-2.18 (m, 2H), 2.95-3.05 (m, 2H), 3.58 (s, 2H), 3.63-3.73 (m, 1H), 3.87 (s, 3H), 3.88 (s, 6H), 3.89 (s, 3H), 3.92 (s, 6H), 4.50 (s, 2H), 6.65-6.72 (m, 2H), 6.69 (s, 2H), 6.76 (s, 2H), 6.87 (dd, 2H, J=9.0 Hz, 9.0 Hz), 7.22 (d, 1H, J=7.6 Hz), 7.25-7.48 (m, 9H). |

Referential Example 186

Synthesis of 1-(tert-butoxycarbonyl)-4-phenylaminopiperidine:

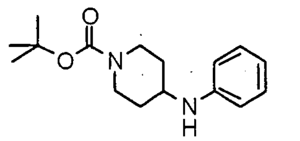

1-(tert-Butoxycarbonyl)-4-piperidone (5.00 g) was reacted with aniline (2.23 g) in the same manner as described in Referential Example 37 to give a white powder of the title compound.
Yield: 3.77 g (57%).
$^1$H-NMR (400 MHz, $CDCl_3$) δ: 1.25-1.38 (m, 2H), 1.47 (s, 9H), 2.00-2.07 (m, 2H), 2.87-2.97 (m, 2H), 3.38-3.53 (m, 2H), 3.96-4.14 (m, 2H), 6.57-6.52 (m, 2H), 6.70 (tt, 1H, J=6.2 Hz, 1.0 Hz), 7.17 (dd, 2H, J=8.6 Hz, 7.2 Hz).

Referential Example 187

Synthesis of 1-(tert-butoxycarbonyl)-4-[N-phenyl-N-[[2-(3,4,5-trimethoxyphenyl)pyridin-4-yl] methyl]amino]piperidine:

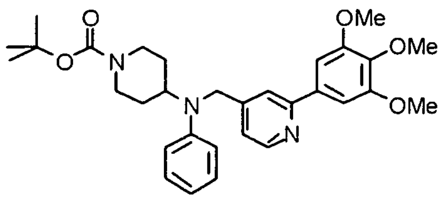

1-(tert-Butoxycarbonyl)-4-phenylaminopiperidine (553 mg) and 4-chloromethyl-2-(3,4,5-trimethoxyphenyl) pyridine (588 mg) were treated in the same manner as described in Preparation Example 9 to give a light yellow amorphous substance of the title compound.
Yeld: 760 mg (71%).
$^1$H-NMR (400 MHz, $CDCl_3$) δ: 1.45 (s, 9H), 1.53-1.63 (m, 2H), 1.83-1.91 (m, 2H), 2.76-2.90 (m, 2H), 3.86-3.97 (m, 1H), 3.89 (s, 3H), 3.93 (s, 6H), 4.14-4.32 (m, 2H), 4.49 (s, 2H), 6.71-6.78 (m, 3H), 7.14 (s, 1H), 7.15 (s, 2H), 7.21 (dd, 2H, J=8.8 Hz, 7.4 Hz), 7.55 (s, 1H), 8.56 (d, 1H, J=5.1 Hz).

Referential Example 188

Synthesis of 4-[N-phenyl-N-[[2-(3,4,5-trimethoxyphenyl)pyridin-4-yl]methyl]amino]piperidine dihydrochloride:

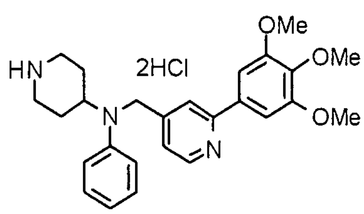

1-(tert-Butoxycarbonyl)-4-[N-phenyl-N-[[2-(3,4,5-trimethoxyphenyl)pyridin-4 -yl]methyl]amino]piperidine (760 mg) was treated in the same manner as described in Referential Example 94 to give light yellow powder of the

title compound.
Yield: 652 mg (90%).

Referential Example 189

Synthesis of 1-(tert-butoxycarbonyl)-4-[N-phenyl-N-[[3-(3,4,5-trimethoxyphenyl)pyridin-5-yl] methyl]amino]piperidine:

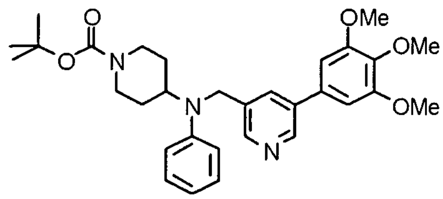

1-(tert-Butoxycarbonyl)-4-N-phenylaminopiperidine (553 mg) and 5-chloromethyl-3-(3,4,5-trimethoxyphenyl) pyridine (588 mg) were treated in the same manner as described in Preparation Example 9 to give a light yellow amorphous substance of the title compound.
Yield: 222 mg (21%).
$^1$H-NMR (400 MHz, $CDCl_3$) δ: 1.45 (s, 9H), 1.52-1.67 (m, 2H), 1.82-1.91 (m, 2H), 2.74-2.87 (m, 2H), 3.88-3.90 (m, 1H), 3.88 (s, 3H), 3.89 (s, 6H), 4.14-4.31 (m, 2H), 4.53 (s, 2H), 6.67 (s, 2H), 6.74-6.80 (m, 3H), 7.21 (dd, 2H, J=8.8 Hz, 7.2 Hz), 7.67 (s, 1H), 8.50 (d, 1H, J=5.3 Hz, 2.2 Hz), 8.66 (d, 1H, J=2.1 Hz).

Referential Example 190

Synthesis of 4-[N-phenyl-N-[[3-(3,4,5-trimethoxyphenyl)pyridin-5-yl]methyl]amino]piperidine dihydrochloride:

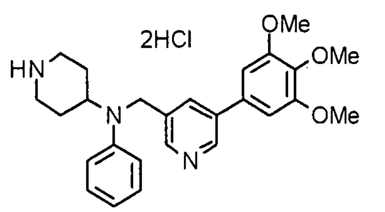

1-(tert-Butoxycarbonyl)-4-[N-phenyl-N-[[3-(3,4,5-trimethoxyphenyl)pyridin-5-yl] methyl]amino]piperidine (222 mg) was treated in the same manner as described in Referential Example 94 to give light yellow powder of the title compound.
Yeld: 197 mg (94%).

Referential Example 191

Synthesis of 1-(tert-butoxycarbonyl)-4-[N-phenyl-N-[3-(3,4,5-trimethoxyphenyl)benzyl]amino] piperidine:

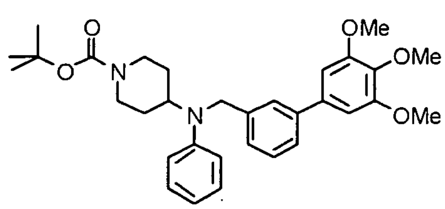

1-(tert-Butoxycarbonyl)-4-phenylaminopiperidine (553 mg) was reacted with 3-(3,4,5-trimethoxyphenyl)benzyl chloride (586 mg) in the same manner as described in Preparation Example 9 to give a light yellow amorphous substance of the title compound.
Yield: 1.06 g (100%).
$^1$H-NMR (400 MHz, $CDCl_3$) δ: 1.45 (s, 9H), 1.52-1.68 (m, 2H), 1.83-1.92 (m, 2H), 2.73-2.86 (m, 2H), 3.88 (s, 3H), 3.89 (s, 6H), 3.94 (tt, 1H, J=11.7 Hz, 3.3 Hz), 4.14-4.30 (m, 2H), 4.52 (s, 2H), 6.69-6.78 (m, 6H), 7.17-7.27 (m, 2H), 7.32-7.42 (m, 3H).

Referential Example 192

Synthesis of 4-[N-phenyl-N-[3-(3,4,5-trimethoxyphenyl)benzyl]amino]piperidine hydrochloride:

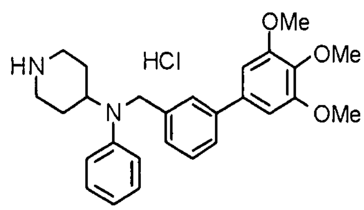

1-(tert-Butoxycarbonyl)-4-[N-phenyl-N-[3-(3,4,5-trimethoxyphenyl)benzyl] amino]piperidine (1.06 g) was treated in the same manner as described in Referential Example 94 to give a light yellow powder of the title compound.
Yield: 909 mg (97%).

Preparation Examples 165 to 169

These compounds were obtained by the reaction of the amines obtained in Referential Examples 188, 190 and 192 with the chloride derivatives obtained in Referential Examples 3 and 48. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 165 | 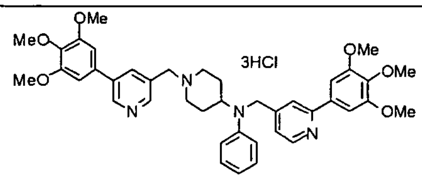 | 53% | 1.63-1.81 (m, 4H), 1.82-1.92 (m, 2H), 2.14-2.24 (m, 2H), 2.95-3.05 (m, 2H), 3.59 (s, 2H), 3.80-4.02 (m, 1H), 3.89 (s, 3H), 3.90 (s, 3H), 3.92 (s, 6H), 3.93 (s, 6H), 4.53 (s, 2H), 6.69-6.77 (m, 5H), 7.13-7.17 (m, 3H), 7.20 (dd, 2H, J=7.6 Hz, 7.6 Hz), 7.55 (s, 1H), 7.76 (s, 1H), 8.51 (d, 1H, J=1.8 Hz), 8.55 (d, 1H, J=5.1 Hz), 8.70 (s, 1H). |
| 166 | 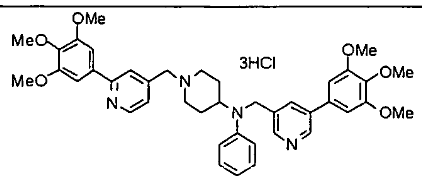 | 50% | 1.85-2.04 (m, 4H), 2.20-2.40 (m, 2H), 2.92-3.25 (m, 2H), 3.60-3.77 (m, 3H), 3.88 (s, 3H), 3.89 (s, 6H), 3.90 (s, 3H), 3.97 (s, 6H), 4.59 (s, 2H), 6.67 (s, 2H), 6.72-6.81 (m, 4H), 7.17-7.30 (m, 4H), 7.68 (s, 1H), 8.50 (s, 1H), 8.62 (d, 1H, J=4.9 Hz), 8.65 (d, 1H, J=2.0 Hz). |

| | | | |
|---|---|---|---|
| 167 | 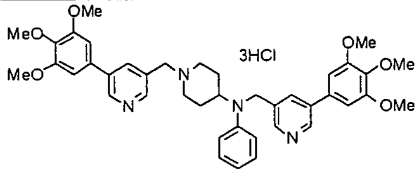 | 43% | 1.72-1.92 (m, 4H), 2.13-2.26 (m, 2H), 2.95-3.04 (m, 2H), 3.59 (s; 2H), 3.78-4.01 (m, 1H), 3.88 (s, 9H), 3.90 (s, 3H), 3.93 (s, 6H), 4.56 (s, 2H), 6.66 (s, 2H), 6.70-6.78 (m, 5H), 7.19 (dd, 2H, J=8.2 Hz, 8.2 Hz), 7.66 (s, 1H), 7.77 (s, 1H), 8.50 (d, 1H, J=2.3 Hz), 8.51 (d, 1H, J=2.2 Hz), 8.65 (d, 1H, J=1.9 Hz), 8.70 (d, 1H, J=2.2 Hz). |
| 168 | 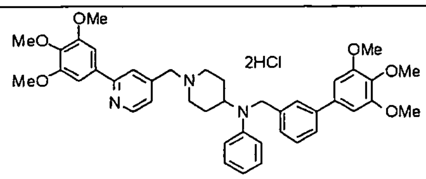 | 82% | 1.75-1.92 (m, 4H), 2.14-2.23 (m, 2H), 2.94-3.01 (m, 2H), 3.57 (s, 2H), 3.80-3.94 (m, 1H), 3.87 (s, 3H), 3.88 (s, 6H), 3.90 (s, 3H), 3.96 (s, 6H), 4.57 (s, 2H), 6.67-6.77 (m, 5H), 7.15-7.27 (m, 5H), 7.34 (dd, 1H, J=7.4 Hz, 7.4 Hz), 7.39 (d, 1H, 7.6 Hz), 7.42 (s, 1H), 7.59 (s, 1H), 8.59 (d, 1H, J=5.1 Hz). |
| 169 | 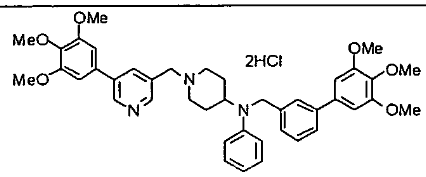 | 65% | 1.72-1.91 (m, 4H), 2.13-2.22 (m, 2H), 2.95-3.03 (m, 2H), 3.59 (s, 2H), 3.79-4.00 (m, 1H), 3.87 (s, 3H), 3.87 (s, 6H), 3.90 (s, 3H), 3.93 (s, 6H), 4.56 (s, 2H), 6.66-6.77 (m, 7H), 7.18 (dd, 2H, J=7.4 Hz, 7.4 Hz), 7.24 (d, 1H, J=7.4 Hz), 7.33 (dd, 1H, J=7.4 Hz, 7.4 Hz), 7.38 (d, 1H, J=7.6 Hz), 7.41 (s, 1H), 7.76 (s, 1H), 8.50 (d, 1H, J=1.6 Hz), 8.69 (d, 1H, J=2.2 Hz). |

Referential Examples 193 to 203

These compounds were prepared by the same procedure as described in Referential Examples 1 to 3. Structures and NMR data are listed below.

| Referential Example | Structure | NMR data (400 MHz, $CDCl_3$) δ |
|---|---|---|
| 193 | 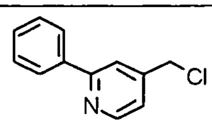 | 4.61 (s, 2H), 7.25 (d, 1H, J=1.2 Hz), 7.41-7.52 (m, 3H), 7.75 (d, 1H, J=0.8 Hz), 7.98-8.02 (m, 2H), 8.69 (d, 1H, J=4.9 Hz). |

| | | |
|---|---|---|
| 194 | 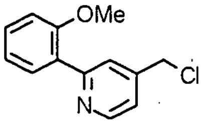 | 3.87 (s, 3H), 4.60 (s, 2H), 7.01 (d, 1H, J=8.4 Hz), 7.08 (t, 1H, J=7.4 Hz), 7.24 (dd, 1H, J=5.1 Hz, 1.4 Hz), 7.38 (dt, 1H, J=7.4 Hz, 1.8 Hz), 7.77 (dd, 1H, J=7.6 Hz, 1.8 Hz), 7.84 (s, 1H), 8.69 (d, 1H, J=5.1 Hz) |
| 195 | 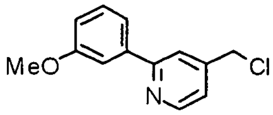 | 3.90 (s, 3H), 4.60 (s, 2H), 6.87-7.03 (1H, m), 7.39 (t, 1H, 7.8Hz), 7.50-7.66 (m, 2H), 7.73 (s, 1H), 8.68 (d, 1H, J=5.1 Hz) |
| 196 | 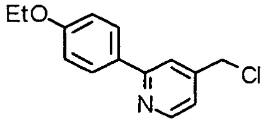 | 1.45 (t, 3H, J=7.0 Hz), 4.12 (q, 2H, J=7.0 Hz), 4.59 (s, 2H), 6.99 (d, 2H, J=8.8 Hz), 7.18 (d, 1H, J=5.1 Hz), 7.20-7.29 (m, 1H), 7.68 (s, 1H), 7.95 (d, 2H, J=8.8 Hz), 8.63 (d, 1H, J=5.1 Hz) |
| 197 | 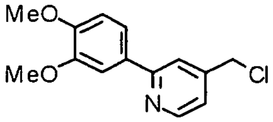 | 3.95 (s, 3H), 4.00 (s, 3H), 4.60 (s, 2H), 6.96 (d, 1H, J=8.4 Hz), 7.21 (d, 1H, J=4.1 Hz), 7.53 (dd, 1H, J=8.4 Hz, 2.0 Hz), 7.67 (d, 1H, J=2.0 Hz), 7.70 (s, 1H), 8.65 (d, 1H, J=5.1 Hz) |
| 198 | 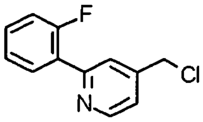 | 4.61 (s, 2H), 7.14-7.21 (m, 1H), 7.21-7.23 (m, 2H), 7.35-7.42 (m, 1H), 7.80 (s, 1H), 7.98 (1H, dt, J=8.0 Hz, 2.0 Hz), 8.73 (d, 1H, J=5.1 Hz) |
| 199 | 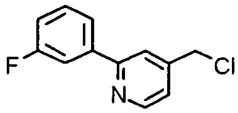 | 4.61 (s, 2H), 7.13 (1H, dt, J=8.4 Hz, 2.8 Hz), 7.28 (1H, d, J=5.0 Hz), 7.40-7.79 (m, 1H), 7.70-7.79 (m, 3H), 8.69 (d, 1H, J=5.0 Hz) |
| 200 | 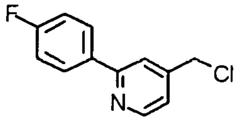 | 4.60 (s, 2H), 7.13-7.20 (m, 2H), 7.25 (1H, d, J=5.1 Hz), 7.70 (s, 1H), 7.95-8.03 (m, 2H), 8.66 (d, 1H, J=5.1 Hz) |
| 201 | 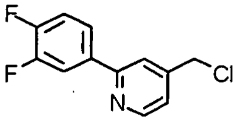 | 4.61 (s, 2H), 7.21-7.30 (m, 2H), 7.69 (s, 1H), 7.73-7.76 (m, 1H), 7.85-7.92 (m, 1H), 8.76 (d, 1H, J=4.9 Hz) |
| 202 | 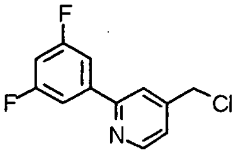 | 4.61 (s, 2H), 6.86-6.91 (m, 1H), 7.31 (1H, d, J=5.1 Hz), 7.51-7.59 (m, 2H), 7.71 (s, 1H), 8.69 (d, 1H, J=5.1 Hz) |
| 203 | 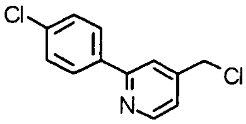 | 4.61 (s, 2H), 7.26 (d, 1H, J=4.9 Hz), 7.45 (d, 2H, J=8.4 Hz), 7.72 (s, 1H), 7.95 (d, 2H, J=8.4 Hz), 8.68 (s, 1H, J=4.9 Hz) |

Referential Example 204

Synthesis of 1-(tert-butoxycarbonyl)-4-[N-(4-methoxyphenyl)-N-[(2-phenylpyridin-4-yl)methyl]amino]piperidine:

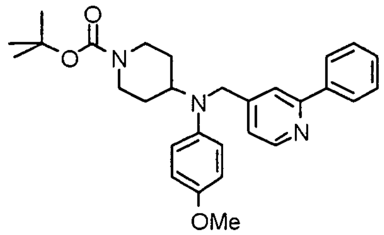

4-(*p*-Anisidino)-1-(tert-butoxycarbonyl)piperidine (612 mg) was reacted with 4-chloromethyl-2-phenylpyridine (204 mg) in the same manner as described in Preparation Example 9 to give the title compound.
Yield: 407 mg (43%).

Referential Example 205

Synthesis of 4-[N-(4-methoxyphenyl)-N-[(2-phenylpyridin-4-yl)methyl]amino]piperidine dihydrochloride:

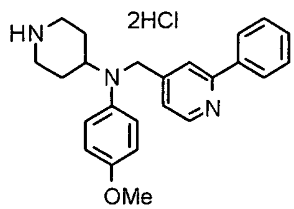

1-(tert-Butoxycarbonyl)-4-[N-(4-methoxyphenyl)-N-[(2-phenylpyridin-4-yl) methyl]amino]piperidine (407 mg) was treated in the same manner as described in Referential Example 94 to give the title compound.
Yield: 365 mg (95%).

Referential Example 206

Synthesis of 1-(tert-butoxycarbonyl)-4-[N-(4-methoxyphenyl)-N-[[2-(2-methoxyphenyl)pyridin-4-yl]methyl]amino] piperidine:

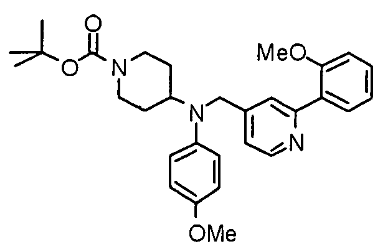

4-(*p*-Anisidino)-1-(tert-butoxycarbonyl)piperidine (306 mg) was reacted with 4-chloromethyl-2-(2-methoxyphenyl)pyridine (234 mg) in the same manner as described in Preparation Example 9 to give the title compound.
Yield: 237mg (72%).

Referential Example 207

Synthesis of 4-[N-(4-methoxyphenyl)-N-[[2-(2-methoxyphenyl)pyridin-4-yl]methyl]amino] piperidine dihydrochloride:

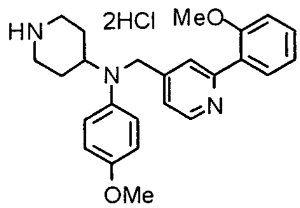

1-(tert-Butoxycarbonyl)-4-[N-(4-methoxyphenyl)-N-[[2-(2-methoxyphenyl) pyridin-4-yl]methyl]amino]piperidine (360 mg) was treated in the same manner as described in Referential Example 94 to give the title compound.
Yield: 365mg (65%).

Referential Example 208

Synthesis of 1-(tert-butoxycarbonyl)-4-[N-(4-methoxyphenyl)-N-[[2-(3-methoxyphenyl)pyridin-4-yl]methyl]amino] piperidine:

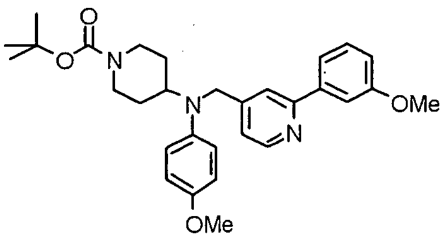

4-(*p*-Anisidino)-1-(tert-butoxycarbonyl)plperidine (306 mg) was reacted with 4-chloromethyl-2-(3-methoxyphenyl)pyridine (234 mg) were condensed in the same manner as described in Preparation Example 9 to give the title compound.
Yield: 550mg (theoretical yield).

Referential Example 209

Synthesis of 4-[N-(4-methoxyphenyl)-N-[[2-(3-methoxyphenyl)pyridin-4-yl]methyl]amino] piperidine dihydrochloride:

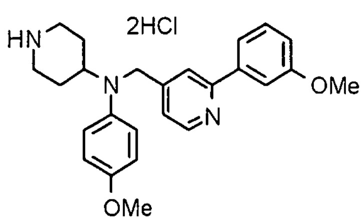

1-(tert-Butoxycarbonyl)-4-[N-(4-methoxyphenyl)-N-[[2-(3-methoxyphenyl) pyridin-4-yl]methyl]amino]piperidine (550 mg) was treated in the same manner as described in Referential Example 94 to give the title compound.
Yield: 436g (85%).

Referential Example 210

Synthesis of 1-(tert-butoxycarbonyl)-4-[N-[[2-(4-ethoxyphenyl)pyridin-4-yl]methyl]-N-(4-methoxyphenyl)]amino] piperidine:

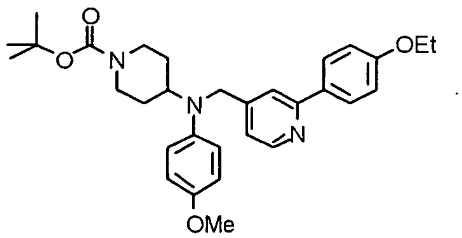

4-(*p*-Anisidino)-1-(tert-butoxycarbonyl)piperidine (306 mg) was reacted with 4-chloromethyl-2-(4-ethoxyphenyl)pyridine (248 mg) in the same manner as described in Preparation Example 9 to give the title compound. Yield: 515 mg (99%).

Referential Example 211

Synthesis of 4-[N-[[2-(4-ethoxyphenyl)pyridin-4-yl]methyl]-N-(4-methoxyphenyl)amino] piperidine dihydrochloride:

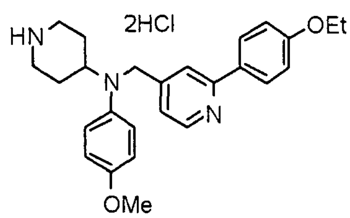

1-(tert-Butoxycarbonyl)-4-[N- [2-(4-ethoxyphenyl)pyridin-4-yl]methyl-N-(4-methoxyphenyl)amino]piperidine (515 mg) was treated in the same manner as described in Referential Example 94 to give the title compound. Yield: 418 mg (80%).

Referential Example 212

Synthesis of 1-(tert-butoxycarbonyl)-4-[N-[[2-(3,4-dimethoxyphenyl)pyridin-4-yl]methyl] -[N-(4-methoxyphenyl)amino] piperidine:

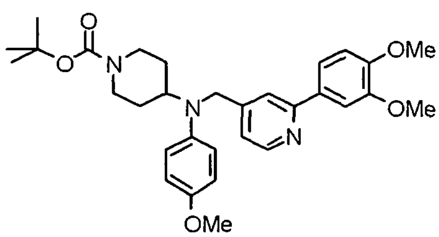

4-(*p*-Anisidino)-1-(tert-butoxycarbonyl)piperidine (306 mg) was reacted with 4-chloromethyl-2-(3,4-dimethoxyphenyl)pyridine (264 mg) in the same manner as described in Preparation Example 9 to give the title compound.

Yield: 600 mg (theoretical yield).

Referential Example 213

Synthesis of 4-[N-[[2-(3,4-dimethoxyphenyl)pyridin-4-yl]methyl]-N-(4-methoxyphenyl)amino] piperidine dihydrochloride:

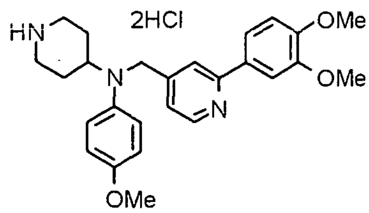

1-(tert-Butoxycarbonyl)-4-[N-[[2-(3,4-dimethoxyphenyl)pyridin-4-yl]methyl]-N-(4-methoxyphenyl)amino]piperidine (600 mg) was treated in the same manner as described in Referential Example 94 to give the title compound. Yield: 416 mg (77%).

Referential Example 214

Synthesis of 1-(tert-butoxycarbonyl)-4-[N-[[2-(2-fluorophenyl)pyridin-4-yl]methyl]-N-(4-methoxyphenyl)amino] piperidine:

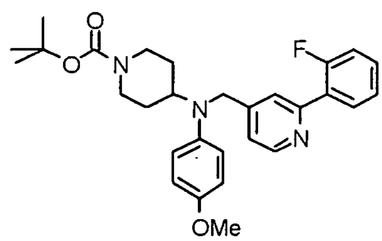

4-(*p*-Anisidino)- (tert-butoxycarbonyl)piperidine (306 mg) was reacted with 4-chloromethyl-2-(2-fluorophenyl) pyridine (222 mg) in the same manner as described in Preparation Example 9 to give the title compound.
Yeld: 530 mg (theoretical yield).

Referential Example 215

Synthesis of 4-[N-[[2-(2-fluorophenyl)pyridin-4-yl]methyl]-N-(4-methoxyphenyl)amino]piperidine dihydrochloride:

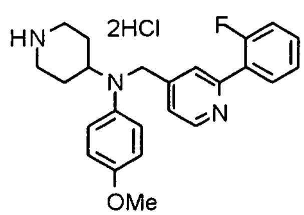

1-(tert-Butoxycarbonyl)-4-[N-[[2-(2-fluorophenyl)pyridin-4-yl]methyl]-N-(4-methoxyphenyl)amino]piperidine (530 mg) was treated in the same manner as described in Referential Example 94 to give the title compound.
Yield: 423mg (85%).

Referential Example 216

Synthesis of 1-(tert-butoxycarbonyl)-4-[N-[[2-(3-fluorophenyl)pyridin-4-yl]methyl]-N-(4-methoxyphenyl)amino] piperidine:

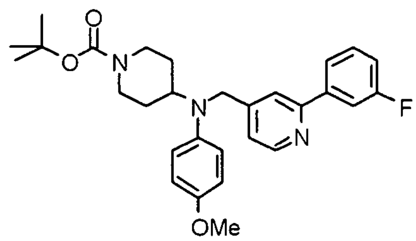

4-(*p*-Anisidino)- (tert-butoxycarbonyl)piperidine (153 mg) was reacted with 4-chloromethyl-2-(3-fluorophenyl) pyridine (111 mg) in the same manner as described in Preparation Example 9 to give the title compound.
Yield: 270 mg (theoretical yield).

Referential Example 217

Synthesis of 4-[N-[2-(3-fluorophenyl)pyridin-4-yl]methyl-N-(4-methoxyphenyl)amino]piperidine dihydrochloride:

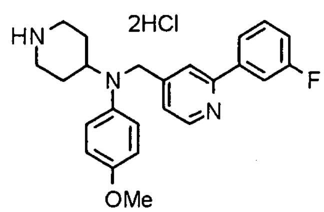

1-(tert-Butoxycarbonyl)-4-[N-[[2-(3-fluorophenyl)pyridin-4-yl]methyl]-N-(4-methoxyphenyl)amino]piperidine (270 mg) was treated in the same manner as described in Referential Example 94 to give the title compound.
Yield: 193 mg (70%).

Referential Example 218

Synthesis of 1-(tert-butoxycarbonyl)-4-[N-[[2-(4-fluorophenyl)pyridin-4-yl]methyl]-N-(4-methoxyphenyl)amino] piperidine:

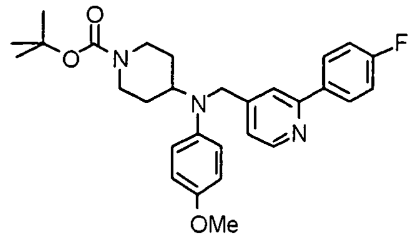

4-(*p*-Anisidino)-1-(tert-butoxycarbonyl)piperidine (306 mg) was reacted with 4-chloromethyl-2-(4-fluorophenyl)pyridine (222 mg) in the same manner as described in Preparation Example 9 to give the title compound. Yeld: 550 mg (theoretical yield).

Referential Example 219

Synthesis of 4-[N-[[2-(4-fluorophenyl)pyridin-4-yl]methyl]-N-(4-methoxyphenyl)amino]piperidine dihydrochloride:

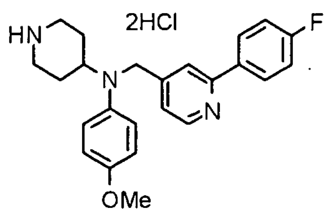

1-(tert-Butoxycarbonyl)-4-[N-[[2-(4-fluorophenyl)pyridin-4-yl]methy]-1-N-(4-methoxyphenyl)amino]piperidine (550 mg) was treated in the same manner as described in Referential Example 94 to give the title compound. Yield: 439 mg (88%).

Referential Example 220

Synthesis of 1-(tert-butoxycarbonyl)-4-[N-[[2-(3,4-difluorophenyl)pyridin-4-yl]methyl]-N-(4-methoxyphenyl)amino] piperidine:

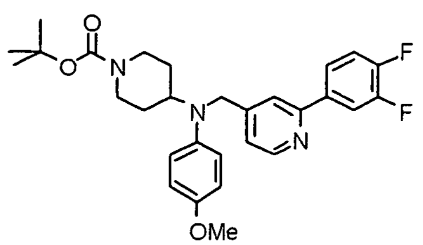

4-(*p*-Anisidino)-1-(tert-butoxycarbonyl)piperidine (306 mg) was reacted with 4-chloromethyl-2-(3,4-difluorophenyl)pyridine (240 mg) in the same manner as described in Preparation Example 9 to give the title compound.

Yield: 590 mg (theoretical yield).

Referential Example 221

Synthesis of 4-[N-[[2-(3,4-difluorophenyl)pyridin-4-yl]methyl] -N-(4-methoxyphenyl)amino]piperidine dihydrochloride:

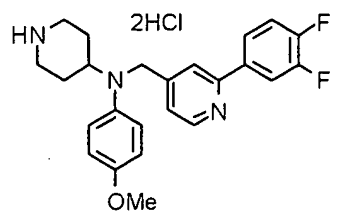

1-(tert-Butoxycarbonyl)-4-[-N-[[2-(3,4-difluorophenyl)pyridin-4-yl]methyl]-N -(4-methoxyphenyl)amino]piperidine (590 mg) was treated in the same manner as described in Referential Example 94 to give the title compound. Yield: 483 mg (93%).

Referential Example 222

Synthesis of 1-(tert-butoxycarbonyl)-4-[N-[[2-(3,5-difluorophenyl)pyridin-4-yl]methyl]-N-(4-methoxyphenyl)amino] piperidine:

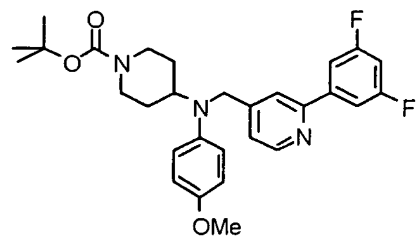

4-(*p*-Anisidino)-1-(tert-butoxycarbonyl)piperidine (306 mg) was reacted with 4-chloromethyl-2-(3,5-difluorophenyl)pyridine (240 mg) in the same manner as described in Preparation Example 9 to give the title compound. Yield: 530 mg (theoretical yield).

Referential Example 223

Synthesis of 4-[N-[[2-(3,5-difluorophenyl)pyridin-4-yl]methyl]-N-(4-methoxyphenyl)amino] piperidine dihydrochloride:

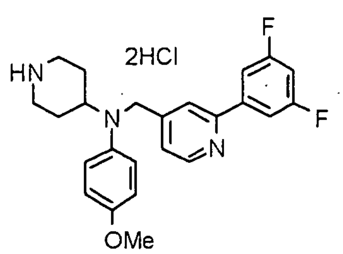

1-(tert-Butoxycarbonyl)-4-[N-[[2-(3,5-difluorophenyl)pyridin-4-yl]methyl]-N-(4-methoxyphenyl)amino]piperidine (530 mg) was treated in the same manner as described in Referential Example 94 to give the title compound. Yield: 418 mg (81%).

Referential Example 224

Synthesis of 1-(tert-butoxycarbonyl)-4-[N-[[2-(4-chlorophenyl)pyridin-4-yl]methyl]-N-(4-methoxyphenyl)amino] piperidine:

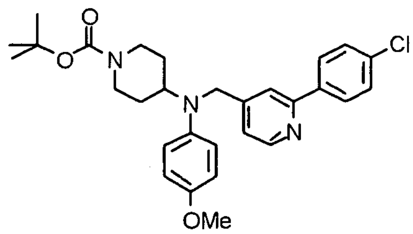

4-(*p*-Anisidino)-1-(tert-butoxycarbonyl)piperidine (306 mg) was reacted with 4-chloromethyl-2-(4-chlorophenyl)pyridine (238 mg) in the same manner as described in Preparation Example 9 to give the title compound. Yield: 600 mg (theoretical yield).

Referential Example 225

Synthesis of 4-[N-[[2-(4-chlorophenyl)pyridin-4-yl]methyl]-N-(4-methoxyphenyl)amino]piperidine dihydrochloride:

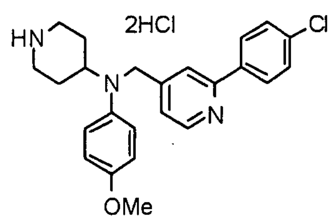

1-(tert-Butoxycarbonyl)-4-[N-[[2-(4-chlorophenyl)pyridin-4-yl]methyl]-N-(4-methoxyphenyl)amino]piperidine: (600 mg) was treated in the same manner as described in Referential Example 94 to give the title compound. Yield: 447 mg (86%).

Preparation Examples 170 to 202

These compounds were obtained by the reaction of the amines obtained in Referential Examples 96, 205, 207, 209, 211, 213, 215, 217, 219, 221, 223 and 225 with the chloride derivatives obtained in Referential Examples 3, 193, 194, 195, 196, 197, 198, 199, 200, 201, 202 and 203. Free bases obtained were then converted to the corresponding hydrochlorides. Yields and NMR data of their free bases are listed below.

| Preparation Example | Structure | Yield | NMR data (400 MHz, measured as free bases, $CDCl_3$) δ |
|---|---|---|---|
| 170 | 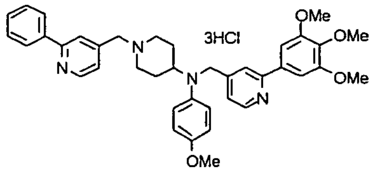 | 47% | 1.67-1.80 (m, 2H), 1.83-1.91 (m, 2H), 2.10-2.19 (m, 2H), 2.93-3.00 (m, 2H), 3.54-3.65 (m, 1H), 3.56 (s, 2H), 3.73 (s, 3H), 3.89 (s, 3H), 3.93 (s, 6H), 4.45 (s, 3H), 6.73 (d, 2H, J=9.4 Hz), 6.78 (d, 2H, J=9.4 Hz), 7.14-7.21 (m, 2H), 7.15 (s, 2H), 7.38-7.49 (m, 3H), 7.57 (s, 1H), 7.68 (s, 1H), 7.97 (d, 1H, J=1.0 Hz), 7.99 (d, 1H, J=1.6 Hz), 8.54 (d, 1H, J=5.1 Hz), 8.61 (d, 1H, J=5.1 Hz). |
| 171 | 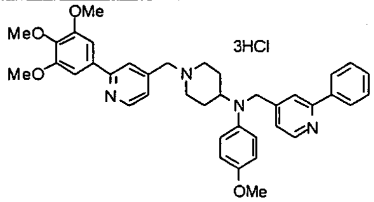 | 55% | 1.62-1.80 (m, 2H), 1.84-1.93 (m, 2H), 2.10-2.20 (m, 2H), 2.93-3.02 (m, 2H), 3.53-3.66 (m, 1H), 3.56 (s, 2H), 3.73 (s, 3H), 3.90 (s, 3H), 3.96 (s, 6H), 4.44 (s, 2H), 6.65-6.83 (m, 4H), 7.14-7.30 (m, 4H), 7.36-7.50 (m, 3H), 7.59 (s, 1H), 7.67 (s, 1H), 7.93 (d, 2H, J=7.0 Hz), 8.54-8.61 (m, 2H). |
| 172 | 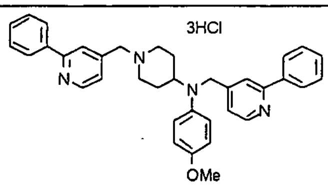 | 54% | 1.67-1.92 (m, 4H), 2.08-2.20 (m, 2H), 2.92-3.01 (m, 2H), 3.52-3.65 (m, 1H), 3.55 (s, 2H), 3.72 (s, 3H), 4.38 (s, 2H), 6.72 (d, 2H, J=9.2 Hz), 6.78 (d, 2H, J=9.0 Hz), 7.18 (dd, 2H, J=4.9 Hz, 4.9 Hz), 7.36-7.50 (m, 6H), 7.67 (s, 1H), |

| | | | |
|---|---|---|---|
| | | | 7.68 (s, 1H), 7.93 (dd, 2H, J=8.4 Hz, 1.2 Hz), 7.98 (dd, 2H, J=8.6 Hz, 1.4 Hz), 8.57 (d, 1H, J=5.1 Hz), 8.60 (d, 1H, J=5.1 Hz). |
| 173 | 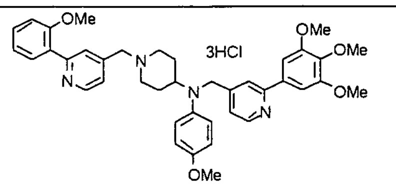 | 100% | 1.66-1.79 (m, 2H), 1.82-1.91 (m, 2H), 2.09-2.20 (m, 2H), 2.93-3.03 (m, 2H), 3.56 (s, 2H), 3.56-3.59 (m, 1H), 3.73 (s, 3H), 3.80 (s, 3H), 3.89 (s, 3H), 3.93 (s, 6H), 4.45 (s, 2H), 6.73 (d, 2H, J=9.3 Hz), 6.78 (d, 2H, J=9.3 Hz), 6.98 (d, 1H, J=8.5 Hz), 7.07 (t, 1H J=7.6 Hz), 7.15 (s, 2H), 7.15-7.19 (m, 2H), 7.33-7.38 (m, 1H), 7.57 (s, 1H), 7.66-7.74 (m, 2H), 8.53 (d, 1H, J=5.1 Hz), 8.61 (d, 1H, J=4.9 Hz). |
| 174 | 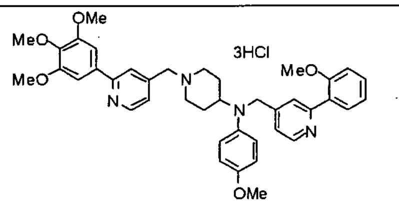 | 94% | 1.70-1.80 (m, 2H), 1.83-1.91 (m, 2H), 2.11-2.18 (m, 2H), 2.92-3.01 (m, 2H), 3.56 (s, 2H), 3.57-3.65 (m, 1H), 3.73 (s, 3H), 3.74 (s, 3H), 3.90 (s, 3H), 3.96 (s, 6H), 4.44 (s, 2H), 6.71 (d, 2H, J=9.0 Hz), 6.78 (d, 2H, J=9.0 Hz), 6.96 (d, 1H, J=8.3 Hz), 7.05 (dt, 1H. J=7.3 Hz, 1.0 Hz), 7.14 (d, 1H, J=5.2 Hz), 7.20 (d, 1H, J=5.2 Hz), 7.22 (2H, s), 7.32-7.37 (m, 1H), 7.59 (s, 1H), 7.71-7.75 (m, 2H), 8.56-8.60 (m, 2H). |
| 175 | 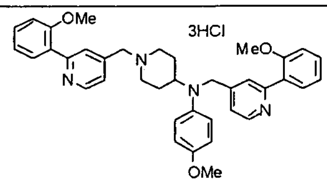 | 98% | 1.67-1.80 (m, 2H), 1.83-1.90 (m, 2H), 2.10-2.19 (m, 2H), 2.94-3.03 (m, 2H), 3.50-3.67 (m, 1H), 3.56 (s, 2H), 3.73 (s, 3H), 3.74 (s, 3H), 3.79 (s, 3H), 4.44 (s, 2H), 6.70 (d, 2H, J=9.3 Hz), 6.78 (d, 2H, J=9.3 Hz), 6.96 (d, 1H, J=8.3 Hz), 6.98 (d, 1H, J=8.8 Hz), 7.04 (dd, 1H, J=7.6 Hz, 1.0 Hz), 7.07 (dd, 1H, 7.6, J=1.0 Hz), 7.12-7.19 (m, 2H), 7.32-7.39 (m, 2H), 7.70-7.75 (m, 4H), 8.58 (d, 1H, J=5.1 Hz), 8.61 (d, 1H, J=4.9 Hz). |
| 176 | 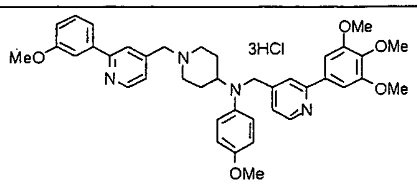 | 100% | 1.68-1.79 (m, 2H), 1.82-1.90 (m, 2H), 2.10-2.19 (m, 2H), 2.90-3.01 (m, 2H), 3.56 (s, 2H), 3.56-3.58 (m, 1H), 3.73 (s, 3H), 3.89 (s, 3H), 3.91 (s, 3H), 3.93 (s, 6H), 4.45(s, |

| | | | |
|---|---|---|---|
| | | | 2H), 6.73 (d, 2H, J=9.3 Hz), 6.78 (d, 2H, J=9.3 Hz), 6.93-6.99 (m, 1H), 7.15 (s, 2H), 7.16-7.20 (m, 2H), 7.37 (t, 1H, J=7.8 Hz), 7.52-7.59 (m, 3H), 7.67 (s, 1H), 8.54 (d, 1H, J=5.1 Hz), 8.60 (d, 1H, J=5.1 Hz). |
| 177 | 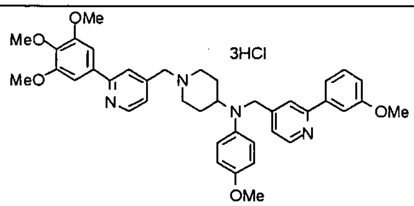 | 100% | 1.68-1.79 (m, 2H), 1.83-1.92 (m, 2H), 2.11-2.16 (m, 2H), 2.91-3.02 (m, 2H), 3.56 (s, 2H), 3.55-3.65 (m, 1H), 3.73 (s, 3H), 3.88 (s, 3H), 3.90 (s, 3H), 3.96 (s, 6H), 4.43 (s, 2H), 6.72 (d, 2H, J=9.3 Hz), 6.78 (d, 2H, J=9.3 Hz), 6.95 (dd, 1H. J=8.3 Hz, 2.7 Hz), 7.16-7.21 (m, 2H), 7.22 (s, 2H), 7.35 (t, 1H, J=7.8 Hz), 7.48 (d, 1H, J=7.8 Hz), 7.53 (t, 1H, J=2.7 Hz), 7.59 (s, 1H), 7.65 (s, 1H), 8.55-8.60 (m, 2H). |
| 178 | 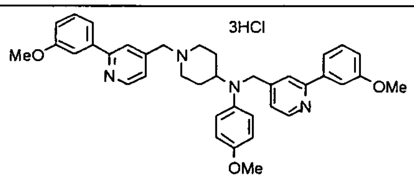 | 100% | 1.65-1.79 (m, 2H), 1.82-1.90 (m, 2H), 2.09-2.19 (m, 2H), 2.92-3.00 (m, 2H), 3.50-3.66 (m, 1H), 3.56 (s, 2H), 3.73 (s, 3H), 3.73 (s, 3H), 3.88 (s, 3H), 3.89 (s, 3H), 4.44 (s, 2H), 6.72 (d, 2H, J=9.3 Hz), 6.78 (d, 2H, J=9.3 Hz), 6.92-6.98 (m, 2H), 7.16-7.21 (m, 2H), 7.34 (d, 1H, J=7.8 Hz), 7.38 (d, 1H, J=8.5 Hz), 7.46-7.59 (m, 4H), 7.65 (s, 1H), 7.67 (s, 1H), 8.57 (dd, 1H, J=5.1 Hz, 0.7 Hz), 8.60 (d, 1H, J=5.1 Hz). |
| 179 | 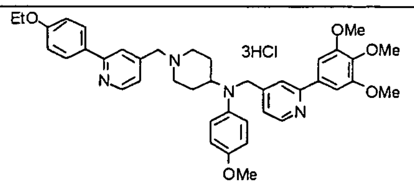 | 76% | 1.44 (t, 3H, J=7.1 Hz), 1.70-1.80 (m, 2H), 1.82-1.91 (m, 2H), 2.10-2.19 (m, 2H), 2.90-3.02 (m, 2H), 3.54 (s, 2H), 3.73-3.78 (m, 1H), 3.73 (s, 3H), 3.88 (s, 3H), 3.93 (s, 6H), 4.09 (q, 2H, J=7.1 Hz), 4.45 (s, 2H), 6.73 (d, 2H, J=9.2 Hz), 6.78 (d, 2H, J=9.2 Hz), 6.97 (d, 2H, J=8.8 Hz), 7.10-7.18 (m, 2H), 7.15 (s, 2H), 7.57 (s, 1H), 7.61 (s, 1H), 7.92 (d, 2H, J=8.8 Hz), 8.52-8.58 (m, 2H). |

| | | | |
|---|---|---|---|
| 180 | 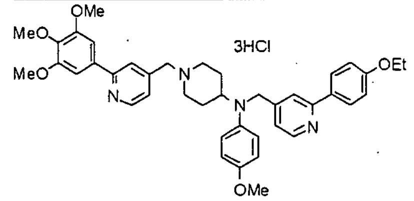 | 93% | 1.43 (t, 3H, J=6.8 Hz), 1.68-1.80 (m, 2H), 1.82-1.92 (m, 2H), 2.10-2.19 (m, 2H), 2.90-3.01 (m, 2H), 3.56 (s, 2H), 3.57-3.64 (m, 1H), 3.73 (s, 3H), 3.90 (s, 3H), 3.96 (s, 6H), 4.08 (q, 2H, J=6.8 Hz), 4.42 (s, 2H), 6.72 (d, 2H, J=9.0 Hz), 6.78 (d, 2H, J=9.3 Hz), 6.95 (d, 2H, J=8.8 Hz), 7.11 (d, 1H, J=5.1 Hz), 7.20 (d, 1H, J=5.1 Hz), 7.22 (s, 2H), 7.58-7.62 (m, 2H), 7.87 (d, 2H, J=8.8 Hz), 8.52 (d, 1H, J=5.1 Hz), 8.58 (d, 1H, J=5.1 Hz). |
| 181 | 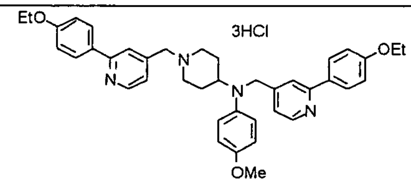 | 100% | 1.43 (t, 3H, J=7.1 Hz), 1.44 (t, 3H, J=7.1 Hz), 1.67-1.78 (m, 2H), 1.82-1.90 (m, 2H), 2.09-2.18 (m, 2H), 2.92-3.00 (m, 2H), 3.54 (s, 2H), 3.55-3.65 (m, 1H), 3.73 (s, 3H), 4.08 (q, 2H, J=7.1 Hz), 4.09 (q, 2H, J=6.8 Hz), 4.42 (s, 2H), 6.71 (d, 2H, J=9.0 Hz), 6.78 (d, 2H, J=9.0 Hz), 6.93-7.00 (m, 4H), 7.10-7.14 (m, 2H), 7.60 (s, 2H), 7.88 (s, 2H), 7.88 (d, 2H, J=8.8 Hz), 7.93 (d, 2H, J=8.8 Hz), 8.52 (d, 1H, J=5.1 Hz), 8.56 (d, 1H, J=4.9 Hz). |
| 182 | 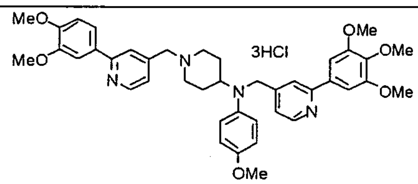 | 100% | 1.68-1.79 (m, 2H), 1.82-1.90 (m, 2H), 2.10-2.19 (m, 2H), 2.90-3.01 (m, 2H), 3.55 (s, 2H), 3.56-3.59 (m, 1H), 3.73 (s, 3H), 3.89 (s, 3H), 3.93 (s, 6H), 3.94 (s, 3H), 3.99 (s, 3H), 4.45 (s, 2H), 6.76 (d, 2H, J=9.5 Hz), 6.78 (d, 2H, J=9.5 Hz), 6.94 (d, 1H, J=8.3 Hz), 7.15 (s, 2H), 7.16-7.19 (m, 2H), 7.49-7.66 (m, 4H), 8.54 (d, 1H, J=4.9 Hz), 8.57 (d, 1H, J=5.1 Hz). |
| 183 | 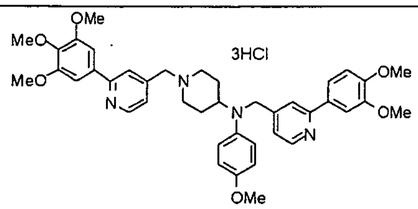 | 100% | 1.68-1.78 (m, 2H), 1.82-1.91 (m, 2H), 2.10-2.18 (m, 2H), 2.93-3.00 (m, 2H), 3.56 (s, 2H), 3.56-3.62 (m, 1H), 3.73 (s, 3H), 3.90 (s, 3H), 3.93 (s, 3H), 3.96 (s, 6H), 3.97 (S, 3H), 4.43 (s, 2H), 6.72 (d, 2H, J=9.3 Hz), 6.78 (d, 2H, J=9.3 Hz), 6.92 (d, 1H, J=8.3 Hz), 7.12 (d, |

| | | | |
|---|---|---|---|
| | | | 1H, J=5.1 Hz), 7.20 (d, 1H, J=5.1 Hz), 7.22 (s, 2H), 7.42 (d, 1H, J=8.5 Hz, 2.2Hz), 7.58-7.63 (m, 3H), 8.53 (d, 1H, J=4.9 Hz), 8.58 (d, 1H, J=5.1 Hz). |
| 184 | 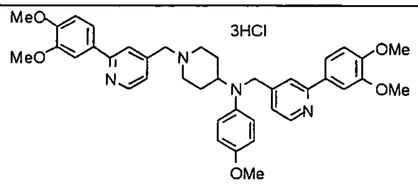 | 89% | 1.67-1.79 (m, 2H), 1.84-1.90 (m, 2H), 2.10-2.19 (m, 2H), 2.93-3.01 (m, 2H), 3.50-3.65 (m, 1H), 3.55 (s, 2H), 3.73 (s, 3H), 3.94 (s, 3H), 3.97 (s, 3H), 3.99 (s, 3H), 4.43 (s, 2H), 6.72 (d, 2H, J=9.3 Hz), 6.78 (d, 2H, J=9.3 Hz), 6.92 (d, 1H, J=8.6 Hz), 6.94 (d, 1H, J=8.3 Hz), 7.14 (d, 1H, J=5.6 Hz), 7.15 (d, 1H, J=6.4 Hz), 7.43 (dd, 1H, J=8.6 Hz, 2.0 Hz), 7.50 (dd, 1H, J=8.3 Hz, 1.9 Hz), 7.60-7.63 (m, 3H), 7.66 (d, 1H, J=2.2 Hz), 8.53 (d, 1H, J=5.1 Hz), 8.57 (d, 1H, J=4.9 Hz). |
| 185 | 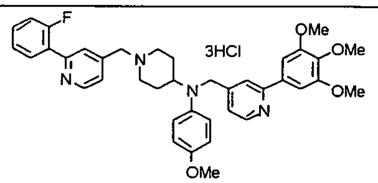 | 100% | 1.68-1.79 (m, 2H), 1.82-1.90 (m, 2H), 2.10-2.20 (m, 2H), 2.93-3.01 (m, 2H), 3.57 (s, 2H), 3.57-3.65 (m, 1H), 3.73 (s, 3H), 3.89 (s, 3H), 3.93 (s, 6H), 4.46 (s, 2H), 6.73 (d, 2H, J=7.3 Hz), 6.78 (d, 2H, J=7.3 Hz), 7.11-7.19 (m, 2H), 7.15 (s, 2H), 7.22-7.29 (m, 2H), 7.34-7.40 (m, 1H), 7.58 (s, 1H), 7.73 (s, 1H), 7.94 (t, 1H, J=8.3 Hz), 8.54 (d, 1H, J=5.1 Hz), 8.64 (d, 1H, J=4.9 Hz). |
| 186 | 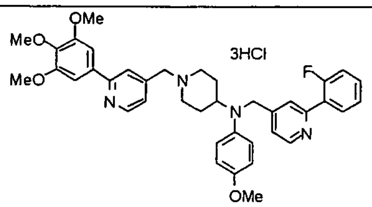 | 88% | 1.68-1.79 (m, 2H), 1.83-1.92 (m, 2H), 2.09-2.16 (m, 2H), 2.93-3.01 (m, 2H), 3.56 (s, 2H), 3.56-3.62 (m, 1H), 3.73 (s, 3H), 3.90 (s, 3H), 3.96 (s, 6H), 4.44(s, 2H), 6.71 (d, 2H, J=9.3 Hz), 6.77 (d, 2H, J=9.3 Hz), 7.10-7.16 (m, 1H), 7.17-7.26 (m,3H), 7.22 (s, 2H), 7.32-7.38 (m, 1H), 7.59 (s, 1H), 7.73 (s, 1H) , 7.92 (dt, 1H, J=8.0 Hz, 2.0 Hz), 8.57-8.61(m, 2H). |

| | | | |
|---|---|---|---|
| 187 | 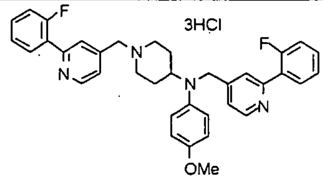 | 100% | 1.66-1.80 (m, 2H), 1.83-1.93 (m, 2H), 2.10-2.20 (m, 2H), 2.92-3.02 (m, 2H), 3.53-3.65 (m, 1H), 3.57 (s, 2H), 3.73 (s, 3H), 4.44 (s, 2H), 6.71 (d, 2H, J=9.0 Hz), 6.78 (d, 2H, J=9.3 Hz), 7.10-7.18 (m, 2H), 7.19-7.29 (m, 4H), 7.32-7.40 (m, 2H), 7.73 (s, 2H), 7.91 (dd, 1H, J=8.1 Hz, 1.4 Hz), 7.95 (dd, 1H, J=7.6 Hz, 1.5 Hz), 8.60 (d, 1H, J=4.9 Hz), 8.64 (d, 1H, J=5.1 Hz). |
| 188 | 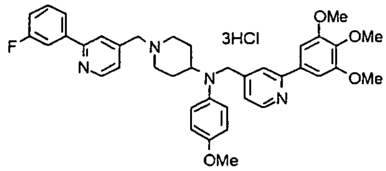 | 96% | 1.67-1.80 (m, 2H), 1.82-1.92 (m, 2H), 2.10-2.20 (m, 2H), 2.91-3.01 (m, 2H), 3.56 (s, 2H), 3.56-3.61(m, 1H), 3.73 (s, 3H), 3.89 (s, 3H), 3.93 (s, 6H), 4.46 (s, 2H), 6.73 (d, 2H, J=9.3 Hz), 6.78 (d, 2H, J=9.3 Hz), 7.06-7.19 (m, 2H), 7.15 (s, 2H), 7.20-7.26 (m, 1H), 7.38-7.45 (m, 1H), 7.56 (s,1H), 766-7.78 (m, 3H), 8.54 (d, 1H, J=5.1 Hz), 8.61 (d, 1H, J=4.9 Hz). |
| 189 | 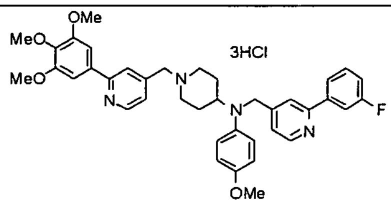 | 92% | 1.65-1.78 (m, 2H), 1.79-1.92 (m, 2H), 2.21-2.26 (m, 2H), 2.90-3.01 (m, 2H), 3.56 (s, 2H), 3.56-3.63 (m, 1H), 3.73 (s, 3H), 3.90 (s, 3H), 3.96 (s, 6H), 4.44 (s, 2H), 6.72 (d, 2H, J=9.3 Hz), 6.78 (d, 2H, J=9.3 Hz), 7.08 (dt, 1H, J=8.3 Hz, 1.7 Hz), 7.18-7.40 (m, 2H), 7.22 (s, 2H), 7.37-7.43 (m, 1H), 7.56-7.72 (m, 4H), 8.55-8.60 (m, 2H). |
| 190 | 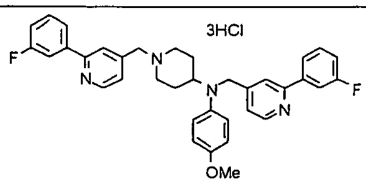 | 55% | 1.66-1.79 (m, 2H), 1.80-1.91 (m, 2H), 2.10-2.20 (m, 2H), 2.88-3.01 (m, 2H), 3.50-3.66 (m, 1H), 3.56 (s, 2H), 3.73 (s, 3H), 4.45 (s, 2H), 6.72 (d, 2H, J=8.5 Hz), 6.79 (d, 2H, J=9.0 Hz), 7.04-7.13 (m, 2H), 7.19-7.25 (m, 2H), 7.35-7.46 (m, 2H), 7.62-7.79 (m, 6H), 8.57 (d, 1H, J=5.1 Hz), 8.61 (d, 1H, J=4.9 Hz). |

| | | | |
|---|---|---|---|
| 191 | 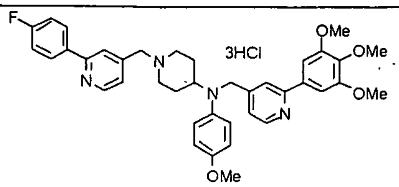 | 100% | 1.68-1.79 (m, 2H), 1.82-1.91(m, 2H), 2.10-2.19 (m, 2H), 2.92-3.00 (m, 2H), 3.55 (s, 2H), 3.56-3.63 (m, 1H), 3.73 (s, 3H), 3.89 (s, 3H), 3.93 (s, 6H), 4.45(s, 2H), 6.73(d, 2H, J=9.3 Hz), 6.78 (d, 2H, J=9.3 Hz), 7.11-7.19 (m, 4H), 7.15 (s, 2H), 7.57 (s, 1H), 7.63 (s, 1H), 7.92-8.01 (m, 2H), 8.54 (d, 1H, J=5.1 Hz), 8.58 (d, 1H, J=5.1 Hz). |
| 192 | 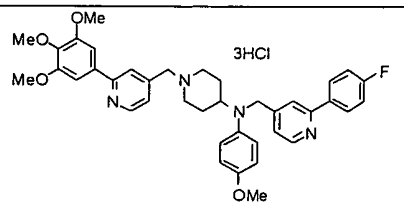 | 100% | 1.68-1.79 (m, 2H), 1.83-1.92 (m, 2H), 2.11-2.19 (m, 2H), 2.93-3.01 (m, 2H), 3.56 (s, 2H), 3.57-3.62 (m, 1H), 3.73 (s, 3H), 3.90 (s, 3H), 3.96 (s, 6H), 4.43 (s, 2H), 6.72 (d, 2H, J=9.3 Hz), 6.78 (d, 2H, J=9.3 Hz), 7.10-7.22 (m, 4H), 7.22 (s, 2H), 7.54-7.66 (m, 2H), 7.88-7.94 (m, 2H), 8.55 (d, 1H, J=4.9 Hz), 8.58 (d, 1H, J=4.9 Hz). |
| 193 | 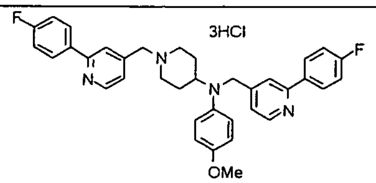 | 90% | 1.66-1.80 (m, 2H), 1.83-1.91 (m, 2H), 2.10-2.19 (m, 2H), 2.92-3.00 (m, 2H), 3.50-3.66 (m, 1H), 3.55 (s, 2H), 3.73 (s, 3H), 4.44 (s, 2H), 6.72 (d, 2H, J=9.3 Hz), 7.78 (d, 2H, J=9.3 Hz), 7.09-7.20 (m, 6H), 7.62 (s, 1H), 7.63 (s, 1H), 7.89-8.00 (m, 4H), 8.55 (d, 1H, J=5.1 Hz), 8.58 (d, 1H, J=4.9 Hz). |
| 194 | 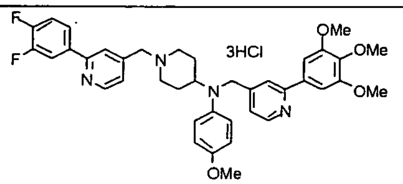 | 36% | 1.68-1.80 (m, 2H), 1.82-1.90 (m, 2H), 2.11-2.19 (m, 2H), 2.91-2.99 (m, 2H), 3.55 (s, 2H), 3.56-3.62 (m, 1H), 3.73 (s, 3H), 3.89 (s, 3H), 3.93 (s, 6H), 4.45 (s, 2H), 6.73 (d, 2H, J=9.3 Hz), 6.78 (d, 2H, J=9.3 Hz), 7.15 (s, 2H), 7.16-7.26 (m, 3H), 7.57 (s, 1H), 7.62 (s, 1H), 7.71 (br, 1H), 7.80-7.90 (m, 1H), 8.54 (d, 1H, J=5.1 Hz), 8.58 (d, 1H, J=4.9 Hz). |
| 195 | 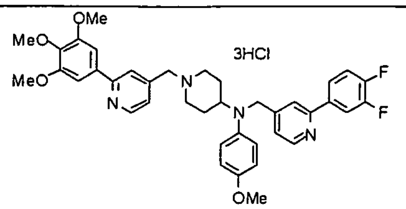 | 100% | 1.60-1.80 (m, 2H), 1.82-1.91 (m, 2H), 2.12-2.19 (m, 2H), 2.91-3.00 (m, 2H), 3.56 (s, 2H), 3.56-3.64 (m, 1H), 3.73 (s, 3H), 3.90 (s, 3H), 3.96 (s, 6H), 4.45 (s, 2H), 6.72 (d, 2H, J=9.0 Hz), 6.78 (d, 2H, J=9.0 Hz), 7.17-7.24 (m, 4H), 7.25-7.27 |

| | | | |
|---|---|---|---|
| | | | (m, 1H), 7.60 (s, 2H), 7.65 (br, 1H), 7.77-7.84 (m, 1H), 8.53-8.61 (m, 2H). |
| 196 | 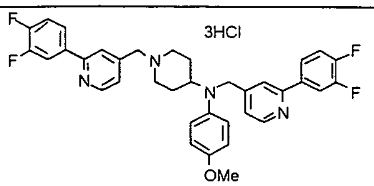 | 100% | 1.66-1.79 (m, 2H), 1.82-1.91 (m, 2H), 2.09-2.20 (m, 2H), 2.90-3.00 (m, 2H), 3.50-3.65 (m, 1H), 3.55 (s, 2H), 3.73 (s, 3H), 4.44 (s, 2H), 6.72 (d, 2H, J=9.3 Hz), 6.79 (d, 2H, J=9.3 Hz), 7.18-7.28 (m, 4H), 7.60 (s, 1H), 7.62 (s, 1H), 7.63-7.68 (m, 1H), 7.70-7.75 (m, 1H), 7.77-7.89 (m, 2H), 8.55 (d, 1H, J=4.9 Hz), 8.58 (d, 1H, J= 5.1 Hz). |
| 197 | 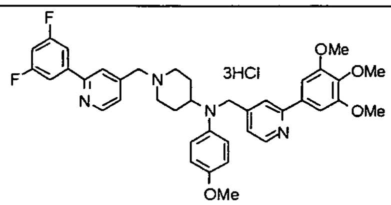 | 100% | 1.68-1.80 (m, 2H), 1.82-1.90 (m, 2H), 2.10-2.21 (m, 2H), 2.90-3.00 (m, 2H), 3.56 (s, 2H), 3.56-3.63 (m, 1H), 3.73 (s, 3H), 3.89 (s, 3H), 3.93 (s, 6H), 4.56 (s, 2H), 6.73 (d, 2H, J=9.3 Hz), 6.78 (d, 2H, J=9.3 Hz), 6.81-6.87 (m, 1H), 7.15 (s, 2H), 7.18 (d, 1H, J=4.2 Hz), 7.22-7.26 (m, 1H), 7.51-7.59 (m, 3H), 7.65 (s, 1H), 8.54 (d, 1H, J=4.9 Hz), 8.59 (d, 1H, J= 5.1 Hz). |
| 198 | 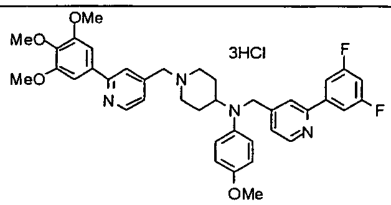 | 100% | 1.65-1.79 (m, 2H), 1.80-1.94 (m, 2H), 2.22-2.25 (m, 2H), 2.90-3.05 (m, 2H), 3.56 (s, 2H), 3.56-3.65 (m, 1H), 3.73 (s, 3H), 3.90 (s, 3H), 3.96 (s, 6H), 4.44 (s, 2H), 6.72 (d, 2H, J=9.2 Hz), 6.78 (d, 2H, J=9.2 Hz), 6.80-6.94 (m, 2H), 7.22 (s, 2H), 7.19-7.28 (m, 1H), 7.45-7.51 (m, 2H), 7.59 (s, 1H), 7.62 (s, 1H), 8.56 (d, 1H, J=4.9 Hz), 8.59 (d, 1H, J= 5.1 Hz). |
| 199 | 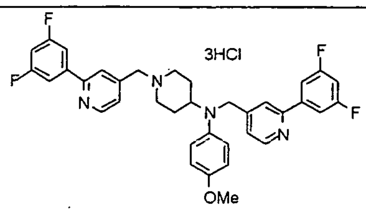 | 100% | 1.67-1.79 (m, 2H), 1.82-1.92 (m, 2H), 2.12-2.20 (m, 2H), 2.92-2.99 (m, 2H), 3.50-3.65 (m, 1H), 3.56 (s, 2H), 3.73 (s, 3H), 4.45 (s, 2H), 6.72 (d, 2H, J=9.0 Hz), 6.79 (d, 2H, J=9.3 Hz), 6.80-6.88 (m, 2H), 7.23-7.27 (m, 2H), 7.48 (dd, 2H, J=8.8 Hz, 2.2 Hz), 7.55 (dd, 2H, J=8.8 Hz, 2.2 Hz), 7.63 (s, 1H), 7.65 (s, 1H), 8.57 (d, 1H, J=4.9 Hz), 8.60 (d, 1H, J=4.9 Hz). |

| | | | |
|---|---|---|---|
| 200 | 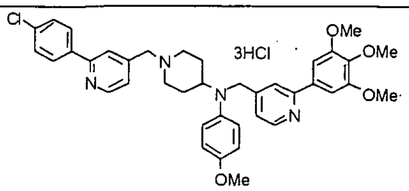 | 84% | 1.68-1.80 (m, 2H), 1.83-1.92 (m, 2H), 2.10-2.21(m, 2H), 2.91-3.00 (m, 2H), 3.56 (s, 2H), 3.57-3.62 (m, 1H), 3.73 (s, 3H), 3.89 (s, 3H), 3.93 (s, 6H), 4.45 (s, 2H), 6.73 (d, 2H, J=9.3 Hz), 6.78 (d, 2H, J=9.3 Hz), 7.15 (s, 2H), 7.17 (d, 1H, J=4.9 Hz), 7.20 (d, 1H, J=5.1 Hz), 7.43 (d, 2H, J=8.3 Hz), 7.57 (s, 1H), 7.65 (s, 1H), 7.93 (d, 2H, J=8.3 Hz), 8.54 (d, 1H, J=4.9 Hz), 8.59 (d, 1H, J=5.1 Hz). |
| 201 | 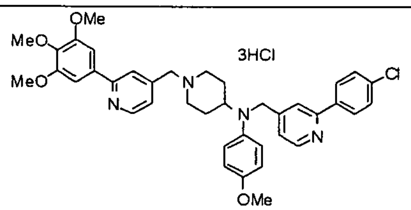 | 72% | 1.65-1.78 (m, 2H), 1.82-1.91 (m, 2H), 2.10-2.16 (m, 2H), 2.91-3.02 (m, 2H), 3.56 (s, 2H), 3.56-3.64 (m, 1H), 3.73 (s, 3H), 3.90 (s, 3H), 3.96 (s, 6H), 4.43 (s, 2H), 6.72 (d, 2H, J=9.3 Hz), 6.78 (d, 2H, J=9.3 Hz), 7.17-7.21 (m, 1H), 7.22 (2H, s), 7.41 (d, 2H, J=8.7 Hz), 7.48 (d, 1H, J=7.8 Hz), 7.59 (s, 1H), 7.63 (s, 1H) 7.87 (d, 2H, J=8.7 Hz), 8.56 (d, 1H, J=4.9 Hz), 8.58 (d, 1H, J=5.1 Hz). |
| 202 | 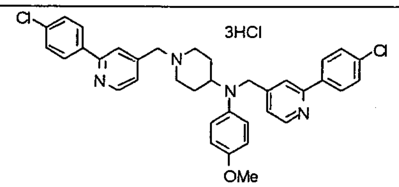 | 94% | 1.67-1.88 (m, 2H), 1.83-1.90 (m, 2H), 2.10-2.17 (m, 2H), 2.92-2.99 (m, 2H), 3.50-3.65 (m, 1H), 3.55 (s, 2H), 3.73 (s, 3H), 4.44 (s, 2H), 6.72 (d, 2H, J=9.0 Hz), 6.78 (d, 2H, J=9.3 Hz), 7.17-7.22 (m, 2H), 7.39-7.45 (m, 4H), 7.63 (s, 1H), 7.65 (s, 1H), 7.88 (d, 2H, J=8.6 Hz), 7.93 (d, 2H, J=8.5 Hz), 8.56 (d, 1H, J=4.9 Hz), 8.59 (d, 1H, J=4.9 Hz). |

Referential Example 226

Synthesis of 4-[N-[3-(3,4,5-trimethoxyphenyl)benzyl]-N-[4-(methylsulfonyl)phenyl]amino] piperidine:

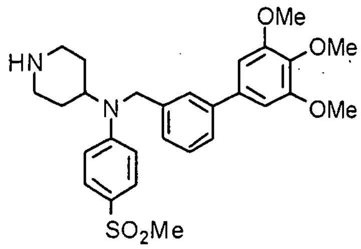

4-[N-[3-(3,4,5-Trimethoxyphenyl)benzyl]-N-[4-(methylthio)phenyl]amino]-piperidine hydrochloride (52mg, prepared in Referential Example 145) was dissolved in dichloromethane (1mL) and 3-chloroperbenzoic acid (69mg) was added to the resulting solution at 0°C. After the mixture was allowed to cool to room temperature and stirred for 3 hours, a saturated sodium hydrogen carbonate solution was added thereto. After separating an organic layer, an aqueous layer was further extracted with chloroform. Combined organic layers were washed with saturated brine, dried over anhydrous sodium sulfate, and concentrated under reduced pressure. The resulting pale yellow oil was used in the next step without purification.

Preparation Example 203

Synthesis of 4-[N-[3-(3,4,5-trimethoxyphenyl)benzyl]-N-[4-(methylsulfonyl)phenyl]amino]-1-[2-(3, 4,5-trimethoxyphenyl)pyridin-4-yl]methyl]piperidine dihydrochloride:

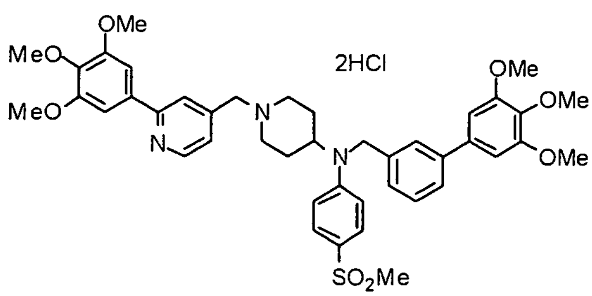

The crude 4-[N-[3-(3,4,5-trimethoxyphenyl)benzyl]-N-[4-(methylsulfonyl)phenyl]-amino]piperidine obtained in Referential Example 226 was reacted with 4-chloromethyl-2-(3,4,5-trimethoxyphenyl)pyridine (29mg) in the same manner as described in Preparation Example 2. By converting the resulting free base to the corresponding dihydrochloride, the title compound was obtained as a pale yellow powder
Yield: 23mg (26% in two steps)
$^1$H-NMR(400MHz, measured as free base,$CDCl_3$) δ:1.70-1.97(m,4H), 2.16-2.28(m,2H), 2.95-3.04(m,2H), 2.99(s,3H), 3.59(s,2H), 3.82(s,3H), 3.87-3.97(m,1H), 3.90(s,3H), 3.91(s,3H), 3.92(s,3H), 3.96(s,9H), 4.65(s,2H), 6.59(s,1H), 6.75 (d,2H,J=9.3Hz), 7.19-7.30(m,7H), 7.39(dd,1H,J=7.6Hz,7.6Hz), 7.60(s,1H), 7.68(d,2H,J=9.0Hz), 8.60(d,1H,J=4.9Hz)

Preparation Example 204

Synthesis of 4-[N-(4-methoxyphenyl)-N-[[3-(3,4, 5-trimethoxyphenyl)pyridin-5-yl] methyl] amino]-1-[[2-(3-methoxyphenyl)pyridin-4-yl]methyl]piperidine trihydrochloride:

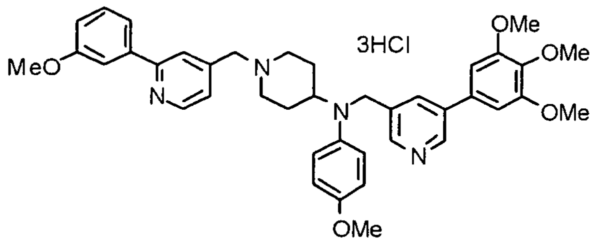

4-[N-(4-Methoxyphenyl)-N-[[3-(3,4,5-trimethoxyphenyl)pyridin-5-yl]methyl] amino]piperidine dihydrochloride (139mg, refer to Referential Example 98) was reacted with 4-chloromethyl-2-(3-methoxyphenyl)pyridine (70mg, refer to Referential Example 195) in the same manner as described in Preparation Example 2 to obtain the title compound as the trihydrochloride.
Yeld: 131mg (66%)
$^1$H-NMR (400MHz, measured as a free base, $CDCl_3$) δ:1.70-1.95(m,4H), 2.05-2.25(m,2H), 2.90-3.08(m,2H), 3.45-3.68 (m,3H), 3.72(s,3H), 3.88(s,3), 3.90(s,9H), 4.46(s,2H), 6.70-6.85(m,4H), 6.96(d,1H,J=8.3Hz), 7.21(br,1H), 7.38(t,1H, J=7.8Hz), 7.55(t,1H,J=7.8Hz), 7.59(s,1H), 7.63-7.75(m,2H), 8.50(s,1H), 8.62(br,1H)

Preparation Example 205

Synthesis of 4-[N-(4-methoxyphenyl)-N-[[3-(3,4,5-trimethoxyphenyl)pyridin-5-yl]amino]-1-[[2-(3,4 -dimethoxyphenyl) pyridin-4-yl]methyl]piperidine:

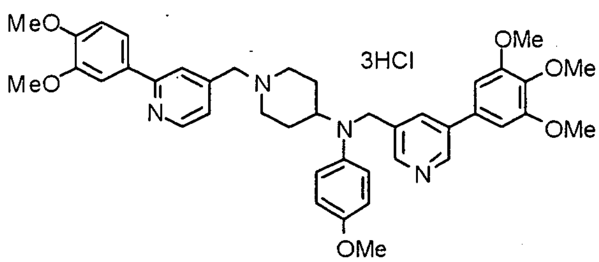

4-[N-(4-Methoxyphenyl)-N-[[3-(3,4,5-trimethoxyphenyl)pyridin-5-yl]methyl] amino]piperidine dihydrochloride (139mg, refer to Referential Example 98) was reacted with 4-chloromethyl-2-(3,4-dimethoxyphenyl)pyridine (80mg, refer to Referential Example 197) in the same manner as described in Preparation Example 2 to obtain the title compound as the trihydrochloride.
Yield: 139mg (67%)
$^1$H-NMR(400MHz, measured as a free base, $CDCl_3$) δ:1.70-1.95 (m,4H),2.05-2.20(m,2H), 2.90-3.05 (m,2H), 3.45-3.60 (m,3H),3.73(s,3H), 3.88(s,3H), 3.89 (s,6H), 3.94 (s,3H), 4.00 (s,3H), 4.46 (s,2H), 6.65 (s,2H), 6.74-6.82 (m,4H), 6.94 (d,1H,J=8.3Hz), 7.15 (br,1H), 7.52 (br,1H), 7.58-7.71 (m,3H), 8.50 (s,1H), 8.57 (d,1H,J=5.2Hz), 8.62 (br,1H)

Preparation Example 206

Synthesis of 4-[N-(4-fluorophenyl)-N-[[3-(3,4,5-trimethoxyphenyl)pyridin-5-yl]methyl]amino]-1-[[2 -(3-methoxyphenyl) pyridin-4-yl]methyl]piperidine trihydrochloride:

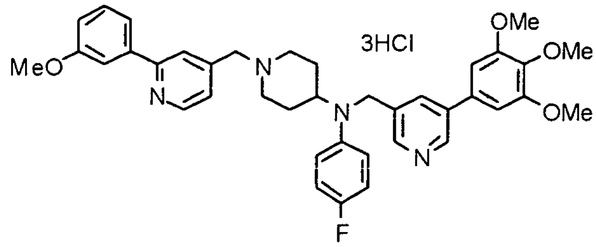

4-[N-(4-Fluorophenyl)-N-[[3-(3,4,5-trimethoxyphenyl)pyridin-5-yl]methyl] amino]piperidine dihydrochloride (135mg, refer to Referential Example 183) was reacted with 4-chloromethyl-2-(3-methoxyphenyl)pyridine (70mg, refer to Referential Example 195) in the same manner as described in Preparation Example 2 to obtain the title compound as the trihydrochloride.
Yield: 178mg (92%)
$^1$H-NMR(400MHz, measured as a free base, $CDCl_3$) δ:1.73-1.95(m,4H), 2.10-2.25(m,2H), 2.93-3.05(m,2H), 3.57(s, 2H), 3.64(br,1H),3.88(s,3H), 3.89(s,9H), 4.51(s,2H), 6.66(s,2H), 6.70-6.76(m,2H), 6.90(t,2H,J=8.3Hz), 6.96(d,1H, J=8.3Hz), 7.21(br,1H), 7.38(t,1H,J=8.0Hz), 7.54(d,1H,J=7.8Hz), 7.58(s,1H), 7.65(s,1H), 7.74(br,1H), 8.50(s,1H), 8.61 (d,1H,J=5.1Hz), 8.65(br,1H)

Preparation Example 207

Synthesis of 4-[N-(4-fluorophenyl)-N-[[3-(3,4,5-trimethoxyphenyl)pyridin-5-yl]methyl]amino]-1-[[2 -(3,4-dimethoxyphenyl)pyridin-4-yl]methyl]piperidine trihydrochloride:

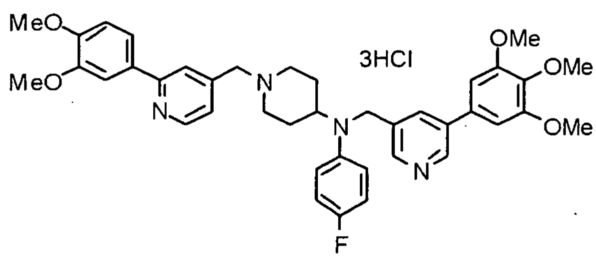

4-[N-(4-Fluorophenyl)-N-[[3-(3,4,5-trimethoxyphenyl)pyridin-5-yl]methyl] amino]piperidine dihydrochloride (135mg, refer to Referential Example 183) was reacted with 4-chloromethyl-2-(3,4-dimethoxyphenyl)pyridine (80mg, refer to Referential Example 197) in the same manner as described in Preparation Example 2 to obtain the title compound as the trihydrochloride.
Yield:195mg (96%)
$^1$H-NMR(400MHz, measured as a free base, $DCl_3$) δ:1.70-1.95(m,4H), 2.10-2.24(m,2H), 2.94-3.09(m,2H), 3.57(s,2H), 3.64(br,1H), 3.88(s,3H), 3.89(s,6H), 3.94(s,3H), 4.00(s,3H), 4.51(s,2H), 6.65(s,2H), 6.69-6.78(m,2H), 6.86-6.97(m, 3H), 7.16(d,1H,J=4.9Hz), 7.51(d,1H,J=8.5Hz), 7.60-7.70(m,3H), 8.50(s,1H), 8.58(d,1H,J=4.9Hz), 8.65(s,1H)

Preparation Example 208

Synthesis of 4-[N-(3,4-difluorophenyl)-N-[[3-(3,4,5-trimethoxyphenyl)pyridin-5-yl]methyl]amino]-1 -[[2-(3-methoxyphenyl)pyridin-4-yl]methyl]piperidine trihydrochloride:

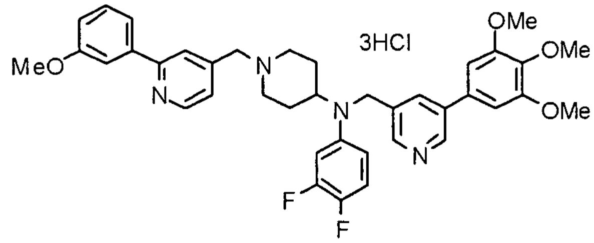

4-[N-(3,4-Difluorophenyl)-N-[[3-(3,4, 5-trimethoxyphenyl)pyridin-5-yl]methyl] amino]piperidine dihydrochloride (160mg, refer to Referential Example 176) was reacted with 4-chloromethyl-2-(3-methoxyphenyl)pyridine (80mg, refer to Referential Example 195) in the same manner as described in Preparation Example 2 to obtain the title compound as the trihydrochloride.
Yield:130mg (57%)
$^1$H-NMR(400MHz, measured as a free base, $CDCl_3$) δ:1.73-1.90(m,4H), 2.01-2.24(m,2H), 2.92-3.05(m,2H), 3.57(s, 2H), 3.67(br,1H), 3.88(s,3H), 3.89(s,3H), 3.90(s,6H), 4.52(s,2H), 6.36-6.42(m,1H), 6.50-6.58(m,1H), 6.67(s,2H), 6.93-7.01(m,2H), 7.20(br,1H), 7.38(t,1H,J=7.8Hz), 7.52-7.62(m,2H), 7.62-7.72(m,2H), 8.48(br,1H), 8.61(br,1H), 8.66 (d,1H,J=2.0Hz)

Preparation Example 209

Synthesis of 4-[N-(4-methylthiophenyl)-N-[[3-(3,4,5-trimethoxyphenyl)pyridin-5-yl]methyl]amino]-1-[[2-(3-methoxyphenyl)pyridin-4-yl]methyl]piperidine:

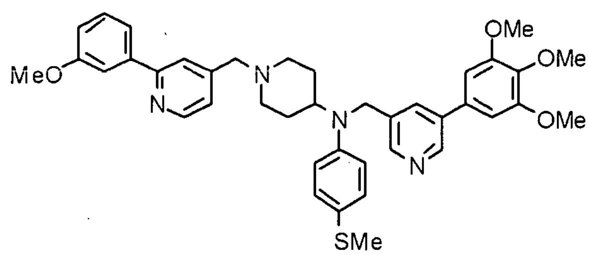

4-[N-(4-Methylthiophenyl)-N-[[3-(3,4,5-trimethoxyphenyl)pyridin-5-yl]methyl] amino]piperidine dihydrochloride (121mg, refer to Referential Example 143) was reacted with 4-chloromethyl-2-(3-methoxyphenyl)pyridine (55mg, refer to Referential Example 195) in the same manner as described in Preparation Example 2 to obtain the title compound.
Yield: 71mg (44%)
$^1$H-NMR(400MHz, measured as a free base, $CDCl_3$) δ:1.72-1.83(m,4H), 2.12-2.20(m,2H), 2.37(s,3H), 2.97(d,2H, J=10.8Hz), 3.56(s,2H), 3.75-3.81(m,1H), 3.86(s,3H), 3.87(s,6H), 4.54(s,2H), 6.64-6.69(m,3H), 6.94(dd,1H,J=7.8Hz, 1.9Hz), 7.17-7.26(m,4H), 7.35(t,1H,J=7.8Hz), 7.51-7.66(m,4H), 8.47(s,1H), 8.59(d,1H,J=4.6Hz), 8.63(s,1H)

Test example 1

A. Materials and methods

Hep3B cells producing EPO under hypoxic condition were used. The cells were inoculated onto a 100 mm

petri dish by 1.5 - 3.0 x $10^6$. As a culture medium, 10 ml of Dulbecco's Modified Eagles' Medium (DMEM)/10 % Fetal Calf Serum (FCS) was used. The next day, the culture medium was exchanged, and compound of Preparation Example 13 (final concentration: 100 nM) was added, then cells were cultured under 1 % $O_2$ at 37 °C for 24 hrs. Groups were prepared in which IL-1β (final concentration: 15 U/ml) or TNF-α (final concentration: 220 U/ml), of which EPO production suppressing action is known (Faquin WC, Schneider TJ, Goldgerg MA, Blood, 79(8):1987-94(1992)), were used solely or in combination with compound of Preparation Example 13 (final concentration: 100 nM). As a control, a group that does not contain any agents was prepared. The amount of EPO in the supernatant obtained after the culture was measured by ELISA (each group; n = 3). The enhancer site and promoter site (144 bp) of a human EPO gene were linked to luciferase reporter vector, which was used for measuring the EPO promoter activity. The EPO promoter activity was evaluated as follows.

1x $10^6$ Hep3B cells were inoculated on 30 mm petri dish (4 mL:DMEM/10 % FCS), and a reporter gene (2 μg) as well as β-galactosidase gene (1 μg) as an internal standard were transfected into the cells by the lipofectin method. The next day, the culture medium was exchanged and the groups, whose group-composition was similar to the above (control group (containing no agents), IL-1β group (final concentration: 15 U/ml), TNF-α (final concentartion: 220 U/ml), compound of Preparation Example 13 (final concentration: 100 nM), or a combination of compound of Preparation Example 13 and IL-1β or TNF-α) were cultured under 1 % $O_2$ or 21 % $O_2$ (as a control) at 37 °C for 24 hrs (for each group; n=4). The extract of the cells after cultured were prepared, then luciferase activity and β galactosidase activity were measured. The EPO promoter activity was expressed by the formula:

$$\text{HI (Hypoxic Induction)} = \frac{1\ \%\ O_2\ \text{luciferase}/\beta\ \text{galactosidase}}{21\ \%\ O_2\ \text{luciferase}/\beta\ \text{galactosidase}}$$

The data obtained were analyzed and evaluated by Student's t-test.

B. Results

Fig. 1 shows the effect of addition of agents on the amount of the EPO protein produced by Hep3B cells under hypoxia (1 % $O_2$). While the amount of the produced EPO protein of control was 424.3±43.0 mU/mg protein, that of the group containing compound of Preparation Example 13 (final concentration: 100 nM) was increased to 829.8±119.0 mU/mg protein, which is twice as much as the control. When IL-1β (15 U/ml) or TNF-α (220 U/ml) was added, the produced amount of EPO protein were reduced to 233.3±19.4 mU/mg protein, 180.8±24.6 mU/mg protein, respectively. But addition of compound of Preparation Example 13 (final concentration: 100 nM) in combination made it rescued to 395.0 ± 72.6 mU/mg protein and 284.8 ± 24.2 mU/mg protein, respectively.

Fig. 2 shows human EPO promoter activity. While the HI of the control was 33.7 ± 4.8 fold, it was significantly increased to 46.1 ± 5.9 fold by the addition of 100 nM of the Preparation Example 13 compound to the control. Although the addition of IL-1β (15 U/ml) or TNF-α (220 U/ml) suppressed the HI to 19.9 ± 6.5, 20.7 ± 4.1 fold, respectively, the simultaneous use of the Preparation Example 13 compound (100 nM) changes it to 45.5 ± 7.1 fold, 49.5 ± 5.4 fold, respectively, meaning that the addition produces not only release of the suppress, but also a significant increase thereof.

These results reveal that the Preparation Example 13 compound can increase EPO production from Hep3B cells under hypoxic condition, and that this increase is caused through transcription process of the gene.

Test example 2

A. Materials and methods

Hep3B cells were inoculated by 1 x $10^6$ on each of 6 cm dishes, and then cultured for 24 hrs. As a culture medium, 3 mL of DMEM/10 % FCS was used. The culture medium was exchanged and the compounds of each of Preparation Examples 13, 23, 29, 36, and 114 were added such that the content thereof was 100 nM final concentration, and they were transferred into the atmosphere of 1 % oxygen at 37 °C for further 24 hrs culture thereof. The supernatant of the culture was recovered, and the amount of EPO was measured by ELISA kit (product of Roche Diagnostics) (two cases in each group). Further, cell extract was prepared and protein amount was determined, and corrected it as production amount per protein amount. As a control, groups not containing the compounds are prepared.

B. Results

Table 1 shows the effect of the compounds on the amount of the EPO produced in the Hep3B cell culture medium under 1 % oxygen. The addition of each compound (100 nM) gave 1.3-2.3 fold production of EPO compared

to the control.

Table 1 -

| Increasing action of EPO production of each compound | | | | | | |
|---|---|---|---|---|---|---|
| Preparation Example No. | Control | 13 | 23 | 29 | 36 | 114 |
| mU/mgProtein | 8.1 | 18.2 | 13.9 | 10.5 | 10.0 | 14.9 |

Test example 3 (*in vivo* mice anemia model)

A. Materials and methods

ICR mice (available from CLEA Japan Inc.) were made to be anemia by taking blood samples by 0.3 mL at a time from the orbital vein of the mouse by use of a heparin-coated microcapillary by 4 times in every 12 hours from 2 days before administering the agents. Further, 1.67 x $10^4$ unit/day of mouse IL-1β (Roche Diagnostics) or 3.33 x $10^5$ unit/day of mouse TNF-α (Roche Diagnostics) was injected intraperitoneally on day 0-3 (the first day of administration of agents was defined as day 0) to induce a suppressed condition of EPO production. To these mice, Preparation Example 13 compound dissolved in 50 % polyethylene glycol solution was administered orally by 1 or 3 mg/kg/day on day 0-5. As a control, 50 % polyethylene glycol solution was administered (each group; n=7-15). On day 3 and 6, 0.3 mL of blood was collected, and hematocrit (Ht) value, hemoglobin (Hb) concentration (Celltac α: NIHON KOHDEN), EPO amount (ELISA kit: Roche Diagnostics) were measured. Also, the blood was stained by methylene-blue, and smears were prepared to calculate Reticulocytes value.

B. Results

Fig. 3 shows the effect of the compounds on Ht value of anemia model mice. In the Figure, a column indicates [average value + standard deviation]. While Ht of control group on day 3 was 42.2 ± 3.6 %, it was significantly reduced, by IL-1β administration or TNF-α administration, to 36.0 ± 3.0 %, 38.3 ± 2.5 %, respectively.
In contrast thereto, the oral administration of Preparation Example 13 compound by 1 or 3 mg/kg/day improved to 37.5 ±2.5%, 37.5 ± 3.1 % (IL-1β) and 38.8 ±2.0%, 40.0 ± 2.7 % (TNF-α), respectively. Similarly on day 6, while Ht value of the control group was 46.4 ± 2.9 %, the administration of IL-1β or TNF-α provided significant reduction of 40.9± 1.5 %, 41.6± 1.8 %, respectively. In contrast, the oral administration of Preparation Example 13 compound by 1 or 3 mg/kg/day improved to of 42.6 ± 2.5 %, 43.3± 2.8 % (IL-1β), respectively, and 43.3± 2.5 %, 43.2± 2.2 % (TNF-α), respectively. In particular, it is observed that in the IL-1β administration group, the Ht value by the administration of Preparation Example 13 compound was significantly improved both in 1 and in 3 mg/kg/day.
Fig. 4 shows the effect of the compound on Hb amount. In the Figure, a column indicates [average value + standard deviation]. While Hb of control group on day 3 was 13.8 ± 0.6 g/dL, it was significantly reduced, by IL-1β administration or TNF-α administration, to 12.3 ± 0.8 g/dL, 12.6 ± 0.7 g/dL, respectively. In contrast thereto, the oral administration of Preparation Example 13 compound by 1 or 3 mg/kg/day improved to 12.7 ± 0.7 g/dL, 12.6 ± 1.0 g/dL (IL-1β), and 12.8 ± 0.6 g/dL, 13.1 ± 0.8g/dL (TNT-α), respectively. Similarly, on day 6, while Hb of control group was 14.8 ± 0.8 g/dL, it was significantly reduced, by IL-1β administration or TNF-α administration, to 13.5 ± 0.5 g/dL, 13.1 ± 0.5 g/dL, respectively. In contrast thereto, the oral administration of Preparation Example 13 compound by 1 or 3 mg/kg/day significantly improved to 14.1 ± 1.1 g/dL, 14.0 ± 0.9 g/dL (IL-1β), and 14.0 ± 0.6 g/dL, 13.8 ± 0.5 g/dL (TNF-α), respectively.
Fig. 5 shows the number of reticulocytes. In this figure, the value of the column indicates [average value + standard deviation]. While the reticulocyte value of control group on day 3 was 32.1 ± 4.4 %, the administration of IL-1β or TNF-α reduced significantly the value to 15.4 ± 3.5 %, 14.2 ± 3.4 %, respectively. By contrast, the administration of Preparation Example 13 compound by 1 or 3 mg/kg/day significantly increased the value to 36.1 ± 4.5 %, 38.1 ± 8.1 % (IL-1β), and 29.1 ± 4.7%, 28.9 ± 4.5 % (TNF-α), respectively. Similarly, on day 6, while reticulocyte value of control group was 16.3 ± 3.1 %, it was significantly reduced, by IL-1β administration or TNF-α administration, to 12.2 ± 2.0 %, 9.4 ± 3.3 %, respectively. In contrast thereto, the oral administration of Preparation Example 13 compound by 1 or 3 mg/kg/day significantly improved to 27.1 ± 2.5 %, 30.4 ± 4.0 % (IL-1β), and 23.3 ± 3.3 %, 26.8 ± 7.5 % (TNF-α), respectively.
Fig. 6 shows the effect of the compound on EPO amount in serum. In this figure, the value of the column indicates [average value + standard deviation]. While the EPO amount of control group on day 3 was 23.7 ± 31.0 mU/mL, the administration of IL-1β or TNF-α reduced significantly the EPO amount to 5.9 ± 14.3 mU/mL, 2.7 ± 4.6 mU/mL. By contrast, the administration of Preparation Example 13 compound by 1 or 3 mg/kg/day significantly increased

the value to 41.8 ± 28.3 mU/mL, 58.9 ± 21.1 mU/mL (IL-1β), and 61.4 ± 70.4 mU/mL, 64.7 ± 92.2 mU/mL (TNF-α), respectively.
Especially, with regard to the group of IL-1β administration, the improvement of EPO by administration of Preparation Example 13 compound was significant at 1 and 3 mg/kg/day. Similarly, on day 6, while EPO amount of control group was 24.8 ± 33.8 mU/mL, it was significantly reduced, by IL-1β administration or TNF-α administration, to 2.9 ± 5.0 mU/mL, 3.3 ± 6.0 mU/mL, respectively. In contrast thereto, the oral administration of Preparation Example 13 compound by 1 or 3 mg/kg/day significantly improved to 22.2 ± 32.8 mU/mL, 37.1 ± 31.1 mU/mL (IL-1β), and 57.4 ± 23.9 mU/mL, 69.9 ± 37.8 mU/mL (TNF-α), respectively. Especially, with regard to the group of TNF-α administration, the improvement of EPO by administration of Preparation Example 13 compound was significant at 1 and 3 mg/kg/day.

The above results has indicated that the Preparation Example 13 compound enhances, in mice model of anemia of chronic disease, EPO production, promotes differentiation to erythrocytes, thereby improving the condition of anemia.

## Claims

1. A preventive or therapeutic agent for pathological conditions caused by reduced production of erythropoietin, comprising as an active ingredient, a cyclic amine compound represented by the following formula (1):

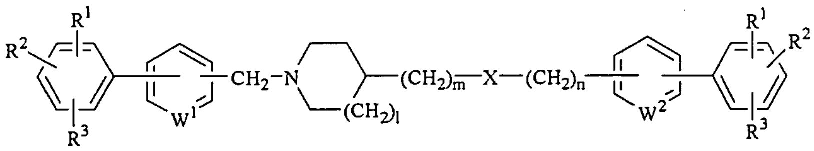

(1)

wherein,
$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;
$W^1$ and $W^2$ each independently represent N or CH;
X represents O, $NR^4$, $CONR^4$ or $NR^4CO$;
$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and
l, m and n each represents a number of 0 or l, or a salt thereof or a solvate thereof.

2. The preventive or therapeutic agent according to claim 1, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

3. The preventive or therapeutic agent according to claim 1, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5- or 6-membered ring containing 1-4 nitrogen atoms.

4. The preventive or therapeutic agent according to claim 3, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

5. The preventive or therapeutic agent according to claim 1, wherein the active ingredient is
4-[N-(4-methoxyphenyl)-N-[[5-(3,4,5-trimethoxyphenyl)pyridine-3-yl]methyl]amino]-1-[[2-(3,4,5-trimethoxyphenyl)pyridine-4-yl]methyl]piperidine;
4-[N-(3,5-dimethoxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;

4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-methyl-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

**6.** A preventive or therapeutic agent for anemia, comprising as an active ingredient, a cyclic amine compound represented by the following formula (1):

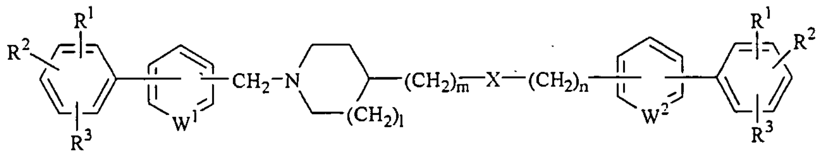

(1)

wherein,
$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;
$W^1$ and $W^2$ each independently represent N or CH;
X represents O, $NR^4$, $CONR^4$ or $NR^4CO$;
$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and
l, m and n each represents a number of 0 or 1, or a salt thereof or a solvate thereof.

**7.** The preventive or therapeutic agent according to claim 6, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

**8.** The preventive or therapeutic agent according to claim 6, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5- or 6-membered ring containing 1-4 nitrogen atoms.

**9.** The preventive or therapeutic agent according to claim 8, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

**10.** The preventive or therapeutic agent according to claim 6, wherein the active ingredient is
4-[N-(4-methoxyphenyl)-N-[[5-(3,4,5-trimethoxyphenyl)pyridine-3-yl]methyl]amino]-1-[[2-(3,4,5-trimethoxyphenyl)pyridine-4-yl]methyl]piperidine;
4-[N-(3,5-dimethoxyphenyl)-N-[[2-(3,4,5-trimethoxyphenyl)piridine-4-yl]methyl] amino]-l-[[2-(3,4,5-trimethoxyphenyl)piridine-4-yl]methyl]piperidine;
4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-methyl-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

**11.** A preventive or therapeutic agent for chronic anemia, renal anemia, anaplastic anemia or pure red cell aplasia,

comprising as an active ingredient, a cyclic amine compound represented by the following formula (1):

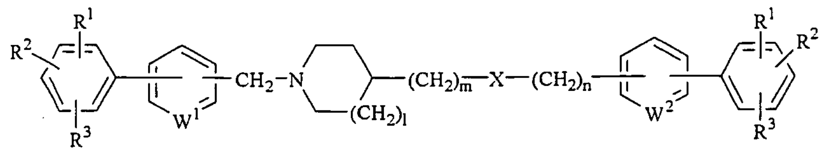

(1)

wherein,

$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;

$W^1$ and $W^2$ each independently represent N or CH;

X represents O, $NR^4$, $CONR^4$ or $NR^4CO$;

$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and

l, m and n each represents a number of 0 or 1, or a salt thereof or a solvate thereof.

**12.** The preventive or therapeutic agent according to claim 11, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

**13.** The preventive or therapeutic agent according to claim 11, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5- or 6-membered ring containing 1-4 nitrogen atoms.

**14.** The preventive or therapeutic agent according to claim 13, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

**15.** The preventive or therapeutic agent according to claim 11, wherein the active ingredient is
4-[N-(4-methoxyphenyl)-N-[[5-(3,4,5-trimethoxyphenyl)pyridine-3-yl]methyl]amino]-1-[[2-(3,4,5-trimethoxyphenyl)pyridine-4-yl]methyl]piperidine;
4-[N-(3,5-dimethoxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-methyl-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

**16.** Use of a cyclic amine compound represented by the following formula (1):

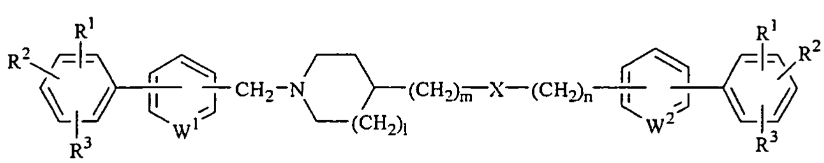

(1)

wherein,

$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;

$W^1$ and $W^2$ each independently represent N or CH;

X represents O, $NR^4$, $CONR^4$ or $NR^4CO$;

$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and

l, m and n each represents a number of 0 or 1, or a salt thereof or a solvate thereof for the manufacture of a preventive or therapeutic agent for pathological conditions caused by reduced production of erythropoietin.

**17.** The use according to claim 16, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

**18.** The use according to claim 16, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5-or 6-membered ring containing 1-4 nitrogen atoms.

**19.** The use according to claim 18, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

**20.** The use according to claim 16, wherein the active ingredient is

4-[N-(4-methoxyphenyl)-N-[[5-(3,4,5'-trimethoxyphenyl)pyridine-3-yl]methyl]amino] -1-[[2-(3,4,5-trimethoxyphenyl)pyridine-4-yl]methyl]piperidine;

4-[N-(3,5-dimethoxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;

4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;

4-[N-methyl-N-[[2-(3,4,5-trimethoxyphenyl)piridine-4-yl]methyl]amino]-1-[[2-(3,4,5-trimethoxyphenyl)piridine-4-yl]methyl]piperidine;

4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

**21.** Use of a cyclic amine compound represented by the following formula (1):

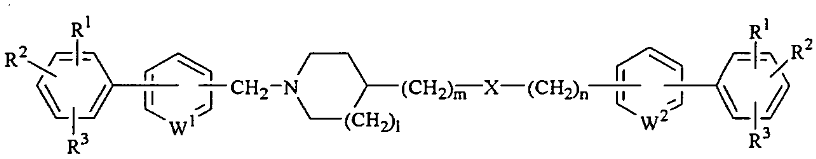

(1)

wherein,

$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;

$W^1$ and $W^2$ each independently represent N or CH;

X represents O, $NR^4$, $CONR^4$ or $NR^4CO$;

$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and

l, m and n each represents a number of 0 or 1, or a salt thereof or a solvate thereof for the manufacture of

a preventive or therapeutic agent for anemia.

**22.** The use according to claim 21, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

**23.** The use according to claim 21, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5- or 6-membered ring containing 1-4 nitrogen atoms.

**24.** The use according to claim 23, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

**25.** The use according to claim 21, wherein the active ingredient is
4-[N-(4-methoxyphenyl)-N-[[5-(3,4,5-trimethoxyphenyl)pyridine-3-yl]methyl]amino]-1-[[2-(3,4,5-trimethoxyphenyl)pyridine-4-yl]methyl]piperidine;
4-[N-(3,5-dimethoxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-methyl-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

**26.** Use of a cyclic amine compound represented by the following formula (1):

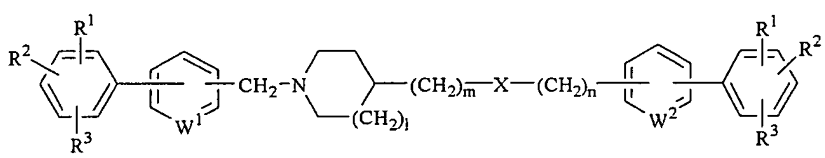

(1)

wherein,
$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;
$W^1$ and $W^2$ each independently represent N or CH;
X represents O, $NR^4$, $CONR^4$ or $NR^4CO$;
$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and
l, m and n each represents a number of 0 or 1, or a salt thereof or a solvate thereof for the manufacture of a preventive or therapeutic agent for chronic anemia, renal anemia, aplastic anemia, or pure red cell aplasia.

**27.** The use according to claim 26, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

**28.** The use according to claim 26, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl

group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5- or 6-membered ring containing 1-4 nitrogen atoms.

**29.** The use according to claim 28, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

**30.** The use according to claim 21, wherein the active ingredient is
4-[N-(4-methoxyphenyl)-N-[[5-(3,4,5-trimethoxyphenyl)pyridine-3-yl]methyl]amino]-1-[[2-(3,4,5-trimethoxyphenyl)pyridine-4-yl]methyl]piperidine;
4-[N-(3,5-dimethoxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-methyl-N-[[2-(3,4,5-trimethoxyphenyl)piridine-4-yl]methyl]amino]-1-[[2-(3,4,5 trimethoxyphenyl)piridine-4-yl]methyl]piperidine;
4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

**31.** A method of treating pathological conditions caused by reduced production of erythropoietin, comprising administering an effective amount of a cyclic amine compound represented by the following formula (1):

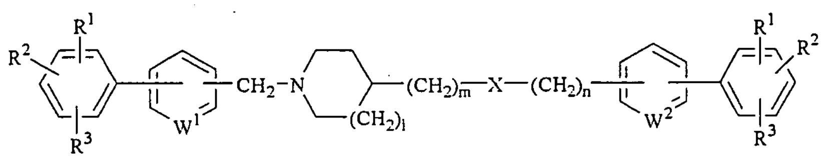

(1)

wherein,

$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;

$W^1$ and $W^2$ each independently represent N or CH;

X represents O, $NR^4$, $CONR^4$ or $NR^4CO$;

$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and

l, m and n each represents a number of 0 or 1, or a salt thereof or a solvate thereof.

**32.** The method according to claim 31, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

**33.** The method according to claim 31, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5- or 6-membered ring containing 1-4 nitrogen atoms.

**34.** The method according to claim 33, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

**35.** The method according to claim 31, wherein the active ingredient is 4-[N-(4-methoxyphenyl)-N-[[5-(3,4,5-trimethoxyphenyl)pyridine-3-yl]methyl]amino]-1-[[2-(3,4,5-trimethoxyphenyl)pyridine-4-yl]methyl]piperidine; 4-[N-(3,5-dimethoxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine; 4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine; 4-[N-methyl-N-[[2-(3,4,5-trimethoxyphenyl)piridine-4-yl]methyl]amino]-1-[[2-(3,4,5-trimethoxyphenyl)piridine-4-yl]methyl]piperidine; 4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

**36.** A method of treating anemia, comprising administering an effective amount of a cyclic amine compound represented by the following formula (1):

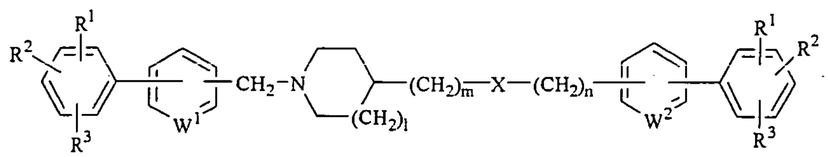

(1)

wherein,

$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;

$W^1$ and $W^2$ each independently represent N or CH;

X represents O, $NR^4$, $CONR^4$ or $NR^4CO$;

$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and

l, m and n each represents a number of 0 or 1, or a salt thereof or a solvate thereof.

**37.** The method according to claim 36, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

**38.** The method according to claim 36, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5- or 6-membered ring containing 1-4 nitrogen atoms.

**39.** The method according to claim 38, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

**40.** The method according to claim 36, wherein the active ingredient is 4-[N-(4-methoxyphenyl)-N-[[5-(3,4,5-trimethoxyphenyl)pyridine-3-yl]methyl]amino]-1-[[2-(3,4,5-trimethoxyphenyl)pyridine-4-yl]methyl]piperidine; 4-[N-(3,5-dimethoxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine; 4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine; 4-[N-methyl-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

**41.** A method of treating chronic anemia, renal anemia, aplastic anemia, or pure red cell aplasia, comprising administering an effective amount of a cyclic amine compound represented by the following formula (1):

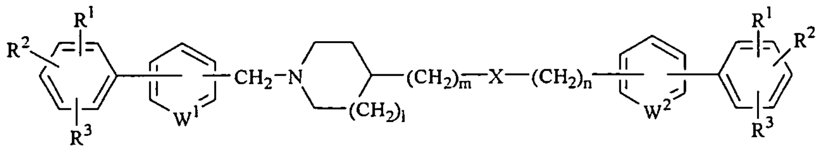

(1)

wherein,

$R^1$, $R^2$ and $R^3$ each independently represent a hydrogen atom, a halogen atom, or hydroxy, alkyl, halogen-substituted alkyl, alkoxy, alkylthio, carboxyl, alkoxycarbonyl or alkanoyl group;

$W^1$ and $W^2$ each independently represent N or CH;

X represents O, $NR^4$, $CONR^4$ or $NR^4CO$;

$R^4$ each represents a hydrogen atom, or an alkyl, alkenyl, alkynyl, substituted or unsubstituted aryl, substituted or unsubstituted heteroaryl, substituted or unsubstituted aralkyl, or substituted or unsubstituted heteroaralkyl group; and

l, m and n each represents a number of 0 or 1, or a salt thereof or a solvate thereof.

**42.** The method according to claim 41, wherein $R^1$, $R^2$ and $R^3$ are each a hydrogen atom, a halogen atom, a hydroxy group, a $C_1$-$C_8$-alkyl group, a halogen-substituted $C_1$-$C_8$-alkyl, an alkoxy group having a $C_1$-$C_8$-alkyl group, an alkylthio group having a $C_1$-$C_8$-alkyl group, a carboxyl group, an alkoxycarbonyl group having a $C_1$-$C_6$-alkyl group, or an alkanoyl group having a $C_1$-$C_6$-alkyl group.

**43.** The method according to claim 41, wherein $R^4$ each represents a hydrogen atom, a $C_1$-$C_8$-alkyl group, $C_3$-$C_8$-alkenyl group, $C_3$-$C_8$-alkynyl group, substituted or unsubstituted $C_6$-$C_{14}$-aryl group, substituted or unsubstituted heteroaryl group having 5- or 6-membered ring containing 1-4 nitrogen atoms, substituted or unsubstituted $C_6$-$C_{14}$-aryl-$C_1$-$C_6$-alkyl group, or $C_1$-$C_6$-alkyl group having heteroaryl group having 5- or 6-membered ring containing 1-4 nitrogen atoms.

**44.** The method according to claim 43, wherein in $R^4$, the substituent of an aryl group, an aryl group of aralkyl group, heteroaryl group, or heteroaryl group of heteroaralkyl group is 1-3 groups selected from the group consisting of alkyl group, alkoxy group, alkylthio group, a halogen atom, a nitro group, an amino group, an acetylamino group, trifluoromethyl group and alkylenedioxy group.

**45.** The method according to claim 41, wherein the active ingredient is
4-[N-(4-methoxyphenyl)-N-[[5-(3,4,5-trimethoxyphenyl)pyridine-3-yl]methyl]amino]-1-[[2-(3,4,5-trimethoxyphenyl)pyridine-4-yl]methyl]piperidine;
4-[N-(3,5-dimethoxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(3,4-methylenedioxyphenyl)-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-methyl-N-[[2-(3,4,5-trimethoxyphenyl)piridine-4-yl]methyl]amino]-1-[[2-(3,4,5-trimethoxyphenyl)piridine-4-yl]methyl]piperidine;
4-[N-(4-(methylthio)phenyl)-N-[[5-(3,4,5-tromethoxyphenyl)piridine-3-yl]methyl] amino]-1-[[2-(3,4,5-tromethoxyphenyl)piridine-4-yl]methyl]piperidine; or a salt thereof.

Fig. 1

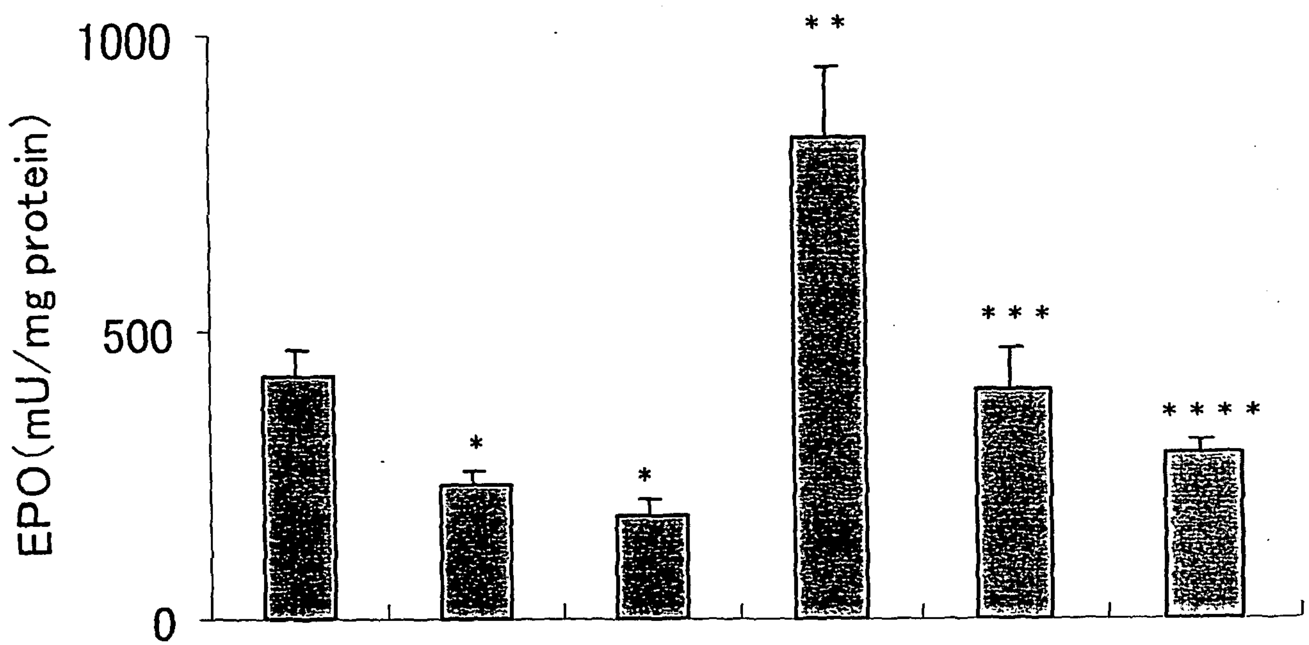


| | | | | | | |
|---|---|---|---|---|---|---|
| Preparation Example 13 Compound (100 nM) | − | − | − | + | + | + |
| IL-1β (15 U/ml) | − | + | − | − | + | − |
| TNF-α (220 U/ml) | − | − | + | − | − | + |

* ; $p<0.005$、Against control group

* * ; $p<0.01$、Against control group

* * * ; $p<0.025$、Against IL-1β (15 U/ml) group

* * * * ; $p<0.01$、Against TNF-α (220 U/ml) group

Fig. 2

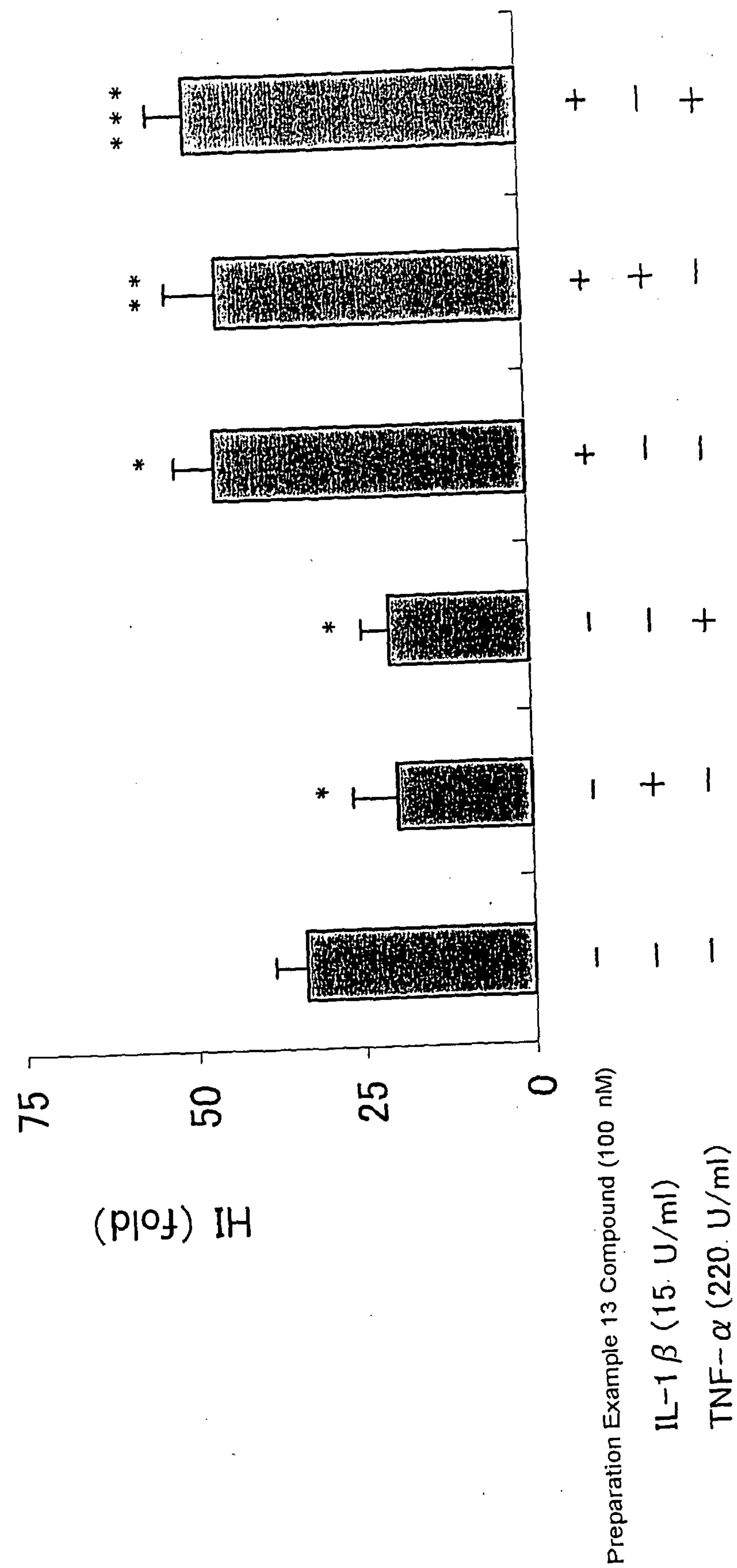


* ; p<0.005, Against control group

* * ; p<0.005, Against IL-1β (15 U/ml) group

* * * ; p<0.005, Against TNF-α (220 U/ml) group

Fig. 3

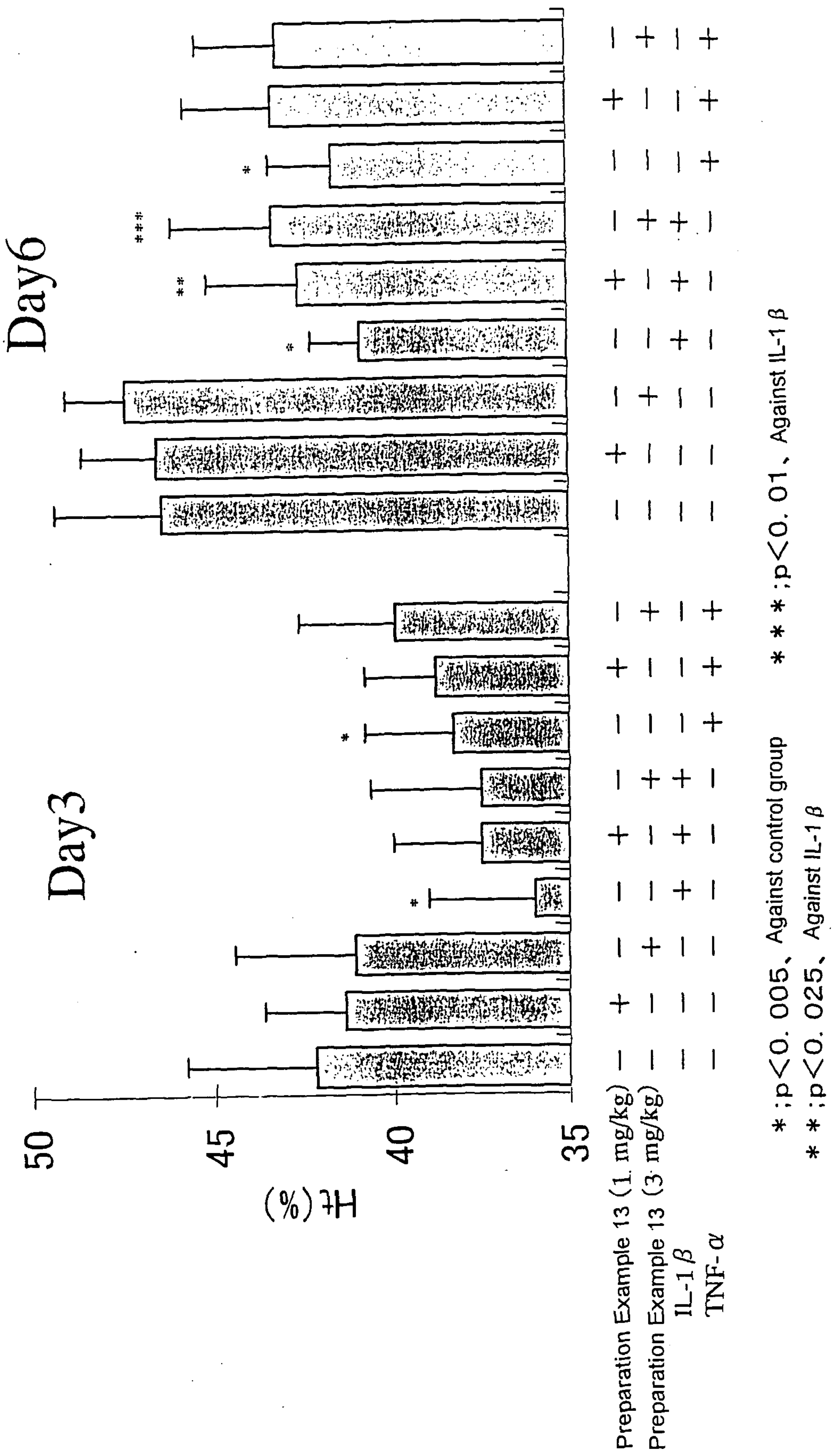
Day3
Day6
Ht(%)
50
45
40
35
Preparation Example 13 (1 mg/kg)
Preparation Example 13 (3 mg/kg)
IL-1β
TNF-α
* ;p<0. 005、Against control group
* * ;p<0. 025、Against IL-1β
* * * ;p<0. 01、Against IL-1β

Fig. 4

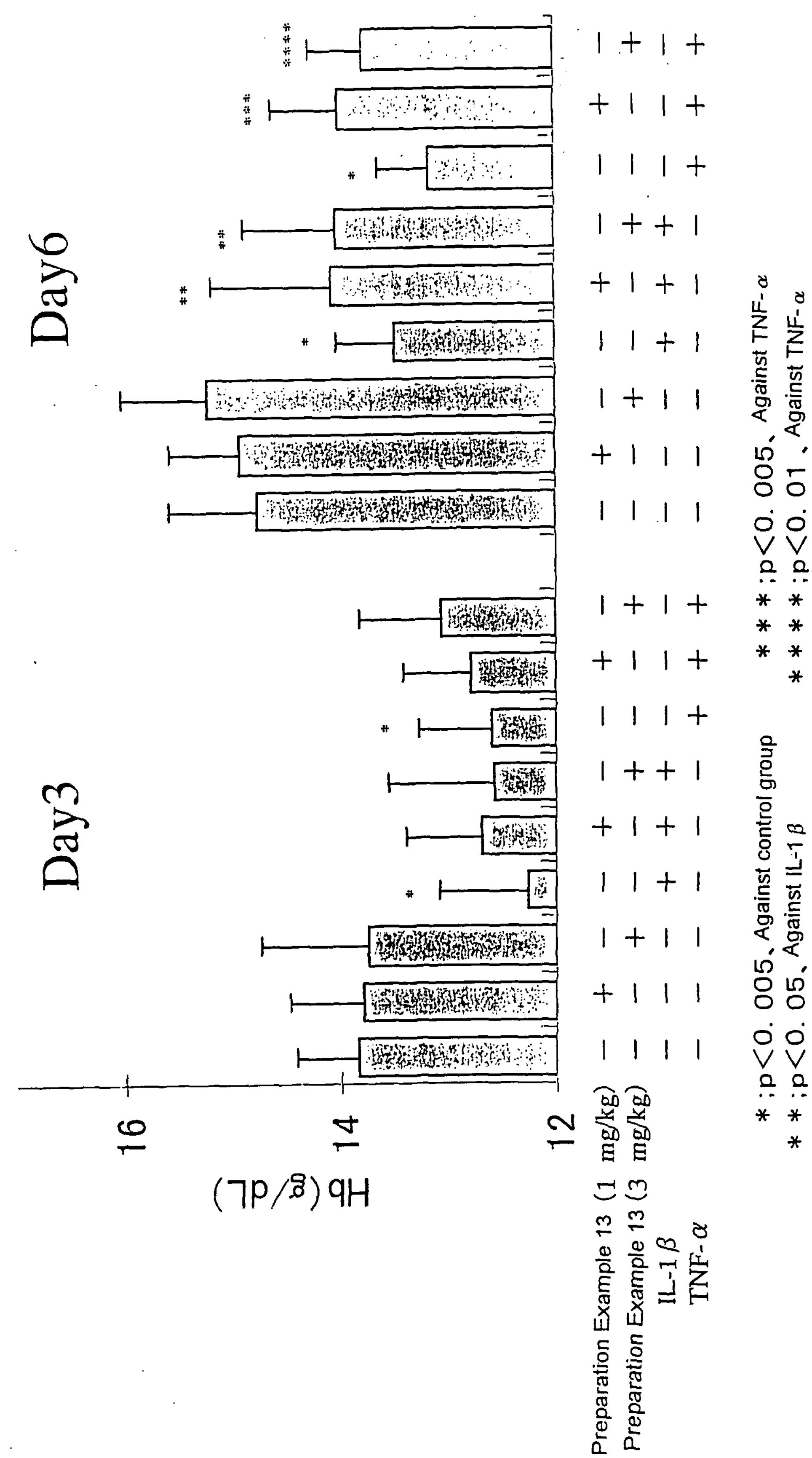
Day3
Day6
Hb (g/dL)
16
14
12
Preparation Example 13 (1 mg/kg)
Preparation Example 13 (3 mg/kg)
IL-1β
TNF-α
*;p<0.005, Against control group
**;p<0.05, Against IL-1β
***;p<0.005, Against TNF-α
****;p<0.01, Against TNF-α

Fig. 5

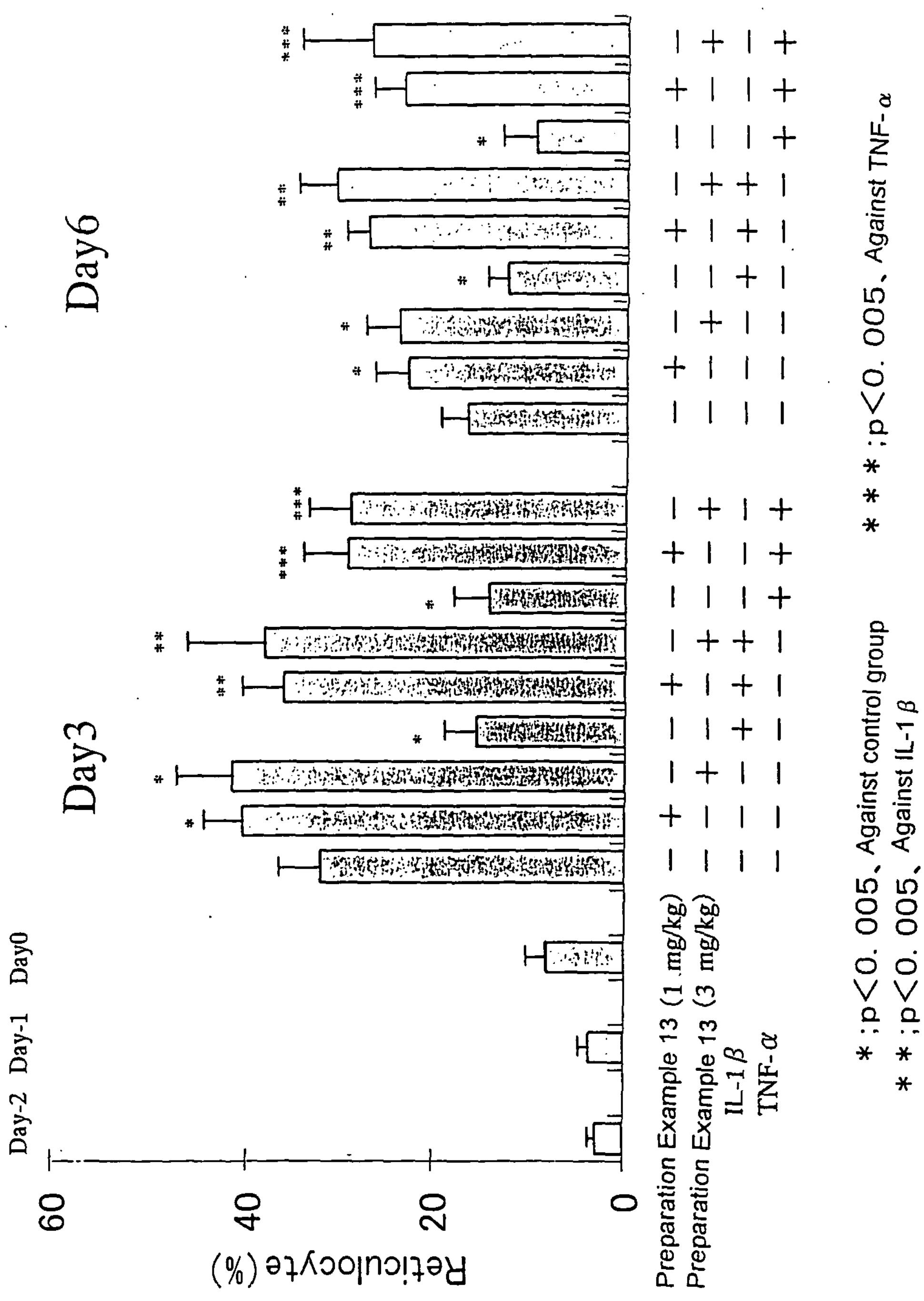
Reticulocyte (%)
60
40
20
0
Day-2
Day-1
Day0
Day3
Day6
Preparation Example 13 (1 mg/kg)
Preparation Example 13 (3 mg/kg)
IL-1β
TNF-α
*; p<0. 005, Against control group
**; p<0. 005, Against IL-1β
***; p<0. 005, Against TNF-α

Fig. 6

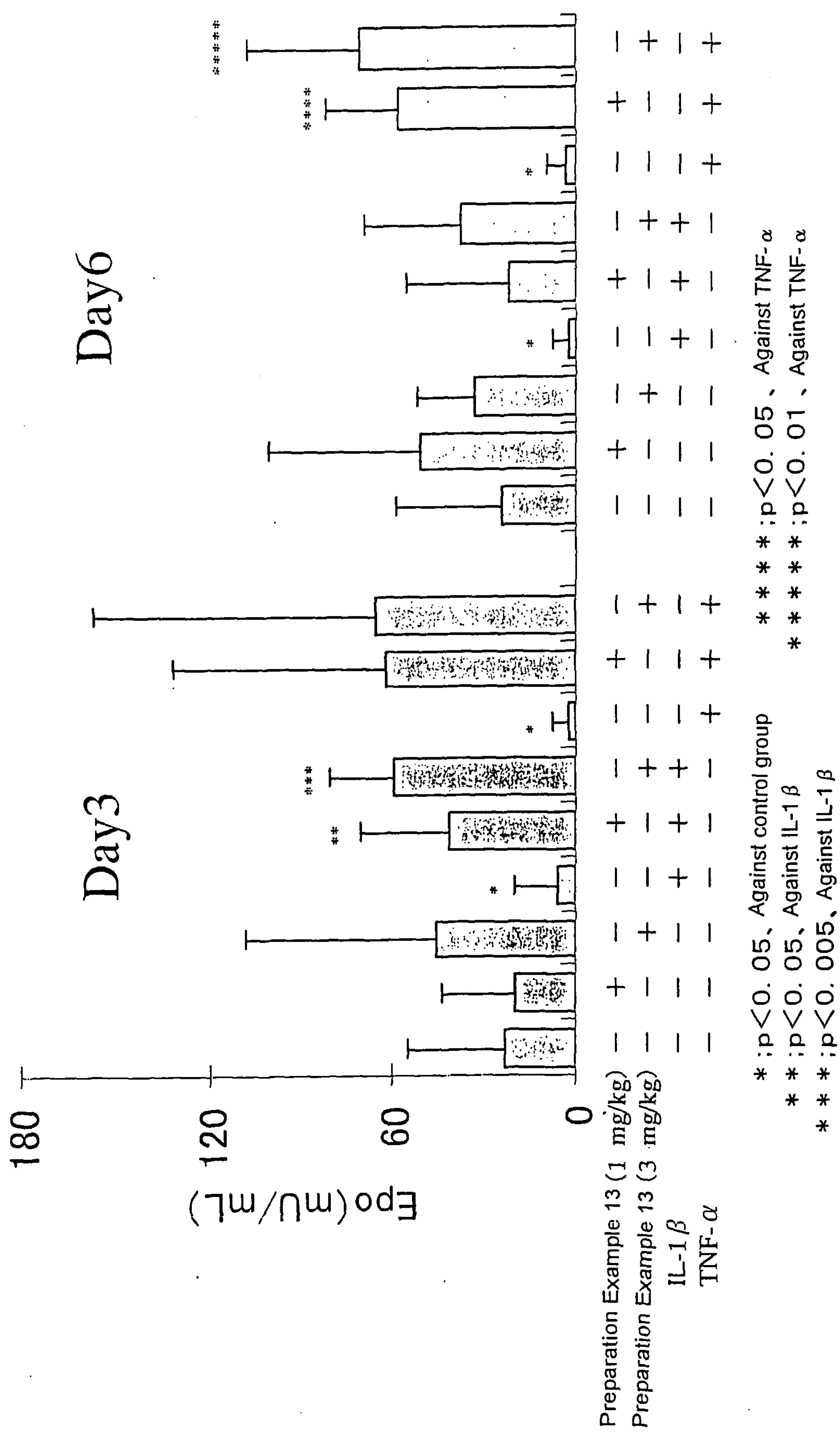
Day3
Day6
Epo (mU/mL)
180
120
60
0
Preparation Example 13 (1 mg/kg)
Preparation Example 13 (3 mg/kg)
IL-1β
TNF-α
* ;p<0. 05、Against control group
* * ;p<0. 05、Against IL-1β
* * * ;p<0. 005、Against IL-1β
* * * * ;p<0. 05 、Against TNF-α
* * * * * ;p<0. 01 、Against TNF-α

# INTERNATIONAL SEARCH REPORT

International application No.
PCT/JP03/15589

A. CLASSIFICATION OF SUBJECT MATTER
Int.Cl[7] C07D211/58, 401/06, 401/14, 405/14, A61K31/4468, 31/4545, 31/444, A61P7/00, 7/06, 43/00

According to International Patent Classification (IPC) or to both national classification and IPC

B. FIELDS SEARCHED

Minimum documentation searched (classification system followed by classification symbols)
Int.Cl[7] C07D211/58, 401/06, 401/14, 405/14, A61K31/4468, 31/4545, 31/444, A61P7/00, 7/06, 43/00

Documentation searched other than minimum documentation to the extent that such documents are included in the fields searched

Electronic data base consulted during the international search (name of data base and, where practicable, search terms used)
REGISTRY(STN), CAPLUS(STN), CAOLD(STN)

C. DOCUMENTS CONSIDERED TO BE RELEVANT

| Category* | Citation of document, with indication, where appropriate, of the relevant passages | Relevant to claim No. |
|---|---|---|
| A | US 6395753 B1 (KOWA CO., LTD.), 28 May, 2002 (28.05.02), & WO 03/020703 A1 & US 6498169 B1 & US 6605620 B1 | 1-30 |
| P,A | WO 03/086397 A1 (KOWA CO., LTD.), 23 October, 2003 (23.10.03), (Family: none) | 1-30 |
| A | WO 01/00589 A1 (BAYER AG.), 04 January, 2001 (04.01.01), & JP 2003-503391 A & EP 1196392 A1 & DE 19929782 A & CA 2377117 A & AU 5974100 A | 1-30 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 February, 2004 (03.02.04) | 24 February, 2004 (24.02.04) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/15589

C (Continuation). DOCUMENTS CONSIDERED TO BE RELEVANT

| Category* | Citation of document, with indication, where appropriate, of the relevant passages | Relevant to claim No. |
|---|---|---|
| A | JP 11-171774 A (Kyowa Hakko Kogyo Co., Ltd.), 29 June, 1999 (29.06.99), (Family: none) | 1-30 |

Form PCT/ISA/210 (continuation of second sheet) (July 1998)

## INTERNATIONAL SEARCH REPORT

International application No.
PCT/JP03/15589

**Box I Observations where certain claims were found unsearchable (Continuation of item 2 of first sheet)**

This international search report has not been established in respect of certain claims under Article 17(2)(a) for the following reasons:

1. [X] Claims Nos.: 31 to 45
   because they relate to subject matter not required to be searched by this Authority, namely:
   Claims 31 to 45 pertain to methods for treatment of the human body by therapy.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)